# EUROPEAN PATENT SPECIFICATION

(11) **EP 4 619 101 B1**
(45) Date of publication and mention of the grant of the patent: **02.09.2026**
(21) Application number: 23805953.9
(22) Date of filing: 14.11.2023
(51) Int. Cl.: A61P 25/00, A61P 27/16, C07D 487/04, A61K 31/53

(54) **NOVEL PYRROLO[1,2-D][1,2,4]TRIAZINONE DERIVATIVES AS NEGATIVE ALLOSTERIC MODULATORS OF MGLU7 RECEPTORS**
NEUE PYRROLO[1,2-D][1,2,4]TRIAZINONDERIVATE ALS NEGATIVE ALLOSTERISCHE MODULATOREN VON MGLU7-REZEPTOREN
NOUVEAUX DÉRIVÉS DE PYRROLO[1,2-D][1,2,4]TRIAZINONE EN TANT QU'EFFECTEURS ALLOSTÉRIQUES NÉGATIFS DES RÉCEPTEURS MGLU7

(30) Priority: 14.11.2022 GB 202216963
(43) Date of publication of application: 24.09.2025
(73) Proprietor: Neurosterix Pharma Sàrl, 1202 Geneva (CH)
(72) Inventor: PAPARIN, Jean-Laurent, 1202 Geneva (CH); ROCHER, Jean-Philippe, 1202 Geneva (CH); STACH, Tanja, 66564 Ottweiler (DE); RUTJES, Floris Petrus Johannes Theodorus, 6605 DD Wijchen (NL); JANSSEN, Freek Jan, 6538 SR Nijmegen (NL); DERKS, Max Theodorus Gerardus Maria, 6536DZ Nijmegen (NL); VAN DER KOLK, Marnix Ruben, 2000 Antwerpen (BE); BOUILLON, Marc, 66123 Saarbruecken (DE); KATTNER, Lars, 66123 Saarbruecken (DE)
(74) Representative: Reddie & Grose LLP
(86) International application number: PCT/EP2023/081726
(87) International publication number: WO 2024/105018

(56) References cited:
- WO-A1-2018/079862
- WO-A1-2019/063596
- WO-A1-2022/212818
- JP-A- 2014 214 124
- DYCK B ET AL: "A Thienopyridazinone-Based Melanin-Concentrating Hormone Receptor 1 Antagonist with Potent in Vivo Anorectic Properties", JOURNAL OF MEDICINAL CHEMISTRY, AMERICAN CHEMICAL SOCIETY, US, vol. 49, no. 13, 29 June 2006 (2006-06-29), pages 3753 - 3756, XP003003117, ISSN: 0022-2623, DOI: 10.1021/JM051263C
- TURGUT ZUHAL: "Reaction of 1,4-Dianion of Methyl 2-Methyl 2-Thienyl Ketone N-Ethoxycarbonylhydrazone with Carboxylic Acid Derivatives and the Synthesis of Pyrazolotriazin-7-ones", BULLETIN OF THE KOREAN CHEMICAL SOCIETY, vol. 23, no. 6, 20 June 2002 (2002-06-20), KR, pages 911 - 914, XP093107677, ISSN: 0253-2964, Retrieved from the Internet <URL:http://journal.kcsnet.or.kr/main/j_search/j_archives.htm?qpage=j_search&spage=b_bkcs&dpage=ar> DOI: 10.5012/bkcs.2002.23.6.911
- PADWA ALBERT ET AL: "1,3-Dipolar cycloaddition reactions of diazopyrazolinones with electron-deficient dipolarophiles", THE JOURNAL OF ORGANIC CHEMISTRY, vol. 48, no. 7, 1 April 1983 (1983-04-01), pages 1069 - 1074, XP093107678, ISSN: 0022-3263, DOI: 10.1021/jo00155a028

## Description

### SUMMARY OF THE INVENTION

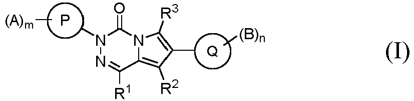

The present invention relates to novel compounds of Formula (I), wherein P, Q, A, B, m, n, R¹, R² and R³ are defined as in Formula (I); which are negative allosteric modulators of the metabotropic glutamate receptor subtype 7 (mGlu7) and which are useful for the treatment or prevention of neurological, ear and psychiatric disorders associated with glutamate dysfunction and diseases in which the mGlu7 subtype of metabotropic receptors is involved. The invention is also directed to pharmaceutical compositions comprising such compounds, to processes of preparing such compounds and such compositions, and to the use of such compounds for the prevention or treatment of neurological, ear and psychiatric disorders and diseases in which mGlu7 is involved.

### BACKGROUND OF THE INVENTION

Glutamate is the primary amino-acid transmitter in the mammalian central nervous system (CNS). Glutamate is associated with numerous physiological functions learning and memory, sensory perception, development of synaptic plasticity, motor control, respiration, and regulation of cardiovascular function. Furthermore, glutamate is at the centre of several different neurological and psychiatric diseases, where there is an imbalance in glutamatergic neurotransmission.

Glutamate mediates synaptic neurotransmission through the activation of ionotropic glutamate receptor channels (iGluRs), the NMDA, AMPA and kainate receptors which are responsible for fast excitatory transmission (Nakanishi et al. (1998) Brain Res. Rev., 26:230-235).

In addition, glutamate activates metabotropic glutamate receptors (mGluRs) which have a modulatory role that contributes to the fine-tuning of synaptic efficacy (Niswender & Conn (2010) Ann. Rev. Pharmacol. Toxicol. 50:295-322). As opposed to iGluRs, mGluRs do not mediate but rather "modulate" synaptic transmission acting at different levels of the tripartite synapse formed by the junction of axon terminals, dendritic spines, and astrocytes. The mGluRs are seven-transmembrane domain-containing G protein-coupled receptors (GPCRs) belonging to family 3 GPCRs along with the calcium-sensing, GABA_{B}, and pheromone receptors. Glutamate activates the mGluRs through binding to a site on the large extracellular amino-terminal domain of the receptor, herein called the orthosteric binding site. This activation induces a conformational change of the rest of the receptor which results in the activation of the G-protein and subsequently to a large variety of intracellular signalling pathways. The mGluR family is composed of eight members. They are classified into three groups (group I comprising mGlu1 and mGlu5; group II comprising mGlu2 and mGlu3; group III comprising mGlu4, mGlu6, mGlu7, and mGlu8) according to sequence homology, pharmacological profile, and nature of intracellular signalling cascades activated (Schoepp et al. (1999) Neuropharmacology, 38:1431-1476).

Among mGlu receptors, the mGlu7 subtype is the most widely distributed and is present pre-synaptically at a broad range of synapses that are postulated to be critical for both normal CNS functions and a range of psychiatric and neurological disorders (Ohishi et al. (1995) J. Comp. Neurol. 360(4):555-570; Kinzie et al. (1995) Neuroscience, 69(1):167-176; Corti et al. (1998) Eur. J. Neurosci, 10(12):3629-3641). mGlu7 is negatively coupled to adenylate cyclase via activation of Gαi-protein, and its activation as a pre-synaptic autoreceptor leads to inhibition of glutamate and GABA release in the synapse (Dalezios et al. (2002) Cereb. Cortex, 12(9):961-974; Cartmell and Schoepp (2000) J. Neurochem., 75:889-907; Somogyi et al. (2003) Eur. J. Neurosci. 17(12):2503-2520) therefore shaping the synaptic responses at glutamatergic synapses as well as being a key regulator of inhibitory GABAergic transmission with the final goal of fine tuning the overall excitability of the brain.

Previously, most available pharmacological tools targeting mGluRs were orthosteric ligands which cross react with several members of the family as they are structural analogs of glutamate (Schoepp et al. (1999) Neuropharmacology, 38:1431-1476). However, with new screening methods, it has become possible to identify molecules selective to individual mGluRs that act through allosteric mechanisms, modulating the receptor by binding to a site different from the highly conserved orthosteric binding site. These types of molecules have been discovered for several mGluRs (reviewed in Hellyer et al. (2017) Curr. Opin. Pharmacol. 32:49-55; Stansley & Conn (2019) Trends Pharmacol. Sci. 40(4):240-52; Dogra & Conn, (2022) Mol. 101(5):275-285). Several small molecules targeting mGlu7 receptors have been identified in recent years (reviewed in Vasquez-Villa & Trabanco (2019) Med. Chem. Comm. 10:193-9). AMN082 was described as being a potent, selective and systemically active mGlu7 allosteric agonist (Mitsukawa et al. (2005) Proc. Natl. Acad. Sci. USA, 102:18712-18717). 7-Hydroxy-3-(4-iodophenoxy)-4H-chromen-4-one (XAP044), an allosteric antagonist of mGlu7 was also recently described (Gee et al. (2014) J. Biol. Chem. 18;289(16):10975-10987), acting via a binding pocket localized in the receptor's extracellular Venus flytrap domain. Finally, several classes of compounds have been described, such as isoxazolopyridinone derivatives, phenylbenzamide derivatives, dihydrobenzoxazolone derivatives, tetrahydrophthalazinone derivatives and pharmacologically characterized as selective mGlu7 negative allosteric modulators (Suzuki et al. (2007) J. Pharmacol. Exp. Ther., 323:147-156; Kalinichev et al. (2013) J. Pharmacol. Exp. Ther. 344(3):624-636; Reed et al. (2017) ACS Med. Chem. Lett. (12):1326-1330 and Duvey et al (2019) WO2019063596).

Specifically, modulators of the mGlu7, and preferably antagonists, inverse agonists, and negative allosteric modulators (NAMs), are reported to hold potential for the treatment of neurological, psychiatric, mood disorders as well as pain and otic disorders, based on experimental studies on laboratory animals, deemed relevant to clinical syndromes.

Combined expression of mGlu7 in brain regions and pharmacological manipulations of mGlu7 in genetically modified mice and wild-type animals reveal an important role for mGlu7 in numerous CNS disorders, including depression, schizophrenia, anxiety, obsessive compulsive disorders and associated symptoms (reviewed by Pallazo et al. (2016) Curr. Neuropharmacol. 14(5): 504-513), and in particular in acute and chronic stress-related disorders (reviewed by Peterlik et al. (2016) Curr Neuropharmacol. 14(5):514-539).

mGlu7 has been shown to be located on limbic system nuclei such as the amygdala, hippocampus and the locus coerulus, regions that are known to be critical for the manifestation of anxiolysis and antidepressant actions (Kinoshita et al. (1998) J. Comp. Neurol., 393(3):332-352; Makoff et al. (1996) Brain Res. Mol. Brain Res., 40(1):165-170; Kinzie et al. (1995) Neuroscience, 69(1):167-176). Moreover, studies in several behavioral models (light-dark box test, elevated plus maze, staircase test, forced swim test and tail suspension test) have shown that mGlu7 knockout animals exhibit an anxiolytic and anti-depressant phenotype but also some deficits in amygdala-dependent behaviors (fear response and conditioned taste aversion) (Cryan et al. (2003) Eur. J. Neuroscience, 17:2409-2417). Therefore, a pharmacological agent aiming at modulating mGlu7 activity may represent a novel therapeutic approach for the treatment of neurological and psychiatric disorders such as anxiety and depression.

Activation of mGlu7 using the allosteric agonist AMN082 increase plasma levels of the stress hormones corticosterone and ACTH (Mitsukawa et al. (2005) PNAS, 102(51):18712-18717). This effect is totally absent in mGlu7 knock-out mice. Those results are in accordance with previous genetic studies showing that mGlu7 is an important regulator of stress response *in vivo* (Mitsukawa et al. (2006) Neuropsychopharm., 31(6):1112-1122). In this paper, Mitsukawa *et al.* demonstrated that mGlu7 ablation causes dysregulation of the HPA axis and increases hippocampal BDNF protein levels, indicating that this receptor might be implicated in stress-related psychiatric disorders such as anxiety, depression, post-traumatic stress syndrome, behaviours induced by innate fear such as acquisition and extinction of conditioned fear or conditioned taste aversion. These data also confirmed previous observations where mGlu7-deficient mice showed marked reduction in fear-mediated freezing responses during electric foot-shocks and impairment in the ability to associate between a taste stimulus and a malaise-evoking LiCl injection (conditioned taste aversion, CTA) (Masugi et al. (1999) J. Neurosc., 19(3):955-963). These mice also demonstrated a deficit in the acquisition and extinction learning of conditioned responses compared to wild type animals (Goddyn et al. (2008) Neurobiol. Learn. Mem., 90(1): 103-111).

Contradictory effects observed with the allosteric agonist AMN082 may be explained by the rapid and long-lasting mGlu7 receptor internalization, coinciding with functional antagonism, and its scarce selectivity in vivo suggests a potential off-target involvement (Sukoff Rizzo et al. (2011) J. Pharmacol. Exp. Ther., 338(1):345-352; Pelkey et al. (2007) Neuropharmacology 52(1):108-117).

The recent discovery of several negative allosteric modulators has contributed to better understanding of functional role of mGlu7 in neural functioning. 6-(4-Methoxyphenyl)-5-methyl-3-pyridin-4-ylisoxazolo[4,5-*c*]pyridin-4(5*H*)-one (MMPIP) administered in vivo has demonstrated anxiolytic, anti-depressant like properties, as well as improved cognitive performance in rodent models (Palazzo et al. (2015) Pain, 156(6):1060-1073). 7-Hydroxy-3-(4-iodophenoxy)-4H-chromen-4-one (XAP044) was shown to produce anti-stress, anti-depressant and anxiolytic-like effects and to reduce freezing in a fear-conditioning paradigm (Gee et al. (2014) J. Biol. Chem. 289(16):10975-10987). Furthermore, (S)-6-(2,4-dimethylphenyl)-2-ethyl-6,7-dihydrobenzo[d]oxazol-4(5H)-one (ADX71743) demonstrated anxiolytic-like effects in the elevated plus maze and marble burying tests, as well as reducing amphetamine-induced hyperactivity without altering baseline locomotor activity (Kalinichev et al. (2013) J. Pharmacol. Exp. Ther. 344(3):624-636). Taken together, these data indicate that inhibiting mGlu7 with a modulator would be useful for the treatment of mood disorders related to anxiety, depression and PTSD.

In addition, mGlu7 receptors have also been implicated in pathways affected during pain. Given its high and wide expression both in the peripheral and central nervous systems, mGlu7 was found to play a role in regulating pain behaviour. The role of mGlu7 in pain was also recently demonstrated using AMN082 injection directly into the central nucleus of the amygdala (CeA) or in the periaqueductal gray (PAG). Under normal conditions, activation of amygdala mGlu7 facilitates pain responses, as shown by a decrease in the spinal withdrawal reflex thresholds and increased audible and ultrasonic vocalizations evoked by brief compression of the knee (Palazzo et al. (2008) Neuropharmacol., 55(4):537-545). In a similar manner, activation of PAG mGlu7 decreased thermoceptive thresholds measured using the tail flick latency in rats (Marabese et al. (2007) J. Neurophysiol., 98:43-53). In rodent models of pain, AMN082 inhibited hyperalgesia (Dolan et al. (2009) Behav. Pharmacol. 20(7):596-604); Osikowicz et al. (2008) Pain 139(1):117-126). In addition, the mGlu7 negative allosteric modulator ADX71743 was shown to reduce visceral pain in a stress-sensitive model of visceral hypersensitivity (Moloney et al. (2015) Neurobiol. Stress 2:28-33). Altogether these data suggest that activation of mGlu7 receptors worsen pain perception and mGlu7 inhibition reduces it, therefore suggesting negative allosteric modulators of this receptor might be useful in the treatment of pain and pain-related disorders.

Genome-wide studies have also shown an association of the mGlu7 receptor with age-related hearing impairment (ARHI), also called presbycusis. This resulted in the identification of a highly significant and replicated single nucleotide polymorphism (SNP) located in GRM7, the gene coding for the mGlu7 receptor (Van Laer et al. (2010) Eur. J. Hum. Genet., 18(6):685-693; Friedman et al. (2009) Hum. Mol. Genet., 18(4):785-796; Newman et al. (2012) Hear Res. 294:125-132; Luo et al. (2013) PLoS One, 8(10):e77153; Haider et al. (2017) Front. Aging Neurosci. 9:346; Matyas et al. (2019) Pathol. Oncol. Res. 25(4):1645-52; Chang et al. (2018) J. Int. Adv. Otol. 14(2):170-175). GRM7 variants were also identified to be in association of noise-induced hearing loss, as reported by Lu et al. (BMC Med. Genet. (2018), 19(1):4) and tinnitus, as reported by Haider et al. (Front. Aging Neurosci. (2017), 9:346). Finally, mGlu7 expression, studied by immunohistochemistry, is located in the neurons of the spiral ganglion, in the inner and outer hair cells of the organ of Corti, and the hair cells of the vestibular apparatus formed by the sacculus, the utriculus and the crista ampullaris (Friedman et al. (2008) WO2008131439). These data suggest that mGlu7 receptor modulators are of potential use in the experimental treatment of otic disorders linked to the inner ear and auditory nervous system such as age-related hearing loss (presbycusis), noise-induced hearing loss, acute and chronic hearing loss, tinnitus, Meniere's disease and vestibular disorders.

Finally, on top of its wide distribution throughout the CNS, mGlu7 shows the highest degree of evolutionary conservation of all mGluRs (Flor et al. (1997) Neuropharmacol., 36:153-159), suggesting an important role for this receptor in CNS functioning. Moreover, it has a relatively low affinity for glutamate (Okamato et al. (1994) J. Biol. Chem., 269:1231-1236), thus it may remain inactive during normal transmission, only becoming active during times of excessive glutamate release (Ferraguti F. and Shigemoto R. (2006) Cell Tissue Res., 326:483-504). Taken together these data strongly highlight the potential of mGlu7 modulators in clinical indications such as neuroprotection (to treat stroke and head injury, ischemic damage and neurotoxicity).

Altogether, these pharmacological and genetic data strongly support the potential of mGlu7 modulators for the treatment of a wide range of disease and associated symptoms across psychiatric, neurological, neurodevelopmental, otic and pain disorders.

Pyrrolotriazinones have been shown to be useful as antifungal and/or antiparasitic agents by Loge et al. in the international publications WO2017021178 and WO2017020944. However, none of the specifically disclosed compounds are structurally related to the compounds of the present invention.

### SUMMARY OF THE INVENTION

The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy.

The invention relates to compounds having metabotropic glutamate receptor 7 modulator activity. The present invention provides a compound according to Formula (I), 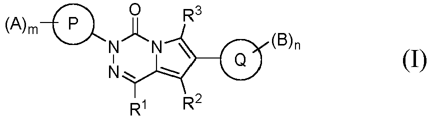 a pharmaceutically acceptable acid or base addition salt thereof, a stereochemically isomeric form thereof or an *N*-oxide form thereof, wherein:
R¹ is selected from the group of (for example the group consisting of) hydrogen, - CH₃ and -CF₃;
R² and R³ are each independently selected from the group of (for example the group consisting of) hydrogen, halogen, -(C₁-C₆)alkyl, -(C₁-C₆)haloalkyl and -CF₃;
P represents a -(C₁-C₆)alkyl, or a cycloalkyl, aryl, heteroaryl, -(C₁-C₆)alkylene-heteroaryl or heterocycle of formula: 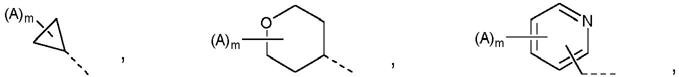 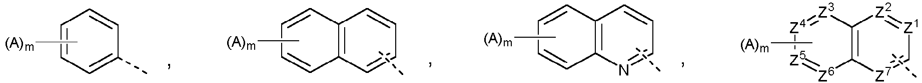 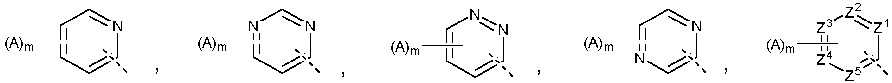 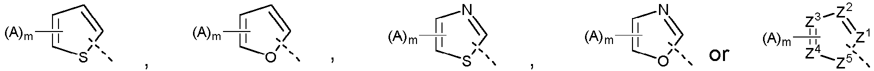
wherein each cycloalkyl ring, aryl ring, heteroaryl ring, -(C₁-C₆)alkylene-heteroaryl ring or heterocycle ring is optionally substituted with m radicals A, wherein m is an integer equal to zero, 1, 2, 3 or 4;
wherein Z¹, Z², Z³, Z⁴, Z⁵, Z⁶ and Z⁷ are each independently selected from C, N, O or S; provided that at least one of Z¹, Z², Z³, Z⁴, Z⁵, Z⁶ and Z⁷ is N;
the or each (A)ₘ is independently selected from the group of (for example the group consisting of) hydrogen, halogen, -CN, -OH, -NO₂, -CF₃, -SH, -NH₂ and an optionally substituted radical selected from the group of (for example the group consisting of) -(C₁-C₆)alkyl, -(C₁-C₆)haloalkyl, -(C₂-C₆)alkynyl, -(C₂-C₆)alkenyl, -(C₃-C₇)cycloalkyl, -(C₁-C₆)alkylene-(C₃-C₇)cycloalkyl, -(C₃-C₈)cycloalkenyl, -(C₁-C₆)cyanoalkyl, -(C₁-C₆)alkylene-heteroaryl, -(C₁-C₆)alkylene-aryl, aryl, heteroaryl, - (C₁-C₆)alkylene-heterocycle, heterocycle, -(C₀-C₆)alkylene-OR⁴, -O-(C₂-C₆)alkylene-OR⁴, -NR⁴(C₂-C₆)alkylene-OR⁵, -(C₃-C₆)alkynylene-OR⁴, -(C₃-C₆)alkynylene-NR⁴R⁵, - (C₃-C₆)alkenylene-OR⁴, -(C₃-C₆)alkenylene-NR⁴R⁵, -(C₀-C₆)alkylene-S-R⁴, -O-(C₂-C₆)alkylene-S-R⁴, -NR⁴-(C₂-C₆)alkylene-S-R⁵, -(C₀-C₆)alkylene-S(=O)-R⁴, -O-(C₁-C₆)alkylene-S(=O)-R⁴, -NR⁴-(C₁-C₆)alkylene-S(=O)-R⁵, -(C₀-C₆)alkylene-S(=O)₂-R⁴, - O-(C₁-C₆)alkylene-S(=O)₂-R⁴, -NR⁴-(C₁-C₆)alkylene-S(=O)₂-R⁵, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkyleneS(=O)₂NR⁴R⁵, -O-(C₁-C₆)alkylene-S(=O)₂NR⁴R⁵, -NR⁴-(C₁-C₆)alkylene-S(=O)₂NR⁵R⁶, -(C₀-C₆)alkylene-NR⁴-S(=O)₂R⁵, -O-(C₂-C₆)alkylene-NR⁴-S(=O)₂R⁵, -NR⁴-(C₂-C₆)alkylene-NR⁵-S(=O)₂R⁶, -(C₀-C₆)alkylene-C(=O)-NR⁴R⁵, -O-(C₁-C₆)alkylene-C(=O)-NR⁴R⁵, -NR⁴-(C₁-C₆)alkylene-C(=O)-NR⁵R⁶, -(C₀-C₆)alkylene-NR⁴C(=O)-R⁵, - O-(C₂-C₆)alkylene-NR⁴C(=O)-R⁵, -NR⁴-(C₂-C₆)alkylene-NR⁵C(=O)-R⁶, -(C₀-C₆)alkylene-OC(=O)-R⁴, -O-(C₂-C₆)alkylene-OC(=O)-R⁴, -NR⁴-(C₂-C₆)alkylene-OC(=O)-R⁵, -(C₀-C₆)alkylene-C(=O)-OR⁴, -O-(C₁-C₆)alkylene-C(=O)-OR⁴, -NR⁴-(C₀-C₆)alkylene-C(=O)-OR⁵, -(C₀-C₆)alkylene-C(=O)-R⁴, -O-(C₁-C₆)alkylene-C(=O)-R⁴, - NR⁴-(C₁-C₆)alkylene-C(=O)-R⁵, -(C₀-C₆)alkylene-NR⁴-C(=O)-OR⁵, -C(=O)-(C₁-C₆)alkylene-NR⁴-C(=O)-OR⁵, -(C₀-C₆)alkylene-O-C(=O)-NR⁴R⁵, -(C₀-C₆)alkylene-NR⁴-C(=O)-NR⁵R⁶, -O-(C₂-C₆)alkylene-NR⁴-C(=O)-NR⁵R⁶, -NR⁴-(C₂-C₆)alkylene-NR⁵-C(=O)-NR⁶R⁷, -(C₀-C₆)alkylene-NR⁴-C(=S)-NR⁵R⁶ and -(C₀-C₆)alkylene-NR⁴-C(=NR⁵)-NR⁶R⁷;
R⁴, R⁵, R⁶ and R⁷ are each independently hydrogen or an optionally substituted radical selected from the group of (for example the group consisting of) -(C₁-C₆)haloalkyl, -(C₁-C₆)alkyl, -(C₁-C₆)cyanoalkyl, -(C₃-C₇)cycloalkyl, -(C₁-C₆)alkylene-(C₃-C₇)cycloalkyl, heteroaryl, -(C₁-C₆)alkylene-heteroaryl, aryl, -(C₁-C₆)alkylene-heterocycle, heterocycle, -(C₁-C₆)alkylene-aryl, -(C₀-C₆)alkylene-O-(C₀-C₆)alkyl, -(C₀-C₆)alkylene-N-((C₀-C₆)alkyl)₂ and -C(=O)-O-(C₁-C₆)alkyl;
Q represents an aryl, heteroaryl or -(C₅-C₇)cycloalkenyl of formula: 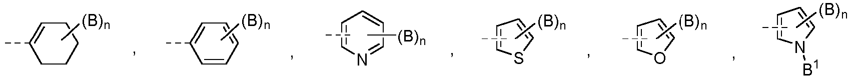 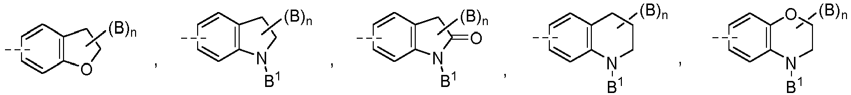 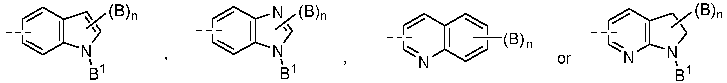
wherein each aryl ring, heteroaryl ring or -(C₅-C₇)cycloalkenyl ring is optionally substituted with n radicals B, wherein n is an integer equal to zero, 1, 2, 3, 4 or 5; wherein B¹ is a radical B;
the or each (B)ₙ is independently selected from the group of (for example the group consisting of) hydrogen, halogen, -CN, -OH, -NO₂, -CF₃, -SH, -NH₂ and an optionally substituted radical selected from the group of (for example the group consisting of) - (C₁-C₆)alkyl, -(C₁-C₆)haloalkyl, -(C₂-C₆)alkynyl, -(C₂-C₆)alkenyl, -(C₃-C₇)cycloalkyl, - (C₁-C₆)alkylene-(C₃-C₇)cycloalkyl, -(C₃-C₈)cycloalkenyl, -(C₁-C₆)cyanoalkyl, -(C₁-C₆)alkylene-heteroaryl, -(C₁-C₆)alkylene-aryl, aryl, heteroaryl, -(C₁-C₆)alkylene-heterocycle, heterocycle, -(C₀-C₆)alkylene-OR⁸, -O-(C₂-C₆)alkylene-OR⁸, -NR⁸(C₂-C₆)alkylene-OR⁹, -(C₃-C₆)alkynylene-OR⁸, -(C₃-C₆)alkynylene-NR⁸R⁹, -(C₃-C₆)alkenylene-OR⁸, -(C₃-C₆)alkenylene-NR⁸R⁹, -(C₀-C₆)alkylene-S-R⁸, -O-(C₂-C₆)alkylene-S-R⁸, -NR⁸-(C₂-C₆)alkylene-S-R⁹, -(C₀-C₆)alkylene-S(=O)-R⁸, -O-(C₁-C₆)alkylene-S(=O)-R⁸, -NR⁸-(C₁-C₆)alkylene-S(=O)-R⁹, -(C₀-C₆)alkylene-S(=O)₂-R⁸, - O-(C₁-C₆)alkylene-S(=O)₂-R⁸, -NR⁸-(C₁-C₆)alkylene-S(=O)₂-R⁹, -(C₀-C₆)alkylene-NR⁸R⁹, -O-(C₂-C₆)alkylene-NR⁸R⁹, -NR⁸-(C₂-C₆)alkylene-NR⁹R¹⁰, -(C₀-C₆)alkyleneS(=O)₂NR⁸R⁹, -O-(C₁-C₆)alkylene-S(=O)₂NR⁸R⁹, -NR⁸-(C₁-C₆)alkyleneS(=O)₂NR⁹R¹⁰, -(C₀-C₆)alkylene-NR⁸-S(=O)₂R⁹, -O-(C₂-C₆)alkylene-NR⁸-S(=O)₂R⁹, - NR⁸-(C₂-C₆)alkylene-NR⁹-S(=O)₂R¹⁰, -(C₀-C₆)alkylene-C(=O)-NR⁸R⁹, -O-(C₁-C₆)alkylene-C(=O)-NR⁸R⁹, -NR⁸-(C₁-C₆)alkylene-C(=O)-NR⁹R¹⁰, -(C₀-C₆)alkylene-NR⁸C(=O)-R⁹, -O-(C₂-C₆)alkylene-NR⁸C(=O)-R⁹, -NR⁸-(C₂-C₆)alkylene-NR⁹C(=O)-R¹⁰, -(C₀-C₆)alkylene-OC(=O)-R⁸, -O-(C₂-C₆)alkylene-OC(=O)-R⁸, -NR⁸-(C₂-C₆)alkylene-OC(=O)-R⁹, -(C₀-C₆)alkylene-C(=O)-OR⁸, -O-(C₁-C₆)alkylene-C(=O)-OR⁸, -NR⁸-(C₁-C₆)alkylene-C(=O)-OR⁹, -(C₀-C₆)alkylene-C(=O)-R⁸, -O-(C₁-C₆)alkylene-C(=O)-R⁸, -NR⁸-(C₁-C₆)alkylene-C(=O)-R⁹, -(C₀-C₆)alkylene-NR⁸-C(=O)-OR⁹, -(C₀-C₆)alkylene-O-C(=O)-NR⁸R⁹, -(C₀-C₆)alkylene-NR^{g}-C(=O)-NR⁹R¹⁰, -O-(C₂-C₆)alkylene-NR⁸-C(=O)-NR⁹R¹⁰, -NR⁸-(C₂-C₆)alkylene-NR⁹-C(=O)-NR¹⁰R¹¹, -(C₀-C₆)alkylene-NR⁸-C(=S)-NR⁹R¹⁰ and -(C₀-C₆)alkylene-NR⁸-C(=NR⁹)-NR¹⁰R¹¹;
R⁸, R⁹, R¹⁰ and R¹¹ are each independently hydrogen or an optionally substituted radical selected from the group of (for example the group consisting of) -(C₁-C₆)haloalkyl, -(C₁-C₆)alkyl, -(C₁-C₆)cyanoalkyl, -(C₃-C₇)cycloalkyl, -(C₁-C₆)alkylene-(C₃-C₇)cycloalkyl, heteroaryl, -(C₁-C₆)alkylene-heteroaryl, aryl, -(C₁-C₆)alkylene-heterocycle, heterocycle, -(C₁-C₆)alkylene-aryl, -(C₀-C₆)alkylene-O-(C₀-C₆)alkyl and - (C₀-C₆)alkylene-N-((C₀-C₆)alkyl)₂;
wherein optionally any two radicals A are combined with the intervening atoms to form a 3 to 10 membered bicyclic heterocycle, aryl or heteroaryl ring, wherein each ring is optionally further substituted with 1 to 5 radicals independently selected from the group of (for example the group consisting of) halogen, -CN, nitro, -(C₁-C₆)alkyl, -(C₃-C₇)alkyl, -(C₀-C₆)alkylene-O-(C₀-C₆)alkyl and -(C₀-C₆)alkylene-N-((C₀-C₆)alkyl)₂;
wherein optionally two of the substituents R⁴, R⁵, R⁶ or R⁷ are combined with the intervening atoms to form a 3 to 10 membered heterocycle, aryl or heteroaryl ring, wherein each ring is optionally further substituted with 1 to 5 radicals independently selected from the group of (for example the group consisting of) halogen, cyano, nitro, -(C₁-C₆)alkyl, -(C₃-C₇)alkyl, -(C₀-C₆)alkylene-O-(C₀-C₆)alkyl and -(C₀-C₆)alkylene-N-((C₀-C₆)alkyl)₂;
wherein optionally two substituents from R⁸, R⁹, R¹⁰ or R¹¹ are combined with the intervening atoms to form a 3 to 10 membered heterocycle, aryl or heteroaryl ring, wherein each ring is optionally further substituted with 1 to 5 radicals independently selected from the group of (for example the group consisting of) halogen, cyano, nitro, -(C₁-C₆)alkyl, -(C₃-C₇)alkyl, -(C₀-C₆)alkylene-O-(C₀-C₆)alkyl and -(C₀-C₆)alkylene-N-((C₀-C₆)alkyl)₂;
wherein optionally any two radicals B are combined with the intervening atoms to form a 3 to 10 membered bicyclic heterocycle, aryl or heteroaryl ring, wherein each ring is optionally further substituted with 1 to 5 radicals independently selected from the group of (for example the group consisting of) halogen, -CN, nitro, -(C₁-C₆)alkyl, -(C₃-C₇)alkyl, -(C₀-C₆)alkylene-O-(C₀-C₆)alkyl and -(C₀-C₆)alkylene-N-((Cₒ-C₆)alkyl)₂.

It has now, surprisingly, been found that the compounds of general Formula (I) show potent activity and selectivity on the mGlu7 receptor. The compounds of the invention demonstrate advantageous properties over compounds of the prior art. Improvements have been observed in one or more of the following characteristics of the compounds of the invention: the potency on the target, the selectivity for the target, the bioavailability, the brain penetration, and the pharmacodynamics.

P may be a -(C₁-C₆)alkyl, or a cycloalkyl, heteroaryl, -(C₁-C₆)alkylene-heteroaryl or heterocycle of formula:

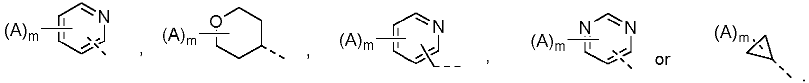

Q may be an aryl, heteroaryl or -(C₅-C₇)cycloalkenyl of formula:

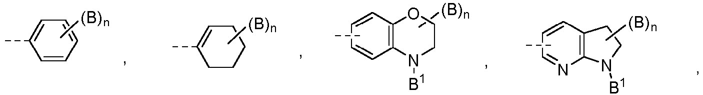

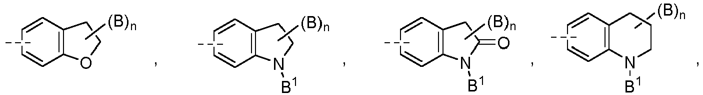

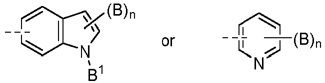

Preferably, P represents a heteroaryl of formula: 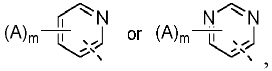 wherein each radical is optionally substituted with m radicals A, wherein m is an integer equal to zero, 1, 2, 3 or 4.

Preferably, Q represents an aryl or heteroaryl of formula: 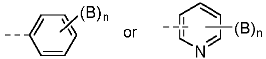 wherein each radical is optionally substituted with n radicals B, wherein n is an integer equal to zero, 1, 2, 3, 4 or 5.

Preferably, P represents a heteroaryl of formula: 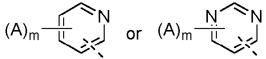 wherein each radical is optionally substituted with m radicals A, wherein m is an integer equal to zero, 1, 2, 3 or 4; and Q represents an aryl or heteroaryl of formula: 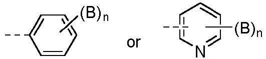 wherein each radical is optionally substituted with n radicals **B,** wherein n is an integer equal to zero, **1, 2,** 3, 4 or 5.

The cycloalkyl, heterocycle, aryl and heteroaryl ring systems of (A)ₘ may be selected from the group of (for example the group consisting of) azetidinyl, 2-azabicyclo[2.2.1]heptan-2-yl, 7-azabicyclo[2.2.1]heptan-7-yl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phthalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents R⁴, R⁵, R⁶ or R⁷.

The cycloalkyl, heterocycle, aryl and heteroaryl ring systems of (B)ₙ may be selected from the group of (for example the group consisting of) azetidinyl, 7-azabicyclo[2.2.1]heptan-7-yl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phthalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents R⁸, R⁹, R¹⁰ or R¹¹.

B¹ may be a radical B as described above. For example, B¹ may be hydrogen, -(C₁-C₆)alkyl or -(C₃-C₇)cycloakyl.

The cycloalkyl, heterocycle, aryl and heteroaryl ring systems of R⁴, R⁵, R⁶, R⁷, R⁸, R⁹, R¹⁰ or R¹¹ may be selected from the group of (for example the group consisting of) azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phthalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted with 1-5 radicals independently selected from hydrogen, halogen, -CN, nitro, -(C₁-C₆)alkyl, -(C₀-C₆)alkylene-O-(C₀-C₆)alkyl and -(C₀-C₆)alkylene-N-((C₀-C₆)alkyl)₂.

For example, R¹ may be hydrogen; and R² and R³ may be each independently selected from the group of (for example the group consisting of) hydrogen and methyl.

The or each (A)ₘ may be independently selected from the group of (for example the group consisting of) hydrogen, halogen, -CN, -OH, -CF₃ and an optionally substituted radical selected from the group of (for example the group consisting of) -(C₁-C₆)alkyl, -(C₁-C₆)haloalkyl, -(C₃-C₇)cycloalkyl, -(C₁-C₆)cyanoalkyl, aryl, heterocycle, -(C₀-C₆)alkylene-OR⁴, -O-(C₂-C₆)alkylene-OR⁴, -NR⁴(C₂-C₆)alkylene-OR⁵, -(C₀-C₆)alkylene-S(=O)₂-R⁴, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-S(=O)₂NR⁴R⁵, -(C₀-C₆)alkylene-C(=O)-NR⁴R⁵, -(C₀-C₆)alkylene-NR⁴C(=O)-R⁵, -(C₀-C₆)alkylene-C(=O)-OR⁴, -(C₀-C₆)alkylene-C(=O)-R⁴, -C(=O)-(C₁-C₆)alkylene-NR⁴-C(=O)-OR⁵ and -NR⁴-(C₀-C₆)alkylene-C(=O)-OR⁵

R⁴, R⁵ and R⁶ may each independently be hydrogen, -(C₁-C₆)alkyl, -(C₁-C₆)haloalkyl, or -C(=O)-O-(C₁-C₆)alkyl.

The or each (B)ₙ may be independently selected from the group of (for example the group consisting of) hydrogen, halogen, -CN, -CF₃, and an optionally substituted radical selected from the group of (for example the group consisting of) -(C₁-C₆)alkyl, -(C₃-C₇)cycloalkyl, aryl, heteroaryl, heterocycle, -(C₀-C₆)alkylene-OR⁸, -NR⁸(C₂-C₆)alkylene-OR⁹, -(C₀-C₆)alkylene-NR⁸R⁹, -(C₀-C₆)alkylene-C(=O)-OR⁸ and -(C₀-C₆)alkylene-C(=O)-R⁸.

R⁸ and R⁹ may each be independently selected from the group of (for example the group consisting of) hydrogen, -(C₁-C₆)haloalkyl, -(C₁-C₆)alkyl, -(C₃-C₇)cycloalkyl and aryl.

Preferably, the compounds of Formula (I) are the compounds according to Formula (II): 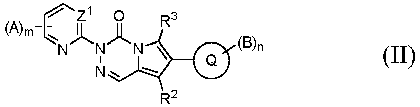 a pharmaceutically acceptable acid or base addition salt thereof, a stereochemically isomeric form thereof or an N-oxide form thereof, wherein Z¹ is selected from C or N and (A)ₘ, Q, R², R³ and (B)ₙ are as defined in any statement set out above.

The compounds of Formula (I) may be the compounds according to Formula (III): 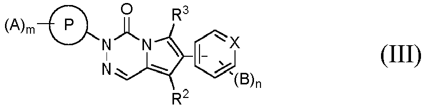 a pharmaceutically acceptable acid or base addition salt thereof, a stereochemically isomeric form thereof or an N-oxide form thereof, wherein X is N or C, and P, (A)ₘ, R², R³ and (B)ₙ are as defined in any statement set out above.

The compounds of Formula (I) may be the compounds according to Formula (IV): 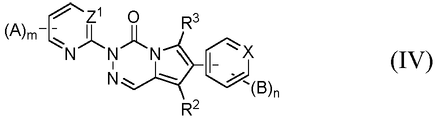 a pharmaceutically acceptable acid or base addition salt thereof, a stereochemically isomeric form thereof or an N-oxide form thereof, wherein Z¹ is selected from C or N, X is selected from C or N, and (A)ₘ, R², R³ and (B)ₙ are as defined in any statement set out above.

The compounds of Formula (I) may be the compounds according to Formula (V): 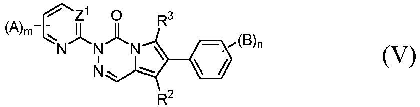 a pharmaceutically acceptable acid or base addition salt thereof, a stereochemically isomeric form thereof or an *N*-oxide form thereof wherein:
Z¹ is selected from C or N, and (A)ₘ, R², R³ and (B)ₙ are as defined in any statement set out above.

For example, R² may be hydrogen or methyl and R³ may be methyl or hydrogen;
The or each (A)ₘ may be independently selected from the group of (for example the group consisting of) hydrogen, halogen, -CF₃ and an optionally substituted radical selected from the group of (for example the group consisting of) -(C₁-C₆)alkyl, -(C₁-C₆)haloalkyl, -(C₃-C₇)cycloalkyl, heterocycle, -(C₀-C₆)alkylene-OR⁴, -O-(C₂-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-C(=O)-NR⁴R⁵, - (C₀-C₆)alkylene-NR⁴C(=O)-R⁵, -(C₀-C₆)alkylene-C(=O)-OR⁴, -(C₀-C₆)alkylene-C(=O)-R⁴, -C(=O)-(C₁-C₆)alkylene-NR⁴-C(=O)-OR⁵ and -NR⁴-(C₀-C₆)alkylene-C(=O)-OR⁵ and R⁴, R⁵ and R⁶ may be each independently hydrogen, -(C₁-C₆)alkyl, -(C₁-C₆)haloalkyl, or -C(=O)-O-(C₁-C₆)alkyl.

The or each (B)ₙ may be independently selected from the group of (for example the group consisting of) hydrogen, halogen and an optionally substituted radical selected from the group of (for example the group consisting of) -(C₁-C₆)alkyl, heterocycle, - (C₀-C₆)alkylene-OR⁸, -NR⁸(C₂-C₆)alkylene-OR⁹, -(C₀-C₆)alkylene-NR⁸R⁹, -(C₀-C₆)alkylene-C(=O)-OR⁸ and -(C₀-C₆)alkylene-C(=O)-R⁸; and
R⁸ and R⁹ may each be independently hydrogen, -(C₁-C₆)alkyl, -(C₃-C₇)cycloalkyl or - (C₁-C₆)haloalkyl.

For example, the or each (A)ₘ may be independently selected from the group of (for example the group consisting of) hydrogen, halogen, and an optionally substituted radical selected from the group of (for example the group consisting of) -(C₁-C₆)alkyl, heterocycle, -(C₀-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁻-(C₂-C₆)alkylene-NR⁵R⁶, -(C₀-C₆)alkylene-C(=O)-OR⁴ and -NR⁴-(C₀-C₆)alkylene-C(=O)-OR⁵.

R⁴, R⁵ and R⁶ may be each independently hydrogen, -(C₁-C₆)alkyl, -(C₁-C₆)haloalkyl or -C(=O)-O-(C₁-C₆)alkyl.

The or each (B)ₙ may be independently selected from the group of (for example the group consisting of) hydrogen, halogen and an optionally substituted radical selected from the group of (for example the group consisting of) -(C₁-C₆)alkyl, heterocycle and -(C₀-C₆)alkylene-OR⁸; and
R⁸ may be hydrogen, -(C₁-C₆)alkyl, -(C₃-C₇)cycloalkyl or -(C₁-C₆)haloalkyl.

Preferably, the compounds of Formula (II) are according to Formula (VI): 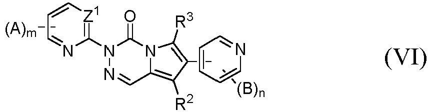 a pharmaceutically acceptable acid or base addition salt thereof, a stereochemically isomeric form thereof or an *N*-oxide form thereof wherein:
Z¹ is selected from C or N, and (A)ₘ, R², R³ and (B)ₙ are as defined in any statement set out above.

For example, R² may be hydrogen or methyl and R³ may be methyl or hydrogen;
the or each (A)ₘ may be independently selected from the group of (for example the group consisting of) hydrogen, halogen, -CF₃ and an optionally substituted radical selected from the group of (for example the group consisting of) -(C₁-C₆)alkyl, -(C₁-C₆)haloalkyl, -(C₃-C₇)cycloalkyl, heterocycle, -(C₀-C₆)alkylene-OR⁴, -O-(C₂-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-C(=O)-NR⁴R⁵, - (C₀-C₆)alkylene-NR⁴C(=O)-R⁵, -(C₀-C₆)alkylene-C(=O)-OR⁴, -(C₀-C₆)alkylene-C(=O)-R⁴, -C(=O)-(C₁-C₆)alkylene-NR⁴-C(=O)-OR⁵ and -NR⁴-(C₀-C₆)alkylene-C(=O)-OR⁵;
R⁴, R⁵ and R⁶ may each be independently hydrogen, -(C₁-C₆)alkyl, -C(=O)-O-(C₁-C₆)alkyl or -(C₁-C₆)haloalkyl;
the or each (B)ₙ may be independently selected from the group of (for example the group consisting of) hydrogen, halogen and an optionally substituted radical selected from the group of (for example the group consisting of) -(C₁-C₆)alkyl, heterocycle, - (C₀-C₆)alkylene-OR⁸, -NR⁸(C₂-C₆)alkylene-OR⁹, -(C₀-C₆)alkylene-NR⁸R⁹, -(C₀-C₆)alkylene-C(=O)-OR⁸ and -(C₀-C₆)alkylene-C(=O)-R⁸; and
R⁸ and R⁹ may be each independently hydrogen -(C₁-C₆)alkyl, -(C₃-C₇)cycloalkyl or - (C₁-C₆)haloalkyl.

Preferably, the compounds of Formula (VI) are the compounds according to Formula (VII): 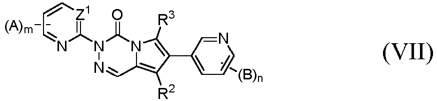 a pharmaceutically acceptable acid or base addition salt thereof, a stereochemically isomeric form thereof or an N-oxide form thereof wherein (A)ₘ, Z¹, R², R³ and (B)ₙ are as defined in any statement set out above.

Preferably, Z¹ is selected from C or N, R² is hydrogen or methyl, and R³ is methyl or hydrogen;
the or each (A)ₘ may be independently selected from the group of (for example the group consisting of) hydrogen, halogen, and an optionally substituted radical selected from the group of (for example the group consisting of) -(C₁-C₆)alkyl, heterocycle, - (C₀-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁴-(C₂-C₆)-alkylene-NR⁵R⁶, -(C₀-C₆)alkylene-C(=O)-OR⁴ and - NR⁴-(C₀-C₆)alkylene-C(=O)-OR⁵;
R⁴, R⁵ and R⁶ may be each independently hydrogen, -(C₁-C₆)alkyl,C₁-C₆)haloalkyl;
the or each (B)ₙ may be independently selected from the group of (for example the group consisting of) hydrogen, halogen and an optionally substituted radical selected from the group of (for example the group consisting of) -(C₁-C₆)alkyl, heterocycle and -(C₀-C₆)alkylene-OR⁸; and
R⁸ may be hydrogen, -(C₁-C₆)alkyl, -(C₃-C₇)cycloalkyl or -(C₁-C₆)haloalkyl.

For example, the or each (A)ₘ may be hydrogen.

Preferably, the compounds of Formula (VI) are the compounds of Formula (VIII): 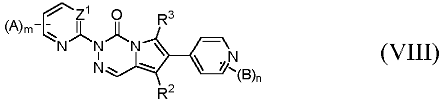 a pharmaceutically acceptable acid or base addition salt thereof, a stereochemically isomeric form thereof or an N-oxide form thereof wherein (A)ₘ, Z¹, R², R³ and (B)ₙ are as defined in any statement set out above.

Preferably, Z¹ is selected from C or N, R² is hydrogen or methyl, R³ is methyl or hydrogen;
the or each (A)ₘ may be independently selected from the group of (for example the group consisting of) hydrogen, halogen, and an optionally substituted radical selected from the group of (for example the group consisting of) -(C₁-C₆)alkyl, heterocycle, - (C₀-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-C(=O)-OR⁴ and - NR⁴-(C₀-C₆)alkylene-C(=O)-OR⁵.

R⁴, R⁵ and R⁶ may be each independently hydrogen, -(C₁-C₆)alkyl, -(C₁-C₆)haloalkyl or -C(=O)-O-(C₁-C₆)alkyl.

The or each (B)ₙ may be independently selected from the group of (for example the group consisting of) hydrogen, halogen and an optionally substituted radical selected from the group of (for example the group consisting of) -(C₁-C₆)alkyl, heterocycle and -(C₀-C₆)alkylene-OR⁸; and
R⁸ may be hydrogen, -(C₁-C₆)alkyl, -(C₃-C₇)cycloalkyl or -(C₁-C₆)haloalkyl.

For example, the or each (A)ₘ may be hydrogen.

For example, the or each (A)ₘ as set out according to any statement above may be independently selected from the group of (for example the group consisting of) hydrogen, halogen, and an optionally substituted radical selected from the group of (for example the group consisting of) heterocycle, -(C₀-C₆)alkylene-OR⁴, -(C₀-C₆)alkylene-C(=O)-OR⁴, -NR⁴-(C₀-C₆)alkylene-C(=O)-OR⁵, -NR⁴(C₂-C₆)alkylene-OR⁵ , -O-(C₂-C₆)alkylene-NR⁴R⁵, -NR⁴-(C₂-C₆)alkylene-NR⁵R⁶, and -(C₀-C₆)alkylene-NR⁴R⁵; and R⁴ and R⁵ may be each independently hydrogen, -(C₁-C₆)alkyl, -(C₁-C₆)haloalkyl, or - C(=O)-O-(C₁-C₆)alkyl.

For example, the or each (B)ₙ as set out according to any statement above may be independently selected from the group of (for example the group consisting of) hydrogen, halogen and an optionally substituted radical selected from the group of (for example the group consisting of) -(C₁-C₆)alkyl, heterocycle and -(C₀-C₆)alkylene-OR⁸; and R⁸ may be hydrogen, -(C₁-C₆)alkyl, -(C₃-C₇)cycloalkyl or -(C₁-C₆)haloalkyl.

For example, the or each (A)ₘ may be independently selected from the group of (for example the group consisting of) hydrogen, halogen, -CH₂CH₂OH, -COOCH₂CH₃, - COH(CH₃)₂, -O-methyl, -N(COOBu^{t})₂, NHCOOBu^{t}, morpholinyl, -NH₂, - NHCH₂CH₂OCH₃, -NHCH₂CH₂N(CH₃)₂, -OCHF₂, -OCH₂CH₂N(CH₃)₂ , CHOH(CH₃)₂, methyl and hydroxy substituted pyrrolidinyl.

For example, the or each (B)ₙ may be independently selected from the group of (for example the group consisting of) hydrogen, halogen, azetidinyl, -OCHF₂, -OCF₃, cyclopropyl, -O-cyclopropyl, -O-methyl, methyl, propyl, -O-cyclobutyl and azabicyclo[2.2.1]heptan-7-yl.

Particular preferred compounds of the invention are compounds as mentioned in the following list, as well as a pharmaceutically acceptable acid or base addition salt thereof, a stereochemically isomeric form thereof or an *N*-oxide form thereof:
7-(2,4-dimethylphenyl)-6,8-dimethyl-3-(2-pyridyl)pyrrolo[1,2-d][1,2,4]triazin-4-one 7-(3-methoxyphenyl)-6,8-dimethyl-3-(2-pyridyl)pyrrolo[1,2-*d*][1,2,4]triazin-4-one 7-[3-(azetidin-1-yl)phenyl]-6,8-dimethyl-3-(2-pyridyl)pyrrolo[1,2-d][1,2,4]triazin-4-one
7-(3-methoxy-2-methyl-phenyl)-6,8-dimethyl-3-(2-pyridyl)pyrrolo[1,2-d][1,2,4]triazin-4-one
7-[3-(azetidin-1-yl)phenyl]-6,8-dimethyl-3-tetrahydropyran-4-yl-pyrrolo[1,2-*d*][1,2,4]triazin-4-one
7-[3-(azetidin-1-yl)phenyl]-6,8-dimethyl-3-(4-pyridyl)pyrrolo[1,2-d][1,2,4]triazin-4-one
7-[3-(azetidin-1-yl)phenyl]-3-(3-fluoro-2-pyridyl)-6,8-dimethyl-pyrrolo[1,2-*d*][1,2,4]triazin-4-one
7-[3-(cyclopropoxy)-2-methyl-phenyl]-6,8-dimethyl-3-(2-pyridyl)pyrrolo[1,2-*d*][1,2,4]triazin-4-one
7-[3-(azetidin-1-yl)phenyl]-6,8-dimethyl-3-(2-pyridylmethyl)pyrrolo[1,2-*d*][1,2,4]triazin-4-one
7-[3-(azetidin-1-yl)phenyl]-3-ethyl-6,8-dimethyl-pyrrolo[1,2-d][1,2,4]triazin-4-one
7-[3-(azetidin-1-yl)phenyl]-3-[5-(2-hydroxyethyl)-2-pyridyl]-6,8-dimethyl-pyrrolo[1,2-*d*][1,2,4]triazin-4-one
7-[3-(azetidin-1-yl)phenyl]-3,6,8-trimethyl-pyrrolo[1,2-*d*][1,2,4]triazin-4-one
7-[3-(azetidin-1-yl)-2-methyl-phenyl]-6,8-dimethyl-3-(2-pyridyl)pyrrolo[1,2-*d*][1,2,4]triazin-4-one
7-(cyclohexen-1-yl)-6,8-dimethyl-3-(2-pyridyl)pyrrolo[1,2-d][1,2,4]triazin-4-one
7-[3-(azetidin-1-yl)phenyl]-3-(5-fluoropyrimidin-2-yl)-6,8-dimethyl-pyrrolo[1,2-*d*][1,2,4]triazin-4-one
7-[5-(azetidin-1-yl)-2-methyl-phenyl]-6,8-dimethyl-3-(2-pyridyl)pyrrolo[1,2-*d*][1,2,4]triazin-4-one
7-[3-(difluoromethoxy)phenyl]-3-(5-fluoropyrimidin-2-yl)-6,8-dimethyl-pyrrolo[1,2-*d*][1,2,4]triazin-4-one
7-[3-(cyclopropoxy)-2-methyl-phenyl]-3-pyrimidin-2-yl-pyrrolo[1,2-*d*][1,2,4]triazin-4-one
ethyl 2-[7-[3-(cyclopropoxy)phenyl]-6,8-dimethyl-4-oxo-pyrrolo[1,2-d][1,2,4]triazin-3-yl]pyrimidine-5-carboxylate
7-[3-(cyclopropoxy)phenyl]-6,8-dimethyl-3-(2-pyridyl)pyrrolo[1,2-d][1,2,4]triazin-4-one
7-[3-(cyclopropoxy)phenyl]-3-(5-fluoropyrimidin-2-yl)-6,8-dimethyl-pyrrolo[1,2-d][1,2,4]triazin-4-one
7-[3-(azetidin-1-yl)phenyl]-3-(5-fluoro-2-pyridyl)-6,8-dimethyl-pyrrolo[1,2-d][1,2,4]triazin-4-one
7-[3-(azetidin-1-yl)phenyl]-6,8-dimethyl-3-pyrimidin-2-yl-pyrrolo[1,2-d][1,2,4]triazin-4-one
7-[3-(azetidin-1-yl)phenyl]-3-[5-(1-hydroxy-1-methyl-ethyl)-2-pyridyl]-6,8-dimethyl-pyrrolo[1,2-*d*][1,2,4]triazin-4-one
7-[3-(azetidin-1-yl)phenyl]-3-(5-methoxypyrimidin-2-yl)-6,8-dimethyl-pyrrolo[1,2-d][1,2,4]triazin-4-one
7-[3-(azetidin-1-yl)phenyl]-3-(5-bromo-2-pyridyl)-6,8-dimethyl-pyrrolo[1,2-d][1,2,4]triazin-4-one
7-[3-(cyclopropoxy)-2-methyl-phenyl]-6,8-dimethyl-3-pyrimidin-2-yl-pyrrolo[1,2-d][1,2,4]triazin-4-one
tert-butyl *N*-[6-[7-[3-(azetidin-1-yl)phenyl]-6,8-dimethyl-4-oxo-pyrrolo[1,2-d][1,2,4]triazin-3-yl]-3-pyridyl]-N-tert-butoxycarbonyl-carbamate
7-[3-(cyclopropoxy)phenyl]-6,8-dimethyl-3-pyrimidin-2-yl-pyrrolo[1,2-*d*][1,2,4]triazin-4-one
7-[3-(azetidin-1-yl)-5-fluoro-phenyl]-6,8-dimethyl-3-(2-pyridyl)pyrrolo[1,2-*d*][1,2,4]triazin-4-one
tert-butyl *N*-[6-[7-[3-(azetidin-1-yl)phenyl]-6,8-dimethyl-4-oxo-pyrrolo[1,2-d][1,2,4]triazin-3-yl]-3-pyridyl]carbamate
7-[3-(azetidin-1-yl)phenyl]-6,8-dimethyl-3-(6-morpholino-3-pyridyl)pyrrolo[1,2-*d*][1,2,4]triazin-4-one
7-[3-(azetidin-1-yl)phenyl]-3-cyclopropyl-6,8-dimethyl-pyrrolo[1,2-*d*][1,2,4]triazin-4-one
3-(5-amino-2-pyridyl)-7-[3-(azetidin-1-yl)phenyl]-6,8-dimethyl-pyrrolo[1,2-d][1,2,4]triazin-4-one
7-[3-(azetidin-1-yl)phenyl]-3-[5-(2-methoxyethylamino)-2-pyridyl]-6,8-dimethyl-pyrrolo[1,2-*d*][1,2,4]triazin-4-one
7-[3-(azetidin-1-yl)phenyl]-3-[5-[2-(dimethylamino)ethylamino]-2-pyridyl]-6,8-dimethyl-pyrrolo[1,2-*d*][1,2,4]triazin-4-one
7-[3-(difluoromethoxy)phenyl]-6,8-dimethyl-3-(2-pyridyl)pyrrolo[1,2-d][1,2,4]triazin-4-one
6,8-dimethyl-3-(2-pyridyl)-7-[3-(trifluoromethoxy)phenyl]pyrrolo[1,2-*d*][1,2,4]triazin-4-one
7-[3-(cyclopropoxy)phenyl]-3-(5-methoxypyrimidin-2-yl)-6,8-dimethyl-pyrrolo[1,2-*d*][1,2,4]triazin-4-one
7-[3-(cyclopropoxy)phenyl]-3-[5-(difluoromethoxy)pyrimidin-2-yl]-6,8-dimethyl-pyrrolo[1,2-*d*][1,2,4]triazin-4-one
7-[3-(cyclopropoxy)phenyl]-3-[5-[2-(dimethylamino)ethoxy]pyrimidin-2-yl]-6,8-dimethyl-pyrrolo[1,2-*d*][1,2,4]triazin-4-one
7-[3-(azetidin-1-yl)phenyl]-3-[5-(difluoromethoxy)pyrimidin-2-yl]-6,8-dimethyl-pyrrolo[1,2-d][1,2,4]triazin-4-one
7-[3-(azetidin-1-yl)phenyl]-3-[5-[2-(dimethylamino)ethoxy]pyrimidin-2-yl]-6,8-dimethyl-pyrrolo[1,2-*d*][1,2,4]triazin-4-one
3-(5-aminopyrimidin-2-yl)-7-[3-(azetidin-1-yl)phenyl]-6,8-dimethyl-pyrrolo[1,2-d][1,2,4]triazin-4-one
7-[3-(cyclopropoxy)phenyl]-3-[5-(1-hydroxy-1-methyl-ethyl)-2-pyridyl]-6,8-dimethyl-pyrrolo[1,2-*d*][1,2,4]triazin-4-one
7-(2,4-dimethylphenyl)-3-(5-methoxypyrimidin-2-yl)-6,8-dimethyl-pyrrolo[1,2-d][1,2,4]triazin-4-one
7-[3-(cyclopropoxy)phenyl]-6,8-dimethyl-3-(5-morpholinopyrimidin-2-yl)pyrrolo[1,2-d][1,2,4]triazin-4-one
3-(5-chloropyrimidin-2-yl)-7-[3-(cyclopropoxy)phenyl]-6,8-dimethyl-pyrrolo[1,2-d][1,2,4]triazin-4-one
7-[3-(cyclopropoxy)-2-methyl-phenyl]-3-(5-fluoropyrimidin-2-yl)-6,8-dimethyl-pyrrolo[1,2-*d*][1,2,4]triazin-4-one
7-[3-(cyclopropoxy)phenyl]-6,8-dimethyl-3-(5-methylpyrimidin-2-yl)pyrrolo[1,2-d][1,2,4]triazin-4-one
7-[3-(cyclopropoxy)phenyl]-3-(3-fluoro-2-pyridyl)-6,8-dimethyl-pyrrolo[1,2-d][1,2,4]triazin-4-one
7-[3-(cyclopropoxy)-2-methyl-phenyl]-6,8-dimethyl-3-(5-methylpyrimidin-2-yl)pyrrolo[1,2-*d*][1,2,4]triazin-4-one
6,8-dimethyl-3-pyrimidin-2-yl-7-[3-(trifluoromethoxy)phenyl]pyrrolo[1,2-*d]* [1,2,4]triazin-4-one
7-[3-(cyclopropoxy)-2-methyl-phenyl]-3-(5-fluoro-2-pyridyl)-6,8-dimethyl-pyrrolo[1,2-*d*][1,2,4]triazin-4-one
7-[3-(cyclopropoxy)phenyl]-3-(5-fluoro-2-pyridyl)-6,8-dimethyl-pyrrolo[1,2-*d]* [1,2,4]triazin-4-one
3-(5-methoxypyrimidin-2-yl)-6,8-dimethyl-7-[3-(trifluoromethoxy)phenyl]pyrrolo[1,2-*d]* [1,2,4]triazin-4-one
7-[3-(difluoromethoxy)phenyl]-3-(5-methoxypyrimidin-2-yl)-6,8-dimethyl-pyrrolo[1,2-*d]* [1,2,4]triazin-4-one
7-[3-(difluoromethoxy)phenyl]-6,8-dimethyl-3-pyrimidin-2-yl-pyrrolo[1,2-*d]* [1,2,4]triazin-4-one
7-[3-(cyclopropoxy)-2-methyl-phenyl]-3-[5-(1-hydroxy-1-methyl-ethyl)-2-pyridyl]-6,8-dimethyl-pyrrolo[1,2-*d*][1,2,4]triazin-4-one
3-(5-fluoropyrimidin-2-yl)-6,8-dimethyl-7-[3-(trifluoromethoxy)phenyl]pyrrolo[1,2-*d]* [1,2,4]triazin-4-one
7-(3-methoxyphenyl)-6-methyl-3-pyrimidin-2-yl-pyrrolo[1,2-*d*][1,2,4]triazin-4-one
6-methyl-7-(4-methyl-2,3-dihydro-1,4-benzoxazin-8-yl)-3-(2-pyridyl)pyrrolo[1,2-*d]* [1,2,4]triazin-4-one
6-methyl-7-(4-methyl-2,3-dihydro-1,4-benzoxazin-8-yl)-3-pyrimidin-2-yl-pyrrolo[1,2-*d]* [1,2,4]triazin-4-one
6-methyl-7-(1-methylindolin-4-yl)-3-(2-pyridyl)pyrrolo[1,2-*d*][1,2,4]triazin-4-one
7-(1-cyclopropylindolin-4-yl)-6-methyl-3-(2-pyridyl)pyrrolo[1,2-*d*][1,2,4]triazin-4-one
7-(1-isopropylindolin-4-yl)-6-methyl-3-(2-pyridyl)pyrrolo[1,2-*d*][1,2,4]triazin-4-one
7-(1-cyclopropylindolin-4-yl)-6-methyl-3-pyrimidin-2-yl-pyrrolo[1,2-*d*][1,2,4]triazin-4-one
7-(1-isopropylindolin-4-yl)-6-methyl-3-pyrimidin-2-yl-pyrrolo[1,2-*d*][1,2,4]triazin-4-one
7-(2,3-dihydrobenzofuran-4-yl)-6-methyl-3-(2-pyridyl)pyrrolo[1,2-*d*][1,2,4]triazin-4-one
7-[3-(difluoromethoxy)phenyl]-6-methyl-3-(2-pyridyl)pyrrolo[1,2-*d*][1,2,4]triazin-4-one
7-[3-(difluoromethoxy)-2-methyl-phenyl]-6-methyl-3-(2-pyridyl)pyrrolo[1,2-*d]* [1,2,4]triazin-4-one
7-(3-methoxy-2-methyl-phenyl)-6-methyl-3-(2-pyridyl)pyrrolo[1,2-*d*][1,2,4]triazin-4-one
7-(2-chloro-3-methoxy-phenyl)-6-methyl-3-(2-pyridyl)pyrrolo[1,2-*d*][1,2,4]triazin-4-one
7-[3-(difluoromethoxy)phenyl]-6-methyl-3-pyrimidin-2-yl-pyrrolo[1,2-*d*][1,2,4]triazin-4-one
7-[3-(difluoromethoxy)-2-methyl-phenyl]-6-methyl-3-pyrimidin-2-yl-pyrrolo[1,2-*d]* [1,2,4]triazin-4-one
7-(3-methoxy-2-methyl-phenyl)-6-methyl-3-pyrimidin-2-yl-pyrrolo[1,2-*d]* [1,2,4]triazin-4-one
7-(2-chloro-3-methoxy-phenyl)-6-methyl-3-pyrimidin-2-yl-pyrrolo[1,2-*d*][1,2,4]triazin-4-one
6-methyl-7-(1-methyl-2-oxo-indolin-4-yl)-3-(2-pyridyl)pyrrolo[1,2-*d*][1,2,4]triazin-4-one
7-(2,4-dimethylphenyl)-6,8-dimethyl-3-pyrimidin-2-yl-pyrrolo[1,2-*d*][1,2,4]triazin-4-one
7-[3-(cyclopropoxy)phenyl]-3-[5-(3-hydroxypyrrolidin-1-yl)pyrimidin-2-yl]-6,8-dimethyl-pyrrolo[1,2-*d*][1,2,4]triazin-4-one
3-(5-aminopyrimidin-2-yl)-7-[3-(cyclopropoxy)phenyl]-6,8-dimethyl-pyrrolo[1,2-*d]* [1,2,4]triazin-4-one
7-[3-(cyclopropoxy)-2-methyl-phenyl]-3-(2-pyridyl)pyrrolo[1,2-*d*][1,2,4]triazin-4-one
7-[3-(cyclopropoxy)phenyl]-6-methyl-3-(2-pyridyl)pyrrolo[1,2-*d*][1,2,4]triazin-4-one
7-[3-(cyclopropoxy)-2-methyl-phenyl]-6-methyl-3-(2-pyridyl)pyrrolo[1,2-*d]* [1,2,4]triazin-4-one
7-[3-(cyclopropoxy)-2-methyl-phenyl]-6-methyl-3-pyrimidin-2-yl-pyrrolo[1,2-*d]* [1,2,4]triazin-4-one
6,8-dimethyl-7-(1-methyl-3,4-dihydro-2H-quinolin-5-yl)-3-pyrimidin-2-yl-pyrrolo[1,2-*d]* [1,2,4]triazin-4-one
6,8-dimethyl-7-(1-methylindolin-4-yl)-3-pyrimidin-2-yl-pyrrolo[1,2-*d*][1,2,4]triazin-4-one
7-[3-(cyclopropoxy)phenyl]-6-methyl-3-pyrimidin-2-yl-pyrrolo[1,2-*d*][1,2,4]triazin-4-one
6-methyl-7-(1-methyl-3,4-dihydro-2H-quinolin-5-yl)-3-pyrimidin-2-yl-pyrrolo[1,2-*d]* [1,2,4]triazin-4-one
6-methyl-7-(1-methylindolin-4-yl)-3-pyrimidin-2-yl-pyrrolo[1,2-d] [1,2,4]triazin-4-one
7-(3-methoxyphenyl)-6,8-dimethyl-3-pyrimidin-2-yl-pyrrolo[1,2-*d*][1,2,4]triazin-4-one
7-[5-(cyclopropoxy)-2-methyl-phenyl]-6-methyl-3-pyrimidin-2-yl-pyrrolo[1,2-d] [1,2,4]triazin-4-one
7-[5-(cyclopropoxy)-2-methyl-phenyl]-6,8-dimethyl-3-pyrimidin-2-yl-pyrrolo[1,2-*d]* [1,2,4]triazin-4-one
3-ethyl-6,8-dimethyl-7-(1-methyl-3,4-dihydro-2*H*-quinolin-5-yl)pyrrolo[1,2-*d]* [1,2,4]triazin-4-one
7-(3-methoxy-2-methyl-phenyl)-6,8-dimethyl-3-pyrimidin-2-yl-pyrrolo[1,2-d] [1,2,4]triazin-4-one
7-(1-cyclopropylindol-4-yl)-6,8-dimethyl-3-pyrimidin-2-yl-pyrrolo[1,2-*d*][1,2,4]triazin-4-one
6,8-dimethyl-7-(1-methylindol-4-yl)-3-pyrimidin-2-yl-pyrrolo[1,2-*d*][1,2,4]triazin-4-one
7-(6-methoxy-2-pyridyl)-6,8-dimethyl-3-pyrimidin-2-yl-pyrrolo[1,2-*d*][1,2,4]triazin-4-one
7-(1-cyclopropylindolin-4-yl)-6,8-dimethyl-3 -pyrimidin-2-yl-pyrrolo[1,2-*d]* [1,2,4]triazin-4-one
3-ethyl-6,8-dimethyl-7-(1-methylindolin-4-yl)pyrrolo[1,2-*d*][1,2,4]triazin-4-one
7-(3-fluoro-5-methoxy-phenyl)-6-methyl-3-(2-pyridyl)pyrrolo[1,2-*d*][1,2,4]triazin-4-one
7-(2-fluoro-5-methoxy-phenyl)-6-methyl-3-(2-pyridyl)pyrrolo[1,2-*d*][1,2,4]triazin-4-one
7-(3-fluoro-5-methoxy-phenyl)-6-methyl-3-pyrimidin-2-yl-pyrrolo[1,2-*d*][1,2,4]triazin-4-one
7-[2-chloro-3-(cyclopropoxy)phenyl]-6-methyl-3-(2-pyridyl)pyrrolo[1,2-*d]* [1,2,4]triazin-4-one
7-(2-fluoro-5-methoxy-phenyl)-6-methyl-3-pyrimidin-2-yl-pyrrolo[1,2-*d*][1,2,4]triazin-4-one
7-(2-methoxy-4-pyridyl)-6-methyl-3-(2-pyridyl)pyrrolo[1,2-*d*][1,2,4]triazin-4-one
7-(4-fluoro-3-methoxy-phenyl)-6-methyl-3-(2-pyridyl)pyrrolo[1,2-*d*][1,2,4]triazin-4-one
7-(2-fluoro-3-methoxy-phenyl)-6-methyl-3-(2-pyridyl)pyrrolo[1,2-*d*][1,2,4]triazin-4-one
7-[3-(cyclopropoxy)-2-fluoro-phenyl]-6-methyl-3-(2-pyridyl)pyrrolo[1,2-*d]* [1,2,4]triazin-4-one
7-[2-chloro-3-(cyclopropoxy)phenyl]-6-methyl-3-pyrimidin-2-yl-pyrrolo[1,2-*d]* [1,2,4]triazin-4-one
7-(4-fluoro-3-methoxy-phenyl)-6-methyl-3-pyrimidin-2-yl-pyrrolo[1,2-*d*][1,2,4]triazin-4-one
7-(2-fluoro-3-methoxy-phenyl)-6-methyl-3-pyrimidin-2-yl-pyrrolo[1,2-*d*][1,2,4]triazin-4-one
7-[3-(cyclopropoxy)-2-fluoro-phenyl]-6-methyl-3-pyrimidin-2-yl-pyrrolo[1,2-*d]* [1,2,4]triazin-4-one
7-(2-methoxy-4-pyridyl)-6-methyl-3-pyrimidin-2-yl-pyrrolo[1,2-*d*][1,2,4]triazin-4-one
8-bromo-7-(2-fluoro-3-methoxy-phenyl)-6-methyl-3-(2-pyridyl)pyrrolo[1,2-*d]* [1,2,4]triazin-4-one
7-(3-fluoro-2-methoxy-4-pyridyl)-6-methyl-3-(2-pyridyl)pyrrolo[1,2-*d*][1,2,4]triazin-4-one
7-(3-fluoro-2-methoxy-4-pyridyl)-6-methyl-3-pyrimidin-2-yl-pyrrolo[1,2-*d]* [1,2,4]triazin-4-one
6-methyl-7-(1-methyl-2,3-dihydropyrrolo[2,3-b]pyridin-4-yl)-3-(2-pyridyl)pyrrolo[1,2-*d]* [1,2,4]triazin-4-one
6-methyl-7-(1-methyl-2,3-dihydropyrrolo[2,3-b]pyridin-4-yl)-3-pyrimidin-2-yl-pyrrolo[1,2-*d*][1,2,4]triazin-4-one
7-[3-(difluoromethoxy)-2-methyl-phenyl]-6,8-dimethyl-3-pyrimidin-2-yl-pyrrolo[1,2-*d]* [1,2,4]triazin-4-one
7-(1-cyclopropyl-3,4-dihydro-2*H*-quinolin-5-yl)-6,8-dimethyl-3-pyrimidin-2-yl-pyrrolo[1,2-*d*][1,2,4]triazin-4-one
7-(4-methoxyphenyl)-6,8-dimethyl-3-pyrimidin-2-yl-pyrrolo[1,2-*d*][1,2,4]triazin-4-one
7-(2-chloro-3-fluoro-phenyl)-6,8-dimethyl-3-pyrimidin-2-yl-pyrrolo[1,2-*d]* [1,2,4]triazin-4-one
7-[3-(cyclobutoxy)phenyl]-6,8-dimethyl-3-pyrimidin-2-yl-pyrrolo[1,2-*d*][1,2,4]triazin-4-one
7-[3-(cyclopropoxy)-2-fluoro-phenyl]-6,8-dimethyl-3-pyrimidin-2-yl-pyrrolo[1,2-*d]* [1,2,4]triazin-4-one
7-(2-fluoro-5-methoxy-phenyl)-6,8-dimethyl-3-(2-pyridyl)pyrrolo[1,2-*d*][1,2,4]triazin-4-one
7-(2-fluoro-5-methoxy-phenyl)-6, 8-dimethyl-3 -pyrimidin-2-yl-pyrrolo[1,2-*d]* [1,2,4]triazin-4-one
7-(2-fluoro-3-methoxy-phenyl)-6,8-dimethyl-3-(2-pyridyl)pyrrolo[1,2-*d*][1,2,4]triazin-4-one
7-(6-methoxy-3-pyridyl)-6,8-dimethyl-3-(2-pyridyl)pyrrolo[1,2-*d*][1,2,4]triazin-4-one
7-(4-methoxyphenyl)-6,8-dimethyl-3-(2-pyridyl)pyrrolo[1,2-*d*][1,2,4]triazin-4-one
7-[3-(7-azabicyclo[2.2.1]heptan-7-yl)phenyl]-6,8-dimethyl-3-pyrimidin-2-yl-pyrrolo[1,2-*d*][1,2,4]triazin-4-one
7-(6-methoxypyridin-3-yl)-6,8-dimethyl-3-(pyrimidin-2-yl)pyrrolo[1,2-d][1,2,4]triazin-4(3H)-one
7-(2-fluoro-3-methoxyphenyl)-6,8-dimethyl-3-(pyrimidin-2-yl)pyrrolo[1,2-d] [1,2,4]triazin-4(3H)-one
7-(4-methoxy-2-methylphenyl)-6,8-dimethyl-3-(pyrimidin-2-yl)pyrrolo[1,2-d] [1,2,4]triazin-4(3H)-one
7-(2-fluoro-4-methoxyphenyl)-6,8-dimethyl-3-(pyrimidin-2-yl)pyrrolo[1,2-d] [1,2,4]triazin-4(3H)-one
7-(2-fluoro-4-methoxyphenyl)-6,8-dimethyl-3-(pyridin-2-yl)pyrrolo[1,2-d][1,2,4]triazin-4(3H)-one
7-(6-methoxy-2-methylpyridin-3-yl)-6,8-dimethyl-3-(pyrimidin-2-yl)pyrrolo[1,2-d] [1,2,4]triazin-4(3H)-one
7-(3-fluoro-2-methoxypyridin-4-yl)-6,8-dimethyl-3-(pyrimidin-2-yl)pyrrolo[1,2-d] [1,2,4]triazin-4(3H)-one
7-(2-methoxypyridin-4-yl)-6,8-dimethyl-3-(pyrimidin-2-yl)pyrrolo[1,2-d][1,2,4]triazin-4(3H)-one
7-(2-(difluoromethoxy)pyridin-4-yl)-6,8-dimethyl-3-(pyridin-2-yl)pyrrolo[1,2-d] [1,2,4]triazin-4(3H)-one
7-(2-(difluoromethoxy)pyridin-4-yl)-6,8-dimethyl-3-(pyrimidin-2-yl)pyrrolo[1,2-d][1,2,4]triazin-4(3H)-one
7-(6-methoxypyridin-3-yl)-6-methyl-3-(pyridin-2-yl)pyrrolo[1,2-d][1,2,4]triazin-4(3H)-one
7-(2-cyclopropoxy-3-methylpyridin-4-yl)-6-methyl-3-(pyridin-2-yl)pyrrolo[1,2-d][1,2,4]triazin-4(3H)-one
7-(6-methoxypyrimidin-3-yl)-6-methyl-3-(pyridin-2-yl)pyrrolo[1,2-d][1,2,4]triazin-4(3H)-one
7-(2-cyclopropoxy-3-methylpyrimidin-4-yl)-6-methyl-3-(pyridin-2-yl)pyrrolo[1,2-d][1,2,4]triazin-4(3H)-one
7-(2-(difluoromethoxy)pyridin-4-yl)-6-methyl-3-(pyridin-2-yl)pyrrolo[1,2-d][1,2,4]triazin-4(3H)-one and
7-(2-(difluoromethoxy)pyrimidin-4-yl)-6-methyl-3-(pyridin-2-yl)pyrrolo[1,2-d][1,2,4]triazin-4(3H)-one.

The above list of compounds can also be represented by the following skeletal formulae:

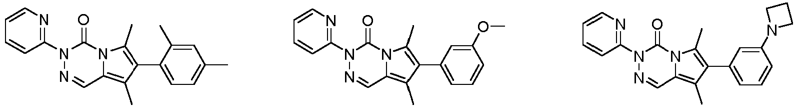

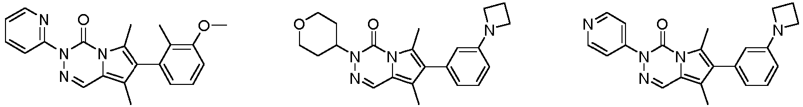

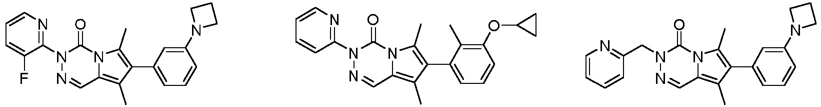

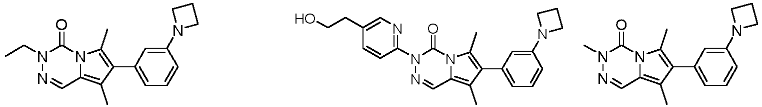

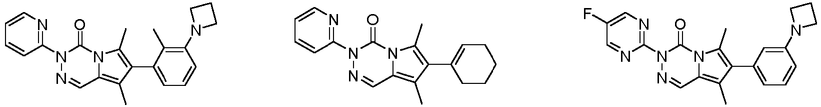

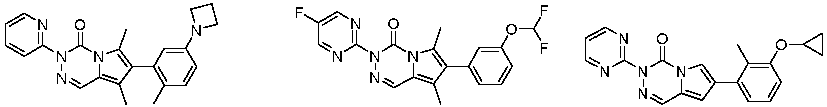

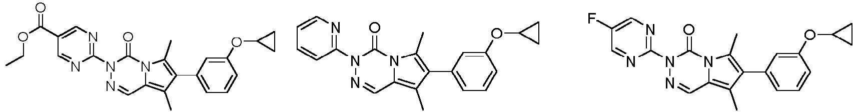

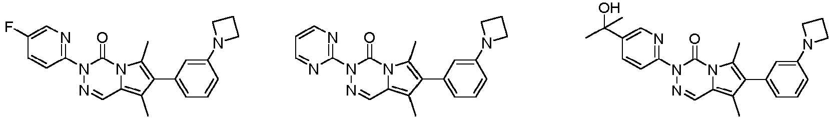

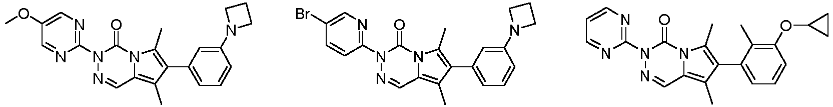

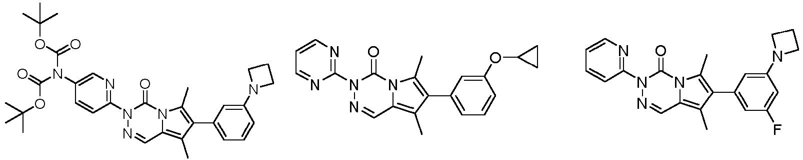

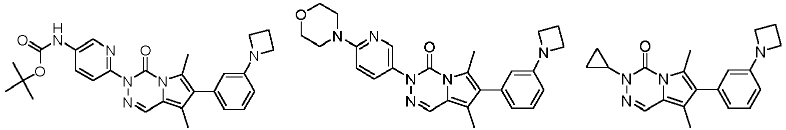

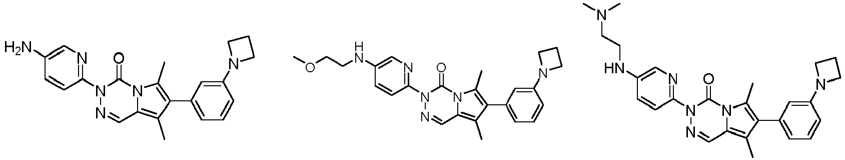

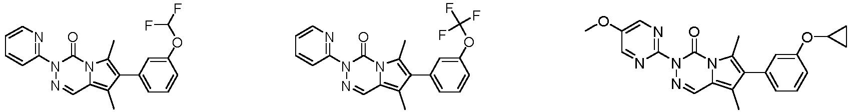

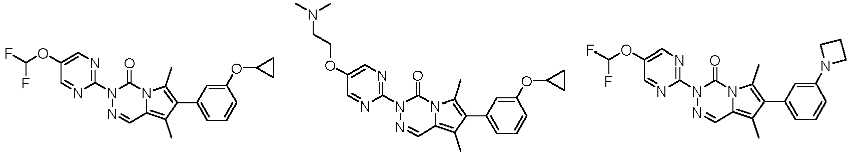

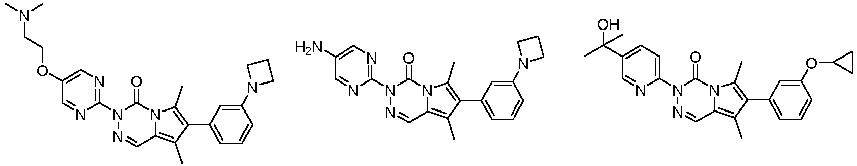

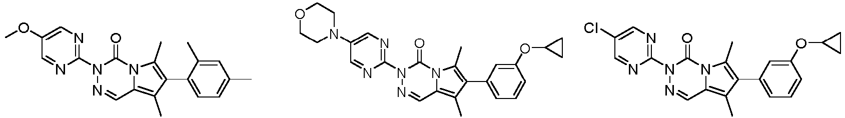

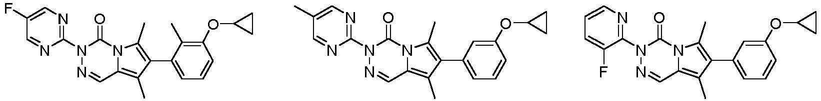

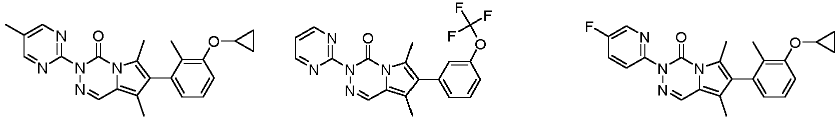

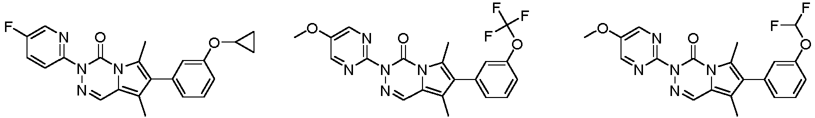

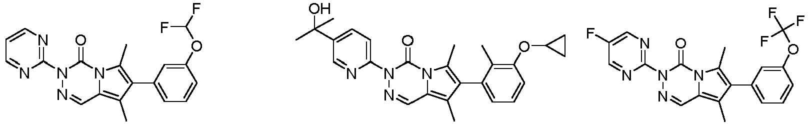

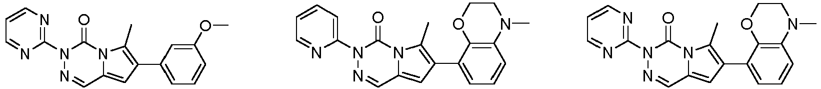

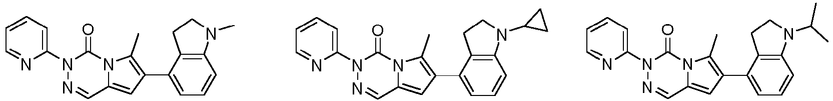

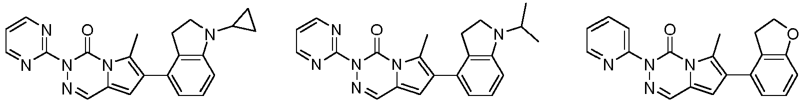

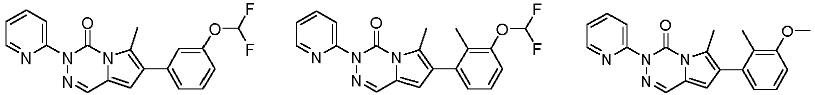

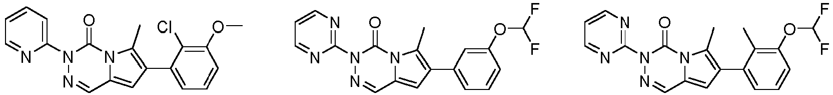

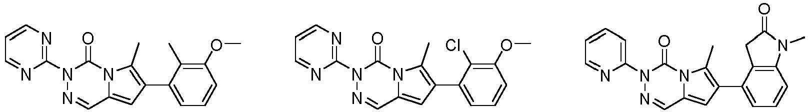

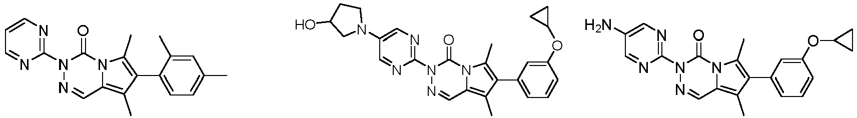

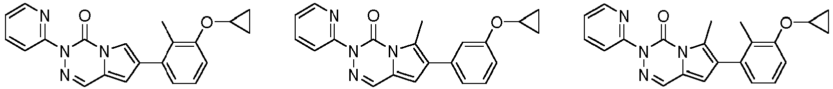

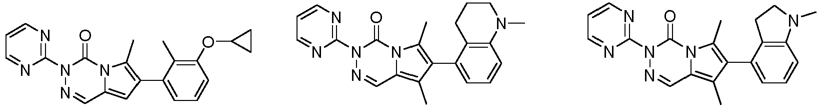

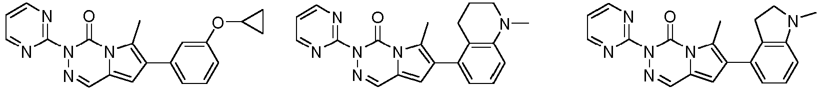

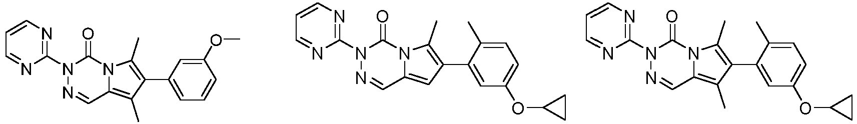

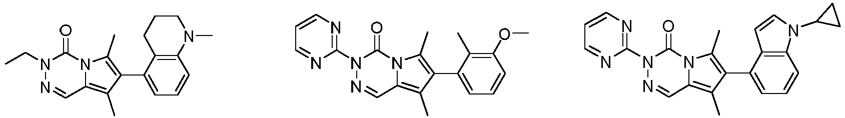

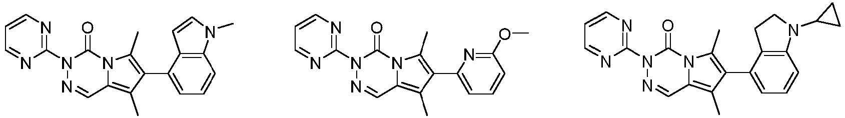

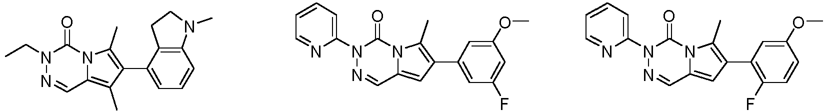

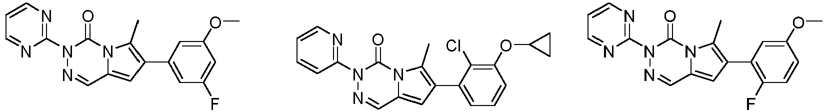

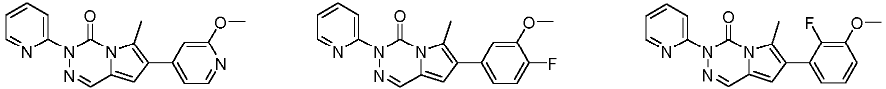

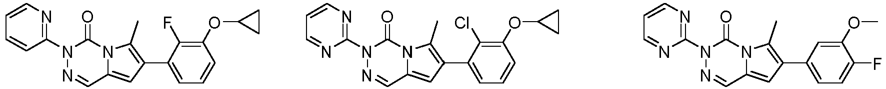

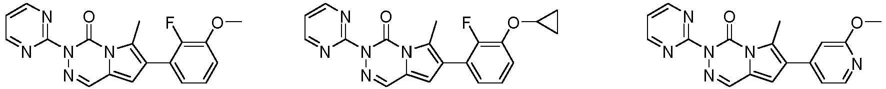

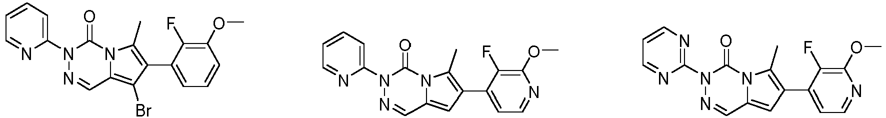

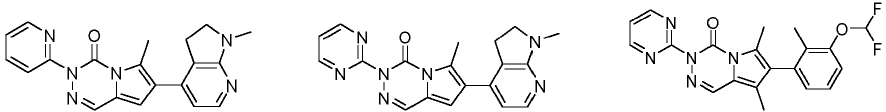

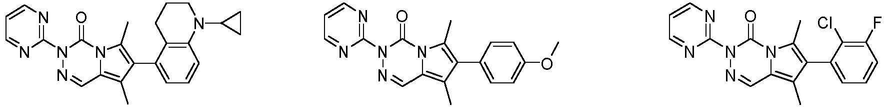

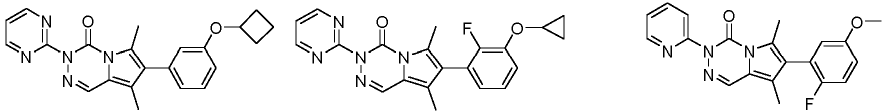

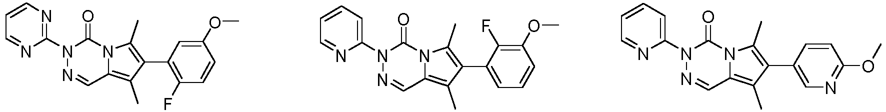

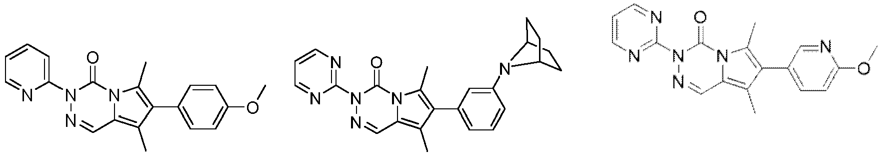

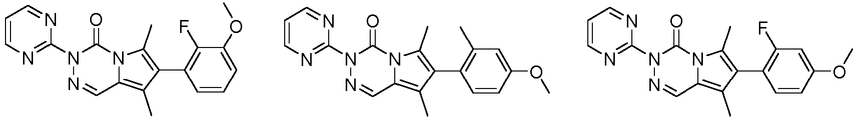

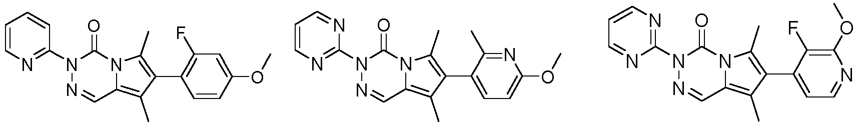

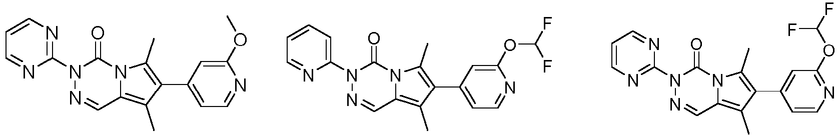

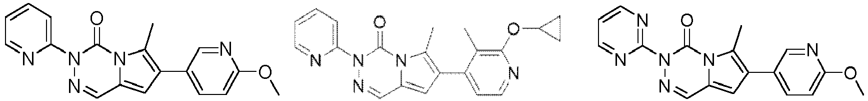

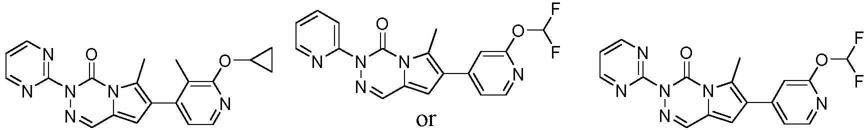

The compounds according to any statement above may exhibit metabotropic glutamate receptor 7 modulator activity.

The disclosed compounds also include all pharmaceutically acceptable isotopic variations, in which at least one atom is replaced by an atom having the same atomic number, but an atomic mass different from the atomic mass usually found in nature. Examples of isotopes suitable for inclusion in the disclosed compounds include, without limitation, isotopes of hydrogen, such as ²H and ³H; isotopes of carbon, such as ¹¹C, ¹³C and ¹⁴C; isotopes of nitrogen, such as ¹⁵N; isotopes of oxygen, such as ¹⁷O and ¹⁸O; isotopes of phosphorus, such as ³¹P, ³²P and ³³P; isotopes of sulfur, such as ³⁵S; isotopes of fluorine, such as ¹⁸F; isotopes of chlorine, such as ³⁶Cl; and isotopes of iodine, such as ¹²⁵I. The invention includes various isotopically labelled compounds as defined herein, for example those into which radioactive isotopes, such as ³H and ¹⁴C, or those into which non-radioactive isotopes, such as ²H and ¹³C are present.

Such isotopically labelled compounds are useful in metabolic studies (with ¹⁴C), reaction kinetic studies (with for example ²H or ³H), detection or imaging techniques, such as positron emission tomography (PET) or single-photon emission computed tomography (SPECT) including drug or substrate tissue distribution assays, or in radioactive treatment of patients. In particular, ¹¹C, ¹⁸F, ¹⁵O and ¹³N or labelled compounds may be particularly desirable for PET studies for examining substrate receptor occupancy. Further, substitution with heavier isotopes, particularly deuterieum (e.g., ²H or D) may afford certain therapeutic advantages resulting from greater metabolic stability, for example, increased in vivo half-life or reduced dosage requirements or an improvemet in therapeutic index. It is understood that deuterium in this context is regarded as a substituent of a compound of Formula (I) to (VIII). Isotopically-labelled compounds of Formula (I) to (VIII) can generally be prepared by conventional techniques known to those skilled in the art or by processes analogous to those described in the accompanying Examples using appropriate isotopically-labelled reagents in place of the non-labelled reagent previously employed.

In an aspect of the present invention there is provided a pharmaceutical composition comprising a compound according to any statement set out above. The pharmaceutical composition may further comprise a pharmaceutically acceptable carrier and/or excipient. The pharmaceutical composition may comprise a therapeutically effective amount of the compound according to any statement set out above.

In an aspect of the present invention there is provided a method of treating or preventing a condition in a mammal comprising administering to a mammal in need of such treatment or prevention, an effective amount of a compound/composition according to any statement set out above.

The treatment or prevention may be affected or facilitated by the modulatory effect of a mGlu7 allosteric modulator such as a mGlu7 negative allosteric modulator.

The condition may be one or more of a central nervous system disorder or an otic disease or disorder or a pain disorder.

The central nervous system disorder may be anxiety disorder such as agoraphobia, generalized anxiety disorder (GAD), obsessive-compulsive disorder (OCD), panic disorder, or post-traumatic stress disorder (PTSD).

The central nervous system disorder may be psychotic disorder such as schizophrenia, delusional disorder, schizoaffective disorder, schizophreniform disorder or substance induced psychotic disorder.

The otic disease and disorder may be one or more of an inner ear impairment, age-related hearing impairment (presbycusis), Meniere's disease, sudden hearing loss, noise induced hearing loss, otitis media, autoimmune inner ear disease, acute tinnitus, chronic tinnitus, drug-induced hearing loss, hidden hearing loss, cisplatin-induced hearing loss, aminoglycosides-induced hearing loss, ototoxicity, central auditory processing disorder or vestibular disorder.

In a further aspect of the present invention, there is provided a method of treating, preventing, ameliorating, controlling or reducing the risk of various neurological and psychiatric disorders associated with glutamate dysfunction in a mammal, comprising administering to a mammal in need of such treatment or prevention, an effective amount of a compound/composition according to any statement set out above. The treatment or prevention may be affected or facilitated by the modulatory effect of mGlu7 negative allosteric modulators.

Preferably, the methods are for the treatment or prevention of a condition in a human.

In a further aspect of the present invention, there is provided the compounds or compositions as set out in any statement above for use as a medicament.

In a further aspect of the present invention, there is provided the compounds or compositions as set out in any statement above for use in a method of treatment or prevention as defined in any statement set out above.

In a furher aspect of the present invention there is provided a use of a compound according to any statement set out above in the manufacture of a medicament for the treatment or prevention of a condition as defined in any statement set out above.

### DEFINITION OF TERMS

Listed below are definitions of various terms used in the specification and claims to describe the present invention.

For the avoidance of doubt it is to be understood that in this specification "(C₁-C₆)" means a carbon radical having 1, 2, 3, 4, 5 or 6 carbon atoms. "(C₀-C₆)" means a carbon radical having 0, 1, 2, 3, 4, 5 or 6 carbon atoms. In this specification "C" means a carbon atom, "N" means a nitrogen atom, "O" means an oxygen atom and "S" means a sulphur atom.

In the case where a subscript is the integer 0 (zero) the radical to which the subscript refers, indicates that the radical is absent, i.e. there is a direct bond between the radicals.

In the case where a subscript is the integer 0 (zero) and the radical to which the subscript refers is alkyl, this indicates the radical is a hydrogen atom.

In this specification, unless stated otherwise, the term "bond" refers to a saturated covalent bond. When two or more bonds are adjacent to one another, they are assumed to be equal to one bond. For example, a radical -A-B-, wherein both A and B may be a bond, the radical is depicting a single bond.

In this specification, unless stated otherwise, the term "alkyl" includes both straight and branched chain alkyl radicals and may be methyl, ethyl, n-propyl, i-propyl, n-butyl, *i-*butyl, s-butyl, t-butyl, n-pentyl, i-pentyl, t-pentyl, neo-pentyl, n-hexyl, i-hexyl or *t-*hexyl. The term "(C₀-C₃)alkyl" refers to an alkyl radical having 0, 1, 2 or 3 carbon atoms and may be methyl, ethyl, n-propyl or i-propyl.

In this specification, unless stated otherwise, the term "alkylene" includes both straight and branched difunctional saturated hydrocarbon radicals and may be methylene (-CH₂-), ethylene (-CH₂-CH₂-), n-propylene (-CH₂-CH₂-CH₂-), i-propylene (-CH-(CH₃)-CH₂-), n-butylene (-CH₂-CH₂-CH₂-CH₂-), i-butylene (-CH₂-CH-(CH₃)-CH₂-), t-butylene (-CH₂-C-(CH₃)-CH₂-), n-pentylene (-CH₂-CH₂-CH₂-CH₂-CH₂-), i-pentylene (-CH₂-CH(CH₃)-CH₂-CH₂-), neo-pentylene (-CH₂-C(CH₃)₂-CH₂-), n-hexylene (-CH₂-CH₂-CH₂-CH₂-CH₂-CH₂-), i-hexylene (-CH₂-CH-(CH₃)-CH₂-CH₂-CH₂-) or neo-hexylene (-CH₂-C(CH₃)₂-CH₂-CH₂-). The term "O-(C₁-C₆)alkylene-aryl" refers to a an alkyl chain having 0, 1, 2, 3, 4, 5 or 6 carbon atoms between an oxygen atom and an aryl group.

In this specification, unless stated otherwise, the term "cycloalkyl" refers to an optionally substituted carbocycle containing no heteroatoms, including mono-, bi-, and tricyclic saturated carbocycles, as well as fused ring systems. Such fused ring systems can include one ring that is partially or fully unsaturated such as a benzene ring to form fused ring systems such as benzo- fused carbocycles. Cycloalkyl includes such fused ring systems as spirofused ring systems. Examples of cycloalkyl include cyclopropyl, cyclobutyl, cyclopentyl, cyclohexyl, bicyclo[1.1.1]pentanyl, decahydronaphthalene, adamantane, indanyl, fluorenyl and 1,2,3,4-tetrahydronaphthalene and the like. The term "(C₃-C₇)cycloalkyl" may be cyclopropyl, cyclobutyl, cyclopentyl, cyclohexyl, cycloheptyl and the like.

In this specification, unless stated otherwise, the term "alkenyl" includes both straight and branched chain alkenyl radicals. The term "(C₂-C₆)alkenyl" refers to an alkenyl radical having 2 to 6 carbon atoms and one or two double bonds, and may be, but is not limited to vinyl, allyl, propenyl, i-propenyl, butenyl, i-butenyl, crotyl, pentenyl, i-pentenyl or hexenyl.

In this specification, unless stated otherwise, the term "alkenylene" includes both straight and branched chain disubstituted alkenyl radicals. The term "(C₂-C₆)alkenylene" refers to an alkenylene radical having 2 to 6 carbon atoms and one or two double bonds, and may be, but is not limited to vinylene, allylene, propenylene, i-propenylene, butenylene, i-butenylene, crotylene, pentenylene, i-pentenylene or hexenylene.

In this specification, unless stated otherwise, the term "alkynyl" includes both straight and branched chain alkynyl radicals. The term (C₂-C₆)alkynyl having 2 to 6 carbon atoms and one or two triple bonds, and may be, but is not limited to ethynyl, propargyl, butynyl, i-butynyl, pentynyl, i-pentynyl or hexynyl.

In this specification, unless stated otherwise, the term "alkynylene" includes both straight and branched chain disubstituted alkynylene radicals. The term (C₂-C₆)alkynylene having 2 to 6 carbon atoms and one or two triple bonds, and may be, but is not limited to ethynylene, propargylene, butynylene, i-butynylene, pentynylene, i-pentynylene or hexynylene.

The term "aryl" refers to an optionally substituted monocyclic or bicyclic hydrocarbon ring system containing at least one unsaturated aromatic ring. Examples and suitable values of the term "aryl" are phenyl, naphthyl, 1,2,3,4-tetrahydronaphthyl, indyl, indenyl and the like.

In this specification, unless stated otherwise, the term "heteroaryl" refers to an optionally substituted monocyclic or bicyclic unsaturated, aromatic ring system containing at least one heteroatom selected independently from N, O or S. Examples of "heteroaryl" may be, but are not limited to benzimidazolyl, benzisothiazolyl benzisoxazolyl, benzofuryl, benzopyrazolyl, benzothiazolyl, benzothiophenyl, benzotriazolyl, benzoxazolyl, furazanyl, furyl, imidazolonyl, imidazolyl, imidazopyridazinyl, imidazopyridyl, indolyl, isoindolyl, isoquinolinyl, isothiazolyl, isoxazolyl, naphthyridinyl, oxadiazolyl, oxazolonyl, oxazolopyridazinyl, oxazolopyridyl, oxazolyl, phthalazinyl, pteridinyl, purinyl, pyrazinyl, pyrazolopyridinyl, pyrazolyl, pyridazinyl, pyridonyl, pyridyl, pyrimidyl, pyrrolyl, quinazolyl, quinolyl, quinoxalinyl, tetrahydrotriazolopyridyl, tetrahydrotriazolopyrimidinyl, tetrazolyl, thiadiazolyl, thiazolonyl, thiazolopyridazinyl, thiazolopyridyl, thiazolyl, thienyl, thionaphthyl, triazinyl and triazolyl.

In this specification, unless stated otherwise, the term "alkylene-aryl", "alkylene-heteroaryl" and "alkylene-cycloalkyl" refers respectively to a substituent that is attached via the alkyl radical to an aryl, heteroaryl or cycloalkyl radical, respectively. The term "(C₁-C₆)alkylene-aryl" includes aryl-C₁-C₆-alkyl radicals such as benzyl, 1-phenylethyl, 2-phenylethyl, 1-phenylpropyl, 2-phenylpropyl, 3-phenylpropyl, 1-naphthylmethyl and 2-naphthylmethyl. The term "(C₁-C₆)alkylene-heteroaryl" includes heteroaryl-C₁-C₆-alkyl radicals, wherein examples of heteroaryl are the same as those illustrated in the above definition, such as 2-furylmethyl, 3-furylmethyl, 2-thienylmethyl, 3-thienylmethyl, 1-imidazolylmethyl, 2-imidazolylmethyl, 3-imidazolylmethyl, 2-oxazolylmethyl, 3-oxazolylmethyl, 2-thiazolylmethyl, 3-thiazolylmethyl, 2-pyridinylmethyl, 3-pyridinylmethyl, 4-pyridinylmethyl, 1-quinolylmethyl and the like.

In this specification, unless stated otherwise, the term "heterocycle" refers to an optionally substituted, monocyclic, bicyclic or tricyclic saturated, partially saturated or unsaturated ring system containing at least one heteroatom selected independently from N, O and S. Bicyclic or tricyclic ring systems may be formed by annelation of two or more rings, by a bridging atom (e.g. O, S, N) or by a bridging group (e.g. alkylene).

Examples of heterocyclic moieties include, but are not limited to: azetidinyl, dihydrofuranyl, dihydrothienyl, dioxolanyl, 1,1-dioxo-thiomorpholinyl, imidazolidinyl, imidazolinyl, isothiazolinyl, isoxazolidinyl, isoxazolinyl, morpholinyl, oxazolidinyl, oxazolinyl, oxetanyl, piperazinonyl, piperazinyl, piperidinonyl, piperidinyl, pyranyl, pyrrolidinonyl, pyrrolidinyl, pyrrolinyl, tetrahydrofuranyl, tetrahydropyranyl, tetrahydrothiopyranyl, thiazolidinyl, thiazolinyl, thiomorpholinyl, thiopyranyl, triazolinyl, and the corresponding benzannulated heterocycles (e.g. dihydrobenzofuranyl, dihydrobenzothiophenyl, dihydrobenzoxazinyl, dihydrofuropyridinyl, dihydroquinolinyl, dihydrothienopyridinyl, indolinyl, pyrrolopyridinyl, tetrahydroquinolinyl, tetrahydroquinoxalinyl, and the like).

**In** this specification, unless stated otherwise, a 5- or 6-membered ring containing one or more atoms independently selected from C, N, O and S, includes aromatic and heteroaromatic rings as well as carbocyclic and heterocyclic rings which may be saturated or unsaturated. Such rings include spirocyclic and bridged bicyclic systems. Examples of such rings may be, but are not limited to dihydrofuranyl, dihydrothienyl, dioxolanyl, 1,1-dioxo-thiomorpholinyl, furazanyl, furyl, imidazolidinyl, imidazolinyl, imidazolonyl, imidazolyl, isothiazolinyl, isothiazolyl, isoxazolidinyl, isoxazolinyl, isoxazolyl, morpholinyl, oxadiazolyl, oxazolidinyl, oxazolinyl, oxazolonyl, oxazolyl, phenyl, piperazinonyl, piperazinyl, piperidinonyl, piperidinyl, pyranyl, pyrazinyl, pyrazolyl, pyridazinyl, pyridonyl, pyridyl, pyrimidyl, pyrrolidinonyl, pyrrolidinyl, pyrrolinyl, pyrrolyl, tetrahydrofuranyl, tetrahydropyranyl, tetrahydrothiopyranyl, tetrazolyl, thiadiazolyl, thiazolidinyl, thiazolinyl, thiazolonyl, thiazolyl, thienyl, thiomorpholinyl, thiopyranyl, triazolinyl, triazinyl, triazolyl, cyclopentyl, cyclopentenyl, cyclohexyl and cyclohexenyl.

**In** this specification, unless stated otherwise, a 3- to 10-membered ring containing one or more atoms independently selected from C, N, O and S, includes aromatic and heteroaromatic rings as well as carbocyclic and heterocyclic rings which may be saturated or unsaturated. Examples of such rings may be, but are not limited to azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phthalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl.

In this specification, unless stated otherwise, the term "halo" or "halogen" may be fluoro, chloro, bromo or iodo.

In this specification, unless stated otherwise, the term "haloalkyl" means an alkyl radical as defined above, substituted with one or more halo radicals. The term "(C₁-C₆)haloalkyl" may include, but is not limited to, fluoromethyl, difluoromethyl, trifluoromethyl, fluoroethyl and difluoroethyl. The term "O-C₁-C₆-haloalkyl" may include, but is not limited to, fluoromethoxy, difluoromethoxy, trifluoromethoxy and fluoroethoxy.

In this specification, unless stated otherwise, the term "cyanoalkyl" means an alkyl radical as defined above, substituted with one or more cyano.

In this specification, unless stated otherwise, the term "optionally substituted" refers to radicals further bearing one or more substituents which may be, acyl, (C₁-C₆)alkyl, - (C₁-C₆)haloalkyl, -(C₃-C₇)cycloalkyl, -(C₁-C₆)alkylene-(C₃-C₇)cycloalkyl, -(C₃-C₇)cycloalkyl-(C₁-C₆)alkylene, -(C₀-C₆)alkylene-(C₃-C₇)spiroalkyl-(C₀-C₆)alkylene, hydroxy, (C₁-C₆)alkylene-oxy, dimethylamino(C₁-C₃)alkyl, mercapto, aryl, heterocycle, heteroaryl, (C₁-C₆)alkylene-aryl, (C₁-C₆)alkylene-heterocycle, (C₁-C₆)alkylene-heteroaryl, halogen, haloalkyl, trifluoromethyl, pentafluoroethyl, haloalkoxy, cyano, cyanomethyl, nitro, amino, amido, amidinyl, oxo, carboxyl, carboxamide, (C₁-C₆)alkylene-oxycarbonyl, carbamate, sulfonamide, ester or sulfonyl.

In this specification, unless stated otherwise, the term "independently" means that where more than one substituent is selected from a number of possible substituents, those substituents may be the same or different.

In this specification, unless stated otherwise, the term "solvate" refers to a complex of variable stoichiometry formed by a solute (e.g. a compound of Formula (I)) and a solvent. The solvent is a pharmaceutically acceptable solvent such as water; such solvent may not interfere with the biological activity of the solute.

In this specification, unless stated otherwise, the term "salt" refers to an acid addition or base addition salt of a compound of the invention. "Salts" include in particular "pharmaceutically acceptable salts".

The pharmaceutically acceptable salts of the invention can be synthesized from a basic or acidic moiety, by conventional chemical methods. When both a basic and an acid group are present in the same molecule, the compounds of the invention may also form internal salts, e.g., zwitterionic molecules.

In this specification, unless stated otherwise, certain compounds may exist in one or more particular geometric, optical, enantiomeric, diastereoisomeric, epimeric, stereoisomeric, tautomeric, conformational, or anomeric forms, including, but not limited to, *cis-* and *trans-forms; E-* and Z-forms; *endo-* and exo-forms, *R-, S-,* and *meso-forms; D-* and L-forms; *d-* and *l*-forms; (+) and (-) forms; keto-, enol-, and enolate-forms; α- and β-forms; axial and equatorial forms; and combinations thereof, collectively referred to as "isomers" or "isomeric forms".

For example, the radical 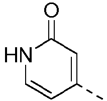 is a tautomer of

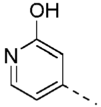

The term "isomer" includes compounds with one or more isotopic substitutions. For example, H may be in any isotopic form, including, but not limited to, ¹H, ²H (D), and ³H (T); C may be in any isotopic form, including, but not limited to, ¹²C, ¹³C, ¹⁴C; O may be in any isotopic form, including, but not limited to, ¹⁶O and ¹⁸O; and the like. F may be in any isotopic form, including, but not limited to, ¹⁹F and ¹⁸F; and the like.

In this specification, unless stated otherwise, the term "negative allosteric modulator of mGlu7" or "allosteric modulator of mGlu7" refers also to a pharmaceutically acceptable acid or base addition salt thereof, a stereochemically isomeric form thereof or an *N-*oxide form thereof.

### PHARMACEUTICAL COMPOSITIONS

Allosteric modulators of mGlu7 described herein, and the pharmaceutically acceptable salts, solvates and hydrates thereof can be used in pharmaceutical preparations in combination with a pharmaceutically acceptable carrier or diluent. Suitable pharmaceutically acceptable carriers include inert solid fillers or diluents and sterile aqueous or organic solutions. The allosteric modulators of mGlu7 will be present in such pharmaceutical compositions in amounts sufficient to provide the desired dosage amount in the range described herein. Techniques for formulation and administration of the compounds of the instant invention can be found in Remington: the Science and Practice of Pharmacy, 19th edition, Mack Publishing Co., Easton, PA (1995).

The amount of allosteric modulators of mGlu7, administered to the subject will depend on the type and severity of the disease or condition and on the characteristics of the subject, such as general health, age, sex, body weight and tolerance to drugs. The skilled artisan will be able to determine appropriate dosages depending on these and other factors. Effective dosages for commonly used CNS drugs are well known to the skilled person. The total daily dose usually ranges from about 0.05 - 2000 mg.

The present invention relates to pharmaceutical compositions which provide from about 0.01 to 1000 mg of the active ingredient per unit dose. The compositions may be administered by any suitable route. For example, orally in the form of capsules and the like, parenterally in the form of solutions for injection, topically in the form of onguents or lotions, ocularly in the form of eye-drops, rectally in the form of suppositories, intranasally or transcutaneously in the form of a delivery system like patches.

For oral administration, the allosteric modulators of mGlu7 thereof can be combined with a suitable solid or liquid carrier or diluent to form capsules, tablets, pills, powders, syrups, solutions, suspensions and the like.

The tablets, pills, capsules, and the like contain from about 0.01 to about 99 weight percent of the active ingredient and a binder such as gum tragacanth, acacias, corn starch or gelatin; excipients such as dicalcium phosphate; a disintegrating agent such as corn starch, potato starch, alginic acid, a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

For parenteral administration the disclosed allosteric modulators of mGlu7, or salts thereof, can be combined with sterile aqueous or organic media to form injectable solutions or suspensions. For example, solutions in sesame or peanut oil, aqueous propylene glycol and the like can be used, as well as aqueous solutions of water-soluble pharmaceutically-acceptable salts of the compounds. Dispersions can also be prepared in glycerol, liquid polyethylene glycols and mixtures thereof in oils. Under ordinary conditions of storage and use, these preparations contain a preservative to prevent the growth of microorganisms.

In addition to the formulations described previously, the compounds may also be formulated as a depot preparation. Such long acting formulations may be administered for example, by subcutaneously implantation or by intramuscular injection. Thus, for example, the compounds may be formulated as an emulsion in an acceptable oil, or ion exchange resins, or as sparingly soluble derivatives, for example, as sparingly soluble salts.

Preferably disclosed allosteric modulators of mGlu7 or pharmaceutical formulations containing these compounds are in unit dosage form for administration to a mammal. The unit dosage form can be any unit dosage form known in the art including, for example, a capsule, an IV bag, a tablet, or a vial. The quantity of active ingredient in a unit dose of composition is an effective amount and may be varied according to the particular treatment involved. It may be appreciated that it may be necessary to make routine variations to the dosage depending on the age and condition of the patient. The dosage will also depend on the route of administration which may be by a variety of routes including oral, aerosol, rectal, transdermal, subcutaneous, intravenous, intramuscular, intraperitoneal and intranasal.

### METHODS OF SYNTHESIS

The compounds according to the invention, in particular the compounds according to the Formula (I) to (VIII), may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (Green T.W. and Wuts P.G.M., (1991) Protecting Groups in Organic Synthesis, John Wiley & Sons). These groups are removed at a convenient stage of the compound synthesis using methods that are readily apparent to those skilled in the art. The selection of process as well as the reaction conditions and order of their execution shall be consistent with the preparation of compounds of Formula (I) to (VIII).

The compounds according to the invention may be represented as a mixture of enantiomers, which may be resolved into the individual pure R- or S-enantiomers. If for instance, a particular enantiomer is required, it may be prepared by asymmetric synthesis or by derivation with a chiral auxiliary, where the resulting diastereomeric mixture is separated and the auxiliary group cleaved to provide the pure desired enantiomers. Alternatively, where the molecule contains a basic functional group such as an amino or an acidic functional group such as carboxyl, this resolution may be conveniently performed by fractional crystallization from various solvents as the salts of an optical active acid or by other methods known in the literature (e.g. chiral column chromatography).

Resolution of the final product, an intermediate or a starting material may be performed by any suitable method known in the art (Eliel E. L., Wilen S. H. and Mander L.N. (1984) Stereochemistry of Organic Compounds, Wiley-Interscience).

Many of the heterocyclic compounds of the invention can be prepared using synthetic routes well known in the art (Katrizky A. R. and. Rees C. W. (1984) Comprehensive Heterocyclic Chemistry, Pergamon Press).

The product from the reaction can be isolated and purified by employing standard techniques, such as extraction, chromatography, recrystallization and distillation.

The compounds of the invention may be prepared by general route of synthesis as disclosed in the following methods.

In one embodiment of the present invention, compounds of Formula (I) may be prepared according to the synthetic sequence illustrated in Scheme 1. 1H-Pyrrole-2-carbaldehyde **g1** may be oxidized in the presence of N-bromosuccinimide, in an appropriate solvent such as carbon tetrachloride, at an appropriate temperature, to afford the intermediate 4-bromo-1H-pyrrole-2-carbaldehyde g2. Intermediate g4 may be prepared by reacting the corresponding intermediate g2 with ethyl hydrazine carboxylate g3 in an appropriate solvent such as toluene, at an appropriate temperature. Intermediate g4 may be converted into bromopyrrolo[1,2-d][1,2,4]triazinone derivatives g5 by condensation in the presence of a base such as sodium hydride, in an appropriate solvent such as DMF, at an appropriate temperature. Intermediates g5 can then be converted into intermediates g7 by suitable reactions known by people skilled in the art of organic synthesis, for example by Suzuki cross coupling reaction, mediated by palladium-complex catalysts such as PdCl₂(dppf) in the presence of a base such as potassium carbonate, in an appropriate solvent such as a mixture of 1,4-dioxane/water. Final compounds g9 may be obtained either by Ullmann coupling reaction with an appropriate aryl halide or heteroaryl halide g8, mediated by copper-complex catalysts such as CuI, in the presence of a base such as potassium phosphate, at an appropriate temperature or by the alkylation of g7 in the presence of a base such as potassium carbonate or cesium carbonate, in an appropriate solvent such as DMF.

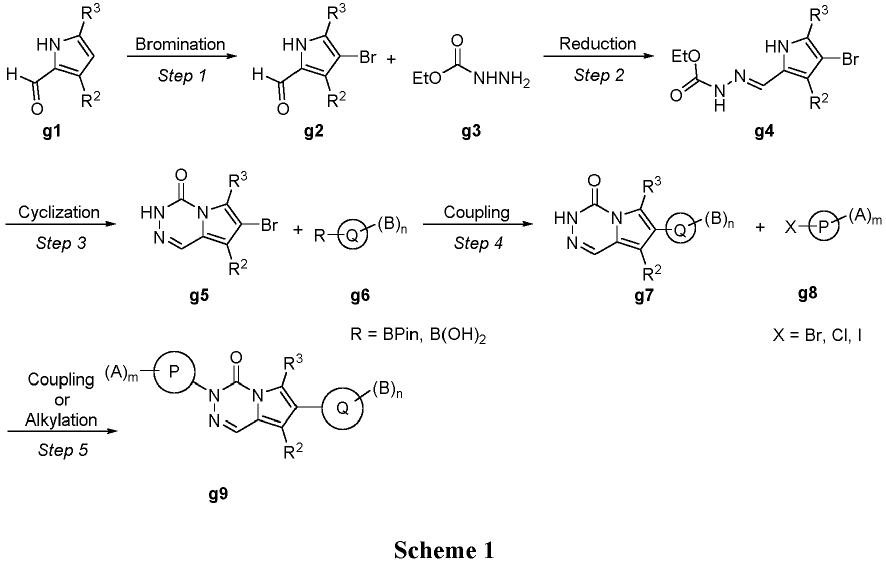

Similarly, final compounds g9 may be prepared according to the synthetic sequence illustrated in Scheme 2. Bromopyrrolo[1,2-d][1,2,4]triazinone derivatives g5, prepared according to Scheme 1 Step 3, may be converted into intermediates **g10** either by Ullmann coupling reaction, mediated by copper-complex catalysts such as CuI, in the presence of a base such as potassium phosphate or by alkylation of g5 in the presence of a base such as potassium carbonate. Final compounds g9 can be obtained by Suzuki cross coupling reaction, mediated by palladium-complex catalysts such as PdCl₂(dppf), in the presence of a base such as potassium carbonate, in an appropriate solvent such as a mixture of 1,4-dioxane/water.

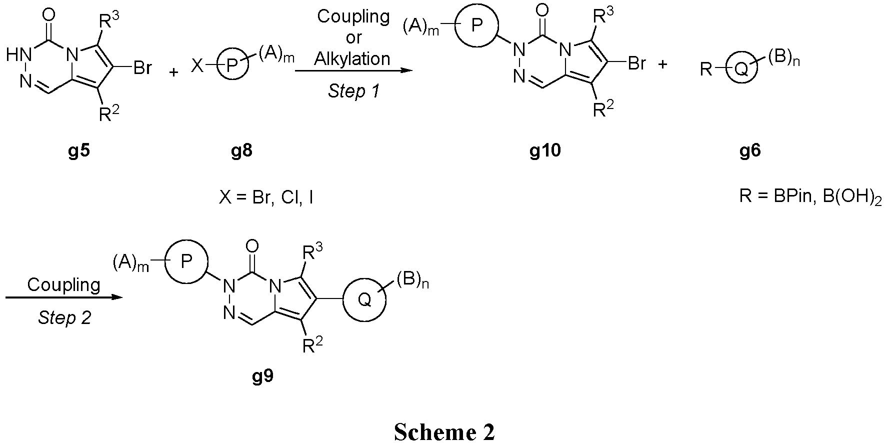

In one embodiment of the present invention, compounds of Formula (II) may be prepared according to the synthetic sequence illustrated in Scheme 3. Final compounds **g12** may be prepared according to the synthetic sequence illustrated in Scheme 3. Intermediates g7, prepared according to Scheme 1 Step 4, may be converted into intermediates **g11** by Ullmann coupling reaction with heteroaryl halide, mediated by copper-complex catalysts such as CuI, in the presence of a base such as potassium phosphate, in an appropriate solvent such as 1,4-dioxane. Deprotection of **g11** with an appropriate acid such as TFA, in an appropriate solvent such as DCM give the final compounds g12.

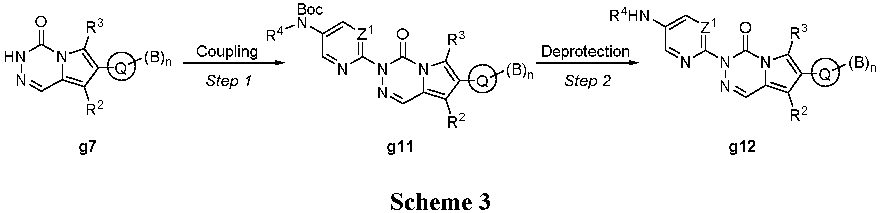

In another specific aspect of Formula (II), final compounds **g14** may be prepared according to the synthetic sequence illustrated in Scheme 4. Intermediates g7, prepared according to Scheme 1 Step 4, may be converted into intermediates **g13** by Ullmann coupling reaction with heteroaryl halide, mediated by copper-complex catalysts such as CuI, in the presence of a base such as *N¹*,*N²*-dimethylethane-1,2-diamine, in an appropriate solvent such as DMF. Final products **g14** can be obtained by nucleophilic substitution of g13 using an appropriate cyclic secondary amine, in an appropriate solvent such as butan-1-ol.

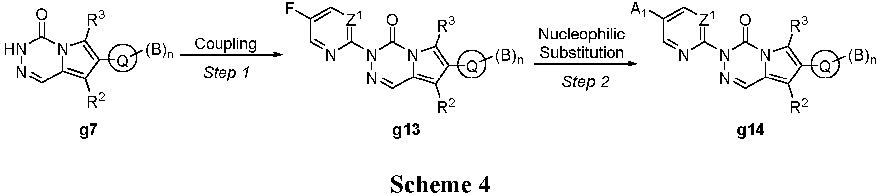

In another specific aspect of Formula (I), final compounds g17 may be prepared according to the synthetic sequence illustrated in Scheme 5. Intermediate compounds g15, prepared according to Scheme 2 Step 1 (R⁴ = H), may be converted into compounds **g16** by Suzuki cross coupling reaction, mediated by palladium-complex catalysts such as PdCl₂(dppf), in the presence of a base such as potassium carbonate, in an appropriate solvent such as a mixture of 1,4-dioxane/water. Bromination of g16 with N-bromosuccinimide in an appropriate solvent such as dry THF, at an appropriate temperature, afford the final compounds g17.

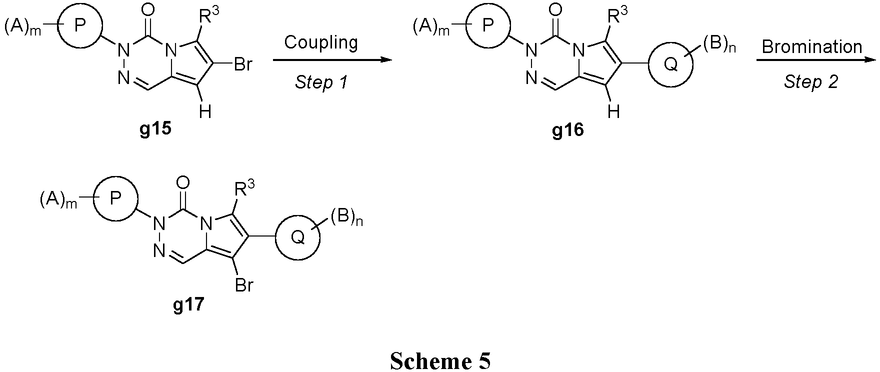

In another specific aspect of Formula (II), final compounds g20 may be prepared according to the synthetic sequence illustrated in Scheme 6. Intermediate compounds g18, prepared according to Scheme 2 Step 1 (R⁴ = H), may be converted into compounds g19 by Miyaura boration, mediated by palladium-complex catalysts such as PdCl₂(dppf), in the presence of a base such as potassium acetate, in an appropriate solvent such as anhydrous 1,4-dioxane. Final compounds g20 can be obtained by Suzuki cross coupling reaction, mediated by palladium-complex catalysts such as PdCl₂(dppf), in the presence of a base such as potassium carbonate, in an appropriate solvent such as a mixture of 1,4-dioxane/water.

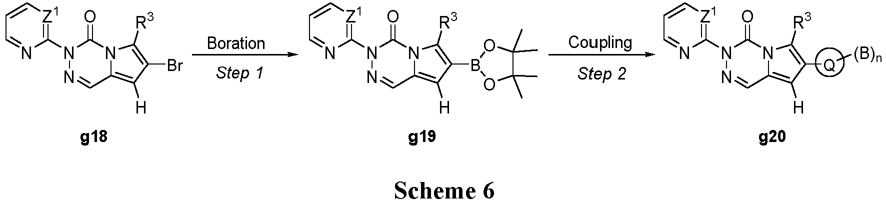

### EXPERIMENTAL

Unless otherwise noted, all starting materials were obtained from commercial suppliers and used without further purification.

Specifically, the following abbreviations may be used in the examples and throughout the specification.

| | |
|---|---|
| ACN (Acetonitrile) | MgSO₄ (Magnesium sulfate) |
| AcOH (Acetic acid) | MHz (Megahertz) |
| BPin (Boronic acid pinacol ester) | min (MinuteS) |
| CCl₄ (Carbon tetrachloride) | mL (Milliliters) |
| CDCl₃ (Deuterated chloroform) | mmol (Millimoles) |
| Cs₂CO₃ (Cesium carbonate) | M.p. (Melting point) |
| Cu(OAc)₂ (Copper (II) acetate) | µm (Micrometers) |
| CuI (Copper (I) iodide) | µl (Microliters) |
| DCM (Dichloromethane) | µmol (Micromoles) |
| DME (1, 2-Dimethoxyethane) | NH₄Cl (Ammonium chloride) |
| DMF (Dimethylformamide) | NaBH₄ (Sodium borohydride) |
| DMPA (*N¹*,*N³*-dimethylpropane-1,3-diamine) | NaH (Sodium hydride) |
| **DMSO** (Dimethyl sulfoxyde) | NaHCO₃ (Sodium bicarbonate) |
| DMSO-*d₆* (Deuterated dimethyl sulfoxide) | NaOH (Sodium hydroxide) |
| EDTA (Ethylenediaminetetraacetic acid) | Na₂CO₃ (Sodium carbonate) |
| ESI (Electrospray Ionization) | Na₂SO₄ (Sodium sulfate) |
| Et₂O (Diethyl ether) | NBS (N-bromosuccinimide) |
| EtOAc (Ethyl acetate) | nd (Not determined) |
| EtOH (Ethanol) | NMR (Nuclear Magnetic Resonance) |
| g (Grams) | PdCl₂(dppf) [1,1'-*Bis*(diphenylphosphino)ferrocene] dichloropalladium(II) |
| h (Hours) | Pd(Ph₃)₄ (Tetrakis(triphenylphosphine)palladium(0)) |
| ¹H (Proton) | Pd(OAc)₂ (Palladium (II) acetate) |
| H₂O (Water) | tBu (Tert-butyl group) |
| HCl (Hydrochloric acid) | psi (Pounds per square inch) |
| Hept (Heptane) | rt (Room temperature) |
| HPLC (High Performance Liquid Chromatography) | RT (Retention Time) |
| Hz (Hertz) | SFC-MS (Supercritical Fluid Chromatography - Mass Spectrometry) |
| K₂CO₃ (Potassium carbonate) | TFA (Trifluoroacetic acid) |
| K₃PO₄ (Potassium phosphate) | THF (Tetrahydrofuran) |
| KOAc (Potassium acetate) | TIC-MS (Total Ion Chromatography - Mass Spectrometry) |
| LC-MS (Liquid Chromatography Mass Spectrometry) | TLC (Thin Layer Chromatography) |
| M (Molar) | UPLC-MS (Ultra Performance Liquid Chromatography-Mass Spectrometry) |
| MeOH (Methanol) | wt% (Weight percent) |
| mg (Milligrams) | Xantphos (4,5-Bis(diphenylphosphino)-9,9-dimethyl xanthene) |

All references to brine refer to a saturated aqueous solution of NaCl. Unless otherwise indicated, all temperatures are expressed in °C (degrees Celsius). All reactions are conducted under an inert atmosphere at room temperature unless otherwise noted.

Most of the reactions were monitored by thin-layer chromatography on pre-coated silica gel plates (TLC Silica gel 60 F₂₅₄, Sigma Aldrich), visualized with UV light. Flash column chromatography was performed on prepacked columns of UltraPure Irregular Silica Gel (40-63µm, 60A) with a capacity of 4g, 12g, 25g, 40g and 80g obtained from Screening Devices BV using a Biotage Isolera One 2.0.8 autocolumn.

### EXAMPLES

### EXAMPLE 1: 7-[3-(azetidin-1-yl)phenyl]-3-ethyl-6,8-dimethyl-pyrrolo[1,2-d][1,2,4]triazin-4-one (Final compound 1-10)

### 4-bromo-3, 5-dimethyl-1H-pyrrole-2-carbaldehyde

According to Scheme 1 Step 1: To a suspension of 3,5-dimethyl-1H-pyrrole-2-carbaldehydE (2.0 g, 16 mmol) in CCl₄ (115 mL) were added NBS (3.0 g, 17 mmol), followed by benzoyl peroxide (98 mg, 0.41 mmol) under argon. The mixture is heated under reflux overnight. The crude mixture is cooled, concentrated *in vacuo* and purified by flash chromatography (Isolera, 80g column, EtOAc:Hept, 50:50) to afford the title compound (2.55 g, 12.6 mmol, 78%) as an olive green solid.

¹H-NMR (500 MHz, DMSO-*d*₆) δ: 12.07 (s, 1H), 9.49 (s, 1H), 2.22 (s, 3H), 2.19 (s, 3H).

### Ethyl (E)-2-((4-bromo-3,5-dimethyl-1H-pyrrole-2-yl)methylene)hydrazine-1-carboxylate

According to Scheme 1 Step 2: To a solution of 4-bromo-3,5-dimethyl-1*H*-pyrrole-2-carbaldehyde (2.55 g, 12.6 mmol) in toluene (50 mL) was added ethyl hydrazine carboxylate (1.97 g, 18.9 mmol). The mixture was heated under reflux overnight. The crude mixture was allowed to cool down to rt. The solid product was filtered and purified by flash chromatography (Isolera, 80g column, EtOAc:Hept, 50:50) to afford the title compound (3.0 g, 10 mmol, 82%) as an olive green solid.

¹H-NMR (500 MHz, DMSO-*d*₆) δ: 11.31 (s, 1H), 10.66 (s, 1H), 7.89 (s, 1H), 4.10 (t, J=7.0 Hz, 2H), 2.14 (s, 3H), 1.99 (s, 3H), 1.22 (t, *J* =7.1 Hz, 3H).

### 7-bromo-6,8-dimethylpyrrolo[1,2-d][1,2,4]triazin-4(3H)-one

According to Scheme I Step 3: A solution of ethyl (*E*)-2-((4-bromo-3,5-dimethyl-1*H-*pyrrole-2-yl)methylene)hydrazine-1-carboxYLate (2.5 g, 8.7 mmol) in DMF (10 mL) was added dropwise to a slurry of NaH (0.11g, 95wt%, 4.3 mmol) in DMF (2.8 mL) at 0°C and then heated at 100°C overnight. After cooling, the solvent was removed *in vacuo* and purified by flash column chromatography (Isolera, 80g column, EtOAc:Hept, 50:50) to afford the title compound (800 mg, 3.30 mmol, 38%) as an orange solid.

¹H-NMR (500 MHz, CDCl₃) δ: 8.84 (s, 1H), 7.81 (s, 1H), 2.79 (s, 3H), 2.24 (s, 3H).

### 7-[3-(azetidin-1-yl)phenyl]-6,8-dimethylpyrrolo[1,2-d][1,2,4]triazin-4(3H)-one

According to Scheme 1 Step 4: To a solution of 7-bromo-6,8-dimethylpyrrolo[1,2-*d*][1,2,4]triazin-4(3*H*)-one (47.1 mg, 0.195 mmol) in a mixture of 1,4-dioxane (0.6 mL) and water (70 µL) were added 1-(3-(4,4,5,5-tetramethyl-1,3,2-dioxaborolan-2-yl)phenyl)azetidine (51.7 mg, 0.295 mmol) and K₂CO₃ (51 mg, 0.37 mmol). The mixture was purged with argon and then PdCl₂(dppf) (10 mg, 14 µmol) was added. The microwave vial was capped, purged again with argon and heated at 110°C in the microwave for 1.5 hour. TLC was then performed and showed that starting material was still present. Therefore, the reaction was heated at 110°C in the microwave for additional 1.5 hour. The reaction was filtered through cElite^{®} and rinsed with DCM (2x 10 mL). The filtrate was concentrated *in vacuo* and purified by flash column chromatography (Isolera, 4g column, EtOAc:Hept, 50:50). The product was washed with Et₂O to afford the title compound (4.0 mg, 13µmol, 11%) as a beige solid.

SFC-MS: RT = 2.96 min; MS m/z [M+H]⁺= 295.1.

### 7-[3-(azetidin-1-yl)phenyl]-3-ethyl-6,8-dimethyl-pyrrolo[1,2-d][1,2,4]triazin-4-one

According to Scheme 1 Step 5: To a solution of 7-[3-(azetidin-1-yl)phenyl]-6,8-dimethylpyrrolo[1,2-*d*][1,2,4]triazin-4(3*H*)-one (50 mg, 0.17 mmol) in DMF (2 mL) weRE added bromoethane (0.19 g, 0.13 mL, 1.7 mmol) and K₂CO₃ (70 mg, 0.51 mmol). The vial was capped and stirred for 20 hours at 120°C. The mixtURe was extracted with Et₂O (3x 10 mL) and washed with brine (1x 25 mL). The organic layer was separated, dried, filtered, concentrated *in vacuo* and purified by flash column chromatography (Isolera, 12g column, EtOAc:Hept, 50:50) to afford the title compound (16 mg, 49 µmol, 29 %).

LC-MS (ESI): RT = 13.33 min; MS m/z [M+H]⁺= 323.1; ¹H-NMR (400 MHz, CDCl₃) δ: 7.85 (s, 1H), 7.26 (t, *J =*7.8 Hz, 1H), 6.60 (ddd, *J =* 7.5, 1.6, 1.0 Hz, 1H), 6.45 (ddd, *J =*8.1*,* 2.4, 1.0 Hz, 1H), 6.32 - 6.26 (m, 1H), 4.10 (q, *J =* 7.2 Hz, 2H), 3.90 (t, *J=* 7.2 Hz, 4H), 2.74 (s, 3H), 2.44 - 2.33 (m, 2H), 2.18 (s, 3H), 1.37 (t, *J =* 7.1 Hz, 3H).

### EXAMPLE 2: 7-[3-(cyclopropoxy)-2-methyl-phenyl]-3-pyrimidin-2-yl-pyrrolo[1,2-d][1,2,4]triazin-4-one (Final compound 1-18)

### 4-bromo-1H-pyrrole-2-carbaldehyde

According to Scheme 1 Step 1: 1*H*-pyrrole-2-carbaldehyde (5000 mg, 52.58 mmol) was added to ACN (100 mL) and was cooled down to 0°C. NBS (9.358 g, 52.58 mmol) was dissolved in ACN (100 mL) and this solution was slowly added to the cooled mixture at 0°C in a period of 30 min using a dropping funnel. The mixture was stirred for 30 miN after which, ice cold water (100 mL) was added. The mixturE was extracted with EtOAc (3x 100 mL) and the combined organic layERs were washed with brine (1x 100 mL), dried over MgSO₄, filtered, and the solvents were removed under reduced pressure. Purification by silica gel chromatography (n-hexane/EtOAc 100:0 → 90:10) was performed to afford the title compound (5616.8 mg, 32.280 mmol, 61.40 %) as white solid.

¹H-NMR (400 MHz, CDCl₃) δ: 9.80 (s, 1H), 9.33 (s, 1H), 7.11 (m, 1H), 6.98 (dd, 1H).

### Ethyl (E)-2-((4-bromo-1H-pyrrol-2-yl)methylene)hydrazine-1-carboxylate

According to Scheme 1 Step 2: To 4-bromo-1*H*-pyrrole-2-carbaldehyde (5397 mg, 31.02 mmol) in toluene (60 mL) was added ethyl hydrazine carboxylate (4.844 g, 46.53 mmol). The mixture was then refluxed for 3 hours and the crude mixture was then allowed to cool to rt. The solvent was removed under reduced pressure and the mixture was further purified by silica gel chromatography (n-hexane/EtOAc 100:0 → 50:50) to afford the title compound (5.911 g, 22.73 mmol, 73.27 %) as a clear white solid.

¹H-NMR (400 MHz, DMSO-*d*₆) δ: 11.66 (s, 1H), 10.88 (s, 1H), 7.82 (s, 1H), 6.93 (dd, *J* =2.9*,* 1.6 Hz, 1H), 6.44 (dd, *J* =2.6*,* 1.6 Hz, 1H), 4.13 (q*, J* =7.1 Hz, 2H), 1.23 (t, *J* =7.1 Hz, 3H).

### 7-bromopyrrolo[1,2-d][1,2,4]triazin-4(3H)-one

According to Scheme 1 Step 3: A solution of ethyl (*E*)-2-((4-bromo-1*H*-pyrrol-2-yl)methylene)hydrazine-1-carboXYlate (5.80 g, 22.3 mmol) in DMF (80 mL) was added dropwise to a slurry of NaH (281.7 mg, 95wt%, 11.15 mmol) in A mixture of DMF (10 mL) and water (2 mL) at 0°C. The reaction mixture was then stirred for approximately 30 min at 0°C after which, the reaction mixture was heated to 100 °C for 20 hours. The reaction mixture was then concentrated under reduced pressure to afford the title compound (4.0 g, 19 mmol, 84 %).

¹H-NMR (400 MHz, CDCl₃) δ: 9.36 (s, 1H), 7.99 - 7.94 (m, 1H), 7.79 (dd, *J* =1.5*,* 0.7 Hz, 1H), 6.77 (d, *J* =1.5 Hz, 1H).

### 7-(3-cyclopropoxy-2-methylphenyl)pyrrolo[1,2-d][1,2,4]triazin-4(3H)-one

According to Scheme 1 Step 4: To a solution of 7-bromopyrrolo[1,2-*d*][1,2,4]triazin-4(3*H*)-one (100 mg, 467 µMOl) in a mixture of 1,4-dioxane (1.5 mL) and water (0.17 mL) were added 2-(3-cyclopropoxy-2-methylphenyl)-4,4,5,5-tetramethyl-1,3,2-dioxaborolane (128 mg, 467 µmol) and K₂CO₃ (187 mg, 1.36 mmol). The mixture was purged with argon after which, PdCl₂(dppf) (37.6 mg, 51.4 µmol) was added. The reaction mixture was purged with argon again and the vial was capped and heated at 110°C for 20 hours. The reaction mixture was diluted with DCM, filtered over Celite^{®} and concentrated under reduced pressure. Purification by silica gel chromatography (n-hexane/EtOAc 100:0 → 80:20) was performed to afford the title compound (68.2 mg, 242 µmol, 51.9 %).

¹H-NMR (400 MHz, CDCl₃) δ: 9.03 (s, 1H), 8.03 (d, *J* =6.2 Hz, 1H), 7.81 (dd, *J =*1.5*,* 0.7 Hz, 1H), 7.27 (dd, *J* =2.1, 1.2 Hz, 1H), 7.04 - 6.95 (m, 1H), 6.84 (d, *J* =1.5 Hz, 1H), 6.81 - 6.75 (m, 0H), 3.84 - 3.75 (m, 1H), 2.24 (s, 3H), 0.88 - 0.80 (m, 4H).

### 7-[3-(cyclopropoxy)-2-methyl-phenyl]-3-pyrimidin-2-yl-pyrrolo[1,2-d][1,2,4]triazin-4-one

According to Scheme 1 Step 5: To 7-(3-cyclopropoxy-2-methylphenyl)pyrrolo[1,2-*d*][1,2,4]triazin-4(3*H*)-one (60.0 mg, 213 µmol) were added CuI (20.3 mg, 107 µmol), 2-bromopyrimidine (102 mg, 640 µmol), K₃PO₄ (113 mg, 533 µmol), 1,10-phenanthroline (23.1 mg, 128 µmol) and 1,4-dioxane (1.62 g, 1.57 mL, 18.3 mmol). The solution was purged with argon for 5 min after which, the vial was capped and heated at 110°C for 20 hours. The mixture was diluted with DCM, filtered over Celite^{®}, concentrated *in vacuo* and further purified using flash column chromatography. The obtained fractions were concentrated *in vacuo,* redissolved in MeOH and further purified by preparative HPLC. The obtained fractions were concentrated *in vacuo,* redissolved in AcOH and freeze-dried to afford the title compound (12.7 mg, 35.3 µmol, 16.6%).

LC-MS: RT = 11.62 min; MS m/z [M+H]⁺= 360.3; ¹H-NMR (400 MHz, CDCl₃) δ: 8.96 (d, *J =*4.8 Hz, 2H), 8.22 (d, *J =*0.7 Hz, 1H), 7.92 (dd, *J* =1.5, 0.7 Hz, 1H), 7.42 (t, *J* =4.8 Hz, 1H), 7.27 (d, *J* =2.1 Hz, 1H), 7.24 (t, *J* =7.9 Hz, 1H), 7.03 (dd, *J =*7.0*,* 1.9 Hz, 1H), 6.89 (d, *J=1.5* Hz, 1H), 3.84 - 3.75 (m, 1H), 2.25 (s, 3H), 0.83 (m, 4H).

### EXAMPLE 3: 7-[3-(azetidin-1-yl)phenyl]-3-[5-(1-hydroxy-1-methyl-ethyl)-2-pyridyl]-6,8-dimethyl-pyrrolo[1,2-d][1,2,4]triazin-4-one (Final compound 1-24)

### 7-[3-(azetidin-1-yl)phenyl]-3-[5-(1-hydroxy-1-methyl-ethyl)-2-pyridyl]-6,8-dimethyl-pyrrolo[1,2-d][1,2,4]triazin-4-one

According to Scheme 1 Step 5: To a solution of 7-[3-(azetidin-1-yl)phenyl]-6,8-dimethylpyrrolo[1,2-*d*][1,2,4]triazin-4(3*H*)-one (45 mg, 0.15 mmol, prepared according Scheme 1 StEP 4 - EXAMPLE 1) in 1,4-dioxane (1.2 mL) were added K₃PO₄ (81 mg, 0.38 mmol), CuI (15 mg, 0.076 mmol), 2-(6-bromopyridin-3-yl)propan-2-ol (99 mg, 0.46 mmol) and DMPA (8.1 mg, 9.9 µL, 0.092 mmol). The solution was purged with argon for 5 min after which, the microwave vial was capped and heated at 110°c overnight in a sand bath. Water (10 mL) was added and the miXTure was extracted with EtOAc (3X 50 mL) and washeD with water (2x 30 mL) and brine (50 mL). The combined organic layers were dried, filtered, concentrated *in vacuo* and purified by flash column chromatography (Isolera, 12g column, EtOAc:Hept, 50:50) to afford the title compound (15 mg, 33 µmol, 14%) as a beige solid.

LC-MS (ESI): RT = 11.47 min; MS m/z [M+H]⁺= 430.4; ¹H-NMR (400 MHz, CDCl₃) δ: 8.73 (d, *J =*2.5 Hz, 1H), 8.02 (s, 1H), 7.97 (dd, *J* =8.4*,* 2.6 Hz, 1H), 7.60 (d*, J =*8.4 Hz, 1H), 7.29 (d, *J =*7.8 Hz, 2H), 6.62 (dt, *J =*7.5*,* 1.3 Hz, 1H), 6.50 - 6.43 (m, 1H), 6.31 (t, *J* =2.0 Hz, 1H), 3.91 (t, *J* =7.2 Hz, 4H), 2.78 (s, 1H), 2.75 (s, 3H), 2.39 (p, *J* =7.2 Hz, 2H), 2.23 (s, 3H), 1.64 (s, 6H).

### EXAMPLE 4: 6,8-dimethyl-3-pyrimidin-2-yl-7-[3-(trifluoromethoxy)phenyl]pyrrolo[1,2-d][1,2,4]triazin-4-one (Final compound 1-53)

### 6,8-dimethyl-7-(3-(trifluoromethoxy)phenyl)pyrrolo[1,2-d][1,2,4]triazin-4(3H)-one

According to Scheme 1 Step 4: To a solution of 7-bromo-6,8-dimethylpyrrolo[1,2-*d*][1,2,4]triazin-4(3H)-one (100 mg, 413 µmol, prepared according to Scheme 1 Step 3 - EXAMplE 1) in a mixture Of 1,4-dioxane (2 mL) and water (0.2 mL) were added 4,4,5,5-tetramethyl-2-(3-(trifluoromethoxy)phenyl)-1,3,2-dioxaborolane (178 mg, 620 µmol) and K₂CO₃ (171 mg, 1.24 mmol). The mixture was purged with argon and PdCl₂(dppf) (75.6 mg, 103 µmol) was added. The reaction mixture was purged again with argon and the vial was capped and heated at 110°C in the microwave for 3 hours. The mixture was then filtered over Celite^{®}, concentrated and further purified by flash column chromatography to afford the title compound (166 mg, 513 µmol, 124 %).

¹H-NMR (400 MHz, CDCl₃) δ: 9.43 (s, 1H), 7.96 (s, 2H), 7.56 - 7.47 (m, 1H), 7.30 - 7.24 (m, 1H), 7.22 (dt, *J* =7.6, 1.3 Hz, 1H), 7.15 (dt, *J* =2.5, 1.3 Hz, 1H), 2.77 (s, 3H), 2.23 (s, 3H).

### 6,8-dimethyl-3-pyrimidin-2-yl-7-[3-(trifluoromethoxy)phenyl]pyrrolo[1,2-d][1,2,4]triazin-4-one

According to Scheme 1 Step 5: To a solution of 6,8-dimethyl-7-(3-(trifluoromethoxy)phenyl)pyrrolo[1,2-*d*][1,2,4]triazin-4(3*H*)-one (45 mg, 90 wt%, 0.13 mmol) in DMF (2.00 mL) were added 2-bromopyrimidine (60 mg, 0.38 mmol) and CuI (12 mg, 63 µmol) and was purged for 5 min with argon. The reaction was then stirred overnight at 110°C. The mixture was diluted with DCM, filtered over Celite^{®}, concentrated *in vacuo* and further purified using flash column chromatography. The obtained fractions were then concentrated *in vacuo,* redissolved in MeOH and further purified by preparative HPLC. Finally, the obtained fractions were concentrated *in vacuo,* redissolved in AcOH and freeze-dried to afford the title compound (6 mg, 0.01 mmol, 10 %, 100% purity).

LC-MS (ESI): RT = 12.42 min; MS m/z [M+H]⁺= 402.2; ¹H-NMR (400 MHz, CDCl₃) δ: 8.96 (s, 2H), 8.10 (s, 1H), 7.52 (t, *J* =8.0 Hz, 1H), 7.40 (t, *J* =4.7 Hz, 1H), 7.33 - 7.19 (m, 2H), 7.17 (dt, *J* =2.5, 1.2 Hz, 1H), 2.78 (s, 3H), 2.26 (s, 3H).

### EXAMPLE 5: 7-[3-(azetidin-1-yl)phenyl]-6,8-dimethyl-3-(2-pyridyl)pyrrolo[1,2-d][1,2,4]triazin-4-one (Final compound 1-3)

### 7-bromo-6,8-dimethyl-3-(pyridin-2-yl)pyrrolo[1,2-d][1,2,4]triazin-4(3H)-one

According to Scheme 2 Step 1: To a solution of 7-bromo-6,8-dimethylpyrrolo[1,2-d][1,2,4]triazin-4(3H)-one (538 mg, 2.22 mmol, prepared according to SCHeme 1 Step 3 -EXAMPLE 1) in DMF (15 mL) were added K₃PO₄ (1.18 g, 5.56 mmol), CuI (212 mg, 1.11 mmol), 2-bromopyridine (1.05 g, 638 µL,6.67 mmol) and DMPA (136 mg, 16.0 µL, 1.33 mmol). The solution was purged with argon for 5 min after which, the microwave vial was capped and heated at 110°C in THe microwave for 1.5 hour. Water (10 mL) was added and the soluTIon was acidified with 2 N HCl until pH = 5. The miXTure was extracted with EtOAc (3X 50 mL) and washeD with water (2x 30 mL) and brine (50 mL). The combined organic layers were dried, filtered, concentrated *in vacuo* and purified by flash column chromatography (Isolera, 25g column, EtOAc:Hept, 50:50) to afford the title compound (365.7 mg, 1.146 mmol, 52%) as a solid.

SFC-MS: RT = 3.06 min; MS m/z [M+H]⁺= 320.9.

### 7-[3-(azetidin-1-yl)phenyl]-6,8-dimethyl-3-(2-pyridyl)pyrrolo[1,2-d][1,2,4]triazin-4-one

According to Scheme 2 Step 2: To a solution of 7-bromo-6,8-dimethyl-3-(pyridin-2-yl)pyrrolo[1,2-*d*][1,2,4]triazin-4(3*H*)-one (40 mg, 0.13 mMOl) in a mixture of 1,4-dioxane (0.6 mL) an water (70 µL) were added 1-(3-(4,4,5,5-tetramethyl-1,3,2-dioxaborolan-2-yl)phenyl)azetidine (49 mg, 0.19 mmol), K₂CO₃ (52 mg, 0.38 mmol). The mixture was purged with argon and then PdCl₂(dppf) (10 mg, 14 µmol) was added. The microwave vial was then capped, purged again with argon and heated at 110°C in the microwave for 3 hours. The reaction was filtered throuGH Celite^{®} and rinsed with DCM (2x 10 mL). The filtrate was concentrated *in vacuo* and purified by flash column chromatography (Isolera, 4g column, EtOAc:Hept, 50:50). The product was crashed out using Et₂O to afford the title compound (20 mg, 54µmol, 43%) as a beige solid.

SFC-MS: RT = 3.94 min; MS m/z [M+H]⁺= 372.1; ¹H- NMR (500 MHz, CDCl₃) δ: 8.65 (ddd, *J* =4.9, 2.0, 0.9 Hz, 1H), 8.04 (s, 1H), 7.87 - 7.81 (m, 1H), 7.66 (dt, *J* =8.2, 1.0 Hz, 1H), 7.35 - 7.25 (m, 2H), 6.62 (dt, *J* =7.5, 1.3 Hz, 1H), 6.46 (ddd, *J* =8.1, 2.4, 1.0 Hz, 1H), 6.31 (t, *J* =1.9 Hz, 1H), 3.91 (t, *J* =7.2 Hz, 4H), 2.75 (s, 3H), 2.45 - 2.34 (m, 2H), 2.23 (s, 3H).

### EXAMPLE 6: 3-(5-amino-2-pyridyl)-7-[3-(azetidin-1-yl)phenyl]-6,8-dimethyl-pyrrolo[1,2-d][1,2,4]triazin-4-one (Final compound 1-34)

### Tert-butyl N-[6-[7-[3-(azetidin-1-yl)phenyl]-6, 8-dimethyl-4-oxo-pyrrolo[1,2-d][1,2,4]triazin-3-yl]-3-pyridyl]carbamate

According to Scheme 3 Step 1: To a solution of 7-[3-(azetidin-1-yl)phenyl]-6,8-dimethylpyrrolo[1,2-*d*][1,2,4]triazin-4(3*H*)-one (40 mg, 0.14 mmol, prepared according Scheme 1 StEP 4 - EXAMPLE 1) in 1,4-dioxane (1.2 mL) were added K₃PO₄ (72 mg, 0.34 mmol), CuI (13 mg, 68 µmol), tert-butyl (6-bromopyridin-3-yl)carbamate (74 mg, 0.27 mmol) and 1,10-phenanthroline (15 mg, 82 µmol). The solution was purged with argon for 5 min after which, the microwave vial was capped and heated overnight at 110°C in a sand bath. The crude reaction mixture was filtered through Celite^{®} and washed with Et₂O, concentrated *in vacuo* and purified by flash column chromatography (Isolera, 12g column, EtOAc:Hept, 50:50) to afford the title compound (40 mg, 75 µmol, 55%) as a beige solid.

LC-MS (ESI): RT = 13.54 min; MS m/z [M+H]⁺= 487.0.

### 3-(5-amino-2-pyridyl)-7-[3-(azetidin-1-yl)phenyl]-6,8-dimethyl-pyrrolo[1,2-d][1,2,4]triazin-4-one

According to Scheme 3 Step 2: To a solution of tert-butyl *N*-[6-[7-[3-(azetidin-1-yl)phenyl]-6,8-dimethyl-4-oxo-pyrrolo[1,2-*d*][1,2,4]triazin-3-yl]-3-pyridyl]caRBamate (8 mg, 0.02 mmol) in DCM (0.5 mL) was added TFA (74 mg, 50 µL, 0.65 mmol). The reaction was stirred overnight at rt. The crude mixture WAs extracted wiTH 5% bicarbonate (20 mL) and DCM (10 mL), dried, concentrated *in vacuo* and purified by flash column chromatography (Isolera, 12g column, EtOAc:Hept, 50:50) to afford the title compound (3.15 mg, 7.8 µmol, 50%) as a white solid.

LC-MS (ESI): RT = 12.59 min; MS m/z [M+H]⁺= 388.2; ¹H-NMR (500 MHz, CDCl₃) δ: 8.31 (s, 2H), 7.98 (s, 1H), 7.27 (s, 1H), 6.64 - 6.59 (m, 1H), 6.46 (ddd, *J* =8.1, 2.4, 0.9 Hz, 1H), 6.31 (t, *J =*2.0 Hz, 1H), 3.95 - 3.85 (m, 4H), 2.74 (s, 3H), 2.37 (dt, *J* =15.3*,* 7.6 Hz, 2H), 2.22 (s, 3H).

### EXAMPLE 7: 7-[3-(cyclopropoxy)phenyl]-6,8-dimethyl-3-(5-morpholinopyrimidin-2-yl)pyrrolo[1,2-d][1,2,4]triazin-4-one (Final compound 1-47)

### 7-[3-(cyclopropoxy)phenyl]-3-(5-fluoropyrimidin-2-yl)-6,8-dimethyl-pyrrolo[1,2-d][1,2,4]triazin-4-one

According to Scheme 4 Step 1: To a solution of 7-(3-cyclopropoxyphenyl)-6,8-dimethylpyrrolo[1,2-*d*][1,2,4]triazin-4(3*H*)-one (61 mg, 0.21 mmol, prepared acCOrding to Scheme 1 Step 4) in DMF (2 mL) were added CuI (20 mg, 0.10 mmol), 2-bromo-5-fluoropyrimidine (37 mg, 0.21 mmol) and *N¹,N²*-dimethylethane-1,2-diamine (11 mg, 13 µL, 0.12 mmol). The solution was purged with argon for 5 min after which, the microwave vial was capped, purged again with argon and heated at 110°C in the microwave for 3 hoURs. After full conversion, water (10 mL) and saturated EDTA (2 mL) were added and the mIXture was extracted with Et₂O (3x 20 ml) and washed with Saturated EDTA (10 mL), water (2x 20 mL) and brine (20 mL). The combined organic layers were dried, filtered, concentrated *in vacuo* and purified by flash column chromatography (Isolera, SILICYCLE 12g column, EtOAc:Hept, 0-20%) to afford the title compound (11 mg, 28 µmol, 14%).

LC-MS (ESI): RT = 12.84 min; MS m/z [M+H]⁺= 392.2; ¹H-NMR (500 MHz, CDCl₃) δ: 8.75 (s, 2H), 8.03 (s, 1H), 7.37 (dd, *J* =8.3, 7.6 Hz, 1H), 7.08 (ddd, *J* =8.2, 2.5, 1.0 Hz, 1H), 6.94 (dd, *J* =2.6, 1.5 Hz, 1H), 6.87 (dt, *J* =7.6, 1.3 Hz, 1H), 3.80 - 3.72 (m, 1H), 2.76 (s, 3H), 2.25 (s, 3H), 0.83 - 0.74 (m, 4H).

### 7-[3-(cyclopropoxy)phenyl]-6,8-dimethyl-3-(5-morpholinopyrimidin-2-yl)pyrrolo[1,2-d][1,2,4]triazin-4-one

According to Scheme 4 Step 2: To a stirred solution of 7-[3-(cyclopropoxy)phenyl]-3-(5-fluoropyrimidin-2-yl)-6,8-dimethyl-pyrrolo[1,2-*d*][1,2,4]triazin-4-one (15.8 mg, 40.6 µmol) in butan-1-ol (1 mL) was added morpholine (35.30 mg, 35.00 µL, 405.7 µmol). The microwave vial was capped and the solution was stirred overnight at 160°C. The crude mixture was concentrated *in vacuo* and purified by flash chromatography to afford the title compound (6.3 mg, 12 µmol, 30%).

LC-MS (ESI): RT = 12.21 min; MS m/z [M+H]⁺= 459.4; ¹H-NMR (500 MHz, CDCl₃) δ: 8.46 (s, 2H), 8.00 (s, 1H), 7.36 (dd, *J* =8.3*,* 7.5 Hz, 1H), 7.07 (ddd, *J* =8.3*,* 2.6, 1.0 Hz, 1H), 6.95 (dd, *J* =2.6*,* 1.5 Hz, 1H), 6.87 (ddd, *J* =7.5*,* 1.6, 1.0 Hz, 1H), 3.92 - 3.89 (m, 4H), 3.48 (q, *J* =7.0 Hz, 2H), 3.31 - 3.27 (m, 4H), 2.76 (s, 3H), 2.24 (s, 3H), 0.84 - 0.77 (m, 4H).

### EXAMPLE 8: 8-bromo-7-(2-fluoro-3-methoxy-phenyl)-6-methyl-3-(2-pyridyl)pyrrolo[1,2-d][1,2,4]triazin-4-one (Final compound 1-115)

### 7-(2-fluoro-3-methoxy-phenyl)-6-methyl-3-(2-pyridyl)pyrrolo[1,2-d][1,2,4]triazin-4-one

According to Scheme 5 Step 1: To a stirred solution of 7-bromo-6-methyl-3-(pyridin-2-yl)pyrrolo[1,2-*d*][1,2,4]triazin-4(3*H*)-one (100 mg, 0.329 mmol, prepared according to Scheme 2 StEP 1) in a mixturE of 1,4-dioxane (10 mL) and water (1 mL) were added 2-(2-fluoro-3-methoxyphenyl)-4,4,5,5-tetramethyl-1,3,2-dioxaborolane (91 mg, 0.36 mmol) and K₂CO₃ (132 mg, 0.954 mmol). Then, PdCl₂(dppf) (29 mg, 0.040 mmol) was added in one portion, and the mixture was stirred at 110 °C overnight in a sealed pressure tube. The reaction mixture was diluted with DCM, filtered over Celite^{®} and concentrated under reduced pressure. A first silica gel chromatography (*n*-hexane/ EtOAc 100:0 → 1:1) afforded the title compound impure. A second silica gel chromatography (DCM/ EtOAc 100:0 → 95:5) gave the title compound (93 mg, 0.266 mmol, 81%) as an off-white solid.

TIC-MS: RT = 4.26 min; MS m/z [M+H]⁺= 351.3.

### 8-bromo-7-(2-fluoro-3-methoxy-phenyl)-6-methyl-3-(2-pyridyl)pyrrolo[1,2-d][1,2,4]triazin-4-one

According to Scheme 5 Step 2: NBS (136 mg, 0.765 mmol) was added to a stirred solution of 7-(2-fluoro-3-methoxy-phenyl)-6-methyl-3-(2-pyridyl)pyrrolo[1,2-d][1,2,4]triazin-4-onE (134 mg, 0.382 mmol) in dry THF (10 mL). After 3 hours, an additional portion of NBS (34 mg) was added and the reaction mixture was stirred overnight at rt. The reacTIon mixture was diluted with DCM (25 mL) And washed with sATurated NaHCO₃ (25 mL) and brine (10 mL). The organic phase was dried over MgSO₄, filtered and the solvent was removed under reduced pressure. Silica gel chromatography (n-hexane/ EtOAc 100:0 → 60:40) was performed to afford the title compound (141 mg, 0.329 mmol, 86%) as a colorless solid.

TIC-MS: RT = 4.63 min; MS m/z [M+H]⁺= 429.2; ¹H-NMR (500 MHz, CDCl₃) δ: 8.71 (dd, *J* =4.9, 1.9 Hz, 1H), 8.13 (s, 1H), 7.95 (td, *J* =7.8, 1.9 Hz, 1H), 7.75 (d, *J* =8.1 Hz, 1H), 7.41 (ddd, *J* =7.4, 5.0, 1.1 Hz, 1H), 7.19 (td, *J* =8.0, 1.4 Hz, 1H), 7.06 (td, *J* =8.2, 1.6 Hz, 1H), 6.88 (ddd, *J =*7.6*,* 6.0, 1.6 Hz, 1H), 3.95 (s, 3H), 2.73 (d, *J* =1.2 Hz, 3H).

### EXAMPLE 9: 6-methyl-7-(1-methyl-2,3-dihydropyrrolo[2,3-b]pyridin-4-yl)-3-(2-pyridyl)pyrrolo[1,2-d][1,2,4]triazin-4-one (Final compound 1-118)

### 6-methyl-3-(pyridin-2-yl)-7-(4,4,5,5-tetramethyl-1,3,2-dioxaborolan-2-yl)pyrrolo[1,2-d][1,2,4]triazin-4(3H)-one

According to Scheme 6 Step 1: A stirred mixture of 7-bromo-6-methyl-3-(pyridin-2-yl)pyrrolo[1,2-*d*][1,2,4]triazin-4(3*H*)-one (200 mg, 0.655 mmol, prepared according to Scheme 2 Step 1), bis(pinacolato)diboron (200 mg, 0.79 mmol) and KOAc (193 mg, 1.97 mmol) in anhydrous 1,4-dioxane (10 mL) was degassed with nitrogen for 10 min. PdCl₂(dppf) (24 mg, 0.033 mmol) was added, and the resulting mixture was stirred in a sealed pressure tube at 100°C. After 18 hours, additional PdCl₂(dppf) (24 mg, 0.033 mmol) was added, and the mixture was stirred for further 24 hours at 100°C. After 42 hours in total, the reacTIon mixture was diluted with DCM (30 mL) and filtered ovER a short plug of Celite^{®}. Water (10 mL) was added, and the layers were separated. The aqueous layer was extracted with DCM (2x 30 mL). The combined orgaNIc layers were washed with brine (30 mL), dried over MgSO₄, filtered, and concentrated *in vacuo.* Silica gel chromatography of the residue (DCM/EtOAc 9:1) gave the title compound impure as a pale-brown solid (105 mg). A second silica gel chromatography (n-hexane/EtOAc 90:10 → 60:40) afforded the title compound (64.2 g, 0.182 mmol, 28%) as an off-white solid.

¹H-NMR (500 MHz, CDCl₃) δ:8.67 (s, 1H), 8.02 (s, 1H), 7.89 (td, *J =* 7.8, 1.8 Hz, 1H), 7.72 (d, *J =* 8.2 Hz, 1H), 7.35 (dd, *J =* 7.3, 4.9 Hz, 1H), 6.90 (s, 1H), 3.00 (s, 3H), 1.34 (s, 12H).

### 6-methyl-7-(1-methyl-2,3-dihydropyrrolo[2,3-b]pyridin-4-yl)-3-(2-pyridyl)pyrrolo[1,2-d][1,2,4]triazin-4-one

According to Scheme 6 Step 2: To a stirred solution of 6-methyl-3-(pyridin-2-yl)-7-(4,4,5,5-tetramethyl-1,3,2-dioxaborolan-2-yl)pyrrolo[1,2-*d*][1,2,4]triazin-4(3*H*)-one (263 mg, 0.75 MMol) in a mixture OF 1,4-dioxane (13 mL) and water (1.3 mL) were added 4-bromo-1-methyl-2,3-dihydro-1*H*-pyrrolo[2,3-b]pyridine (155 mg, 0.82 mmol) and K₂CO₃ (300 mg, 2.17 mmol). PdCl₂(dppf) (66 mg, 0.090 mmol) was then added in one portion, and the mixture was stirred overnight at 110 °C in a sealed pressure tube. After 20 hours, the reacTIon mixture was diluted with DCM (50 mL), filtered over a plug of MgSO₄ and concentrated under reduced pressure. A first silica gel chromatography (EtOAc/MeOH 100:0 → 99:1) of the black residue gave the desired product (223 mg) contaminated with Pd catalyst residues as a brown solid. A second silica gel chromatography (DCM/EtOAc 100:0 → 0:100) afforded the title compound (211 mg) as an amber solid. The final third silica gel chromatography (DCM/MeOH 100:0 → 97:3) eventually yielded the desired product (193 mg) as a light-orange solid. Precipitation from DCM/n-pentane (1:5) provided the title compound (158 mg, 0.44 mmol, 59%) as a powdery beige solid.

TIC-MS: RT = min; MS m/z ES⁺=; ¹H-NMR (500 MHz, CDCl₃) δ: 8.65 (ddd, *J* =4.9, 1.9, 0.8 Hz, 1H), 8.05 (s, 1H), 7.92 - 7.90 (m, 1H), 7.87 (td, *J* =7.8, 1.9 Hz, 1H), 7.66 (dt, *J* =8.0, 0.9 Hz, 1H), 7.35 (ddd, *J* =7.5, 4.9, 1.0 Hz, 1H), 6.67 (s, 1H), 6.40 (d, *J* =5.6 Hz, 1H), 3.51 (t, *J* =8.2 Hz, 2H), 3.02 (s, 3H), 2.92 (t, *J* =8.3 Hz, 2H), 2.78 (s, 3H).

The compounds in the following Table have been synthezised according to the same methods as previous Examples 1 to 9, as denoted in the column denoted as "Exp. nr". The compounds denoted with the asterisk have been exemplified in the Examples.

**Table 1: Compounds prepared according to the Examples.**

| 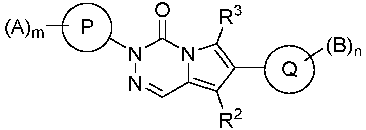 | | | | | |
|---|---|---|---|---|---|
| **Co.nr.** | **Exp nr.** | 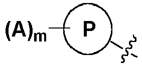 | **R³** | **R²** | 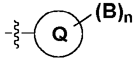 |
| **1-1** | **5** | 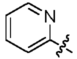 | Me | Me | 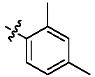 |
| **1-2** | **5** | 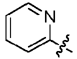 | Me | Me | 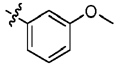 |
| **1-3** | **5*** | 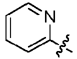 | Me | Me | 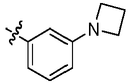 |
| **1-4** | **5** | 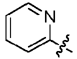 | Me | Me | 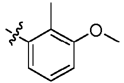 |
| **1-5** | **1** | 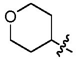 | Me | Me | 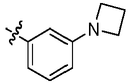 |
| **1-6** | **3** | 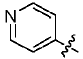 | Me | Me | 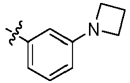 |
| **1-7** | **3** | 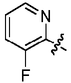 | Me | Me | 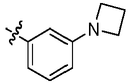 |
| **1-8** | **5** | 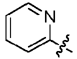 | Me | Me | 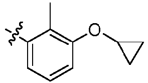 |
| **1-9** | **1** | 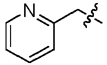 | Me | Me | 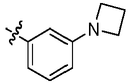 |
| **1-10** | **1*** | 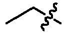 | Me | Me | 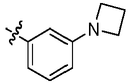 |
| **1-11** | **3** | 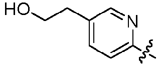 | Me | Me | 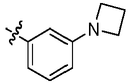 |
| **1-12** | **1** | 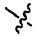 | Me | Me | 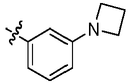 |
| **1-13** | **3** | 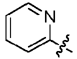 | Me | Me | 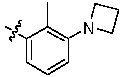 |
| **1-14** | **5** | 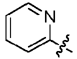 | Me | Me | 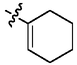 |
| **1-15** | **3** | 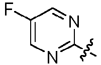 | Me | Me | 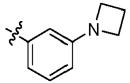 |
| **1-16** | **3** | 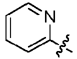 | Me | Me | 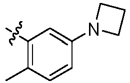 |
| **1-17** | **4** | 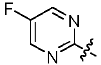 | Me | Me | 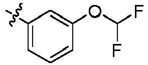 |
| **1-18** | **2*** | 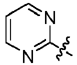 | H | H | 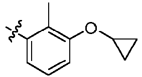 |
| **1-19** | **2** | 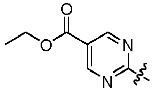 | Me | Me | 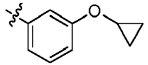 |
| **1-20** | **3** | 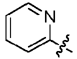 | Me | Me | 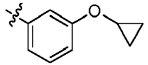 |
| **1-21** | **3** | 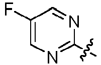 | Me | Me | 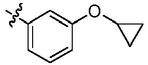 |
| **1-22** | **3** | 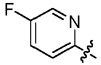 | Me | Me | 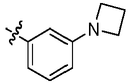 |
| **1-23** | **2** | 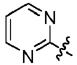 | Me | Me | 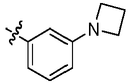 |
| **1-24** | **3*** | 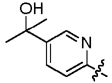 | Me | Me | 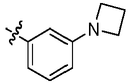 |
| **1-25** | **3** | 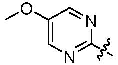 | Me | Me | 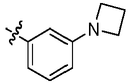 |
| **1-26** | **2** | 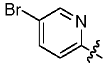 | Me | Me | 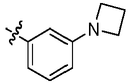 |
| **1-27** | **3** | 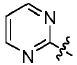 | Me | Me | 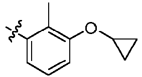 |
| **1-28** | **2** | 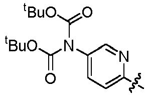 | Me | Me | 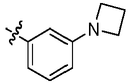 |
| **1-29** | **3** | 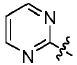 | Me | Me | 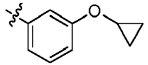 |
| **1-30** | **5** | 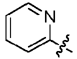 | Me | Me | 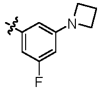 |
| **1-31** | **2** | 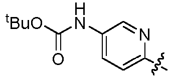 | Me | Me | 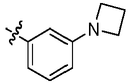 |
| **1-32** | **2** | 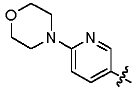 | Me | Me | 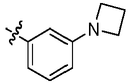 |
| **1-33** | **1** | 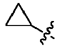 | Me | Me | 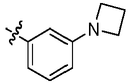 |
| **1-34** | **6*** | 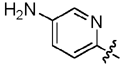 | Me | Me | 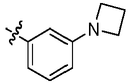 |
| **1-35** | **6** | 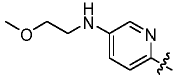 | Me | Me | 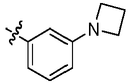 |
| **1-36** | **6** | 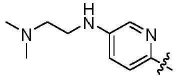 | Me | Me | 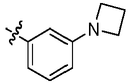 |
| **1-37** | **5** | 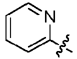 | Me | Me | 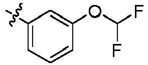 |
| **1-38** | **5** | 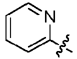 | Me | Me | 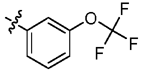 |
| **1-39** | **3** | 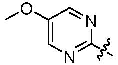 | Me | Me | 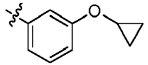 |
| **1-40** | **3** | 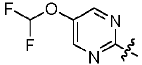 | Me | Me | 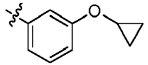 |
| **1-41** | **3** | 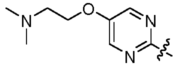 | Me | Me | 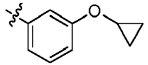 |
| **1-42** | **2** | 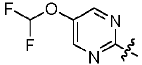 | Me | Me | 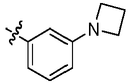 |
| **1-43** | **2** | 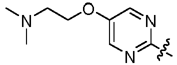 | Me | Me | 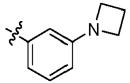 |
| **1-44** | **6** | 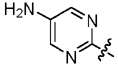 | Me | Me | 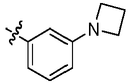 |
| **1-45** | **2** | 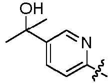 | Me | Me | 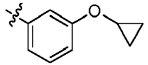 |
| **1-46** | **3** | 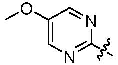 | Me | Me | 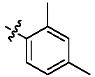 |
| **1-47** | **7*** | 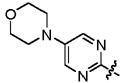 | Me | Me | 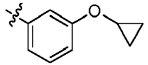 |
| **1-48** | **2** | 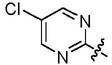 | Me | Me | 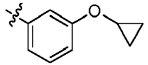 |
| **1-49** | **5** | 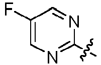 | Me | Me | 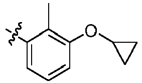 |
| **1-50** | **2** | 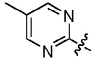 | Me | Me | 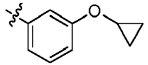 |
| **1-51** | **2** | 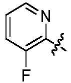 | Me | Me | 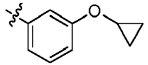 |
| **1-52** | **2** | 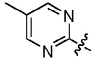 | Me | Me | 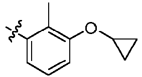 |
| **1-53** | **4*** | 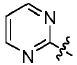 | Me | Me | 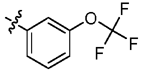 |
| **1-54** | **2** | 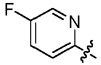 | Me | Me | 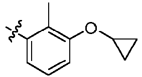 |
| **1-55** | **2** | 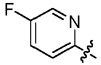 | Me | Me | 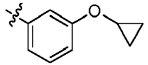 |
| **1-56** | **4** | 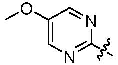 | Me | Me | 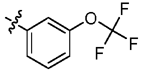 |
| **1-57** | **4** | 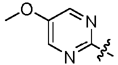 | Me | Me | 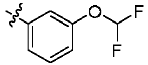 |
| **1-58** | **4** | 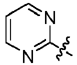 | Me | Me | 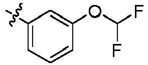 |
| **1-59** | **2** | 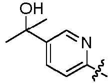 | Me | Me | 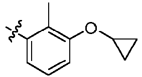 |
| **1-60** | **4** | 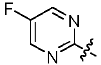 | Me | Me | 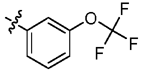 |
| **1-61** | **5** | 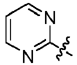 | Me | H | 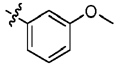 |
| **1-62** | **5** | 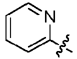 | Me | H | 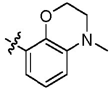 |
| **1-63** | **5** | 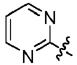 | Me | H | 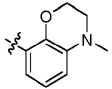 |
| **1-64** | **5** | 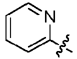 | Me | H | 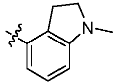 |
| **1-65** | **5** | 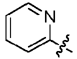 | Me | H | 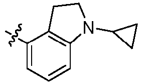 |
| **1-66** | **5** | 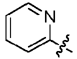 | Me | H | 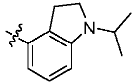 |
| **1-67** | **5** | 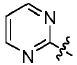 | Me | H | 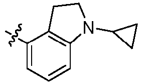 |
| **1-68** | **5** | 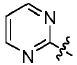 | Me | H | 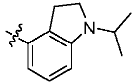 |
| **1-69** | **5** | 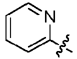 | Me | H | 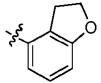 |
| **1-70** | **5** | 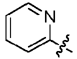 | Me | H | 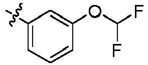 |
| **1-71** | **5** | 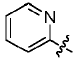 | Me | H | 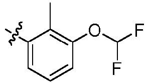 |
| **1-72** | **5** | 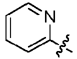 | Me | H | 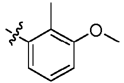 |
| **1-73** | **5** | 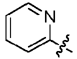 | Me | H | 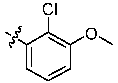 |
| **1-74** | **5** | 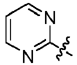 | Me | H | 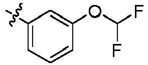 |
| **1-75** | **5** | 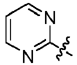 | Me | H | 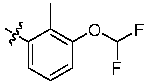 |
| **1-76** | **5** | 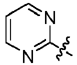 | Me | H | 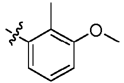 |
| **1-77** | **5** | 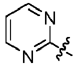 | Me | H | 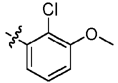 |
| **1-78** | **5** | 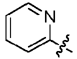 | Me | H | 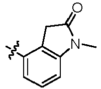 |
| **1-79** | **5** | 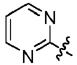 | Me | Me | 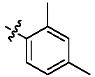 |
| **1-80** | **7** | 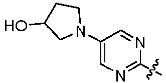 | Me | Me | 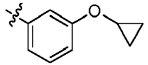 |
| **1-81** | **6** | 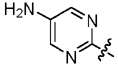 | Me | Me | 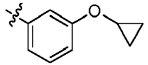 |
| **1-82** | **5** | 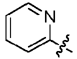 | H | H | 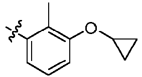 |
| **1-83** | **5** | 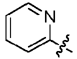 | Me | H | 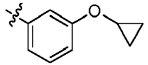 |
| **1-84** | **5** | 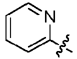 | Me | H | 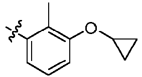 |
| **1-85** | **5** | 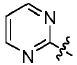 | Me | H | 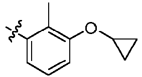 |
| **1-86** | **5** | 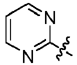 | Me | Me | 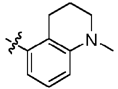 |
| **1-87** | **5** | 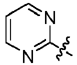 | Me | Me | 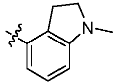 |
| **1-88** | **5** | 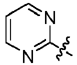 | Me | H | 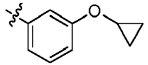 |
| **1-89** | **5** | 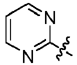 | Me | H | 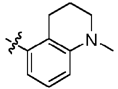 |
| **1-90** | **5** | 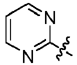 | Me | H | 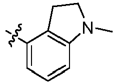 |
| **1-91** | **5** | 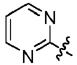 | Me | Me | 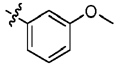 |
| **1-92** | **5** | 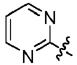 | Me | H | 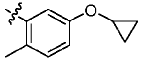 |
| **1-93** | **5** | 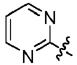 | Me | Me | 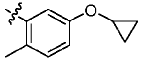 |
| **1-94** | **5** | 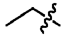 | Me | Me | 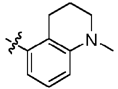 |
| **1-95** | **5** | 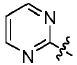 | Me | Me | 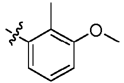 |
| **1-96** | **5** | 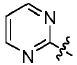 | Me | Me | 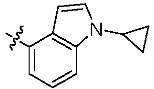 |
| **1-97** | **5** | 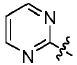 | Me | Me | 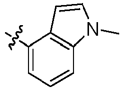 |
| **1-98** | **5** | 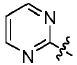 | Me | Me | 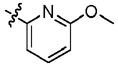 |
| **1-99** | **5** | 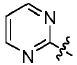 | Me | Me | 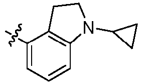 |
| **1-100** | **5** | 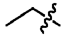 | Me | Me | 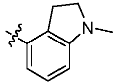 |
| **1-101** | **5** | 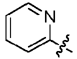 | Me | H | 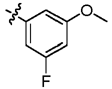 |
| **1-102** | **5** | 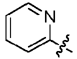 | Me | H | 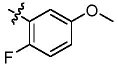 |
| **1-103** | **5** | 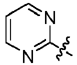 | Me | H | 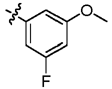 |
| **1-104** | **5** | 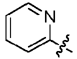 | Me | H | 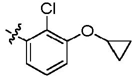 |
| **1-105** | **5** | 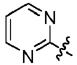 | Me | H | 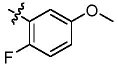 |
| **1-106** | **5** | 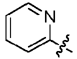 | Me | H | 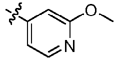 |
| **1-107** | **5** | 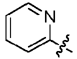 | Me | H | 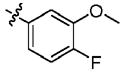 |
| **1-108** | **5** | 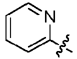 | Me | H | 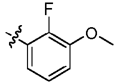 |
| **1-109** | **5** | 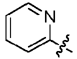 | Me | H | 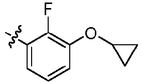 |
| **1-110** | **5** | 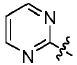 | Me | H | 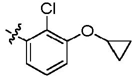 |
| **1-111** | **5** | 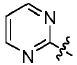 | Me | H | 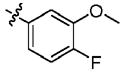 |
| **1-112** | **5** | 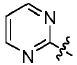 | Me | H | 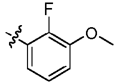 |
| **1-113** | **5** | 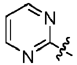 | Me | H | 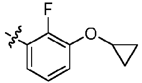 |
| **1-114** | **5** | 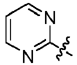 | Me | H | 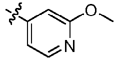 |
| **1-115** | **8*** | 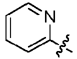 | Me | Br | 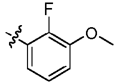 |
| **1-116** | **5** | 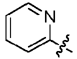 | Me | H | 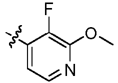 |
| **1-117** | **5** | 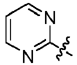 | Me | H | 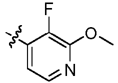 |
| **1-118** | **9*** | 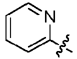 | Me | H | 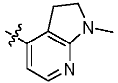 |
| **1-119** | **9** | 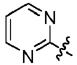 | Me | H | 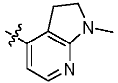 |
| **1-120** | **5** | 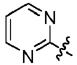 | Me | Me | 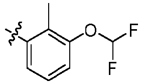 |
| **1-121** | **2** | 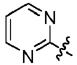 | Me | Me | 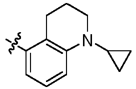 |
| **1-122** | **2** | 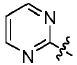 | Me | Me | 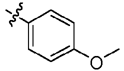 |
| **1-123** | **2** | 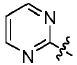 | Me | Me | 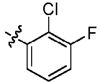 |
| **1-124** | **2** | 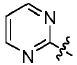 | Me | Me | 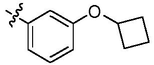 |
| **1-125** | **2** | 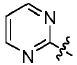 | Me | Me | 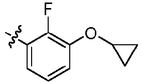 |
| **1-126** | **2** | 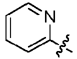 | Me | Me | 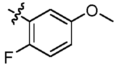 |
| **1-127** | **2** | 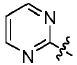 | Me | Me | 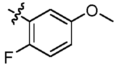 |
| **1-128** | **2** | 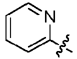 | Me | Me | 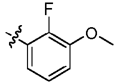 |
| **1-129** | **2** | 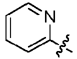 | Me | Me | 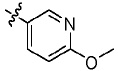 |
| **1-130** | **2** | 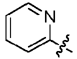 | Me | Me | 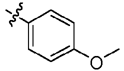 |
| **1-131** | **5** | 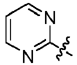 | Me | Me | 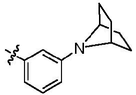 |
| **1-132** | **5** | 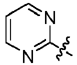 | Me | Me | 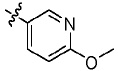 |
| **1-133** | **5** | 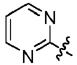 | Me | Me | 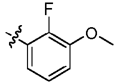 |
| **1-134** | **5** | 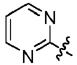 | Me | Me | 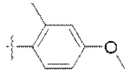 |
| **1-135** | **5** | 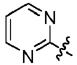 | Me | Me | 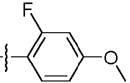 |
| **1-136** | **5** | 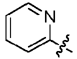 | Me | Me | 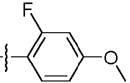 |
| **1-137** | **5** | 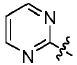 | Me | Me | 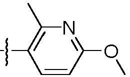 |
| **1-138** | **5** | 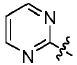 | Me | Me | 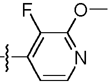 |
| **1-139** | **5** | 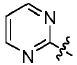 | Me | Me | 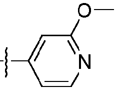 |
| **1-140** | **5** | 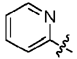 | Me | Me | 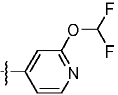 |
| **1-141** | **5** | 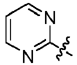 | Me | Me | 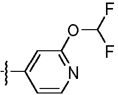 |
| **1-142** | **5** | 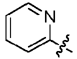 | Me | H | 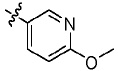 |
| **1-143** | **5** | 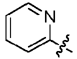 | Me | H | 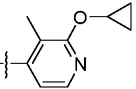 |
| **1-144** | **5** | 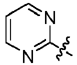 | Me | H | 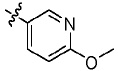 |
| **1-145** | **5** | 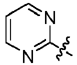 | Me | H | 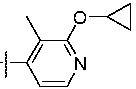 |
| **1-146** | **5** | 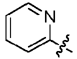 | Me | H | 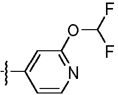 |
| **1-147** | **5** | 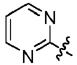 | Me | H | 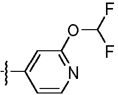 |

### Physico-Chemical Data

### LC-MS method:

Liquid chromatography-mass spectrometry (LC-MS) was performed on a LC-MS system, consisting of a Dionex UltiMate 3000 pump, autosampler, column compartment, and detector (Thermo Fisher Scientific, Dreieich, Germany) and ESI quadrupole MS (MSQ Plus or ISQ EC, Thermo Fisher Scientific, Dreieich, Germany).

### Method 1:

Reversed phase (C₁₈), full scan (positive and negative) 100 - 1000 m/z; eluents: H₂O + 0.1 Formic Acid (A) and MeCN + 0.1 Formic Acid (B): 0 min 5% B → 1 min 5% B → 6.8 min 100% B (linear gradient from 5-100% B within 5.8 min) → 8 min 100% B (1.2 min 100% B). Purity of the final compounds was determined by LS-MS using the area percentage method on the UV trace recorded at a wavelength of 254 nm.

### Method 2:

Reverse phase (Cis) was carried out on Accucore^{™} C₁₈ cartridge (100 x 3 mm); eluents: H₂O + 0.1% Formic acid (A) and MeCN + 0.1% Formic acid (B). Gradient conditions used: Linear gradient from 0-95% B within 10 min. Purity was determined by the area percentage method of the obtained PDA spectra.

Liquid chromatography-mass spectrometry (LC-MS) was also performed on a LC-MS system consisting of a Thermo Finnigan LCQ Fleet coupled to a Shimadzu Preparative HPLC system (DGU-20AR_{3R} degasser, two LC-20AD pumps, SPD-20A PDA detector and a CTO-20A column oven) equipped with a Accucore^{™} C₁₈ cartridge (100 x 3 mm).

### Method 3:

Reverse phase (C₁₈); eluents: H₂O + 0.1% Formic acid (A) and MeCN + 0.1% Formic acid (B). Gradient conditions used: Linear gradient from 5-100% B within 20 min. Purity was determined by the area percentage method of the obtained PDA spectra.

### SFC-MS method:

Supercritical fluid chromatography-mass spectrometry (SFC-MS) was performed on a SFC-MS system consisting of a Waters Acquity UPC2 SFC System (convergence manager, sample manager, binary solvent manager, column manager, PDA detector, isocratic solvent manager and QDa detector) with Viridis HSS C₁₈ SB column or a Viridis BEH column.

### Method 4:

Reverse phase (SB-C₁₈ column); Supercritical CO₂; Gradient conditions used: Linear gradient from 10-30% (90/9/1 MeOH/H₂O/Formic acid) in CO₂ within 6 min. Purity was determined by the area percentage method of the obtained PDA spectra.

### Method 5:

Reverse phase (SB-C₁₈ column); Supercritical CO₂; Gradient conditions used: Linear gradient from 2-30% (90/9/1 MeOH/H₂O/Formic acid) in CO₂ within 7 min. Purity was determined by the area percentage method of the obtained PDA spectra.

### Method 6:

Reverse phase (BEH-C₁₈ column); Supercritical CO₂; Gradient conditions used: Linear gradient from 2-30% (90/9/1 MeOH/H₂O/Formic acid) in CO₂ within 12 min. Purity was determined by the area percentage method of the obtained PDA spectra.

### Method 7:

Reverse phase (SB-C₁₈ column); Supercritical CO₂; Gradient conditions used: Linear gradient from 2-30% (90/9/1 MeOH/H₂O/Formic acid) in CO₂ within 12 min. Purity was determined by the area percentage method of the obtained PDA spectra.

### NMR:

¹H-NMR spectra were recorded on a Bruker Avance III 400 MHz, a Bruker Avance III 500 MHz or a Bruker Avance I 500 (500 MHz) spectrometer. Chemical shifts are expressed in parts per million (ppm, δ units). Splitting patterns describe apparent multiplicities and are designated as s (singlet), d (doublet), t (triplet), q (quadruplet), m (multiplet), br (broad). Coupling constants (*J*) are given in Hertz (Hz).

**Table 2: Physico-chemical data. (RT means retention time in minutes; [MH]⁺ means the protonated mass of the compound (free base); nd = not determined).**

| **Co.Nr.** | **RT (min)** | **[MH]⁺** | **MS method** | **¹H-NMR** |
|---|---|---|---|---|
| **1-1** | 5.09 | 345.1 | Method 5 | (500 MHz, CDCl₃) δ: 8.65 (dd, *J =* 5.0, 1.9 Hz, 1H), 8.03 (s, 1H), 7.85 (td, *J =* 7.8, 2.0 Hz, 1H), 7.67 (dd, *J =* 8.1*,* 1.1 Hz, 1H), 7.32 (ddd, *J =* 7.4, 4.9, 1.0 Hz, 1H), 7.13 (d, *J =* 1.7 Hz, 1H), 7.06 (dd, *J =* 7.6, 1.7 Hz, 1H), 6.98 (d, *J =* 7.6 Hz, 1H), 2.56 (s, 3H), 2.38 (s, 3H), 2.07 (s, 3H), 2.06 (s, 3H). |
| **1-2** | 13.30 | 347.3 | Method 3 | (500 MHz, CDCl₃) δ: 8.65 (ddd, *J =* 4.8*,* 2.0, 0.8 Hz, 1H), 8.04 (s, 1H), 7.85 (td, *J =* 7.8, 2.0 Hz, 1H), 7.66 (dt, *J =* 8.0, 1.0 Hz, 1H), 7.37 (t, *J =* 7.9 Hz, 1H), 7.31 (ddd, *J =* 7.4, 4.8, 1.0 Hz, 1H), 6.92 (ddd, *J*= 8.3, 2.6, 1.0 Hz, 1H), 6.85 (dt, *J =* 7.5, 1.3 Hz, 1H), 6.80 (dd, *J = 2.7,* 1.5 Hz, 1H), 3.85 (s, 3H), 2.75 (s, 3H), 2.23 (s, 3H). |
| **1-3** | 3.94 | 372.1 | Method 6 | (500 MHz, CDCl₃) δ: 8.65 (ddd, *J =* 4.9*,* 2.0, 0.9 Hz, 1H), 8.04 (s, 1H), 7.87 - 7.81 (m, 1H), 7.66 (dt, *J =* 8.2, 1.0 Hz, 1H), 7.35 - 7.25 (m, 2H), 6.62 (dt, *J =* 7.5, 1.3 Hz, 1H), 6.46 (ddd, *J* = 8.1, 2.4, 1.0 Hz, 1H), 6.31 (t, *J =* 1.9 Hz, 1H), 3.91 (t, *J =* 7.2 Hz, 4H), 2.75 (s, 3H), 2.45 - 2.34 (m, 2H), 2.23 (s, 3H). |
| **1-4** | 2.99 | 361.1 | Method 6 | (400 MHz, CDCl₃) δ: 8.69 - 8.59 (m, 1H), 8.04 (s, 1H), 7.89 - 7.81 (m, 1H), 7.67 (dt, *J =* 8.1, 0.9 Hz, 1H), 7.36 - 7.28 (m, 1H), 7.27 - 7.19 (m, 1H), 6.89 (dd, *J =* 8.3*,* 1.1 Hz, 1H), 6.72 (dd, *J =* 7.7*,* 1.1 Hz, 1H), 3.89 (s, 3H), 2.57 (s, 3H), 2.08 (s, 3H), 1.97 (s, 3H). |
| **1-5** | 1.76 | 379.2 | Method 6 | (400 MHz, CDCl₃) δ: 7.89 (s, 1H), 7.31 - 7.20 (m, 1H), 6.59 (dt, *J =* 7.4*,* 1.3 Hz, 1H), 6.45 (ddd, *J =* 8.1, 2.4, 1.0 Hz, 1H), 6.33 - 6.24 (m, 1H), 4.91 (tt, *J =* 11.7, 4.1 Hz, 1H), 4.10 (dd, *J =* 11.5, 4.6 Hz, 2H), 3.90 (t, *J =* 7.2 Hz, 4H), 3.56 (td, *J =* 12.0, 1.9 Hz, 2H), 2.74 (s, 3H), 2.39 (q, *J =* 7.2 Hz, 2H), 2.29 - 2.09 (m, 2H), 2.18 (s, 3H), 1.76 (ddd, *J =* 12.3, 4.2, 1.9 Hz, 2H). |
| **1-6** | 6.97 | 372.2 | Method 7 | (400 MHz, CDCl₃) δ: 8.68 (s, 2H), 8.03 (s, 1H), 7.82 - 7.76 (m, 2H), 7.28 (t, *J =* 7.8 Hz, 1H), 6.60 (dt, *J =* 7.5, 1.3 Hz, 1H), 6.47 (ddd, *J =* 8.1, 2.4, 1.0 Hz, 1H), 6.29 (dd, *J =* 2.4*,* 1.5 Hz, 1H), 3.91 (t, *J =* 7.2 Hz, 4H), 2.75 (s, 3H), 2.45 - 2.31 (m, 2H), 2.22 (s, 3H), 2.21 (s, 3H). |
| **1-7** | 4.83 | 390.2 | Method 7 | (400 MHz, CDCl₃) δ: 8.46 (ddd, *J =* 4.7, 1.6, 0.8 Hz, 1H), 8.03 (s, 1H), 7.62 (ddd, *J =* 9.0, 8.3, 1.5 Hz, 1H), 7.43 (ddd, *J =* 8.3, 4.7, 3.7 Hz, 1H), 7.28 (t, *J =* 7.8 Hz, 1H), 6.62 (ddd, *J =* 7.5, 1.6, 1.0 Hz, 1H), 6.47 (ddd, *J =* 8.1, 2.4, 1.0 Hz, 1H), 6.31 (dd, *J =* 2.4*,* 1.5 Hz, 1H), 3.91 (t, *J =* 7.2 Hz, 4H), 2.74 (s, 3H), 2.47 - 2.34 (m, 2H), 2.24 (s, 3H). |
| **1-8** | 13.54 | 387.4 | Method 3 | (500 MHz, CDCl₃) δ: 8.77 - 8.57 (m, 1H), 8.03 (s, 1H), 7.88 (td, *J =* 7.8, 1.9 Hz, 1H), 7.70 (d, *J =* 8.1 Hz, 1H), 7.34 (ddd, *J =* 7.5, 4.9, 1.1 Hz, 1H), 7.31 - 7.26 (m, 1H), 7.24 (t, *J* = 7.8 Hz, 1H), 6.76 (dd, *J=* 7.3, 1.4 Hz, 1H), 3.80 (dq, *J =* 6.9, 4.5 Hz, 1H), 2.59 (s, 3H), 2.10 (s, 3H), 1.94 (s, 3H), 0.87 - 0.74 (m, 4H). |
| **1-9** | 5.86 | 386.2 | Method 7 | (400 MHz, CDCl₃) δ: 8.59 (ddd, *J =* 4.9*,* 1.8, 1.0 Hz, 1H), 7.90 (s, 1H), 7.65 (td, *J =* 7.7, 1.8 Hz, 1H), 7.32 - 7.22 (m, 2H), 7.18 (ddd, *J =* 7.6, 4.9, 1.2 Hz, 1H), 6.60 (ddd, *J =* 7.6, 1.6, 1.0 Hz, 1H), 6.52 - 6.41 (m, 1H), 6.29 (dd, *J =* 2.4, 1.5 Hz, 1H), 5.37 (s, 2H), 3.94 - 3.85 (m, 4H), 2.73 (s, 3H), 2.44 - 2.32 (m, 2H), 2.19 (s, 3H). |
| **1-10** | 13.33 | 323.1 | Method 3 | (400 MHz, CDCl₃) δ: 7.85 (s, 1H), 7.26 (t, *J =* 7.8 Hz, 1H), 6.60 (ddd, *J =* 7.5, 1.6, 1.0 Hz, 1H), 6.45 (ddd, *J =* 8.1, 2.4, 1.0 Hz, 1H), 6.32 - 6.26 (m, 1H), 4.10 (q, *J =* 7.2 Hz, 2H), 3.90 (t, *J =* 7.2 Hz, 4H), 2.74 (s, 3H), 2.44 - 2.33 (m, 2H), 2.18 (s, 3H), 1.37 (t, *J =* 7.1 Hz, 3H). |
| **1-11** | 11.40 | 373.1 [M+H-CH₂C H₂OH] | Method 3 | (400 MHz, CDCl₃) δ: 8.50 (d, *J =* 2.4 Hz, 1H), 7.80 (dd, *J =* 8.1, 2.4 Hz, 1H), 7.39 (d, *J =* 8.2 Hz, 1H), 7.31 - 7.21 (m, 2H), 6.67 - 6.60 (m, 1H), 6.46 - 6.39 (m, 1H), 6.36 - 6.31 (m, 1H), 3.95 (d, *J =* 6.5 Hz, 2H), 3.90 (t, *J =* 7.2 Hz, 4H), 2.96 (t, *J =* 6.4 Hz, 2H), 2.38 (m, *J =* 7.1 Hz, 2H), 2.24 (s, 6H). One proton not observed |
| **1-12** | 12.39 | 309.0 | Method 3 | (400 MHz, CDCl₃) δ: 7.82 (s, 1H), 7.26 (t, *J =* 7.8 Hz, 1H), 6.59 (ddd, *J =* 7.5, 1.5, 1.0 Hz, 1H), 6.45 (ddd, *J =* 8.2, 2.4, 1.0 Hz, 1H), 6.32 - 6.26 (m, 1H), 3.90 (t, *J =* 7.2 Hz, 4H), 3.68 (s, 3H), 2.74 (s, 3H), 2.38 (dq, *J =* 7.7, 7.1 Hz, 2H), 2.18 (s, 3H). |
| **1-13** | 10.17 | 386.1 | Method 3 | (400 MHz, CDCl₃) δ: 8.65 (ddd, *J =* 4.9*,* 2.0, 0.9 Hz, 1H), 8.03 (s, 1H), 7.85 (td, *J =* 7.7, 1.9 Hz, 1H), 7.67 (dt, *J* = 8.1*,* 1.0 Hz, 1H), 7.32 (ddd, *J =* 7.4, 4.9, 1.1 Hz, 1H), 7.16 (t, *J =* 7.8 Hz, 1H), 6.60 (ddd, *J =* 17.9, 7.8, 1.2 Hz, 2H), 3.95 (dq, *J =* 11.5, 6.9 Hz, 4H), 2.58 (s, 3H), 2.30 (m, *J =* 7.2 Hz, 2H), 2.06 (d, *J =* 14.4 Hz, 3H), 1.93 (s, 3H). |
| **1-14** | 13.61 | 321.3 | Method 3 | (400 MHz, CDCl₃) δ: 8.63 (ddd, J = 4.9, 1.9, 0.8 Hz, 1H), 7.97 (s, 1H), 7.83 (d, J = 0.7 Hz, 1H), 7.64 (dt, J = 8.1, 1.0 Hz, 1H), 7.36 - 7.26 (m, 1H), 5.60 (tt, J = 3.8, 1.8 Hz, 1H), 2.70 (s, 3H), 2.20 (s, 5H), 2.11 (dd, J = 5.2, 3.1 Hz, 2H), 1.81 - 1.67 (m, 4H). |
| **1-15** | 11.73 | 391.2 | Method 3 | (500 MHz, CDCl₃) δ: 8.75 (s, 2H), 8.02 (s, 1H), 7.28 (t, *J =* 7.8 Hz, 2H), 6.61 (dt, *J* = 7.6*,* 1.2 Hz, 1H), 6.49 - 6.42 (m, 1H), 6.32 - 6.27 (m, 1H), 3.91 (t, *J =* 7.2 Hz, 4H), 2.75 (s, 3H), 2.39 (m, *J =* 7.2 Hz, 2H), 2.23 (s, 3H). |
| **1-16** | 10.86 | 386.1 | Method 3 | (500 MHz, CDCl₃) δ: 8.68 - 8.63 (m, 1H), 8.03 (s, 1H), 7.90 - 7.82 (m, 1H), 7.67 (d, *J =* 8.1 Hz, 1H), 7.35 - 7.28 (m, 1H), 7.14 (d, *J =* 8.3 Hz, 1H), 6.44 (dd, *J =* 8.2, 2.6 Hz, 1H), 6.20 (d, *J =* 2.6 Hz, 1H), 3.86 (t, *J =* 7.2 Hz, 4H), 2.58 (s, 3H), 2.36 (m, *J =* 7.2 Hz, 2H), 2.09 (s, 3H), 1.97 (s, 3H). |
| **1-17** | 12.07 | 402.1 | Method 3 | (400 MHz, CDCl₃) δ: 8.76 (s, 2H), 8.04 (s, 1H), 7.46 (t, *J =* 7.9 Hz, 1H), 7.14 (dddd, *J =* 8.9, 7.6, 2.0, 1.0 Hz, 2H), 7.05 (t, *J =* 2.2 Hz, 1H), 6.66 (d, *J =* 73.7 Hz, 1H), 2.75 (s, 3H), 2.23 (s, 3H). |
| **1-18** | 11.62 | 360.3 | Method 3 | (400 MHz, CDCl₃) δ: 8.96 (d, *J =* 4.8 Hz, 2H), 8.22 (d, *J =* 0.7 Hz, 1H), 7.92 (dd, *J =* 1.5, 0.7 Hz, 1H), 7.42 (t, *J =* 4.8 Hz, 1H), 7.27 (d, *J =* 2.1 Hz, 1H), 7.24 (t*, J =* 7.9 Hz, 1H), 7.03 (dd, *J =* 7.0, 1.9 Hz, 1H), 6.89 (d, *J =* 1.5 Hz, 1H), 3.84 - 3.75 (m, 1H), 2.25 (s, 3H), 0.83 (m, 4H). |
| **1-19** | 13.40 | 446.3 | Method 3 | (400 MHz, CDCl₃) δ: 9.40 (s, 2H), 8.07 (s, 1H), 7.41 - 7.34 (m, 1H), 7.08 (ddd, *J =* 8.4, 2.5, 1.0 Hz, 1H), 6.94 (dd, *J =* 2.5*,* 1.5 Hz, 1H), 6.87 (dt, *J =* 7.7*,* 1.2 Hz, 1H), 4.48 (q, *J =* 7.1 Hz, 2H), 3.77 (m, *J* = 4.8 Hz, 1H), 2.77 (s, 3H), 2.25 (s, 3H), 1.45 (t, *J =* 7.1 Hz, 3H), 0.81 (d, *J =* 3.5 Hz, 4H). |
| **1-20** | 12.85 | 373.3 | Method 3 | (500 MHz, CDCl₃) δ: 8.65 (dd, *J =* 4.8, 1.9 Hz, 1H), 8.05 (s, 1H), 7.85 (td, *J* = 7.7, 1.9 Hz, 1H), 7.66 (d, *J =* 8.1 Hz, 1H), 7.45 - 7.29 (m, 2H), 7.08 (dd, *J =* 8.2, 2.5 Hz, 1H), 6.95 (t, *J =* 2.0 Hz, 1H), 6.88 (dt, *J =* 7.5, 1.2 Hz, 1H), 3.77 (tt, *J =* 6.2, 3.7 Hz, 1H), 2.77 (s, 3H), 2.24 (s, 3H), 0.83 - 0.76 (m, 4H). |
| **1-21** | 12.84 | 392.2 | Method 3 | (500 MHz, CDCl₃) δ: 8.75 (s, 2H), 8.03 (s, 1H), 7.37 (dd, *J =* 8.3, 7.6 Hz, 1H), 7.08 (ddd, *J =* 8.2, 2.5, 1.0 Hz, 1H), 6.94 (dd, *J =* 2.6, 1.5 Hz, 1H), 6.87 (dt, *J =* 7.6, 1.3 Hz, 1H), 3.80 - 3.72 (m, 1H), 2.76 (s, 3H), 2.25 (s, 3H), 0.83 - 0.74 (m, 4H). |
| **1-22** | 12.51 | 390.1 | Method 3 | (500 MHz, CDCl₃) δ: 8.48 (d, *J =* 3.0 Hz, 1H), 8.02 (s, 1H), 7.66 (dd, *J =* 8.8, 4.0 Hz, 1H), 7.55 (ddd, *J =* 8.8, 7.4, 3.0 Hz, 1H), 7.28 (t, *J =* 7.8 Hz, 1H), 6.61 (dt, *J* = 7.5, 1.2 Hz, 1H), 6.46 (ddd, *J* = 8.1, 2.4, 1.0 Hz, 1H), 6.30 (t, *J =* 2.0 Hz, 1H), 3.91 (t, *J =* 7.2 Hz, 4H), 2.74 (s, 3H), 2.39 (m, *J =* 7.2 Hz, 2H), 2.23 (s, 3H). |
| **1-23** | 11.23 | 373.1 | Method 3 | (500 MHz, CDCl₃) δ: 8.91 (d, *J =* 4.9 Hz, 2H), 8.03 (s, 1H), 7.35 (t, *J =* 4.8 Hz, 1H), 7.28 (t, *J =* 7.8 Hz, 1H), 6.62 (ddd, *J =* 7.5, 1.6, 1.1 Hz, 1H), 6.46 (ddd, *J =* 8.1, 2.3, 0.9 Hz, 1H), 6.31 (dd, *J =* 2.4*,* 1.5 Hz, 1H), 3.91 (t, *J =* 7.2 Hz, 4H), 2.76 (s, 3H), 2.43 - 2.35 (m, 2H), 2.23 (s, 3H). |
| **1-24** | 11.47 | 430.4 | Method 3 | (400 MHz, CDCl₃) δ: 8.73 (d, *J =* 2.5 Hz, 1H), 8.02 (s, 1H), 7.97 (dd, *J =* 8.4, 2.6 Hz, 1H), 7.60 (d, *J =* 8.4 Hz, 1H), 7.29 (d, *J =* 7.8 Hz, 2H), 6.62 (dt, *J= 7.5,* 1.3 Hz, 1H), 6.50 - 6.43 (m, 1H), 6.31 (t, *J =* 2.0 Hz, 1H), 3.91 (t, *J =* 7.2 Hz, 4H), 2.78 (s, 1H), 2.75 (s, 3H), 2.39 (m, *J =* 7.2 Hz, 2H), 2.23 (s, 3H), 1.64 (s, 6H). |
| **1-25** | 11.61 | 403.1 | Method 3 | (400 MHz, CDCl₃) δ: 8.53 (s, 2H), 8.00 (s, 1H), 7.29 (d, *J =* 7.8 Hz, 1H), 6.62 (dt, *J =* 7.6, 1.2 Hz, 1H), 6.46 (ddd, *J =* 8.1, 2.4, 1.0 Hz, 1H), 6.31 (t, *J =* 1.9 Hz, 1H), 3.99 (s, 3H), 3.91 (t, *J =* 7.2 Hz, 4H), 2.75 (s, 3H), 2.45 - 2.33 (m, 2H), 2.23 (s, 3H). |
| **1-26** | 13.65 | 450.1 452.1 | Method 3 | (500 MHz, CDCl₃) δ: 8.70 - 8.66 (m, 1H), 8.03 (s, 1H), 7.94 (dd, *J =* 8.6, 2.5 Hz, 1H), 7.60 (dd, *J =* 8.5, 0.7 Hz, 1H), 7.28 (t, *J =* 7.8 Hz, 1H), 6.61 (dt, *J =* 7.5, 1.2 Hz, 1H), 6.46 (ddd, *J =* 8.1*,* 2.4, 1.0 Hz, 1H), 6.30 (dd, *J =* 2.3*,* 1.5 Hz, 1H), 3.91 (t, *J =* 7.2 Hz, 4H), 2.74 (s, 3H), 2.44 - 2.33 (m, 2H), 2.22 (s, 3H). |
| **1-27** | 12.76 | 388.4 | Method 3 | 500 MHz, CDCl₃) δ: 8.92 (d, *J =* 4.9 Hz, 2H), 8.04 (s, 1H), 7.36 (t, *J =* 4.8 Hz, 1H), 7.29 - 7.24 (m, 1H), 7.22 (t, *J =* 7.8 Hz, 1H), 6.74 (dd, *J =* 7.4, 1.4 Hz, 1H), 3.83 - 3.71 (m, 1H), 2.58 (s, 3H), 2.08 (s, 3H), 1.91 (s, 3H), 0.85 - 0.74 (m, 4H). |
| **1-28** | 15.10 | 587.2 | Method 3 | (400 MHz, CDCl₃) δ: 8.43 (dd, *J =* 2.6, 0.7 Hz, 1H), 8.05 (s, 1H), 7.74 (dd, *J =* 8.6, 0.7 Hz, 1H), 7.62 (dd, *J =* 8.6, 2.7 Hz, 1H), 7.29 (d, *J =* 7.8 Hz, 1H), 6.61 (dt, *J =* 7.7*,* 1.1 Hz, 1H), 6.47 (ddd, *J =* 8.2, 2.4, 1.0 Hz, 1H), 6.31 (t, *J =* 1.9 Hz, 1H), 3.91 (t, *J =* 7.2 Hz, 4H), 2.76 (s, 3H), 2.39 (m, *J =* 7.2 Hz, 2H), 2.23 (s, 3H), 1.44 (s, 18H). |
| **1-29** | 12.05 | 374.3 | Method 3 | (400 MHz, CDCl₃) δ: 8.91 (d, *J =* 4.9 Hz, 2H), 8.05 (s, 1H), 7.40 - 7.32 (m, 2H), 7.07 (ddd, *J =* 8.3, 2.6, 1.0 Hz, 1H), 6.96 (dd, *J =* 2.6, 1.5 Hz, 1H), 6.91 - 6.84 (m, 1H), 3.83 - 3.69 (m, 1H), 2.78 (s, 3H), 2.25 (s, 3H), 0.82 - 0.73 (m, 4H). |
| **1-30** | 13.73 | 390.4 | Method 3 | (400 MHz, CDCl₃) δ: 8.65 (ddd, *J =* 4.9*,* 2.0, 0.8 Hz, 1H), 8.03 (s, 1H), 7.85 (ddd, *J =* 8.2, 7.5, 1.9 Hz, 1H), 7.65 (dt, *J* = 8.1*,* 1.0 Hz, 1H), 7.32 (ddd, *J =* 7.4, 4.9, 1.0 Hz, 1H), 6.30 (ddd, *J =* 9.4*,* 2.3, 1.3 Hz, 1H), 6.13 (dt*, J =* 10.9, 2.2 Hz, 1H), 6.04 (dd, *J* = 2.2, 1.3 Hz, 1H), 3.91 (t, *J =* 7.3 Hz, 4H), 2.75 (s, 3H), 2.46 - 2.34 (m, 2H), 2.22 (s, 3H). |
| **1-31** | 13.54 | 487.0 | Method 3 | (500 MHz, CDCl₃) δ: 8.47 (s, 1H), 8.18 (s, 1H), 8.09 (s, 1H), 7.67 (s, 1H), 7.29 (t, *J =* 7.8 Hz, 1H), 7.20 (s, 1H), 6.61 (d, *J =* 7.3 Hz, 1H), 6.48 (dd, *J =* 8.0, 2.4 Hz, 1H), 6.31 (s, 1H), 3.90 (t, *J =* 7.2 Hz, 4H), 2.72 (s, 2H), 2.38 (m, *J =* 7.2 Hz, 2H), 2.22 (s, 2H), 1.48 (s, 9H). |
| **1-32** | 12.86 | 457.4 | Method 3 | (500 MHz, CDCl₃) δ: 8.41 (d, *J =* 2.7 Hz, 1H), 7.97 (s, 1H), 7.72 (dd, *J =* 9.0*,* 2.7 Hz, 1H), 7.28 (d, *J =* 7.8 Hz, 1H), 6.69 (d, *J =* 9.0 Hz, 1H), 6.61 (dt, *J =* 7.5*,* 1.2 Hz, 1H), 6.46 (ddd, *J =* 8.1, 2.4, 1.0 Hz, 1H), 6.30 (t, *J =* 2.0 Hz, 1H), 3.95 - 3.80 (m, 8H), 3.55 (dd, *J*= 5.7, 4.0 Hz, 4H), 2.74 (s, 3H), 2.39 (m, *J =* 7.2 Hz, 2H), 2.22 (s, 3H). |
| **1-33** | 14.28 | 335.0 | Method 3 | (400 MHz, CDCl₃) δ: 7.80 (s, 1H), 7.29 - 7.24 (m, 1H), 6.59 (ddd, *J =* 7.5, 1.6, 1.0 Hz, 1H), 6.45 (ddd, *J =* 8.1, 2.4, 1.0 Hz, 1H), 6.30 - 6.26 (m, 1H), 3.90 (t, *J =* 7.2 Hz, 4H), 3.62 - 3.53 (m, 1H), 2.75 (s, 3H), 2.44 - 2.32 (m, 2H), 2.17 (s, 3H), 1.10 - 0.96 (m, 4H). |
| **1-34** | 12.59 | 388.2 | Method 3 | (500 MHz, CDCl₃) δ: 8.31 (s, 2H), 7.98 (s, 1H), 7.27 (s, 1H), 6.64 - 6.59 (m, 1H), 6.46 (ddd, *J =* 8.1, 2.4, 0.9 Hz, 1H), 6.31 (t, *J =* 2.0 Hz, 1H), 3.95 - 3.85 (m, 4H), 2.74 (s, 3H), 2.37 (dt, *J =* 15.3, 7.6 Hz, 2H), 2.22 (s, 3H). |
| **1-35** | 12.87 | 445.4 | Method 3 | (500 MHz, CDCl₃) δ: 8.01 (d, *J =* 3.0 Hz, 1H), 7.98 (s, 1H), 7.37 (d, *J =* 8.6 Hz, 1H), 7.27 (t, *J =* 7.8 Hz, 1H), 7.03 (dd, *J =* 8.6, 3.0 Hz, 1H), 6.61 (dt, *J =* 7.6, 1.3 Hz, 1H), 6.45 (ddd, *J =* 8.1*,* 2.4, 1.0 Hz, 1H), 6.33 - 6.29 (m, 1H), 4.27 (s, 1H), 3.90 (t, *J =* 7.2 Hz, 4H), 3.62 (dd, *J =* 5.6, 4.7 Hz, 2H), 3.40 (s, 3H), 3.34 (q, *J =* 4.4 Hz, 2H), 2.74 (s, 3H), 2.43 - 2.33 (m, 2H), 2.21 (s, 3H). |
| **1-36** | 1.44 | 458.4 | Method 3 | (500 MHz, CDCl₃) δ: 8.01 (d, *J =* 3.0 Hz, 1H), 7.98 (s, 1H), 7.36 (d, *J =* 8.5 Hz, 1H), 7.28 (d, *J =* 7.8 Hz, 1H), 7.01 (dd, *J =* 8.7, 3.0 Hz, 1H), 6.62 (dt, *J =* 7.6, 1.2 Hz, 1H), 6.45 (ddd, *J* = 8.2*,* 2.4, 1.0 Hz, 1H), 6.33 - 6.29 (m, 1H), 4.57 (t, *J =* 5.0 Hz, 1H), 3.91 (t, *J =* 7.2 Hz, 4H), 3.18 (dt, *J =* 6.7, 5.2 Hz, 2H), 2.74 (s, 3H), 2.61 - 2.56 (m, 2H), 2.39 (m, *J =* 7.2 Hz, 2H), 2.26 (s, 6H), 2.22 (s, 3H). |
| **1-37** | 13.31 | 383.3 | Method 3 | (400 MHz, CDCl₃) δ: 8.65 (ddd, *J =* 4.9*,* 2.0, 0.8 Hz, 1H), 8.05 (s, 1H), 7.86 (ddd, *J =* 8.1, 7.4, 1.9 Hz, 1H), 7.66 (dt, *J* = 8.1*,* 1.0 Hz, 1H), 7.46 (t, *J =* 7.9 Hz, 1H), 7.33 (ddd, *J =* 7.4*,* 4.9, 1.1 Hz, 1H), 7.18 - 7.10 (m, 2H), 7.08 - 7.02 (m, 1H), 6.57 (t, *J =* 73.7 Hz, 1H), 2.75 (s, 3H), 2.23 (s, 3H). |
| **1-38** | 14.20 | 401.3 | Method 3 | (400 MHz, CDCl₃) δ: 8.65 (ddd, *J =* 4.9*,* 2.0, 0.9 Hz, 1H), 8.06 (s, 1H), 7.86 (ddd, *J =* 8.1, 7.4, 1.9 Hz, 1H), 7.66 (dt, *J =* 8.1*,* 0.9 Hz, 1H), 7.49 (dd, *J* = 8.2*,* 7.6 Hz, 1H), 7.33 (ddd, *J =* 7.5, 4.9, 1.0 Hz, 1H), 7.28 - 7.17 (m, 2H), 7.15 (dq, *J =* 2.3, 1.2 Hz, 1H), 2.76 (s, 3H), 2.23 (s, 3H). |
| **1-39** | 14.25 | 404.3 | Method 3 | (500 MHz, CDCl₃) δ: 8.53 (s, 2H), 8.01 (s, 1H), 7.36 (t, *J =* 7.9 Hz, 1H), 7.07 (ddd, *J =* 8.3*,* 2.6, 0.9 Hz, 1H), 6.95 (dd, *J =* 2.6, 1.5 Hz, 1H), 6.87 (dt, *J =* 7.7, 1.3 Hz, 1H), 3.99 (s, 3H), 3.80 - 3.73 (m, 1H), 2.76 (s, 3H), 2.24 (s, 3H), 0.87 - 0.72 (m, 4H). |
| **1-40** | 14.81 | 440.3 | Method 3 | (500 MHz, CDCl₃) δ: 8.76 (d, *J =* 1.0 Hz, 2H), 8.04 (s, 1H), 7.37 (t, *J =* 7.9 Hz, 1H), 7.08 (ddd, *J =* 8.2, 2.5, 1.0 Hz, 1H), 6.95 (dd, *J =* 2.6*,* 1.5 Hz, 1H), 6.87 (dt, *J* = 7.6*,* 1.3 Hz, 1H), 6.65 (t, *J =* 71.2 Hz, 1H), 3.80 - 3.73 (m, 1H), 2.77 (s, 3H), 2.25 (s, 3H), 0.85 - 0.74 (m, 4H). |
| **1-41** | 10.28 | 461.2 | Method 3 | (400 MHz, CDCl₃) δ: 8.56 (s, 2H), 8.01 (s, 1H), 7.36 (t, *J =* 7.9 Hz, 1H), 7.07 (ddd, *J =* 8.3*,* 2.6, 1.0 Hz, 1H), 6.95 (dd, *J =* 2.6, 1.5 Hz, 1H), 6.87 (dt, *J =* 7.6, 1.3 Hz, 1H), 4.23 (t, *J =* 5.5 Hz, 2H), 3.76 (tt, *J =* 5.9, 4.1 Hz, 1H), 2.80 (t, *J =* 5.5 Hz, 2H), 2.76 (s, 3H), 2.37 (s, 6H), 2.24 (s, 3H), 0.86 - 0.74 (m, 4H). |
| **1-42** | 13.96 | 439.2 | Method 3 | (500 MHz, CDCl₃) δ: 8.76 (s, 2H), 8.03 (s, 1H), 7.28 *(t, J =* 7.8 Hz, 1H), 6.64 (t, *J =* 71.6 Hz, 1H), 6.61 (dt, *J =* 7.3*,* 1.2 Hz, 1H), 6.52 - 6.44 (m, 1H), 6.30 (t, *J =* 2.0 Hz, 1H), 3.91 (td, *J = 7*.2, 5.1 Hz, 4H), 2.75 (s, 3H), 2.39 (m, *J =* 7.0, 3.0 Hz, 2H), 2.23 (s, 3H). |
| **1-43** | 1.24 | 460.1 | Method 3 | (400 MHz, CDCl₃) δ: 8.55 (s, 2H), 8.00 (s, 1H), 7.28 (t*, J =* 7.8 Hz, 1H), 6.61 (dt, *J =* 7.8*,* 1.2 Hz, 1H), 6.46 (ddd, *J =* 8.2, 2.4, 1.0 Hz, 1H), 6.31 (t, *J =* 1.9 Hz, 1H), 4.24 (t, *J =* 5.5 Hz, 2H), 3.91 (t, *J =* 7.2 Hz, 4H), 2.85 - 2.78 (m, 2H), 2.75 (s, 3H), 2.38 (q, *J =* 7.3 Hz, 2H), 2.38 (s, 6H), 2.22 (s, 3H). |
| **1-44** | 12.59 | 388.2 | Method 3 | (500 MHz, CDCl₃) δ: 8.31 (s, 2H), 7.98 (s, 1H), 7.27 (s, 1H), 6.64 - 6.59 (m, 1H), 6.46 (ddd, *J =* 8.1, 2.4, 0.9 Hz, 1H), 6.31 (t, *J =* 2.0 Hz, 1H), 3.95 - 3.85 (m, 4H), 2.74 (s, 3H), 2.37 (dt, *J =* 15.3, 7.6 Hz, 2H), 2.22 (s, 3H). |
| **1-45** | 12.66 | 431.4 | Method 3 | (400 MHz, CDCl₃) δ: 8.73 - 8.68 (m, 1H), 8.03 (s, 1H), 8.00 - 7.93 (m, 1H), 7.58 (dd, *J =* 8.3, 0.7 Hz, 1H), 7.36 (dd, *J =* 8.3, 7.5 Hz, 1H), 7.06 (ddd, *J =* 8.3, 2.6, 1.0 Hz, 1H), 6.94 (dd, *J =* 2.5, 1.5 Hz, 1H), 6.86 (dt, *J* = 7.6, 1.2 Hz, 1H), 3.79 - 3.71 (m, 1H), 2.74 (s, 3H), 2.30 (s, 1H), 2.22 (s, 3H), 1.62 (s, 6H), 0.89 - 0.71 (m, 4H). |
| **1-46** | 13.06 | 376.2 | Method 3 | (400 MHz, CDCl₃) δ: 8.55 (s, 2H), 8.02 (s, 1H), 7.15 (d, *J* = 1.4 Hz, 1H), 7.08 (dd, *J =* 7.7*,* 1.9 Hz, 1H), 7.00 (d, *J =* 7.7 Hz, 1H), 4.01 (s, 3H), 2.58 (s, 3H), 2.40 (s, 3H), 2.08 (d, *J =* 7.9 Hz, 6H). |
| **1-47** | 12.21 | 459.4 | Method 3 | (500 MHz, CDCl₃) δ: 8.46 (s, 2H), 8.00 (s, 1H), 7.36 (dd, *J =* 8.3, 7.5 Hz, 1H), 7.07 (ddd, *J =* 8.3, 2.6, 1.0 Hz, 1H), 6.95 (dd, *J =* 2.6, 1.5 Hz, 1H), 6.87 (ddd, *J =* 7.5, 1.6, 1.0 Hz, 1H), 3.92 - 3.89 (m, 4H), 3.48 (q, *J =* 7.0 Hz, 2H), 3.31 - 3.27 (m, 4H), 2.76 (s, 3H), 2.24 (s, 3H), 0.84 - 0.77 (m, 4H). |
| **1-48** | 13.12 | 408.1 | Method 3 | (500 MHz, CDCl₃) δ: 8.83 (s, 2H), 8.04 (s, 1H), 7.37 (dd, *J =* 8.3, 7.5 Hz, 1H), 7.08 (ddd, *J =* 8.3, 2.5, 1.0 Hz, 1H), 6.94 (dd, *J =* 2.6, 1.5 Hz, 1H), 6.86 (ddd, *J* = 7.5, 1.6, 1.0 Hz, 1H), 3.80 - 3.73 (m, 1H), 2.76 (s, 3H), 2.24 (s, 3H), 0.85 - 0.77 (m, 4H). |
| **1-49** | 13.40 | 406.3 | Method 3 | (400 MHz, CDCl₃) δ: 8.78 (s, 2H), 8.05 (s, 1H), 7.32 - 7.20 (m, 4H), 6.75 (dd, *J =* 7.3, 1.5 Hz, 1H), 3.85 - 3.75 (m, 1H), 2.59 (s, 3H), 2.12 (d, *J =* 11.3 Hz, 5H), 1.93 (s, 3H), 0.88 - 0.82 (m, 4H). |
| **1-50** | 12.41 | 388.2 | Method 3 | (400 MHz, CDCl₃) δ: 8.74 - 8.68 (m, 2H), 8.03 (s, 1H), 7.36 (dd, *J =* 8.3, 7.5 Hz, 1H), 7.07 (ddd, *J =* 8.3, 2.6, 1.0 Hz, 1H), 6.95 (dd, *J =* 2.6, 1.5 Hz, 1H), 6.87 (ddd, *J= 7.5,* 1.6, 1.0 Hz, 1H), 3.81 - 3.72 (m, 1H), 2.76 (s, 3H), 2.40 (d, *J =* 3.4 Hz, 3H), 2.24 (s, 3H), 0.83 - 0.76 (m, 4H). |
| **1-51** | 13.27 | 391.3 | Method 3 | (400 MHz, CDCl₃) δ: 8.46 (ddd, *J =* 4.6, 1.4, 0.8 Hz, 1H), 8.04 (s, 1H), 7.62 (ddd, *J =* 9.0, 8.2, 1.5 Hz, 1H), 7.48 - 7.42 (m, 1H), 7.42 - 7.33 (m, 1H), 7.07 (ddd, *J =* 8.3*,* 2.6, 1.0 Hz, 1H), 6.96 (dd, *J =* 2.6*,* 1.5 Hz, 1H), 6.88 (dt, *J =* 7.7, 1.3 Hz, 1H), 3.81 - 3.72 (m, 1H), 2.75 (s, 3H), 2.25 (s, 3H), 0.83 - 0.77 (m, 4H). |
| **1-52** | nd | nd | | (400 MHz, CDCl₃) δ: 8.72 (d, *J =* 0.9 Hz, 2H), 8.01 (s, 1H), 7.27 - 7.17 (m, 2H), 6.73 (dd, *J =* 7.3, 1.5 Hz, 1H), 3.82 - 3.73 (m, 1H), 2.56 (s, 3H), 2.40 (s, 3H), 2.07 (s, 3H), 1.90 (s, 3H), 0.85 - 0.79 (m, 4H). |
| **1-53** | 12.42 | 402.2 | Method 3 | (400 MHz, CDCl₃) δ: 8.96 (s, 2H), 8.10 (s, 1H), 7.52 (t, *J =* 8.0 Hz, 1H), 7.40 (t, *J =* 4.7 Hz, 1H), 7.33 - 7.19 (m, 2H), 7.17 (dt, *J =* 2.5, 1.2 Hz, 1H), 2.78 (s, 3H), 2.26 (s, 3H). |
| **1-54** | 13.77 | 405.3 | Method 3 | (500 MHz, CDCl₃) δ: 8.50 (d, *J =* 3.0 Hz, 1H), 8.05 (s, 1H), 7.70 (dd, *J =* 8.8, 4.0 Hz, 1H), 7.59 (ddd, *J =* 8.9,7.4,3.0 Hz, 1H), 7.30 (d, *J =* 1.4 Hz, 1H), 7.24 (t, *J =* 7.8 Hz, 1H), 6.75 (dd, *J =* 7.4, 1.4 Hz, 1H), 3.80 (td, *J =* 6.3, 5.5, 3.2 Hz, 1H), 2.59 (s, 3H), 2.10 (s, 3H), 1.94 (s, 3H), 0.85 (d, *J =* 5.7 Hz, 4H). |
| **1-55** | 13.11 | 391.2 | Method 3 | (500 MHz, CDCl₃) δ: 8.48 (d, *J =* 2.9 Hz, 1H), 8.07 (s, 1H), 7.68 (dd, *J =* 8.8, 4.0 Hz, 1H), 7.59 (ddd, *J =* 8.8, 7.4, 3.0 Hz, 1H), 7.38 (t, *J =* 7.9 Hz, 1H), 7.09 (ddd, *J =* 8.3, 2.6, 1.0 Hz, 1H), 6.93 (dd, *J* = 2.5*,* 1.5 Hz, 1H), 6.88 (dt, *J =* 7.5, 1.2 Hz, 1H), 3.76 (tt, *J =* 6.2, 3.5 Hz, 1H), 2.75 (s, 3H), 2.25 (s, 3H), 0.88 - 0.77 (m, 4H). |
| **1-56** | 12.93 | 432.2 | Method 3 | (400 MHz, CDCl₃) δ: 8.55 (s, 2H), 8.04 (s, 1H), 7.51 (t, *J =* 7.9 Hz, 1H), 7.25 (ddt, *J =* 8.8*,* 7.6, 1.2 Hz, 2H), 7.16 (dt, *J =* 2.5, 1.2 Hz, 1H), 4.02 (s, 3H), 2.77 (s, 3H), 2.25 (s, 3H). |
| **1-57** | 11.95 | 414.2 | Method 3 | (500 MHz, CDCl₃) δ: 8.56 (s, 2H), 8.04 (s, 1H), 7.48 (t, *J =* 7.9 Hz, 1H), 7.19 - 7.12 (m, 2H), 7.07 (t, *J =* 2.0 Hz, 1H), 6.59 (d, *J =* 75.0 Hz, 1H), 4.02 (s, 3H), 2.77 (s, 3H), 2.25 (s, 3H). |
| **1-58** | 11.41 | 384.2 | Method 3 | (500 MHz, CDCl₃) δ: 8.92 (d, *J =* 4.8 Hz, 2H), 8.05 (s, 1H), 7.46 (t, *J =* 7.9 Hz, 1H), 7.37 (t, *J =* 4.8 Hz, 1H), 7.17 - 7.10 (m, 2H), 7.05 (t, *J =* 2.0 Hz, 1H), 6.57 (t, *J =* 73.7 Hz, 1H), 2.76 (s, 3H), 2.23 (s, 3H). |
| **1-59** | 12.81 | 445.4 | Method 3 | (500 MHz, CDCl₃) δ: 8.72 (d, *J =* 2.5 Hz, 1H), 8.05 (s, 1H), 7.99 (dd, *J =* 8.4, 2.5 Hz, 1H), 7.64 (d, *J =* 8.4 Hz, 1H), 7.30 - 7.19 (m, 2H), 6.73 (dd, *J =* 7.4, 1.4 Hz, 1H), 3.76 (tt, *J =* 6.0, 3.2 Hz, 1H), 3.72 (s, 1H), 2.55 (s, 3H), 2.08 (s, 3H), 1.71 (s, 3H), 1.64 (s, 6H), 0.92 - 0.78 (m, 4H). |
| **1-60** | 13.03 | 420.2 | Method 3 | (400 MHz, CDCl₃) δ: 8.78 (s, 2H), 8.07 (s, 1H), 7.52 (t, *J =* 8.0 Hz, 2H), 7.25 (tt, *J =* 7.6, 1.3 Hz, 1H), 7.16 (s, 1H), 2.78 (s, 3H), 2.26 (s, 3H). |
| **1-61** | 4.01 | 334.2 | Method 1 | (500 MHz, CDCl₃) δ: 8.93 (d, *J =* 4.9 Hz, 2H), 8.06 (s, 1H), 7.40 - 7.34 (m, 2H), 7.00 (ddd, *J =* 7.6, 1.6, 1.0 Hz, 1H), 6.95 (dd, *J =* 2.6, 1.5 Hz, 1H), 6.91 (ddd, *J =* 8.3, 2.6, 0.9 Hz, 1H), 6.78 (s, 1H), 3.86 (s, 3H), 2.91 (s, 3H). |
| **1-62** | 4.32 | 374.3 | Method 1 | (500 MHz, CDCl₃) δ: 8.66 (ddd, *J= 4.9,* 2.0, 0.9 Hz, 1H), 8.05 (s, 1H), 7.86 (td, *J =* 7.8, 2.0 Hz, 1H), 7.67 (dt, *J =* 8.0, 1.0 Hz, 1H), 7.33 (ddd, *J =* 7.4, 4.9, 1.1 Hz, 1H), 6.92 (t, *J =* 7.9 Hz, 1H), 6.75 (d, *J =* 6.9 Hz, 2H), 6.65 (dd, *J =* 7.5, 1.6 Hz, 1H), 4.36 - 4.30 (m, 2H), 3.36 - 3.29 (m, 2H), 2.96 (s, 3H), 2.75 (s, 3H). |
| **1-63** | 3.99 | 375.3 | Method 1 | (500 MHz, CDCl₃) δ: 8.91 (d, *J =* 4.9 Hz, 2H), 8.05 (s, 1H), 7.35 (t, *J =* 4.8 Hz, 1H), 6.92 (t, *J =* 7.8 Hz, 1H), 6.77 (s, 1H), 6.74 (dd, *J =* 8.2, 1.5 Hz, 1H), 6.65 (dd, *J =* 7.6, 1.5 Hz, 1H), 4.34 - 4.29 (m, 2H), 3.36 - 3.28 (m, 2H), 2.95 (s, 3H), 2.76 (s, 3H). |
| **1-64** | 4.02 | 358.3 | Method 1 | (500 MHz, CDCl₃) δ: 8.64 (d, *J =* 5.1 Hz, 1H), 8.03 (s, 1H), 7.85 (td, *J =* 7.8, 1.9 Hz, 1H), 7.65 (d, *J =* 8.1 Hz, 1H), 7.32 (ddd, *J =* 7.5, 4.8, 1.0 Hz, 1H), 7.17 (t, *J =* 7.7 Hz, 1H), 6.66 (s, 2H), 6.55 (d, *J =* 7.5 Hz, 1H), 3.33 (d, *J =* 8.4 Hz, 2H), 2.86 (t, *J =* 8.0 Hz, 2H), 2.81 (s, 3H), 2.74 (s, 3H). |
| **1-65** | 5.05 | 384.3 | Method 1 | (500 MHz, CDCl₃) δ: 8.68 - 8.61 (m, 1H), 8.03 (s, 1H), 7.85 (ddd, *J =* 8.1, 7.5, 2.0 Hz, 1H), 7.65 (dt, *J =* 8.0, 0.9 Hz, 1H), 7.32 (ddd, *J =* 7.4, 4.9, 1.1 Hz, 1H), 7.16 (t, *J =* 7.7 Hz, 1H), 6.87 (d, *J =* 7.8 Hz, 1H), 6.67 (d, *J =* 7.6 Hz, 1H), 6.65 (s, 1H), 3.40 (t, *J =* 8.0 Hz, 2H), 2.85 - 2.79 (m, 2H), 2.74 (s, 3H), 2.18 (m, *J =* 4.9 Hz, 1H), 0.71 (d, *J =* 5.8 Hz, 4H). |
| **1-66** | 4.18 | 386.2 | Method 1 | (500 MHz, CDCl₃) δ: 8.68 - 8.62 (m, 1H), 8.05 (s, 1H), 7.87 (td, *J =* 7.7, 2.0 Hz, 1H), 7.67 (dt, *J =* 8.1, 1.0 Hz, 1H), 7.34 (ddd, *J =* 7.5, 4.8, 1.0 Hz, 1H), 7.15 (s, 1H), 6.68 (s, 1H), 6.65 - 6.37 (m, 2H), 3.89 (s, 1H), 3.39 (s, 2H), 2.86 (s, 1H), 2.75 (s, 3H), 1.31 - 1.14 (m, 6H). |
| **1-67** | 4.75 | 385.3 | Method 1 | (500 MHz, CDCl₃) δ: 8.92 (d, *J =* 4.8 Hz, 2H), 8.05 (s, 1H), 7.37 (t, *J =* 4.9 Hz, 1H), 7.18 (t, *J =* 7.7 Hz, 1H), 6.89 (d, *J =* 7.7 Hz, 1H), 6.69 (d, *J =* 7.7 Hz, 2H), 3.42 (t, *J =* 8.0 Hz, 2H), 2.82 (t, *J =* 8.0 Hz, 2H), 2.76 (s, 3H), 2.24 - 2.16 (m, 1H), 0.73 (d, *J =* 5.9 Hz, 4H). |
| **1-68** | 3.72 | 387.3 | Method 1 | (500 MHz, CDCl₃) δ: 8.92 (d, *J =* 4.8 Hz, 2H), 8.05 (s, 1H), 7.37 (t, *J =* 4.9 Hz, 1H), 7.13 (s, 1H), 6.69 (s, 1H), 6.62 - 6.40 (m, 2H), 3.87 (m, *J =* 7.1 Hz, 1H), 3.37 (s, 2H), 2.84 (t, *J =* 8.5 Hz, 2H), 2.76 (s, 3H), 1.20 (d, *J =* 6.7 Hz, 6H). |
| **1-69** | 4.30 | 345.3 | Method 1 | (500 MHz, CDCl₃) δ: 8.71 - 8.66 (m, 1H), 8.08 (s, 1H), 7.96 - 7.87 (m, 1H), 7.73 (dt, *J =* 8.1, 1.0 Hz, 1H), 7.37 (ddd, *J =* 7.5, 4.9, 1.1 Hz, 1H), 7.22 - 7.14 (m, 1H), 6.81 (ddd, *J =* 11.6, 7.8, 0.9 Hz, 2H), 6.70 (s, 1H), 4.59 (t, *J =* 8.7 Hz, 2H), 3.12 (t, *J =* 8.6 Hz, 2H), 2.78 (s, 3H). |
| **1-70** | 4.52 | 369.2 | Method 1 | (500 MHz, CDCl₃) δ: 8.67 (ddd, *J= 4.9,* 2.0, 0.8 Hz, 1H), 8.07 (s, 1H), 7.89 (td, *J =* 7.8, 1.9 Hz, 1H), 7.69 (dt, *J =* 8.1*,* 1.0 Hz, 1H), 7.44 (t, *J =* 7.9 Hz, 1H), 7.36 (ddd, *J =* 7.5, 4.9, 1.0 Hz, 1H), 7.28 - 7.25 (m, 1H), 7.18 (t, *J =* 2.1 Hz, 1H), 7.14 - 7.10 (m, 1H), 6.75 (s, 1H), 6.57 (t, *J =* 73.7 Hz, 1H), 2.89 (s, 3H). |
| **1-71** | 4.69 | 383.3 | Method 1 | (500 MHz, CDCl₃) δ: 8.72 - 8.64 (m, 1H), 8.08 (s, 1H), 7.93 - 7.86 (m, 1H), 7.71 (dt, *J =* 8.0, 0.9 Hz, 1H), 7.36 (ddd, *J =* 7.5, 4.9, 1.0 Hz, 1H), 7.28 - 7.22 (m, 1H), 7.13 (dd, *J =* 8.2, 1.2 Hz, 1H), 7.06 (dd, *J =* 7.6, 1.2 Hz, 1H), 6.62 (s, 1H), 6.56 (t, *J =* 74.1 Hz, 1H), 2.64 (s, 3H), 2.14 (s, 3H). |
| **1-72** | 4.61 | 347.2 | Method 1 | (500 MHz, CDCl₃) δ: 8.71 (dd, *J =* 4.9, 1.9 Hz, 1H), 8.09 (s, 1H), 7.93 (td, *J =* 7.8, 1.9 Hz, 1H), 7.79 (d, *J* = 8.1 Hz, 1H), 7.39 (ddd, *J =* 7.5, 4.9, 1.0 Hz, 1H), 7.22 (t, *J* = 7.9 Hz, 1H), 6.89 (dd, *J* = 8.2, 1.1 Hz, 1H), 6.81 (dd, *J=* 7.7, 1.1 Hz, 1H), 6.64 (s, 1H), 3.89 (s, 3H), 2.65 (s, 3H), 2.07 (s, 3H). |
| **1-73** | 4.39 | 367.2 | Method 1 | (500 MHz, CDCl₃) δ: 8.67 (ddd, *J =* 4.9, 2.0, 0.9 Hz, 1H), 8.08 (s, 1H), 7.89 (ddd, *J =* 8.1, 7.4, 1.9 Hz, 1H), 7.71 (dt, *J =* 8.1*,* 0.9 Hz, 1H), 7.35 (ddd, *J =* 7.5, 4.9, 1.1 Hz, 1H), 7.32 - 7.26 (m, 1H), 6.98 (dd, *J =* 8.3, 1.4 Hz, 1H), 6.91 (dd, *J =* 7.7, 1.4 Hz, 1H), 6.73 (s, 1H), 3.96 (s, 3H), 2.70 (s, 3H). |
| **1-74** | 4.25 | 370.2 | Method 1 | (500 MHz, CDCl₃) δ: 8.93 (d, *J =* 4.9 Hz, 2H), 8.07 (s, 1H), 7.44 (t, *J =* 7.9 Hz, 1H), 7.39 (t, *J =* 4.8 Hz, 1H), 7.28 - 7.23 (m, 1H), 7.18 (t, *J* = 2.0 Hz, 1H), 7.14 - 7.09 (m, 1H), 6.77 (s, 1H), 6.57 (t, *J =* 73.7 Hz, 1H), 2.90 (s, 3H). |
| **1-75** | 4.41 | 384.2 | Method 1 | (500 MHz, CDCl₃) δ: 8.93 (d, *J=* 4.8 Hz, 2H), 8.07 (s, 1H), 7.39 (t, *J =* 4.8 Hz, 1H), 7.25 (t, *J =* 7.9 Hz, 1H), 7.13 (dd, *J =* 8.3, 1.3 Hz, 1H), 7.07 (dd, *J =* 7.6, 1.3 Hz, 1H), 6.64 (s, 1H), 6.56 (t, *J =* 74.0 Hz, 1H), 2.65 (s, 3H), 2.13 (s, 3H). |
| **1-76** | 4.29 | 348.3 | Method 1 | (500 MHz, CDCl₃) δ: 8.93 (d, *J=* 4.8 Hz, 2H), 8.06 (s, 1H), 7.38 (t, *J =* 4.9 Hz, 1H), 7.22 (t, *J =* 7.9 Hz, 1H), 6.88 (dd, *J* = 8.3, 1.1 Hz, 1H), 6.81 (dd, *J* = 7.7, 1.1 Hz, 1H), 6.64 (s, 1H), 3.88 (s, 3H), 2.65 (s, 3H), 2.06 (s, 3H). |
| **1-77** | 4.07 | 368.2 | Method 1 | (500 MHz, CDCl₃) δ: 8.92 (d, *J =* 4.8 Hz, 2H), 8.07 (s, 1H), 7.37 (t, *J =* 4.8 Hz, 1H), 7.29 (t, *J =* 8.0 Hz, 1H), 6.98 (dd, *J =* 8.3, 1.4 Hz, 1H), 6.91 (dd, *J =* 7.7*,* 1.4 Hz, 1H), 6.74 (s, 1H), 3.96 (s, 3H), 2.71 (s, 3H). |
| **1-78** | 3.68 | 372.3 | Method 1 | (500 MHz, CDCl₃) δ: 8.69 (dd, *J =* 5.0, 1.9 Hz, 1H), 8.09 (s, 1H), 7.92 (td, *J =* 7.8, 1.8 Hz, 1H), 7.76 (d, *J =* 8.1 Hz, 1H), 7.42 - 7.32 (m, 2H), 6.97 (d, *J =* 7.8 Hz, 1H), 6.83 (d*, J =* 7.8 Hz, 1H), 6.67 (s, 1H), 3.40 (s, 2H), 3.23 (s, 3H), 2.73 (s, 3H). |
| **1-79** | 11.89 | 346.2 | Method 3 | (400 MHz, CDCl₃) δ: 8.95 (d, *J =* 4.8 Hz, 2H), 8.06 (s, 1H), 7.38 (t, *J =* 4.8 Hz, 1H), 7.15 (dd, *J =* 1.9, 0.9 Hz, 1H), 7.12 - 7.05 (m, 1H), 7.00 (d, *J =* 7.6 Hz, 1H), 2.59 (s, 3H), 2.40 (s, 3H), 2.09 (s, 3H), 2.07 (s, 3H). |
| **1-80** | 11.49 | 459.4 | Method 3 | (400 MHz, CDCl₃) δ: 8.15 (s, 2H), 7.99 (s, 1H), 7.40 - 7.32 (m, 1H), 7.06 (ddd, *J =* 8.3, 2.6, 1.0Hz, 1H), 6.95 (dd, *J =* 2.6*,* 1.5 Hz, 1H), 6.87 (dt, *J* = 7.6*,* 1.2 Hz, 1H), 4.71 (s, 1H), 3.81 - 3.72 (m, 1H),3.66 - 3.55 (m, 2H), 3.50 (d, *J =* 7.0 Hz, 1H), 3.37 (d, *J =* 10.6 Hz, 1H), 2.76 (s, 3H), 2.23 (s, 3H), 2.22 -2.14 (m, 2H), 0.83 - 0.77 (m, 4H). |
| **1-81** | 7.65 | 389.1 | Method 2 | (500 MHz, CDCl₃) δ: 8.31 (s, 2H), 7.99 (s, 1H), 7.39 - 7.34 (m, 1H), 7.06 (ddd, *J =* 8.2, 2.6, 1.0 Hz, 1H), 6.95 (dd, *J =* 2.6*,* 1.5 Hz, 1H), 6.87 (dt, *J* = 7.6*,* 1.3 Hz, 1H), 3.91 (s, 2H), 3.80 - 3.73 (m, 1H), 2.76 (s, 3H), 2.23 (s, 3H), 0.81 - 0.79 (m, 4H). |
| **1-82** | 2.98 | 359.1 | Method 5 | (400 MHz, CDCl₃) δ: 8.71 (s, 1H), 8.25 (s, 1H), 7.93 (td, *J =* 7.9*,* 1.8 Hz, 1H), 7.90 (d, *J =* 1.5 Hz, 1H), 7.79 (d, *J =* 8.2 Hz, 1H), 7.41 (dd, *J =* 7.4, 4.8 Hz, 0H), 7.27 (m, 1H), 7.26 (t, 1H), 7.03 (dd, *J =* 6.9, 2.0 Hz, 1H), 6.89 (d, *J =* 1.6 Hz, 1H), 3.85 - 3.76 (m, 1H), 2.26 (s, 1H), 0.90 - 0.79 (m, 4H). |
| **1-83** | 2.94 | 359.1 | Method 5 | (400 MHz, CDCl₃) δ: 8.73 (s, 1H), 8.10 (s, 1H), 8.04 - 7.95 (m, 1H), 7.83 (d, *J =* 8.2 Hz, 1H), 7.50 - 7.43 (m, 1H), 7.39 (td, *J =* 7.7, 0.8 Hz, 1H), 7.12 - 7.10 (m, 1H), 7.10 - 7.07 (m, 1H), 7.07 - 7.01 (m, 1H), 6.81 (s, 1H), 3.81 (m, *J =* 4.5 Hz, 1H), 2.92 (s, 3H), 0.84 (d, *J =* 4.6 Hz, 4H). |
| **1-84** | 2.88 | 373.2 | Method 5 | (400 MHz, CDCl₃) δ: 8.73 (d, *J =* 4.9 Hz, 1H), 8.09 (s, 1H), 7.99 (ddd, *J =* 8.1*,* 7.4, 1.8 Hz, 1H), 7.83 (d, *J =* 8.1 Hz, 1H), 7.45 (ddd, *J =* 7.5*,* 5.0, 1.0 Hz, 1H), 7.28 (d, *J =* 2.1 Hz, 1H), 7.24 (dd, *J =* 8.0, 7.3 Hz, 1H), 6.84 (dd, *J =* 7.3*,* 1.5 Hz, 1H), 6.67 (s, 1H), 3.85 - 3.76 (m, 1H), 2.66 (s, 3H), 2.04 (s, 3H), 0.91 - 0.79 (m, 4H) |
| **1-85** | 2.99 | 374.2 | Method 5 | (400 MHz, CDCl₃) δ: 8.94 (d, *J =* 4.8 Hz, 2H), 8.07 (s, 1H), 7.39 (t, *J =* 4.8 Hz, 1H), 7.28 - 7.25 (m, 1H), 7.23 (dd, *J* = 8.0, 7.2 Hz, 1H), 6.84 (dd, *J =* 7.2, 1.5 Hz, 1H), 6.65 (s, 1H), 3.83 - 3.74 (m, 1H), 2.67 (s, 3H), 2.02 (s, 3H), 0.84 - 0.77 (m, 4H). |
| **1-86** | 7.53 | 387.2 | Method 2 | (400 MHz, CDCl₃) δ: 8.94 (d, *J =* 4.8 Hz, 2H), 8.05 (s, 1H), 7.38 (t, *J =* 4.8 Hz, 1H), 7.20 - 7.12 (m, 1H), 6.73 (dd, *J =* 8.3, 1.2 Hz, 1H), 6.49 (dd, *J =* 7.5*,* 1.2 Hz, 1H), 3.28 (t, *J =* 5.8 Hz, 2H), 2.99 (s, 3H), 2.60 (s, 3H), 2.47 - 2.29 (m, 2H), 2.11 (s, 3H), 1.99 - 1.88 (m, 2H). |
| **1-87** | 6.98 | 373.2 | Method 2 | (400 MHz, CDCl₃) δ: 8.92 (d, *J =* 4.9 Hz, 2H), 8.03 (s, 1H), 7.36 (t, *J =* 4.8 Hz, 1H), 7.15 (t, *J =* 7.7 Hz, 1H), 6.51 (dd, *J =* 7.7*,* 2.2 Hz, 2H), 3.31 (dt, *J =* 9.1, 7.7 Hz, 2H), 2.81 (s, 3H), 2.77 - 2.58 (m, 5H), 2.14 (s, 3H). |
| **1-88** | 3.09 | 360.2 | Method 5 | (400 MHz, CDCl₃) δ: 8.94 (d, *J =* 4.8 Hz, 2H), 8.08 (s, 1H), 7.40 (d, *J =* 4.8 Hz, 1H), 7.37 (dd, *J =* 8.2, 0.7 Hz, 1H), 7.12 - 7.10 (m, 1H), 7.08 (ddd, *J =* 8.2, 2.5, 1.0 Hz, 1H), 7.04 (ddd, *J =* 7.6, 1.6, 1.0 Hz, 1H), 6.80 (s, 1H), 3.80 (m, *J =* 4.5 Hz, 1H), 2.94 (s, 3H), 0.83 (d, *J =* 4.5 Hz, 4H). |
| **1-89** | 3.29 | 373.2 | Method 5 | (400 MHz, CDCl₃) δ: 8.94 (d, *J =* 4.9 Hz, 2H), 8.06 (s, 1H), 7.39 (t, *J =* 4.9 Hz, 1H), 7.14 (dd, *J =* 8.3, 7.5 Hz, 1H), 6.67 (d, *J =* 1.2 Hz, 1H), 6.66 (s, 1H), 6.53 (dd, *J=* 7.5, 1.1 Hz, 1H), 3.27 (t, 2H), 2.97 (s, 3H), 2.69 (s, 3H), 2.52 (t, *J =* 6.4 Hz, 2H), 1.93 (m, 2H). |
| **1-90** | 3.28 | 359.2 | Method 5 | (400 MHz, CDCl₃) δ: 8.94 (d, *J =* 4.9 Hz, 2H), 8.07 (s, 1H), 7.39 (t, *J =* 4.8 Hz, 1H), 7.18 (tt, *J =* 7.7, 0.8 Hz, 1H), 6.72 (s, 1H), 6.64 (dd, *J =* 7.6, 0.9 Hz, 1H), 6.52 (dd, *J =* 7.8, 0.9 Hz, 1H), 3.34 (t, *J =* 8.1 Hz, 2H), 2.86 (t, *J =* 8.1 Hz, 2H), 2.82 (s, 3H), 2.79 (s, 3H). |
| **1-91** | 7.51 | 347.2 | Method 2 | (400 MHz, CDCl₃) δ: 8.94 (d, *J =* 4.9 Hz, 2H), 8.07 (s, 1H), 7.44 - 7.35 (m, 2H), 7.01 - 6.73 (m, 3H), 3.87 (s, 3H), 2.78 (s, 3H), 2.26 (s, 3H). |
| **1-92** | 2.96 | 373.2 | Method 5 | (400 MHz, CDCl₃) δ: 8.94 (d, *J =* 4.8 Hz, 2H), 8.08 (s, 1H), 7.39 (t, *J =* 4.9 Hz, 1H), 7.21 (d, *J =* 8.4 Hz, 1H), 7.01 (dd, *J =* 8.4, 2.7 Hz, 1H), 6.89 (d, *J =* 2.7 Hz, 1H), 6.67 (s, 1H), 3.79 - 3.70 (m, 1H), 2.70 (s, 3H), 2.14 (s, 3H), 0.84 - 0.74 (m, 4H). |
| **1-93** | 2.92 | 388.2 | Method 5 | (400 MHz, CDCl₃) δ: 8.93 (d, *J =* 4.9 Hz, 2H), 8.05 (s, 1H), 7.37 (t, *J =* 4.9 Hz, 1H), 7.23 - 7.19 (m, 1H), 7.02 (dd, *J* = 8.4, 2.7 Hz, 1H), 6.80 (d, *J* = 2.7 Hz, 1H), 3.78 - 3.69 (m, 1H), 2.61 (s, 3H), 2.11 (s, 3H), 2.03 (s, 3H), 0.78 (ddd, *J =* 6.3, 3.2, 2.1 Hz, 4H). |
| **1-94** | 7.57 | 337.2 | Method 2 | (400 MHz, CDCl₃) δ: 7.87 (s, 1H), 7.13 (t, *J =* 7.9 Hz, 1H), 6.66 (dd, *J =* 8.3*,* 1.1 Hz, 1H), 6.42 (dd, *J =* 7.5, 1.2 Hz, 1H), 4.13 (q, *J =* 7.1 Hz, 2H), 3.26 (t, *J =* 5.8 Hz, 2H), 2.96 (s, 3H), 2.59 (s, 3H), 2.46 - 2.26 (m, 2H), 2.05 (s, 3H), 1.95 - 1.85 (m, 2H), 1.40 (t, *J =* 7.1 Hz, 3H). |
| **1-95** | 7.90 | 362.2 | Method 2 | (400 MHz, CDCl₃) δ: 8.94 (s, 2H), 8.06 (s, 1H), 7.42 - 7.35 (m, 1H), 7.24 (t, *J =* 7.9 Hz, 1H), 6.91 (dd, *J =* 8.3, 1.1 Hz, 1H), 6.74 (dd, *J* = 7.7, 1.1 Hz, 1H), 3.90 (s, 3H), 2.60 (s, 3H), 2.10 (s, 4H), 1.99 (s, 3H). |
| **1-96** | 8.43 | 397.2 | Method 2 | (400 MHz, CDCl₃) δ: 8.95 (d, *J =* 4.9 Hz, 2H), 8.10 (s, 1H), 7.63 (d, *J =* 8.2 Hz, 1H), 7.38 (t, *J =* 4.8 Hz, 1H), 7.35 - 7.27 (m, 1H), 7.18 (d, *J =* 3.3 Hz, 1H), 7.01 (dd, *J =* 7.2*,* 1.0 Hz, 1H), 6.17 - 6.11 (m, 1H), 3.41 (ddd, *J* = 8.2*,* 6.8, 4.0 Hz, 1H), 2.70 (s, 3H), 2.19 (s, 3H), 1.17 - 1.04 (m, 4H). |
| **1-97** | 7.67 | 371.1 | Method 2 | (500 MHz, CDCl₃) δ: 8.95 (d, *J =* 4.8 Hz, 2H), 8.10 (s, 1H), 7.37 (s, 2H), 7.36 - 7.31 (m, 1H), 7.12 (d, *J* = 3.2 Hz, 1H), 7.01 (dd, *J =* 7.1, 0.9 Hz, 1H), 6.20 (d, *J =* 3.1 Hz, 1H), 3.88 (s, 3H), 2.70 (s, 3H), 2.20 (s, 3H). |
| **1-98** | 2.99 | 349.1 | Method 4 | (500 MHz, CDCl₃) δ: 8.95 (d, *J =* 4.8 Hz, 2H), 8.09 (s, 1H), 7.69 (dd, *J =* 10.8, 4.9 Hz, 1H), 7.38 (t, *J =* 4.9 Hz, 1H), 6.96 (d, *J =* 7.2 Hz, 1H), 6.75 (d, *J =* 8.3 Hz, 1H), 4.00 (s, 3H), 2.95 (s, 3H), 2.42 (s, 3H). |
| **1-99** | 3.30 | 399.2 | Method 4 | (500 MHz, CDCl₃) δ: 8.92 (d, *J =* 4.8 Hz, 2H), 8.03 (s, 1H), 7.36 (t, *J =* 4.9 Hz, 1H), 7.15 (t, *J =* 7.7 Hz, 1H), 6.86 (m, 1H), 6.54 (m, 1H), 3.38 (m, 2H), 2.63 (m, 5H), 2.18 (m, 1H), 2.15 (s, 3H), 0,70 (m, 4H). |
| **1-100** | 12.12 | 323.4 | Method 3 | (500 MHz, CDCl₃) δ: 7.85 (s, 1H), 7.14 (t, *J =* 7.7 Hz, 1H), 6.49 (m, *J =* 10.9, 7.8, 1.0 Hz, 2H), 4.10 (q, *J =* 7.1 Hz, 2H), 3.29 (m, 2H), 2.80 (s, 3H), 2.73 - 2.64 (m, 2H), 2.62 (s, 3H), 2.09 (s, 3H), 1.38 (t, *J =* 7.1 Hz, 3H). |
| **1-101** | 4.51 | 351.3 | Method 1 | (500 MHz, CDCl₃) δ: 8.69 (dd, *J =* 5.3, 1.9 Hz, 1H), 8.08 (s, 1H), 7.96 - 7.88 (m, 1H), 7.74 (dt, *J =* 8.1, 1.0 Hz, 1H), 7.38 (ddd, *J* = 7.5, 4.9, 1.0 Hz, 1H), 6.76 - 6.68 (m, 3H), 6.62 (dt, *J =* 10.6, 2.3 Hz, 1H), 3.84 (s, 3H), 2.89 (s, 3H). |
| **1-102** | 4.38 | 351.3 | Method 1 | (400 MHz, CDCl₃) δ: 8.71 - 8.64 (m, 1H), 8.08 (s, 1H), 7.89 (td, *J =* 7.7*,* 1.9 Hz, 1H), 7.71 (dt, *J =* 8.1, 1.0 Hz, 1H), 7.36 (ddd, *J =* 7.5, 4.9, 1.1 Hz, 1H), 7.09 (t, *J =* 9.1 Hz, 1H), 6.90 - 6.81 (m, 2H), 6.76 (d, *J =* 1.2 Hz, 1H), 3.82 (s, 3H), 2.80 (d, *J =* 1.4 Hz, 3H). |
| **1-103** | 4.21 | 352.3 | Method 1 | (400 MHz, CDCl₃) δ: 8.92 (d, *J =* 4.9 Hz, 2H), 8.06 (s, 1H), 7.38 (t, *J =* 4.9 Hz, 1H), 6.77 - 6.67 (m, 3H), 6.62 (dt, *J =* 10.6, 2.3 Hz, 1H), 3.84 (s, 3H), 2.90 (s, 3H). |
| **1-104** | 4.80 | 393.3 | Method 1 | (500 MHz, CDCl₃) δ: 8.71 - 8.65 (m, 1H), 8.08 (s, 1H), 7.90 (td, *J =* 7.8*,* 1.9 Hz, 1H), 7.73 (d, *J =* 8.1 Hz, 1H), 7.36 (ddd, *J =* 8.4*,* 6.6, 3.3 Hz, 2H), 7.29 (t*, J =* 7.9 Hz, 1H), 6.91 (dd, *J =* 7.5, 1.5 Hz, 1H), 6.73 (s, 1H), 3.85 (tt, *J =* 6.0, 3.1 Hz, 1H), 2.70 (s, 3H), 0.94 - 0.82 (m, 5H). |
| **1-105** | 4.06 | 352.3 | Method 1 | (400 MHz, CDCl₃) δ: 8.92 (d, *J =* 4.9 Hz, 2H), 8.07 (s, 1H), 7.38 (t, *J =* 4.8 Hz, 1H), 7.09 (t, *J =* 9.1 Hz, 1H), 6.90 - 6.80 (m, 2H), 6.78 (d, *J =* 1.2 Hz, 1H), 3.82 (s, 3H), 2.81 (d*, J =* 1.4 Hz, 3H). |
| **1-106** | 3.67 | 334.3 | Method 1 | (400 MHz, CDCl₃) δ: 8.69 - 8.61 (m, 1H), 8.24 (dd, *J =* 5.4, 0.7 Hz, 1H), 8.07 (s, 1H), 7.91 - 7.83 (m, 1H), 7.65 (d, *J =* 8.1 Hz, 1H), 7.35 (ddd, *J =* 7.5, 4.9, 1.0 Hz, 1H), 6.98 (dd, *J =* 5.4, 1.5 Hz, 1H), 6.82 (dd, *J =* 1.5, 0.7 Hz, 1H), 6.77 (s, 1H), 4.02 (s, 3H), 2.92 (s, 3H). |
| **1-107** | 4.36 | 351.3 | Method 1 | (400 MHz, CDCl₃) δ: 8.67 (s, 1H), 8.07 (s, 1H), 7.89 (td, *J* = 7.8, 1.8 Hz, 1H), 7.69 (d, *J =* 8.1 Hz, 1H), 7.36 (dd, *J =* 7.4, 4.9 Hz, 1H), 7.15 (dd, *J =* 11.1, 8.3 Hz, 1H), 7.03 - 6.93 (m, 1H), 6.95 - 6.86 (m, 1H), 6.73 (s, 1H), 3.94 (s, 3H), 2.88 (s, 3H). |
| **1-108** | 4.26 | 351.3 | Method 1 | (400 MHz, CDCl₃) δ: 8.71 - 8.62 (m, 1H), 8.08 (s, 1H), 7.89 (td, *J =* 7.8*,* 1.9 Hz, 1H), 7.70 (d, *J =* 8.1 Hz, 1H), 7.35 (ddd, *J =* 7.5, 4.9, 1.0 Hz, 1H), 7.14 (td, *J =* 8.0, 1.4 Hz, 1H), 6.99 (td, *J =* 8.1*,* 1.6 Hz, 1H), 6.90 (ddd, *J =* 7.8, 6.2, 1.6 Hz, 1H), 6.76 (d, *J =* 1.2 Hz, 1H), 3.93 (s, 3H), 2.79 (d, *J =* 1.5 Hz, 3H). |
| **1-109** | 4.65 | 377.3 | Method 1 | (400 MHz, CDCl₃) δ: 8.71 - 8.61 (m, 1H), 8.08 (s, 1H), 7.89 (td, *J* = 7.8, 1.9 Hz, 1H), 7.71 (d, *J =* 8.1 Hz, 1H), 7.39 - 7.28 (m, 2H), 7.14 (td, *J* = 8.0, 1.4 Hz, 1H), 6.91 (ddd, *J =* 7.8, 6.2, 1.6 Hz, 1H), 6.75 (s, 1H), 3.85 (tt, *J =* 6.0, 3.1 Hz, 1H), 2.78 (d, *J =* 1.6 Hz, 3H), 0.86 (ddd, *J =* 18.2, 6.7, 4.3 Hz, 4H). |
| **1-110** | 4.51 | 394.2 | Method 1 | (400 MHz, CDCl₃) δ: 8.92 (d, *J =* 4.8 Hz, 2H), 8.07 (s, 1H), 7.38 (t, *J =* 4.8 Hz, 1H), 7.35 (dd, *J =* 8.3*,* 1.6 Hz, 1H), 7.31 - 7.26 (m, 1H), 6.92 (dd, *J =* 7.5, 1.6 Hz, 1H), 6.74 (s, 1H), 3.85 (tt, *J =* 5.9, 3.2 Hz, 1H), 2.70 (s, 3H), 0.94 - 0.79 (m, 4H). |
| **1-111** | 4.06 | 352.3 | Method 1 | (500 MHz, CDCl₃) δ: 8.93 (s, 2H), 8.06 (s, 1H), 7.39 (t, *J =* 4.9 Hz, 1H), 7.14 (dd, *J =* 11.2, 8.2 Hz, 1H), 6.98 (d, *J =* 8.0 Hz, 1H), 6.95 - 6.87 (m, 1H), 6.74 (s, 1H), 3.93 (s, 3H), 2.88 (s, 3H). |
| **1-112** | 3.93 | 352.3 | Method 1 | (400 MHz, CDCl₃) δ: 8.92 (d, *J =* 4.8 Hz, 2H), 8.07 (s, 1H), 7.38 (t, *J =* 4.8 Hz, 1H), 7.14 (td, *J =* 8.0, 1.4 Hz, 1H), 6.99 (td, *J* = 8.1, 1.6 Hz, 1H), 6.90 (ddd, *J =* 7.8, 6.2, 1.6 Hz, 1H), 6.78 (d, *J =* 1.2 Hz, 1H), 3.94 (s, 3H), 2.80 (d, *J =* 1.4 Hz, 3H). |
| **1-113** | 4.36 | 377.1 | Method 1 | (400 MHz, CDCl₃) δ: 8.92 (d, *J =* 4.8 Hz, 2H), 8.07 (s, 1H), 7.38 (t, *J =* 4.8 Hz, 1H), 7.36 - 7.29 (m, 1H), 7.14 (td, *J =* 8.0, 1.4 Hz, 1H), 6.91 (ddd, *J =* 7.8, 6.2, 1.6 Hz, 1H), 6.76 (d, *J =* 1.2 Hz, 1H), 3.85 (tt, *J =* 6.0, 3.0 Hz, 1H), 2.80 (d, *J =* 1.4 Hz, 3H), 0.93 - 0.79 (m, 4H). |
| **1-114** | 3.28 | 335.3 | Method 1 | (500 MHz, CDCl₃) δ: 8.93 (d, *J =* 4.9 Hz, 2H), 8.23 (d, *J =* 5.3 Hz, 1H), 8.07 (s, 1H), 7.39 (t, *J =* 4.9 Hz, 1H), 6.95 (dd, *J =* 5.3, 1.5 Hz, 1H), 6.82 - 6.76 (m, 2H), 4.00 (s, 3H), 2.93 (s, 3H). |
| **1-115** | 4.63 | 429.2 | Method 1 | (500 MHz, CDCl₃) δ: 8.71 (dd, *J* = 4.9, 1.9 Hz, 1H), 8.13 (s, 1H), 7.95 (td, *J* = 7.8, 1.9 Hz, 1H), 7.75 (d, *J* = 8.1 Hz, 1H), 7.41 (ddd, *J* = 7.4, 5.0, 1.1 Hz, 1H), 7.19 (td, *J =* 8.0, 1.4 Hz, 1H), 7.06 (td, *J* = 8.2, 1.6 Hz, 1H), 6.88 (ddd, *J* = 7.6, 6.0, 1.6 Hz, 1H), 3.95 (s, 3H), 2.73 (d, *J =* 1.2 Hz, 3H). |
| **1-116** | 4.03 | 352.3 | Method 1 | (500 MHz, CDCl₃) δ: 8.67 (d, *J* = 4.8 Hz, 1H), 8.09 (s, 1H), 7.97 (d, *J* = 5.2 Hz, 1H), 7.93 - 7.87 (m, 1H), 7.69 (d, *J* = 8.1 Hz, 1H), 7.37 (ddd, *J* = 7.5, 4.9, 1.1 Hz, 1H), 6.87 (t, *J* = 4.9 Hz, 1H), 6.78 (d, *J* = 1.2 Hz, 1H), 4.08 (s, 3H), 2.82 (d, *J =* 1.5 Hz, 3H). |
| **1-117** | 3.70 | 353.3 | Method 1 | (500 MHz, CDCl₃) δ: 8.93 (d, *J =* 4.9 Hz, 2H), 8.09 (s, 1H), 7.98 (d, *J* = 5.2 Hz, 1H), 7.39 (t, *J =* 4.8 Hz, 1H), 6.88 (dd, *J* = 5.2, 4.6 Hz, 1H), 6.80 (d, *J* = 1.2 Hz, 1H), 4.09 (s, 3H), 2.83 (d, *J =* 1.5 Hz, 3H). |
| **1-118** | 2.52 | 359.4 | Method 1 | (500 MHz, CDCl₃) δ: 8.65 (ddd, *J* = 4.9, 1.9, 0.8 Hz, 1H), 8.05 (s, 1H), 7.92 - 7.90 (m, 1H), 7.87 (td, *J =* 7.8, 1.9 Hz, 1H), 7.66 (dt, *J* = 8.0, 0.9 Hz, 1H), 7.35 (ddd, *J* = 7.5, 4.9, 1.0 Hz, 1H), 6.67 (s, 1H), 6.40 (d, *J* = 5.6 Hz, 1H), 3.51 (t, *J =* 8.2 Hz, 2H), 3.02 (s, 3H), 2.92 (t, *J* = 8.3 Hz, 2H), 2.78 (s, 3H). |
| **1-119** | 2.24 | 360.39 | Method 1 | (500 MHz, CDCl₃) δ: 8.92 (d, *J =* 4.9 Hz, 2H), 8.06 (s, 1H), 7.89 (d, *J* = 5.6 Hz, 1H), 7.39 (t, *J =* 4.9 Hz, 1H), 6.68 (s, 1H), 6.41 (d, *J* = 5.6 Hz, 1H), 3.55 (t, *J =* 8.2 Hz, 2H), 3.06 (s, 3H), 2.94 (t, *J* = 8.2 Hz, 2H), 2.79 (s, 3H). |
| **1-120** | 2.65 | 398.1 | Method 4 | (500 MHz, CDCl₃) δ: 8.92 (d, *J =* 4.9 Hz, 2H), 8.04 (s, 1H), 7.36 (t, *J* = 4.8 Hz, 1H), 7.26 (m, 1H), 7.14 (m, 1H), 6.99 (m, 1H), 6.57 (t, *J* = 74.0 Hz, 1H), 2.58 (s, 3H), 2.07 (s, 3H), 2.04 (s, 3H). |
| **1-121** | 3.41 | 413.2 | Method 4 | (500 MHz, CDCl₃) δ: 8.91 (d, *J =* 4.8 Hz, 2H), 8.02 (s, 1H), 7.35 (t, *J* = 4.8 Hz, 1H), 7.22 (m, 1H), 7.13 (m, 1H), 6.48 (m, 1H), 3.25 (m, 2H), 2.58 (s, 3H), 2.32 (m, 3H), 2.08 (s, 3H), 1.84 (m, 2H), 0.84 (m, 2H), 0.65 (m, 2H). |
| **1-122** | 3.03 | 348.1 | Method 4 | (500 MHz, CDCl₃) δ: 8.91 (d, *J =* 4.7 Hz, 2H), 8.03 (s, 1H), 7.34 (t, *J* = 4.8, 1H), 7.19 (m, 2H), 6.99 (m, 2H), 3.85 (s, 3H), 2.74 (s, 3H), 2.21 (s, 3H). |
| **1-123** | 2.90 | 370.1 | Method 4 | (500 MHz, CDCl₃) δ: 8.92 (d, *J* = 4.9 Hz, 2H), 8.05 (s, 1H), 7.37 (t, *J* = 4.8 Hz, 1H), 7.32 (ddd, *J* = 8.3, 7.6, 5.3 Hz, 1H), 7.22 (td, *J* = 8.5, 1.5 Hz, 1H), 7.04 (dt, *J* = 7.6, 1.3 Hz, 1H), 2.62 (s, 3H), 2.13 (s, 3H). |
| **1-124** | 3.20 | 388.2 | Method 4 | (500 MHz, CDCl₃) δ: 8.91 (d, *J =* 4.9 Hz, 2H), 8.04 (s, 1H), 7.35 (t, *J =* 4.9 Hz, 1H), 7.32 (t, *J =* 7.9 Hz, 1H), 6.82 (m, 2H), 6.71 (m, 1H), 4.67 (m, 1H), 2.75 (s, 3H), 2.46 (m, 2H), 2.23 (s, 3H), 2.20 (m, 2H), 1.87 (m, 1H), 1.70 (m, 1H). |
| **1-125** | 2.98 | 392.1 | Method 4 | (500 MHz, CDCl₃) δ: 8.91 (d, *J =* 4.8 Hz, 2H), 8.05 (s, 1H), 7.36 (m, 1H), 7.34 (m, 1H), 7.15 (td, *J =* 7.9, 1.4 Hz, 1H), 6.81 (ddd, *J* = 7.7, 6.1, 1.6 Hz, 1H), 3.86 (m, 1H), 2.70 (s, 3H), 2.19 (s, 3H), 0.88 (m, 4H). |
| **1-126** | 2.73 | 365.1 | Method 4 | (500 MHz, CDCl₃) δ: 8.65 (ddd, *J* = 4.9, 1.9, 0.8 Hz, 1H), 8.05 (s, 1H), 7.85 (ddd, *J* = 8.1, 7.5, 2.0 Hz, 1H), 7.65 (d, *J* = 8.0 Hz, 1H), 7.32 (ddd, *J* = 7.5, 4.9, 1.1 Hz, 1H), 7.10 (t, *J =* 9.0 Hz, 1H), 6.90 (dt, *J =* 9.0, 3.6 Hz, 1H), 6.73 (dd, *J* = 5.8, 3.2 Hz, 1H), 3.82 (s, 3H), 2.70 (3H), 2.20 (3H). |
| **1-127** | 2.77 | 366.1 | Method 4 | (500 MHz, CDCl₃) δ: 8.91 (d, *J =* 4.8 Hz, 2H), 8.05 (s, 1H), 7.35 (t, *J =* 4.8 Hz, 1H), 7.10 (t, *J =* 9.0 Hz, 1H), 6.90 (ddd, *J* = 9.0, 3.9, 3.1 Hz, 1H), 6.73 (dd, *J* = 5.8, 3.2 Hz, 1H), 3.82 (s, 3H), 2.71 (d, 3H), 2.20 (s, 3H). |
| **1-128** | 2.92 | 366.1 | Method 4 | (500 MHz, CDCl₃) δ: 8.66 (d, *J =* 4.8 Hz, 1H), 8.05 (s, 1H), 7.85 (td, *J* = 7.7, 1.9 Hz, 1H), 7.66 (d, *J* = 8.2 Hz, 1H), 7.32 (ddd, *J* = 7.4, 4.8, 1.0 Hz, 1H), 7.15 (td, *J* = 8.0, 1.4 Hz, 1H), 7.02 (td, *J* = 8.1, 1.6 Hz, 1H), 6.80 (ddd, *J* = 7.7, 6.1, 1.6 Hz, 1H), 3.95 (s, 3H), 3.70 (s, 1H), 2.69 (d, *J* = 0.8 Hz, 3H), 2.19 (d, *J =* 0.8 Hz, 3H). |
| **1-129** | 2.98 | 348.1 | Method 4 | (500 MHz, CDCl₃) δ: 8.65 (dd, *J =* 4.9, 1.1 Hz, 1H), 8.09 (d, *J* = 2.5 Hz, 1H), 8.05 (s, 1H), 7.86 (ddd, *J* = 8.0, 7.5, 1.9 Hz, 1H), 7.65 (d, *J* = 8.1 Hz, 1H), 7.50 (dd, *J* = 8.5, 2.4 Hz, 1H), 7.33 (ddd, *J* = 7.5, 4.8, 1.1 Hz, 1H), 6.85 (dd, *J* = 8.5, 0.8 Hz, 1H), 4.00 (s, 3H), 3.70 (s, 1H), 2.74 (s, 3H), 2.22 (s, 3H). |
| **1-130** | 2.89 | 347.0 | Method 4 | (500 MHz, CDCl₃) δ: 8.65 (dd, *J =* 4.9, 2.0 Hz, 1H), 8.04 (s, 1H), 7.85 (td, *J* = 7.8, 1.9 Hz, 1H), 7.66 (d, *J* = 8.1 Hz, 1H), 7.32 (ddd, *J* = 7.5, 4.9, 1.0 Hz, 1H), 7.19 (m, 1H), 7.00 (m, 1H), 3.86 (s, 3H), 2.73 (s, 3H), 2.22 (s, 3H). |
| **1-131** | 4.73 | 413.2 | Method 2 | (500 MHz, CDCl₃) δ: 8.94 (d, *J =* 4.9 Hz, 2H), 8.06 (s, 1H), 7.37 (t, *J* = 4.8 Hz, 1H), 7.29 (t, *J =* 7.8 Hz, 1H), 6.92 (dd, *J* = 8.2, 2.4 Hz, 1H), 6.78 (t, *J* = 2.0 Hz, 1H), 6.70 (dt, *J* = 7.4, 1.2 Hz, 1H), 4.23 (m, *J* = 2.4 Hz, 2H), 2.77 (s, 3H), 2.24 (s, 3H), 1.89 - 1.83 (m, 4H), 1.49 (t, *J* = 6.5 Hz, 4H). |
| **1-132** | 2.93 | 349.0 | Method 2 | (500 MHz, CDCl₃) δ 8.91 (d, *J =* 4.8 Hz, 2H), 8.08 (dd, *J* = 2.4, 0.8 Hz, 1H), 8.04 (s, 1H), 7.50 (dd, *J* = 8.5, 2.4 Hz, 1H), 7.36 (t, *J* = 4.8 Hz, 1H), 6.85 (dd, *J* = 8.5, 0.8 Hz, 1H), 3.99 (s, 3H), 2.74 (s, 3H), 2.22 (s, 3H) |
| **1-133** | nd | nd | nd | (500 MHz, CDCl₃) δ 8.91 (d, *J =* 4.8 Hz, 2H), 8.05 (s, 1H), 7.36 (t, *J* = 4.8 Hz, 1H), 7.15 (td, *J* = 8.0, 1.4 Hz, 1H), 7.02 (td, *J* = 8.1, 1.6 Hz, 1H), 6.80 (ddd, *J* = 7.7, 6.1, 1.6 Hz, 1H), 3.94 (s, 3H), 2.70 (d, *J =* 0.8 Hz, 3H), 2.19 (d, *J* = 0.8 Hz, 3H) |
| **1-134** | 3.07 | 362.1 | Method 2 | (500 MHz, CDCl₃) δ 8.93 (d, *J =* 4.8 Hz, 1H), 8.05 (s, 1H), 7.37 (t, *J* = 4.8 Hz, 0H), 7.02 (d, *J* = 8.3 Hz, 1H), 6.87 (d, *J* = 2.7 Hz, 0H), 6.81 (dd, *J* = 8.3, 2.7 Hz, 1H), 3.86 (s, 2H), 2.58 (s, 1H), 2.08 (d, *J* = 3.9 Hz, 3H) |
| **1-135** | 2.93 | 366.0 | Method 2 | (500 MHz, CDCl₃) δ 8.91 (d, *J =* 4.8 Hz, 2H), 8.04 (s, 1H), 7.35 (t, *J* = 4.8 Hz, 1H), 7.13 (t, *J* = 8.5 Hz, 1H), 6.82 - 6.78 (m, 1H), 6.75 (dd, *J* = 11.5, 2.5 Hz, 1H), 3.86 (s, 3H), 2.69 (d, *J* = 0.8 Hz, 3H), 2.18 (d, *J =* 0.8 Hz, 3H) |
| **1-136** | 2.81 | 365.0 | Method 2 | (500 MHz, CDCl₃) δ 8.69 - 8.57 (m, 1H), 8.04 (s, 1H), 7.85 (td, *J* = 7.7, 1.9 Hz, 1H), 7.66 (dt, *J* = 8.1, 1.0 Hz, 1H), 7.32 (ddd, *J* = 7.5, 4.9, 1.0 Hz, 1H), 7.13 (t, *J* = 8.4 Hz, 1H), 6.80 (dd, *J* = 8.5, 2.6 Hz, 1H), 6.75 (dd, *J* = 11.5, 2.5 Hz, 1H), 3.86 (s, 3H), 2.68 (s, 3H), 2.18 (s, 3H) |
| **1-137** | 3.34 | 363.1 | Method 2 | (500 MHz, CDCl₃) δ 8.94 (d, 2H), 8.06 (s, 1H), 7.39 (t, 1H), 7.32 (d, 1H), 6.68 (d, 1H), 4.00 (s, 3H), 2.61 (s, 3H), 2.26 (s, 3H), 2.11 (s, 3H) |
| **1-138** | 2.87 | 367.1 | Method 2 | (500 MHz, CDCl₃) δ 8.94 (d, *J =* 4.8 Hz, 1H), 8.08 (s, 1H), 8.01 (d, *J* = 5.1 Hz, 1H), 7.39 (t, *J* = 4.8 Hz, 1H), 6.79 (dd, *J* = 5.1, 4.4 Hz, 1H), 4.11 (s, 2H), 2.75 (d, *J* = 1.0 Hz, 2H), 2.24 (d, *J =* 0.9 Hz, 2H) |
| **1-139** | 2.92 | 349.1 | Method 2 | (500 MHz, CDCl₃) δ 8.94 (d, *J =* 4.9 Hz, 2H), 8.27 (d, *J* = 5.2 Hz, 1H), 8.07 (s, 1H), 7.39 (t, *J =* 4.9 Hz, 1H), 6.83 (dd, *J* = 5.2, 1.4 Hz, 1H), 6.68 (s, 1H), 4.02 (s, 3H), 2.79 (s, 3H), 2.27 (s, 3H) |
| **1-140** | 6.46 | 384.0 | Method 2 | (500 MHz, CDCl₃) δ 8.71 - 8.64 (m, 1H), 8.33 - 8.24 (m, 1H), 8.09 (s, 1H), 7.96 - 7.86 (m, 1H), 7.73 - 7.65 (m, 1H), 7.56 (d, J = 5.7 Hz, 1H), 7.44 - 7.31 (m, 1H), 7.09 - 7.03 (m, 1H), 6.91 - 6.83 (m, 1H, 2.80 (s, 3H), 2.28 (s, 3H) |
| **1-141** | 5.98 | 385.2 | Method 2 | (500 MHz, CDCl₃) δ 8.95 (d, J = 4.9 Hz, 2H), 8.30 (d, J = 5.1 Hz, 1H), 8.09 (s, 1H), 7.56 (s, 1H), 7.44 - 7.38 (m, 1H), 7.06 (dd, J = 5.1, 1.4 Hz, 1H), 6.85 (d, J = 1.4 Hz, 1H), 2.81 (s, 3H), 2.28 (s, 3H) |
| **1-142** | 3.12 | 334.2 | Method 1 | (500 MHz, CDCl₃): δ = 8.66 (ddd, J = 4.9, 1.9, 0.8 Hz, 1H), 8.22 (dd, J = 2.4, 0.8 Hz, 1H), 8.07 (s, 1H), 7.88 (td, J = 7.8, 1.9 Hz, 1H), 7.67 (dt, J = 8.1, 1.0 Hz, 1H), 7.64 (dd, J = 8.5, 2.5 Hz, 1H), 7.35 (ddd, J = 7.5, 4.9, 1.0 Hz, 1H), 6.86 (dd, J = 8.5, 0.8 Hz, 1H), 6.72 (s, 1H), 4.00 (s, 3H), 2.86 (s, 3H) |
| **1-143** | 3.48 | 374.2 | Method 1 | (500 MHz, CDCl₃): δ = 8.66 (ddd, J = 4.8, 1.9, 0.9 Hz, 1H), 8.12 (d, J = 5.2 Hz, 1H), 8.07 (s, 1H), 7.88 (ddd, J = 8.1, 7.4, 1.9 Hz, 1H), 7.66 (dt, J = 8.1, 1.0 Hz, 1H), 7.35 (ddd, J = 7.4, 4.8, 1.0 Hz, 1H), 6.79 (d, J = 5.2 Hz, 1H), 6.61 (s, 1H), 4.38 (tt, J = 6.3, 3.0 Hz, 1H), 2.66 (s, 3H), 2.02 (s, 3H), 0.95 - 0.73 (m, 4H) |
| **1-144** | 2.84 | 335.1 | Method 1 | (500 MHz, CDCl₃): δ = 8.92 (d, J = 4.8 Hz, 2H), 8.23 (d, J = 2.4 Hz, 1H), 8.07 (s, 1H), 7.65 (dd, J = 8.5, 2.5 Hz, 1H), 7.38 (t, J = 4.8 Hz, 1H), 6.86 (d, J = 8.5 Hz, 1H), 6.74 (s, 1H), 4.00 (s, 3H), 2.87 (s, 3H) |
| **1-145** | 3.22 | 375.3 | Method 1 | (500 MHz, CDCl₃): δ = 8.92 (d, J = 4.9 Hz, 2H), 8.10 (d, J = 5.2 Hz, 1H), 8.06 (s, 1H), 7.39 (t, J = 4.9 Hz, 1H), 6.77 (d, J = 5.1 Hz, 1H), 6.63 (s, 1H), 4.35 (dt, J = 6.3, 3.3 Hz, 1H), 2.66 (s, 3H), 2.00 (s, 3H), 0.82 (dd, J = 34.6, 4.4 Hz, 4H) |
| **1-146** | 3.43 | 370.1 | Method 1 | (500 MHz, CDCl₃): δ = 8.70 - 8.65 (m, 1H), 8.25 (d, J = 5.2 Hz, 1H), 8.09 (s, 1H), 7.90 (td, J = 7.8, 1.9 Hz, 1H), 7.68 (dt, J = 8.1, 1.0 Hz, 1H), 7.52 (t, J = 73.0 Hz, 1H), 7.38 (ddd, J = 7.5, 4.9, 1.0 Hz, 1H), 7.16 (dd, J = 5.3, 1.5 Hz, 1H), 6.95 (d, J = 1.4 Hz, 1H), 6.78 (s, 1H), 2.94 (s, 3H) |
| **1-147** | 3.22 | 371.1 | Method 1 | (500 MHz, CDCl₃): δ = 8.93 (d, J = 4.9 Hz, 2H), 8.25 (d, J = 5.3 Hz, 1H), 8.09 (s, 1H), 7.52 (t, J = 73.0 Hz, 1H), 7.41 (t, J = 4.8 Hz, 1H), 7.21 - 7.11 (m, 1H), 6.95 (s, 1H), 6.80 (s, 1H), 2.95 (s, 3H) |

### PHARMACOLOGY

The compounds provided in the present invention are negative allosteric modulators of mGlu7. As such, these compounds are expected to have their effect at mGlu7 by virtue of their ability to block the function of the receptor after binding to a site that is not the orthosteric glutamate recognition site.

Some of the compounds of Formula (I) have been tested according to some of the following methods.

### Example A

### mGlu7 assay on HEK-expressing human mGLU7

### Transfection and Cell culture

The cDNA encoding the human metabotropic glutamate 7 receptor (hmGlu7), (accession number NM_181874.2, NCBI Nucleotide database browser), was subcloned into an expression vector containing also the hygromycin resistance gene. In parallel, the cDNA encoding a G protein allowing redirection of the activation signal to intracellular calcium flux was subcloned into a different expression vector containing also the Puromycin resistance gene. Transfection of both these vectors into HEK293 cells with PolyFect reagent (Qiagen) according to supplier's protocol, and hygromycin and puromycin treatment allowed selection of antibiotic resistant cells which had integrated stably one or more copies of the plasmids. Positive cellular clones expressing hmGlu7 were identified in a functional assay measuring changes in calcium fluxes in response to glutamate and L-AP4 or known mGlu7 orthosteric antagonists.

HEK-293 cells expressing hmGlu7 were maintained in media containing DMEM, Fetal Bovine Serum (10%), GlutaMAX^{™} (2 mM), penicillin (100 units/mL), streptomycin (100 µg/mL), geneticin (100 µg/mL) and hygromycin-B (40 µg/mL) and puromycin (1 µg/mL) at 37°C with 5% CO₂ in a humidified atmosphere.

### Fluorescent cell based- Ca²⁺ mobilization assay

Human mGlu7 HEK-293 cells were plated out 24 hours prior to a fluorescent cell-based calcium mobilization assay using FLIPR³⁸⁴ assay (Molecular Device, Sunnyvale, CA, USA) in black-walled, clear-bottomed, poly-L-ornithine-coated 384-well plates at a density of 25,000 cells/well in a glutamine/glutamate free DMEM medium containing fetal bovine serum (10%), penicillin (100 units/mL), streptomycin (100 µg/mL) and doxycyline (1 µg/ml) at 37°C with 5% CO₂ in a humidified atmosphere.

On the day of the assay, the medium was aspirated and the cells were loaded with a 3 µM solution of Fluo4-AM (LuBioScience, Lucerne, Switzerland) in 0.03% pluronic acid. After 1 hour at 37°C/5% CO₂, the non incorporated dye was removed by washing cell plate with the assay buffer. All assays were performed in a pH 7.4 buffered-Solution containing 20 mM HEPES, 143 mM NaCl, 6 mM KCl, 1 mM MgSO₄, 1 mM CaCl₂, 0.125 mM sulfinpyrazone and 0.1% glucose.

After 10 s of basal fluorescence recording, various concentrations of the compounds of the invention were added to the cells. Changes in fluorescence levels were first monitored for 180 s in order to detect any agonist activity of the compounds. Then the cells were stimulated by an EC₈₀ L-AP4 concentration for an additional 110 s in order to measure inhibiting activities of the compounds of the invention. EC₈₀ L-AP4 concentration is the concentration giving 80% of the maximal glutamate response.

The concentration-response curves of L-AP4 or representative compounds of the present invention were generated using the Prism GraphPad software (Graph Pad Inc, San Diego, USA). The curves were fitted to a four-parameter logistic equation:$\left(\text{Y} = \text{Bottom} + \left(\text{Top} - \text{Bottom}\right) \text{/} \left(1 + 10 \left(\left({\text{LogIC}}_{50} - \text{X}\right)\right. * \text{Hill Slope}\right)\right.$ allowing determination of IC₅₀ values.

The compounds of this application have IC₅₀ values less than 10 µM.

The Table 3 below represents the mean IC₅₀ obtained from at least three independent experiments of selected molecules performed in duplicate.

**Table 3: Activity data for selected compounds**

| **Co.Nr.** | **Ca²⁺ Flux*** | **Co.nr.** | **Ca²⁺ Flux*** | **Co.nr.** | **Ca²⁺ Flux*** |
|---|---|---|---|---|---|
| **1-1** | +++ | **1-51** | ++ | **1-101** | ++ |
| **1-2** | +++ | **1-52** | ++ | **1-102** | +++ |
| **1-3** | +++ | **1-53** | ++ | **1-103** | +++ |
| **1-4** | ++ | **1-54** | ++ | **1-104** | +++ |
| **1-5** | ++ | **1-55** | ++ | **1-105** | ++ |
| **1-6** | ++ | **1-56** | ++ | **1-106** | +++ |
| **1-7** | +++ | **1-57** | ++ | **1-107** | +++ |
| **1-8** | ++ | **1-58** | +++ | **1-108** | ++ |
| **1-9** | ++ | **1-59** | +++ | **1-109** | +++ |
| **1-10** | ++ | **1-60** | ++ | **1-110** | +++ |
| **1-11** | + | **1-61** | ++ | **1-111** | +++ |
| **1-12** | ++ | **1-62** | +++ | **1-112** | ++ |
| **1-13** | +++ | **1-63** | +++ | **1-113** | +++ |
| **1-14** | + | **1-64** | +++ | **1-114** | +++ |
| **1-15** | +++ | **1-65** | +++ | **1-115** | ++ |
| **1-16** | ++ | **1-66** | ++ | **1-116** | ++ |
| **1-17** | +++ | **1-67** | ++ | **1-117** | +++ |
| **1-18** | ++ | **1-68** | +++ | **1-118** | +++ |
| **1-19** | + | **1-69** | +++ | **1-119** | ++ |
| **1-20** | +++ | **1-70** | ++ | **1-120** | ++ |
| **1-21** | +++ | **1-71** | +++ | **1-121** | ++ |
| **1-22** | +++ | **1-72** | +++ | **1-122** | ++ |
| **1-23** | +++ | **1-73** | +++ | **1-123** | +++ |
| **1-24** | +++ | **1-74** | +++ | **1-124** | ++ |
| **1-25** | +++ | **1-75** | +++ | **1-125** | ++ |
| **1-26** | + | **1-76** | +++ | **1-126** | ++ |
| **1-27** | +++ | **1-77** | +++ | **1-127** | +++ |
| **1-28** | + | **1-78** | +++ | **1-128** | +++ |
| **1-29** | +++ | **1-79** | + | **1-129** | +++ |
| **1-30** | +++ | **1-80** | ++ | **1-130** | ++ |
| **1-31** | ++ | **1-81** | ++ | **1-131** | ++ |
| **1-32** | ++ | **1-82** | ++ | **1-132** | +++ |
| **1-33** | ++ | **1-83** | + | **1-133** | ++ |
| **1-34** | +++ | **1-84** | ++ | **1-134** | +++ |
| **1-35** | ++ | **1-85** | ++ | **1-135** | +++ |
| **1-36** | ++ | **1-86** | ++ | **1-136** | +++ |
| **1-37** | +++ | **1-87** | ++ | **1-137** | ++ |
| **1-38** | ++ | **1-88** | +++ | **1-138** | ++ |
| **1-39** | ++ | **1-89** | +++ | **1-139** | ++ |
| **1-40** | ++ | **1-90** | +++ | **1-140** | ++ |
| **1-41** | ++ | **1-91** | +++ | **1-141** | + |
| **1-42** | +++ | **1-92** | +++ | **1-142** | ++ |
| **1-43** | ++ | **1-93** | +++ | **1-143** | +++ |
| **1-44** | +++ | **1-94** | ++ | **1-144** | ++ |
| **1-45** | ++ | **1-95** | ++ | **1-145** | +++ |
| **1-46** | ++ | **1-96** | +++ | **1-146** | ++ |
| **1-47** | ++ | **1-97** | ++ | **1-147** | +++ |
| **1-48** | ++ | **1-98** | ++ | | |
| **1-49** | ++ | **1-99** | ++ | | |
| **1-50** | ++ | **1-100** | ++ | | |

| | | | | | |
|---|---|---|---|---|---|
| ***Table legend:** (+): 1 µM < IC₅₀ <10 µM (++): 100 nM < IC₅₀ <1 µM (+++): IC₅₀ < 100 nM | | | | | |

The results shown in Table 3 demonstrate that the compounds described in the present invention are negative allosteric modulators of human mGlu7 receptors.

### Example B

### Water associated zero maze:

The procedure can be performed as described previously by Ritov and Richter-Levin, 2014 with minor modifications. The apparatus consists of annular platform with two opposite, enclosed quadrants (with walls 35 cm height) and two open quadrants (with borders 5 mm height). The plastic tank that holds this platform is filled up with water (22 ± 2°C, 50 cm deep), arising to 10 cm below the platform level. Thus, the annular platform and the plastic tank comprise one unified arena. For the tests, rats are first habituated to the room for 4 min and then are placed into one of the open quadrants facing a closed part of the apparatus. Rats are allowed to explore the arena for a 5 mins session. During this time rats behavior is tracked, recorded and analyzed by the Etho-Vision system (Noldus Information Technology, Wageningen, Netherlands). Behavioral measures that are analyzed include the time spent in the open quadrants, distance traveled in the open quadrants, distance travelled in the closed quadrants and total freezing behavior. The impact of exposure to various stressors and/or compounds are assessed using these parameters. Pre-treatment time and route of administration of the different tested compounds are defined based of their pharmacokinetic properties.

### Example C

### Elevated plus maze:

The elevated plus maze (EPM) test can be conducted using Sprague-Dawley male rats. The EPM is made of plastic that has two open arms (50 cm × 10 cm) and two closed arms of the same size with walls 40 cm high, elevated 86 cm above the ground. Both arms are made of black Plexiglas. The average illumination level on the open arms is 187 LUX and 100 LUX on the closed arms. At the beginning of the experiment, rats are brought into a holding room directly next to the testing room and allowed to habituate to the environment for 30 min. At the commencement of testing, rats are placed in the center of the maze, facing one of the open arms and observed for 5 min. During this time rats behavior is tracked, recorded and analyzed by the Etho-Vision system (Noldus Information Technology, Wageningen, Netherlands). Behavioral measures that are analyzed include the time spent in the open arms, number of entries in the open arms as well as the distance travelled. Pre-treatment time and route of administration of the different tested compounds are defined based of their pharmacokinetic properties.

### Example D

### Fear conditioning model of post-traumatic stress disorder in the rat:

The fear-conditioning arena (30 cm × 20 cm × 25 cm, Med Associates) is made of Plexiglas in different contexts. The system is placed in a sound-proof ventilated box. The arena floor consists of grid floor (19 parallel 0.48 cm diameter stainless steel rods, 1.6 cm apart) above a stainless steel waste pan. All rods are wired to a shock generator and scrambler. A speaker is mounted in the chamber wall to provide the source of the auditory stimuli. Fear conditioning procedure is performed over two days. The first day (training), rats are placed in the training context (context A) and after a 120 s acclimation period, they received five pairiNGs of the CS and US. The CS tone (78 dB, 2 kHz, 5 ms rise/fall time) is presented for 30 s and co-terminated wITh a brief US footshock (O.5 s, 0.66 mA). The inter-tone interval (tone onset to next tone onset) ranged from 60s. The conditioning chambers are cleaned between subjects with 70% ethanol. The time-spent freezing during delivery of the CS tone is scored (CS freezing). The second day (test day), animals are placed in a new context (context B) and are exposed to the CS (120s) after 60s of acclimation. Time-spent in freezing is measured during both acclimation and CS. Tested compounds are administrated prior and/or after training phase as well as testing phase. Pre-treatment time and route of administration of the different tested compounds are adjusted based of their pharmacokinetic properties.

### Example E

### Noise-induced hearing loss (NIHL) model in the mice:

Young adult males CBA/CaJ mice can be used to assess the effect of tested compound on NIHL. Animals are exposed to octave band noise (8-16Khz) at a sound pressure level of 110dB over 2 hours. Tested compounds are administrated prior and/or after noise exposure. Hearing function is measured using auditory brainstrem response (ABR) audiograms or Distorsion Product of Autoacoustic Emissions (DPOAE) at different timepoint 24 hours, 2 and 4 weeks post acoustic trauma. Pre-treatment time and route of administration of the different tested compounds are adjusted based of their pharmacokinetic properties. The experimental groups are compared to the vehicle treated group through the measure of, for example, ABR Threshold, or ABR Threshold shift.

### Example F

### Colorectal Distension test of visceral pain in rat.

Male stress-sensitive Wistar Kyoto rats (250-300 g) are used in this study. Animals are fasted overnight (16 h) and on the day of testing, are anaesthetised using isoflurane. 6 cm latex balloon is inserted into the colorectal cavity, 1 cm from the anus. The animals are allowed to recover for 20 min before colorectal distension commenced. The paradigm used is an ascending phasic distension from 0 mmHg to 80 mmHg over 8 min using a computer-driven electronic barostat. The parameters measured are the threshold pressure (mmHg) that evoked visually identifiable visceral pain behaviour, and the total number of pain behaviours. Postures defined as visceral pain behaviours are abdominal retractions and/or abdominal withdrawal reflex.

Tested compounds are administrated prior colorectal distension. Pre-treatment time and route of administration of the different tested compounds are adjusted based of their pharmacokinetic properties.

### FORMULATION EXAMPLES

I. Typical examples of recipes for the formulation of the invention are as follows:

### 1. Tablets

| | |
|---|---|
| Active ingredient | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

In this Example, active ingredient can be replaced by the same amount of any of the compounds according to the present invention, in particular by the same amount of any of the exemplified compounds.

### 2. Suspension

An aqueous suspension is prepared for oral administration so that each 1 milliliter contains 1 to 5 mg of one of the active compounds, 50 mg of sodium carboxymethyl cellulose, 1 mg of sodium benzoaTE, 500 mg of sorbitol and water ad 1 mL.

### 3. Injectable

A parenteral composition is prepared by stirring 1.5 % by weight of active ingredient of the invention in 10% by volume propylene glycol and water.

### 4. Ointment

| | |
|---|---|
| Active ingredient | 5 to 1000 mg |
| Stearyl alcohol | 3 g |
| Lanoline | 5 g |
| White petroleum | 15 g |
| Water | ad 100 g |

In this Example, active ingredient can be replaced with the same amount of any of the compounds according to the present invention, in particular by the same amount of any of the exemplified compounds.

## Claims

1. A compound having the Formula (I): 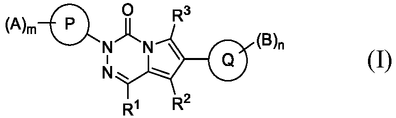 a pharmaceutically acceptable acid or base addition salt thereof, a stereochemically isomeric form thereof or an *N*-oxide form thereof, wherein:
R¹ is selected from the group of hydrogen, -CH₃ and -CF₃;
R² and R³ are each independently selected from the group of hydrogen, halogen, -(C₁-C₆)alkyl, -(C₁-C₆)haloalkyl and -CF₃;
P represents a -(C₁-C₆)alkyl, or a cycloalkyl, aryl, heteroaryl, -(C₁-C₆)alkylene-heteroaryl or heterocycle of formula: 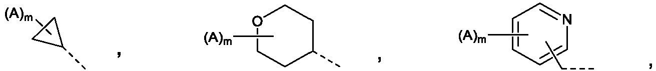 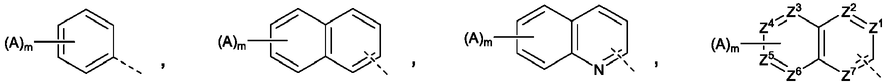 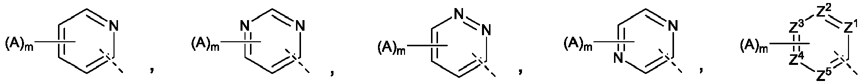 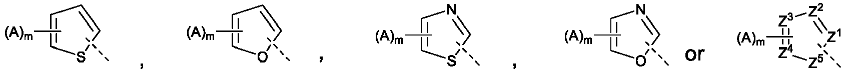
wherein each cycloalkyl, aryl, heteroaryl, -(C₁-C₆)alkylene-heteroaryl or heterocycle ring is optionally substituted with m radicals A, wherein m is an integer equal to zero, 1, 2, 3 or 4;
wherein Z¹, Z², Z³, Z⁴, Z⁵, Z⁶ and Z⁷ are each independently selected from C, N, O or S; provided that at least one of Z¹, Z², Z³, Z⁴, Z⁵, Z⁶ and Z⁷ is N;
the or each (A)ₘ is independently selected from the group of hydrogen, halogen, -CN, - OH, -NO₂, -CF₃, -SH, -NH₂ and an optionally substituted radical selected from the group of -(C₁-C₆)alkyl, -(C₁-C₆)haloalkyl, -(C₂-C₆)alkynyl, -(C₂-C₆)alkenyl, -(C₃-C₇)cycloalkyl, -(C₁-C₆)alkylene-(C₃-C₇)cycloalkyl, -(C₃-C₈)cycloalkenyl, -(C₁-C₆)cyanoalkyl, -(C₁-C₆)alkylene-heteroaryl, -(C₁-C₆)alkylene-aryl, aryl, heteroaryl, -(C₁-C₆)alkylene-heterocycle, heterocycle, -(C₀-C₆)alkylene-OR⁴, -O-(C₂-C₆)alkylene-OR⁴, - NR⁴(C₂-C₆)alkylene-OR⁵, -(C₃-C₆)alkynylene-OR⁴, -(C₃-C₆)alkynylene-NR⁴R⁵, -(C₃-C₆)alkenylene-OR⁴, -(C₃-C₆)alkenylene-NR⁴R⁵, -(C₀-C₆)alkylene-S-R⁴, -O-(C₂-C₆)alkylene-S-R⁴, -NR⁴-(C₂-C₆)alkylene-S-R⁵, -(C₀-C₆)alkylene-S(=O)-R⁴, -O-(C₁-C₆)alkylene-S(=O)-R⁴, -NR⁴-(C₁-C₆)alkylene-S(=O)-R⁵, -(C₀-C₆)alkylene-S(=O)₂-R⁴, - O-(C₁-C₆)alkylene-S(=O)₂-R⁴, -NR⁴-(C₁-C₆)alkylene-S(=O)₂-R⁵, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-S(=O)₂NR⁴R⁵, -O-(C₁-C₆)alkylene-S(=O)₂NR⁴R⁵, -NR⁴-(C₁-C₆)alkylene-S(=O)₂NR⁵R⁶, -(C₀-C₆)alkylene-NR⁴-S(=O)₂R⁵, -O-(C₂-C₆)alkylene-NR⁴-S(=O)₂R⁵, -NR⁴-(C₂-C₆)alkylene-NR⁵-S(=O)₂R⁶, -(C₀-C₆)alkylene-C(=O)-NR⁴R⁵, -O-(C₁-C₆)alkylene-C(=O)-NR⁴R⁵, -NR⁴-(C₁-C₆)alkylene-C(=O)-NR⁵R⁶, -(C₀-C₆)alkylene-NR⁴C(=O)-R⁵, - O-(C₂-C₆)alkylene-NR⁴C(=O)-R⁵, -NR⁴-(C₂-C₆)alkylene-NR⁵C(=O)-R⁶, -(C₀-C₆)alkylene-OC(=O)-R⁴, -O-(C₂-C₆)alkylene-OC(=O)-R⁴, -NR⁴-(C₂-C₆)alkylene-OC(=O)-R⁵, -(C₀-C₆)alkylene-C(=O)-OR⁴, -O-(C₁-C₆)alkylene-C(=O)-OR⁴, -NR⁴-(C₀-C₆)alkylene-C(=O)-OR⁵, -(C₀-C₆)alkylene-C(=O)-R⁴, -O-(C₁-C₆)alkylene-C(=O)-R⁴, - NR⁴-(C₁-C₆)alkylene-C(=O)-R⁵, -(C₀-C₆)alkylene-NR⁴-C(=O)-OR⁵, -C(=O)-(C₁-C₆)alkylene-NR⁴-C(=O)-OR⁵, -(C₀-C₆)alkylene-O-C(=O)-NR⁴R⁵, -(C₀-C₆)alkylene-NR⁴-C(=O)-NR⁵R⁶, -O-(C₂-C₆)alkylene-NR⁴-C(=O)-NR⁵R⁶, -NR⁴-(C₂-C₆)alkylene-NR⁵-C(=O)-NR⁶R⁷, -(C₀-C₆)alkylene-NR⁴-C(=S)-NR⁵R⁶ and -(C₀-C₆)alkylene-NR⁴-C(=NR⁵)-NR⁶R⁷;
R⁴, R⁵, R⁶ and R⁷ are each independently hydrogen or an optionally substituted radical selected from the group of -(C₁-C₆)haloalkyl, -(C₁-C₆)alkyl, -(C₁-C₆)cyanoalkyl, -(C₃-C₇)cycloalkyl, -(C₁-C₆)alkylene-(C₃-C₇)cycloalkyl, heteroaryl, -(C₁-C₆)alkylene-heteroaryl, aryl, -(C₁-C₆)alkylene-heterocycle, heterocycle, -(C₁-C₆)alkylene-aryl, -(C₀-C₆)alkylene-O-(C₀-C₆)alkyl, -(C₀-C₆)alkylene-N-((C₀-C₆)alkyl)₂ and -C(=O)-O-(C₁-C₆)alkyl;
Q represents an aryl, heteroaryl or -(C₅-C₇)cycloalkenyl of formula: 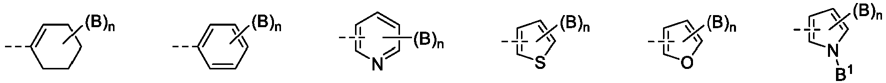 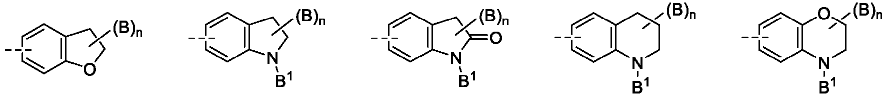 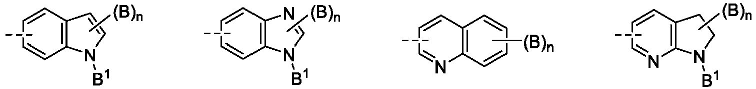
wherein each aryl, heteroaryl or -(C₅-C₇)cycloalkenyl ring is optionally substituted with n radicals B, wherein n is an integer equal to zero, 1, 2, 3, 4 or 5; wherein B¹ is a radical B;
the or each (B)ₙ is independently selected from the group of hydrogen, halogen, -CN, - OH, -NO₂, -CF₃, -SH, -NH₂ and an optionally substituted radical selected from the group of -(C₁-C₆)alkyl, -(C₁-C₆)haloalkyl, -(C₂-C₆)alkynyl, -(C₂-C₆)alkenyl, -(C₃-C₇)cycloalkyl, -(C₁-C₆)alkylene-(C₃-C₇)cycloalkyl, -(C₃-C₈)cycloalkenyl, -(C₁-C₆)cyanoalkyl, -(C₁-C₆)alkylene-heteroaryl, -(C₁-C₆)alkylene-aryl, aryl, heteroaryl, -(C₁-C₆)alkylene-heterocycle, heterocycle, -(C₀-C₆)alkylene-OR⁸, -O-(C₂-C₆)alkylene-OR⁸, -NR⁸(C₂-C₆)alkylene-OR⁹, -(C₃-C₆)alkynylene-OR⁸, -(C₃-C₆)alkynylene-NR⁸R⁹, -(C₃-C₆)alkenylene-OR⁸, -(C₃-C₆)alkenylene-NR⁸R⁹, -(C₀-C₆)alkylene-S-R⁸, -O-(C₂-C₆)alkylene-S-R⁸, -NR⁸-(C₂-C₆)alkylene-S-R⁹, -(C₀-C₆)alkylene-S(=O)-R⁸, -O-(C₁-C₆)alkylene-S(=O)-R⁸, -NR⁸-(C₁-C₆)alkylene-S(=O)-R⁹, -(C₀-C₆)alkylene-S(=O)₂-R⁸, - O-(C₁-C₆)alkylene-S(=O)₂-R⁸, -NR⁸-(C₁-C₆)alkylene-S(=O)₂-R⁹, -(C₀-C₆)alkylene-NR⁸R⁹, -O-(C₂-C₆)alkylene-NR⁸R⁹, -NR⁸-(C₂-C₆)alkylene-NR⁹R¹⁰, -(C₀-C₆)alkylene-S(=O)₂NR⁸R⁹, -O-(C₁-C₆)alkylene-S(=O)₂NR⁸R⁹, -NR⁸-(C₁-C₆)alkylene-S(=O)₂NR⁹R¹⁰, -(C₀-C₆)alkylene-NR⁸-S(=O)₂R⁹, -O-(C₂-C₆)alkylene-NR⁸-S(=O)₂R⁹, -NR⁸-(C₂-C₆)alkylene-NR⁹-S(=O)₂R¹⁰, -(C₀-C₆)alkylene-C(=O)-NR⁸R⁹, -O-(C₁-C₆)alkylene-C(=O)-NR⁸R⁹, -NR⁸-(C₁-C₆)alkylene-C(=O)-NR⁹R¹⁰, -(C₀-C₆)alkylene-NR⁸C(=O)-R⁹, - O-(C₂-C₆)alkylene-NR⁸C(=O)-R⁹, -NR⁸-(C₂-C₆)alkylene-NR⁹C(=O)-R¹⁰, -(C₀-C₆)alkylene-OC(=O)-R⁸, -O-(C₂-C₆)alkylene-OC(=O)-R⁸, -NR⁸-(C₂-C₆)alkylene-OC(=O)-R⁹, -(C₀-C₆)alkylene-C(=O)-OR⁸, -O-(C₁-C₆)alkylene-C(=O)-OR⁸, -NR⁸-(C₁-C₆)alkylene-C(=O)-OR⁹, -(C₀-C₆)alkylene-C(=O)-R⁸, -O-(C₁-C₆)alkylene-C(=O)-R⁸, - NR⁸-(C₁-C₆)alkylene-C(=O)-R⁹, -(C₀-C₆)alkylene-NR⁸-C(=O)-OR⁹, -(C₀-C₆)alkylene-O-C(=O)-NR⁸R⁹, -(C₀-C₆)alkylene-NR⁸-C(=O)-NR⁹R¹⁰, -O-(C₂-C₆)alkylene-NR⁸-C(=O)-NR⁹R¹⁰, -NR⁸-(C₂-C₆)alkylene-NR⁹-C(=O)-NR¹⁰R¹¹, -(C₀-C₆)alkylene-NR⁸-C(=S)-NR⁹R¹⁰ and -(C₀-C₆)alkylene-NR⁸-C(=NR⁹)-NR¹⁰R¹¹;
R⁸, R⁹, R¹⁰ and R¹¹ are each independently hydrogen or an optionally substituted radical selected from the group of -(C₁-C₆)haloalkyl, -(C₁-C₆)alkyl, -(C₁-C₆)cyanoalkyl, -(C₃-C₇)cycloalkyl, -(C₁-C₆)alkylene-(C₃-C₇)cycloalkyl, heteroaryl, -(C₁-C₆)alkylene-heteroaryl, aryl, -(C₁-C₆)alkylene-heterocycle, heterocycle, -(C₁-C₆)alkylene-aryl- -(C₀-C₆)alkylene-O-(C₀-C₆)alkyl and -(C₀-C₆)alkylene-N-((C₀-C₆)alkyl)₂;
wherein optionally any two radicals A are combined with the intervening atoms to form a 3 to 10 membered bicyclic heterocycle, aryl or heteroaryl ring, wherein each ring is optionally further substituted with 1 to 5 radicals independently selected from the group of halogen, -CN, nitro, -(C₁-C₆)alkyl, -(C₃-C₇)alkyl, -(C₀-C₆)alkylene-O-(C₀-C₆)alkyl and -(C₀-C₆)alkylene-N-((C₀-C₆)alkyl)₂;
wherein optionally two of the substituents R⁴, R⁵, R⁶ or R⁷ are combined with the intervening atoms to form a 3 to 10 membered heterocycle, aryl or heteroaryl ring, wherein each ring is optionally further substituted with 1 to 5 radicals independently selected from the group of halogen, cyano, nitro, -(C₁-C₆)alkyl, -(C₃-C₇)alkyl, -(C₀-C₆)alkylene-O-(C₀-C₆)alkyl and -(C₀-C₆)alkylene-N-((C₀-C₆)alkyl)₂;
wherein optionally two substituents from R⁸, R⁹, R¹⁰ or R¹¹ are combined with the intervening atoms to form a 3 to 10 membered heterocycle, aryl or heteroaryl ring, wherein each ring is optionally further substituted with 1 to 5 radicals independently selected from the group of halogen, cyano, nitro, -(C₁-C₆)alkyl, -(C₃-C₇)alkyl, -(C₀-C₆)alkylene-O-(C₀-C₆)alkyl and -(C₀-C₆)alkylene-N-((C₀-C₆)alkyl)₂; and
wherein optionally any two radicals B are combined with the intervening atoms to form a 3 to 10 membered bicyclic heterocycle, aryl or heteroaryl ring, wherein each ring is optionally further substituted with 1 to 5 radicals independently selected from the group of halogen, -CN, nitro, -(C₁-C₆)alkyl, -(C₃-C₇)alkyl, -(C₀-C₆)alkylene-O-(C₀-C₆)alkyl and -(C₀-C₆)alkylene-N-((C₀-C₆)alkyl)₂.

2. The compound according to claim 1 having the Formula (I), wherein P represents a -(C₁-C₆)alkyl, or a cycloalkyl, heteroaryl, -(C₁-C₆)alkylene-heteroaryl or heterocycle of formula: 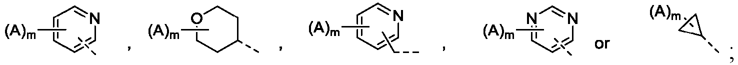 and/or wherein Q represents an aryl, heteroaryl or -(C₅-C₇)cycloalkenyl of formula:

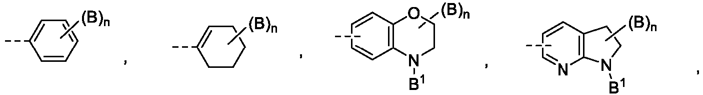

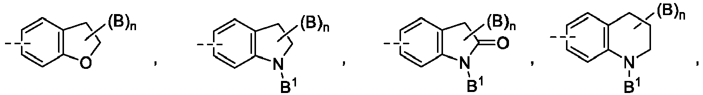

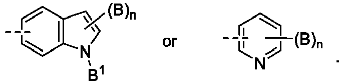

3. The compound according to any preceding claim having the Formula (I), wherein P represents a heteroaryl of formula: 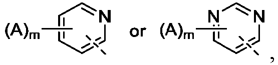 wherein each radical is optionally substituted with m radicals A, wherein m is an integer equal to zero, 1, 2, 3 or 4; and/or wherein Q represents an aryl or heteroaryl group of formula: 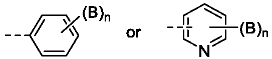 wherein each radical is optionally substituted with n radicals B, wherein n is an integer equal to zero, 1, 2, 3, 4 or 5; and/or wherein:
the cycloalkyl, heterocycle, aryl and heteroaryl ring systems of (A)ₘ are selected from the group of azetidinyl, 2-azabicyclo[2.2.1]heptan-2-yl, 7-azabicyclo[2.2.1]heptan-7-yl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phthalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents R⁴, R⁵, R⁶ or R⁷; and/or wherein:
the cycloalkyl, heterocycle, aryl and heteroaryl ring systems of (B)ₙ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phthalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents R⁸, R⁹, R¹⁰ or R¹¹; and/or wherein:
the cycloalkyl, heterocycle, aryl and heteroaryl ring systems of R⁴, R⁵, R⁶, R⁷, R⁸, R⁹, R¹⁰ or R¹¹ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phthalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted with 1-5 radicals independently selected from hydrogen, halogen, -CN, nitro, -(C₁-C₆)alkyl, -(C₀-C₆)alkylene-O-(C₀-C₆)alkyl and -(C₀-C₆)alkylene-N-((C₀-C₆)alkyl)₂.

4. The compound according to any preceding claim having the Formula (I), wherein R¹ is hydrogen; and/or wherein
R² and R³ are each independently selected from the group of hydrogen and methyl; and/or
wherein the or each (A)ₘ is independently selected from the group of hydrogen, halogen, -CN, -OH, -CF₃ and an optionally substituted radical selected from the group of -(C₁-C₆)alkyl, -(C₁-C₆)haloalkyl, -(C₃-C₇)cycloalkyl, -(C₁-C₆)cyanoalkyl, aryl, heterocycle, - (C₀-C₆)alkylene-OR⁴, -O-(C₂-C₆)alkylene-OR⁴, -NR⁴(C₂-C₆)alkylene-OR⁵, -(C₀-C₆)alkylene-S(=O)₂-R⁴, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-S(=O)₂NR⁴R⁵, -(C₀-C₆)alkylene-C(=O)-NR⁴R⁵, - (C₀-C₆)alkylene-NR⁴C(=O)-R⁵, -(C₀-C₆)alkylene-C(=O)-OR⁴, -(C₀-C₆)alkylene-C(=O)-R⁴, -C(=O)-(C₁-C₆)alkylene-NR⁴-C(=O)-OR⁵ and -NR⁴-(C₀-C₆)alkylene-C(=O)-OR⁵ and/or
wherein R⁴, R⁵ and R⁶ are each independently hydrogen, -(C₁-C₆)alkyl, -(C₁-C₆)haloalkyl or -C(=O)-O-(C₁-C₆)alkyl; and/or
wherein the or each (B)ₙ is independently selected from the group of hydrogen, halogen, -CN, -CF₃, and an optionally substituted radical selected from the group of -(C₁-C₆)alkyl, -(C₃-C₇)cycloalkyl, aryl, heteroaryl, heterocycle, -(C₀-C₆)alkylene-OR⁸, -NR⁸(C₂-C₆)alkylene-OR⁹, -(C₀-C₆)alkylene-NR⁸R⁹, -(C₀-C₆)alkylene-C(=O)-OR⁸ and -(C₀-C₆)alkylene-C(=O)-R⁸; and/or
wherein R⁸ and R⁹ are each independently selected from the group of hydrogen, -(C₁-C₆)haloalkyl, -(C₁-C₆)alkyl, -(C₃-C₇)cycloalkyl and aryl.

5. The compound according to any preceding claim having the Formula (II): 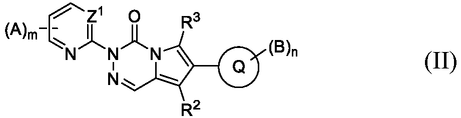
a pharmaceutically acceptable acid or base addition salt thereof, a stereochemically isomeric form thereof or an *N*-oxide form thereof, wherein Z¹ is selected from C or N; or having the Formula (III): 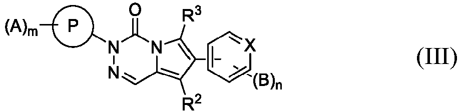
a pharmaceutically acceptable acid or base addition salt thereof, a stereochemically isomeric form thereof or an *N*-oxide form thereof, wherein X is C or N.

6. The compound according to any preceding claim having the Formula (IV): 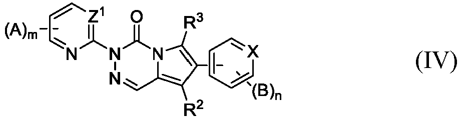
a pharmaceutically acceptable acid or base addition salt thereof, a stereochemically isomeric form thereof or an *N*-oxide form thereof, wherein Z¹ is selected from C or N, and X is selected from C or N, for example having the Formula (V): 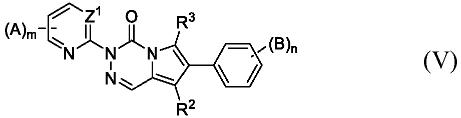
a pharmaceutically acceptable acid or base addition salt thereof, a stereochemically isomeric form thereof or an *N*-oxide form thereof; for example having the Formula (VI): 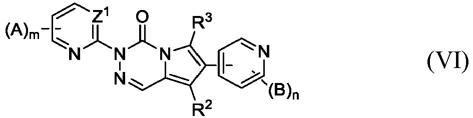
a pharmaceutically acceptable acid or base addition salt thereof, a stereochemically isomeric form thereof or an *N*-oxide form thereof.

7. The compound according to any preceding claim wherein:
R² is hydrogen or methyl;
R³ is methyl or hydrogen;
the or each (A)ₘ is independently selected from the group of hydrogen, halogen, -CF₃ and an optionally substituted radical selected from the group of -(C₁-C₆)alkyl, -(C₁-C₆)haloalkyl, -(C₃-C₇)cycloalkyl, heterocycle, -(C₀-C₆)alkylene-OR⁴, -O-(C₂-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-C(=O)-NR⁴R⁵, - (C₀-C₆)alkylene-NR⁴C(=O)-R⁵, -(C₀-C₆)alkylene-C(=O)-OR⁴, -(C₀-C₆)alkylene-C(=O)-R⁴, -C(=O)-(C₁-C₆)alkylene-NR⁴-C(=O)-OR⁵ and -NR⁴-(C₀-C₆)alkylene-C(=O)-OR⁵;
R⁴, R⁵ and R⁶ are each independently hydrogen, -(C₁-C₆)alkyl, -(C₁-C₆)haloalkyl or - C(=O)-O-(C₁-C₆)alkyl;
the or each (B)ₙ is independently selected from the group of hydrogen, halogen and an optionally substituted radical selected from the group of -(C₁-C₆)alkyl, heterocycle, -(C₀-C₆)alkylene-OR⁸, -NR⁸(C₂-C₆)alkylene-OR⁹, -(C₀-C₆)alkylene-NR⁸R⁹, -(C₀-C₆)alkylene-C(=O)-OR⁸ and -(C₀-C₆)alkylene-C(=O)-R⁸; and
R⁸ and R⁹ are each independently hydrogen -(C₁-C₆)alkyl, -(C₃-C₇)cycloalkyl or -(C₁-C₆)haloalkyl.

8. The compound according to any preceding claim having the Formula (VII): 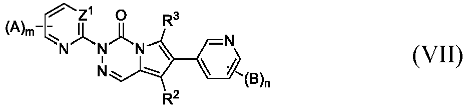 a pharmaceutically acceptable acid or base addition salt thereof, a stereochemically isomeric form thereof or an *N*-oxide form thereof wherein:
Z¹ is selected from C or N;
R² is hydrogen or methyl;
R³ is methyl or hydrogen;
the or each (A)ₘ is independently selected from the group of hydrogen, halogen, and an optionally substituted radical selected from the group of -(C₁-C₆)alkyl, heterocycle, -(C₀-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁴-(C₂-C₆)alkylene-NR⁵R^{6,} -(C₀-C₆)alkylene-C(=O)-OR⁴ and - NR⁴-(C₀-C₆)alkylene-C(=O)-OR⁵;
R⁴, R⁵ and R⁶ are each independently hydrogen, -(C₁-C₆)alkyl, -(C₁-C₆)haloalkyl or - C(=O)-O-(C₁-C₆)alkyl;
the or each (B)ₙ is independently selected from the group of hydrogen, halogen and an optionally substituted radical selected from the group of -(C₁-C₆)alkyl, heterocycle and - (C₀-C₆)alkylene-OR⁸; and
R⁸ is hydrogen -(C₁-C₆)alkyl, -(C₃-C₇)cycloalkyl or -(C₁-C₆)haloalkyl; or having the Formula (VIII): 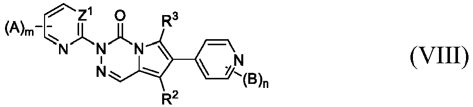
a pharmaceutically acceptable acid or base addition salt thereof, a stereochemically isomeric form thereof or an *N*-oxide form thereof wherein:
Z¹ is selected from C or N;
R² is hydrogen or methyl;
R³ is methyl or hydrogen;
the or each (A)ₘ is independently selected from the group of hydrogen, halogen, and an optionally substituted radical selected from the group of -(C₁-C₆)alkyl, heterocycle, -(C₀-C₆)alkylene-OR⁴, -NR⁴(C₂-C₆)alkylene-OR⁵, -(C₀-C₆)alkylene-NR⁴R⁵, -O-(C₂-C₆)alkylene-NR⁴R⁵, -NR⁴-(C₂-C₆)alkylene-NR⁵R⁶, -(C₀-C₆)alkylene-C(=O)-OR⁴ and - NR⁴-(C₀-C₆)alkylene-C(=O)-OR⁵;
R⁴, R⁵ and R⁶ are each independently hydrogen, -(C₁-C₆)alkyl, -(C₁-C₆)haloalkyl or - C(=O)-O-(C₁-C₆)alkyl;
the or each (B)ₙ is independently selected from the group of hydrogen, halogen and an optionally substituted radical selected from the group of -(C₁-C₆)alkyl, heterocycle and - (C₀-C₆)alkylene-OR⁸; and
R⁸ is hydrogen, -(C₁-C₆)alkyl, -(C₃-C₇)cycloalkyl or -(C₁-C₆)haloalkyl.

9. The compound according to any preceding claim wherein the or each (A)ₘ is independently selected from the group of hydrogen, halogen, and an optionally substituted radical selected from the group of heterocycle, -(C₀-C₆)alkylene-OR⁴, -(C₀-C₆)alkylene-C(=O)-OR⁴, -NR⁴-(C₀-C₆)alkylene-C(=O)-OR⁵, -NR⁴(C₂-C₆)alkylene-OR⁵ , -O-(C₂-C₆)alkylene-NR⁴R⁵, -NR⁴-(C₂-C₆)alkylene-NR⁵R⁶ and -(C₀-C₆)alkylene-NR⁴R⁵; and R⁴, R⁵ and R⁶ are each independently hydrogen, -(C₁-C₆)alkyl, -(C₁-C₆)haloalkyl, or -C(=O)-O-(C₁-C₆)alkyl; and/or wherein the or each (B)ₙ is independently selected from the group of hydrogen, halogen and an optionally substituted radical selected from the group of -(C₁-C₆)alkyl, heterocycle and -(C₀-C₆)alkylene-OR⁸; and R⁸ is hydrogen, -(C₁-C₆)alkyl, -(C₃-C₇)cycloalkyl or -(C₁-C₆)haloalkyl; and/or wherein the or each (A)ₘ is independently selected from the group of hydrogen, halogen, -CH₂CH₂OH, - COOCH₂CH₃, -COH(CH₃)₂, -O-methyl, -N(COOBu^{t})₂, NHCOOBu^{t}, morpholinyl, -NH₂, -NHCH₂CH₂OCH₃, -NHCH₂CH₂N(CH₃)₂, -OCHF₂, -OCH₂CH₂N(CH₃)₂, CHOH(CH₃)₂, methyl and hydroxy substituted pyrrolidinyl; and/or the or each (B)ₙ is independently selected from the group of hydrogen, halogen, azetidinyl, -OCHF₂, -OCF₃, cyclopropyl, -O-cyclopropyl, -O-methyl, methyl, propyl, -O-cyclobutyl and azabicyclo[2.2.1]heptan-7-yl.

10. The compound according to claims 1 to 9, wherein the compound can exist as optical isomers, and wherein the compound is either a racemic mixture or one or both of the individual optical isomers.

11. The compound according to claims 1 to 10, wherein said compound is one or more selected from: 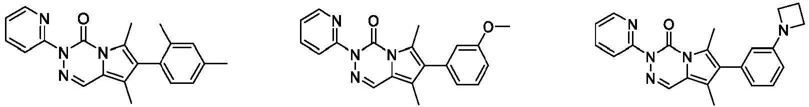 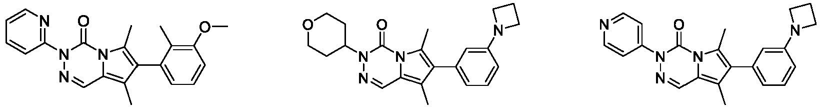 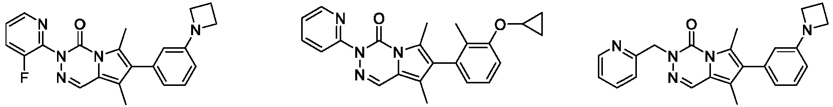 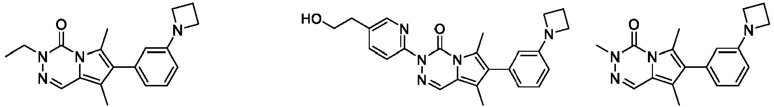 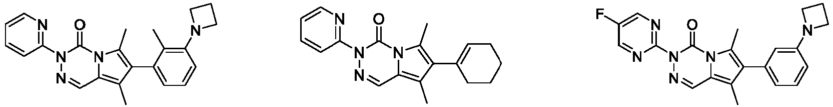 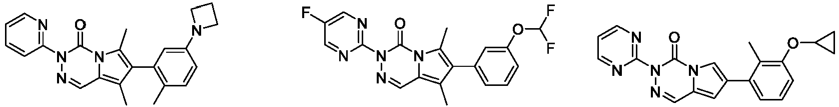 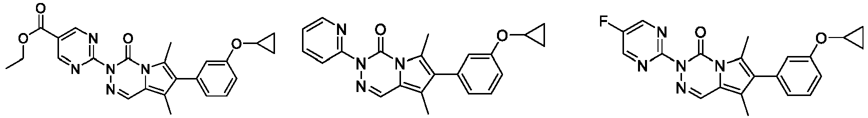 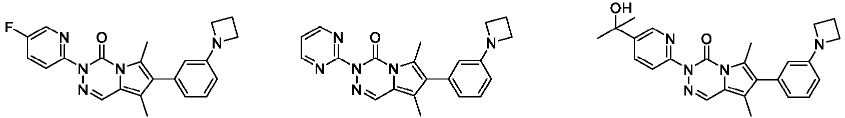 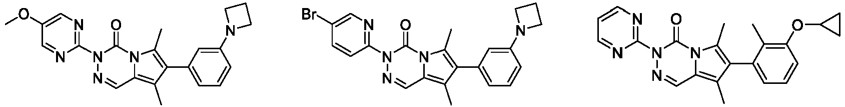 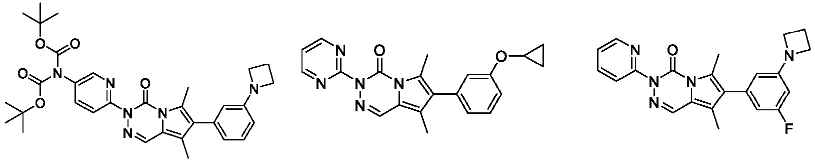 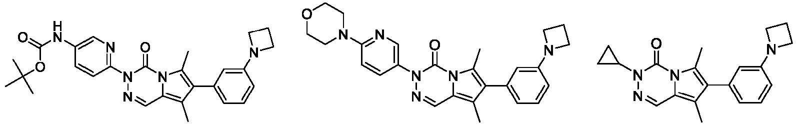 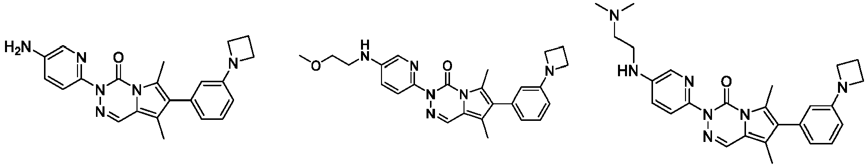 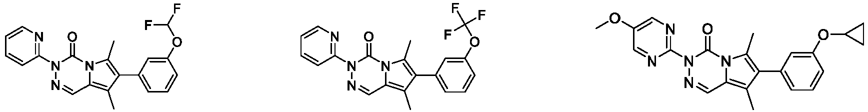 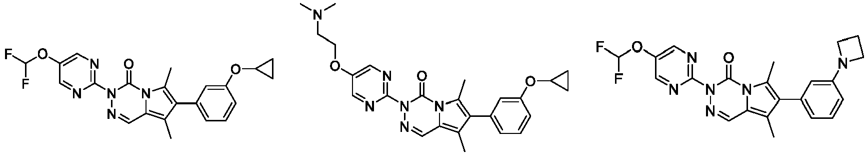 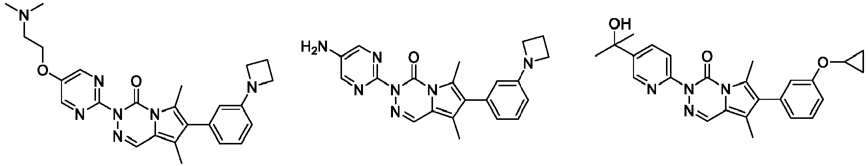 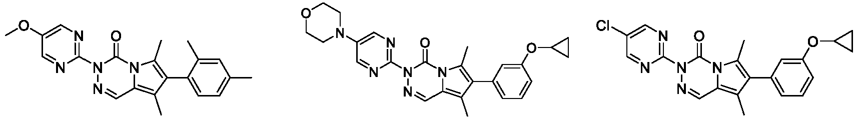 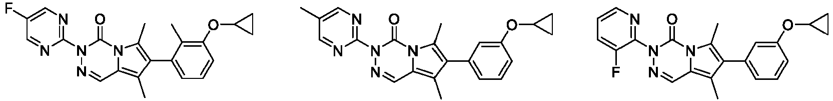 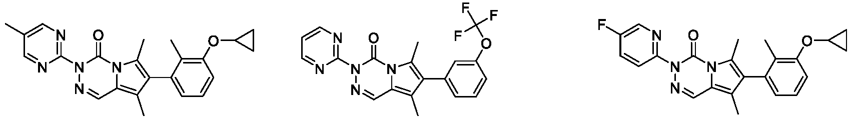 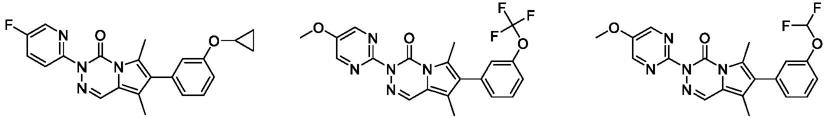 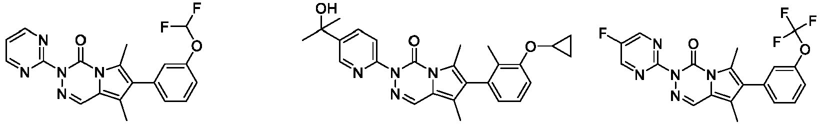 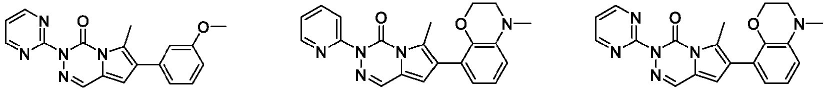 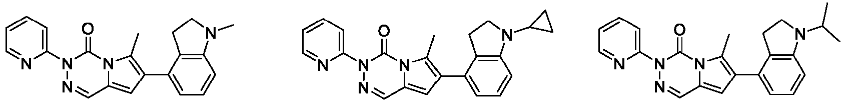 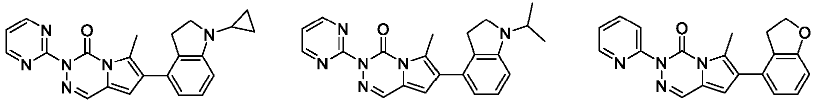 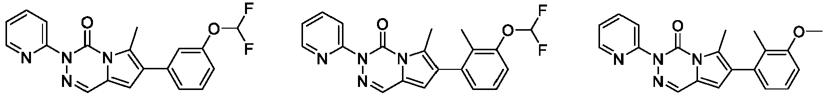 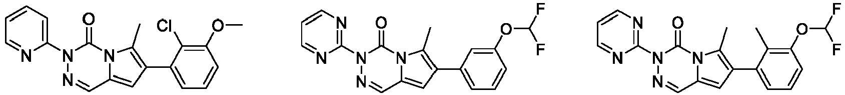 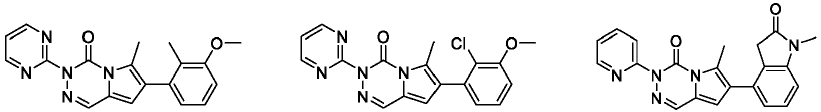 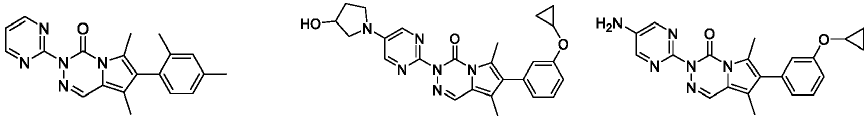 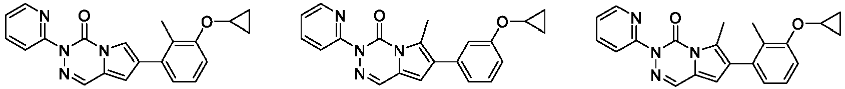 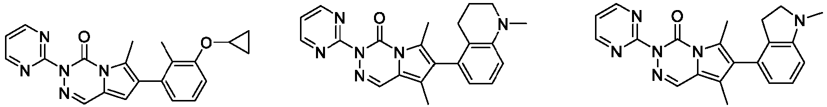 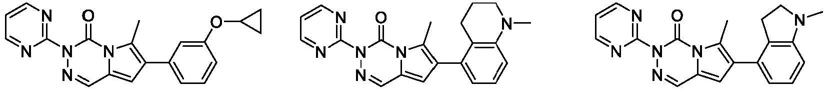 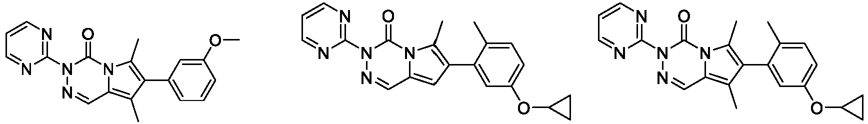 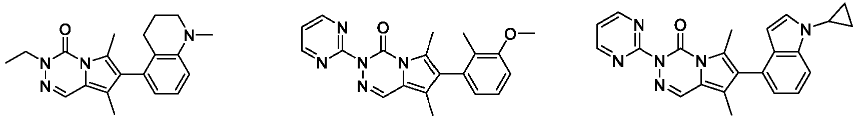 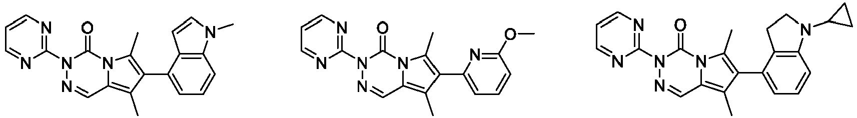 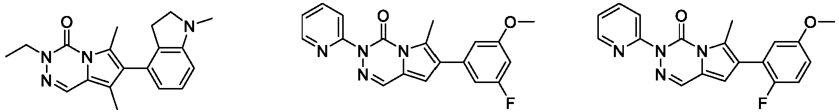 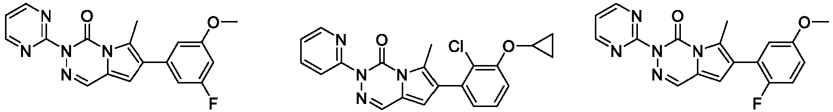 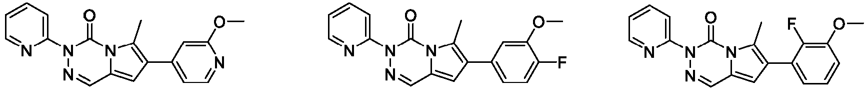 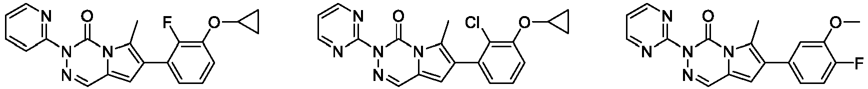 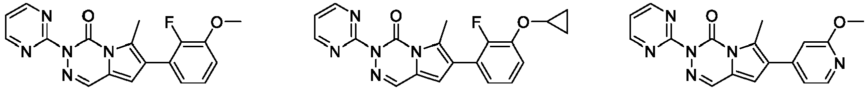 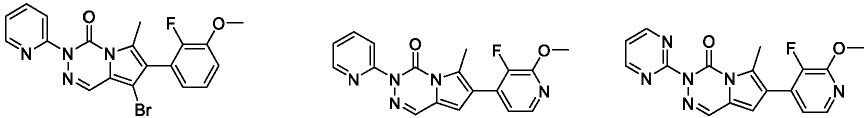 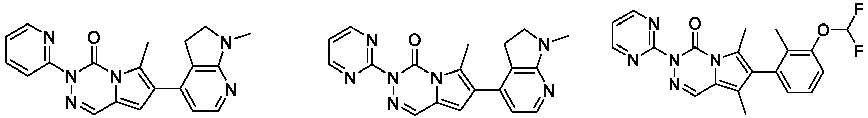 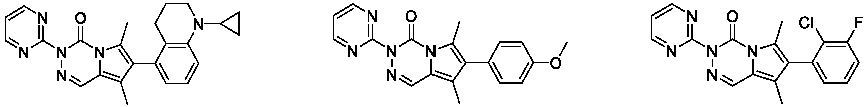 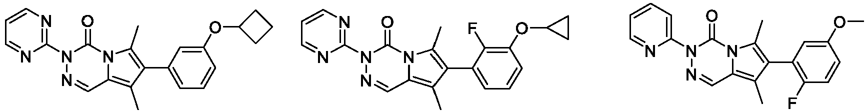 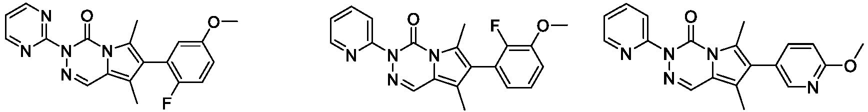 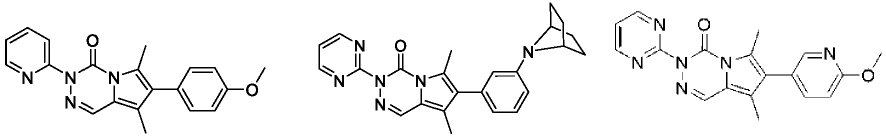 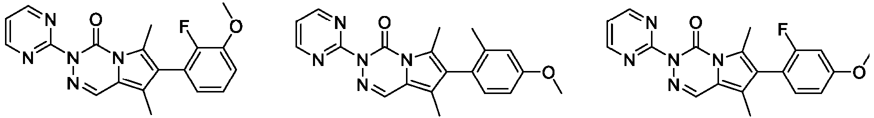 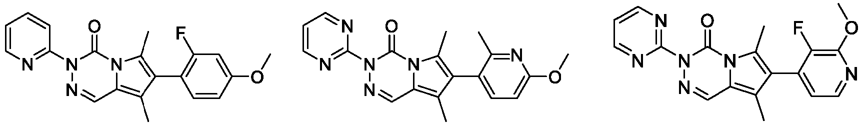 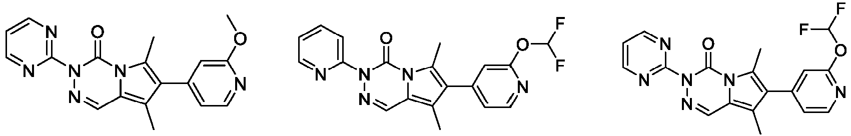 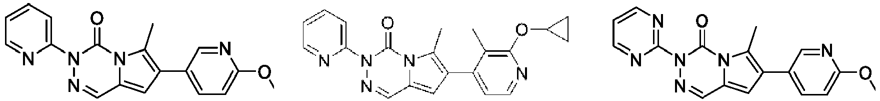 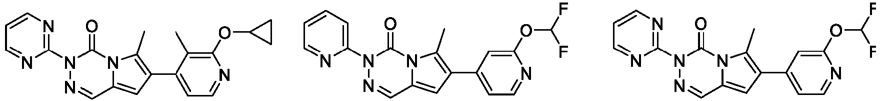 and a pharmaceutically acceptable acid or base addition salt thereof, a stereochemically isomeric form thereof or an *N*-oxide form thereof.

12. A pharmaceutical composition comprising a therapeutically effective amount of a compound according to any one of claims 1 to 11 and a pharmaceutically acceptable carrier and/or excipient.

13. A compound or composition according to any one of claims 1 to 12 for use in the treatment or prevention of a condition in a mammal affected or facilitated by the modulatory effect of a mGlu7 allosteric modulator, such as a mGlu7 negative allosteric modulator; or for use in treating, preventing, ameliorating, controlling or reducing the risk of various neurological and psychiatric disorders associated with glutamate dysfunction in a mammal, comprising administering to a mammal in need of such treatment or prevention, an effective amount of the compound/composition; for example wherein the treatment or prevention is affected or facilitated by the modulatory effect of a mGlu7 negative allosteric modulator.

14. A compound or composition for use according to claim 13, wherein the condition is one or more of a central nervous system disorder, an otic disease or disorder or a pain disorder; for example
wherein the central nervous disorder is an anxiety disorder such as agoraphobia, generalized anxiety disorder (GAD), obsessive-compulsive disorder (OCD), panic disorder, or post-traumatic stress disorder (PTSD); for example
wherein the central nervous system disorder is a psychotic disorder selected from the group of schizophrenia, delusional disorder, schizoaffective disorder, schizophreniform disorder and substance induced psychotic disorder; for example
wherein the otic disease and disorder is one or more of an inner ear impairment, age-related hearing impairment (presbycusis), Meniere's disease, sudden hearing loss, noise induced hearing loss, otitis media, autoimmune inner ear disease, acute tinnitus, chronic tinnitus, drug-induced hearing loss, hidden hearing loss, cisplatin-induced hearing loss, aminoglycosides-induced hearing loss, ototoxicity, central auditory processing disorder or vestibular disorder; for example
wherein the pain disorder is one or more of neuropathic pain, inflammatory pain, visceral pain, acute pain, chronic pain, severe pain, intractable pain, post-traumatic pain, postoperative pain, headache pain or cancer pain.

15. A compound or composition according to any one of claims 1 to 12 for use as a medicament.

## Patentansprüche

1. Verbindung mit der Formel (I): 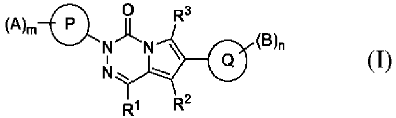 ein pharmazeutisch verträgliches Säure- oder Basenadditionssalz davon, eine stereochemisch isomere Form davon oder eine *N*-Oxid-Form davon, wobei:
R¹ ausgewählt ist aus der Gruppe von Wasserstoff, -CH₃ und -CF₃;
R² und R³ jeweils unabhängig voneinander ausgewählt sind aus der Gruppe von Wasserstoff, Halogen, -(C₁-C₆)-Alkyl, -(C₁-C₆)-Haloalkyl und -CF₃;
P für ein -(C₁-C₆)-Alkyl, oder ein Cycloalkyl, Aryl, Heteroaryl, -(C₁-C₆)-Alkylen-Heteroaryl oder Heterocyclus der folgenden Formel steht: 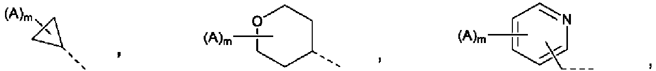 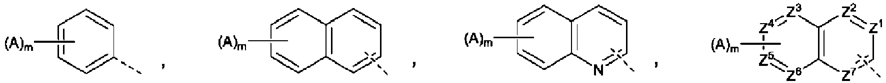 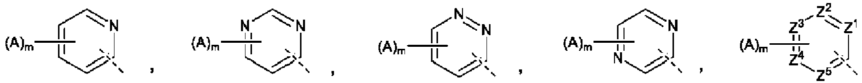 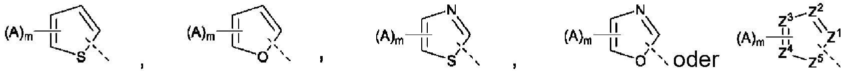
wobei jeder Cycloalkyl-, Aryl-, Heteroaryl-, -(C₁-C₆)-Alkylen-Heteroaryl- oder Heterocyclusring wahlweise mit m Resten A substituiert ist, wobei m eine ganze Zahl gleich Null, 1, 2, 3 oder 4 ist;
wobei Z¹, Z², Z³, Z⁴, Z⁵, Z⁶ und Z⁷ jeweils unabhängig voneinander ausgewählt sind aus C, N, O oder S; vorausgesetzt, dass mindestens eines von Z¹, Z², Z³, Z⁴, Z⁵, Z⁶ und Z⁷ N ist; wobei das oder jedes (A)ₘ unabhängig voneinander ausgewählt ist aus der Gruppe von Wasserstoff, Halogen, -CN, -OH, -NO₂, -CF₃, -SH, -NH₂ und einem wahlweise substituierten Rest, ausgewählt aus der Gruppe -(C₁-C₆)-Alkyl, -(C₁-C₆)-Haloalkyl, -(C₂-C₆)-Alkynyl, -(C₂-C₆)-Alkenyl, -(C₃-C₇)-Cycloalkyl, -(C₁-C₆)-Alkylen-(C₃-C₇)-Cycloalkyl, -(C₃-C₈)-Cycloalkenyl, -(C₁-C₆)-Cyanoalkyl, -(C₁-C₆)-Alkylen-Heteroaryl, -(C₁-C₆)-Alkylen-Aryl, Aryl, Heteroaryl, - (C₁-C₆)-Alkylen-Heterocyclus, Heterocyclus, -(C₀-C₆)-Alkylen-OR⁴, -O-(C₂-C₆)-Alkylen-OR⁴, -NR⁴(C₂-C₆)-Alkylene-OR⁵, -(C₃-C₆)-Alkynylen-OR⁴, -(C₃-C₆)-Alkynylen-NR⁴R⁵, -(C₃-C₆)-Alkenylen-OR⁴, -(C₃-C₆)-Alkenylen-NR⁴R⁵, -(C₀-C₆)-Alkylen-S-R⁴, -O-(C₂-C₆)-Alkylen-S-R⁴, -NR⁴-(C₂-C₆)-Alkylen-S-R⁵, -(C₀-C₆)-Alkylen-S(=O)-R⁴, -O-(C₁-C₆)-Alkylen-S(=O)-R⁴, -NR⁴-(C₁-C₆)-Alkylen-S(=O)-R⁵, -(C₀-C₆)-Alkylen-S(=O)₂-R⁴, - O-(C₁-C₆)-Alkylen-S(=O)₂-R⁴, - NR⁴-(C₁-C₆)-Alkylen-S(=O)₂-R⁵, -(C₀-C₆)-Alkylen-NR⁴R⁵, -O-(C₂-C₆)-Alkylen-NR⁴R⁵, -NR⁴-(C₂-C₆)-Alkylen-NR⁵R⁶, -(C₀-C₆)-Alkylen-S(=O)₂NR⁴R⁵, -O-(C₁-C₆)-Alkylen-S(=O)₂NR⁴R⁵, - NR⁴-(C₁-C₆)-Alkylen-S(=O)₂NR⁵R⁶, -(C₀-C₆)-Alkylen-NR⁴-S(=O)₂R⁵, -O-(C₂-C₆)-Alkylen-NR⁴-S(=O)₂R⁵, -NR⁴-(C₂-C₆)-Alkylen-NR⁵-S(=O)₂R⁶, -(C₀-C₆)-Alkylen-C(=O)-NR⁴R⁵, -O-(C₁-C₆)-Alkylen- C(=O)-NR⁴R⁵, -NR⁴-(C₁-C₆)-Alkylen-C(=O)-NR⁵R⁶, -(C₀-C₆)-Alkylen-NR⁴C(=O)-R⁵, - O-(C₂-C₆)-Alkylen-NR⁴C(=O)-R⁵, -NR⁴-(C₂-C₆)-Alkylen-NR⁵C(=O)-R⁶, -(C₀-C₆)-Alkylen-OC(=O)-R⁴, -O-(C₂-C₆)-Alkylen-OC(=O)-R⁴, -NR⁴-(C₂-C₆)-Alkylen-OC(=O)-R⁵, - (C₀-C₆)-Alkylen-C(=O)-OR⁴, -O-(C₁-C₆)-Alkylen-C(=O)-OR⁴, -NR⁴-(C₀-C₆)-Alkylen-C(=O)-OR⁵, -(C₀-C₆)-Alkylen-C(=O)-R⁴, -O-(C₁-C₆)-Alkylen-C(=O)-R⁴, - NR⁴-(C₁-C₆)-Alkylen-C(=O)-R⁵, -(C₀-C₆)-Alkylen-NR⁴-C(=O)-OR⁵, -C(=O)-(C₁-C₆)-Alkylen-NR⁴-C(=O)-OR⁵, -(C₀-C₆)-Alkylen-O-C(=O)-NR⁴R⁵, -(C₀-C₆)-Alkylen-NR⁴-C(=O)-NR⁵R⁶, -O-(C₂-C₆)-Alkylen-NR⁴-C(=O)-NR⁵R⁶, -NR⁴-(C₂-C₆)-Alkylen-NR⁵-C(=O)-NR⁶R⁷, -(C₀-C₆)-Alkylen-NR⁴-C(=S)-NR⁵R⁶ und -(C₀-C₆)-Alkylen-NR⁴-C(=NR⁵)-NR⁶R⁷;
R⁴, R⁵, R⁶ und R⁷ jeweils unabhängig voneinander Wasserstoff oder ein wahlweise substituierter Rest sind, ausgewählt aus der Gruppe von -(C₁-C₆)-Haloalkyl, -(C₁-C₆)-Alkyl, -(C₁-C₆)-Cyanoalkyl, -(C₃-C₇)-Cycloalkyl, -(C₁-C₆)-Alkylen-(C₃-C₇)-Cycloalkyl, Heteroaryl, - (C₁-C₆)-Alkylen-Heteroaryl, Aryl, -(C₁-C₆)-Alkylen-Heterocyclus, Heterocyclus, -(C₁-C₆)-Alkylen-Aryl, -(C₀-C₆)-Alkylen-O-(C₀-C₆)-Alkyl, -(C₀-C₆)-Alkylen-N-((C₀-C₆)-Alkyl)₂ und - C(=O)-O-(C₁-C₆)-Alkyl;
Q für ein Aryl, Heteroaryl oder -(C₅-C₇)-Cycloalkenyl der folgenden Formel steht: 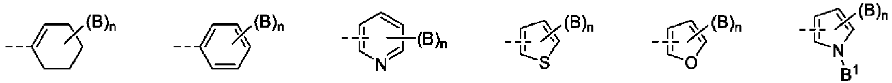 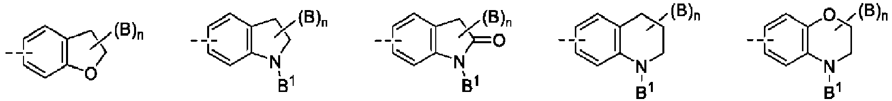 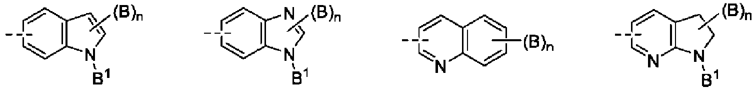
wobei jeder Aryl-, Heteroaryl- oder -(C₅-C₇)-Cycloalkenylring wahlweise mit n Resten B substituiert ist, wobei n eine ganze Zahl gleich Null, 1, 2, 3, 4 oder 5 ist; wobei B1 ein Rest B ist;
wobei das oder jedes (B)ₙ unabhängig ausgewählt ist aus der Gruppe von Wasserstoff, Halogen, -CN, - OH, -NO₂, -CF₃, -SH, -NH₂ und einem wahlweise substituierten Rest, ausgewählt aus der Gruppe -(C₁-C₆)-Alkyl, -(C₁-C₆)-Haloalkyl, -(C₂-C₆)-Alkynyl, -(C₂-C₆)-Alkenyl, -(C₃-C₇)-Cycloalkyl, -(C₁-C₆)-Alkylene-(C₃-C₇)-Cycloalkyl, -(C₃-C₈)-Cycloalkenyl, - (C₁-C₆)-Cyanoalkyl, -(C₁-C₆)-Alkylen-Heteroaryl, -(C₁-C₆)-Alkylen-Aryl, Aryl, Heteroaryl, - (C₁-C₆)-Alkylene-Heterocyclus, Heterocyclus, -(C₀-C₆)-Alkylen-OR⁸, -O-(C₂-C₆)-Alkylen-OR⁸, -NR⁸(C₂-C₆)-Alkylen-OR⁹, -(C₃-C₆)-Alkynylen-OR⁸, -(C₃-C₆)-Alkynylen-NR⁸R⁹, -(C₃-C₆)-Alkenylen-OR⁸, -(C₃-C₆)-Alkenylen-NR⁸R⁹, -(C₀-C₆)-Alkylen-S-R⁸, -O-(C₂-C₆)-Alkylen-S-R⁸, -NR⁸-(C₂-C₆)-Alkylen-S-R⁹, -(C₀-C₆)-Alkylen-S(=O) -R⁸, -O-(C₁-C₆)-Alkylen-S(=O) -R⁸, -NR⁸-(C₁-C₆)-Alkylen-S(=O) -R⁹, -(C₀-C₆)-Alkylen-S(=O) ₂-R⁸, -O-(C₁-C₆)-Alkylen-S(=O)₂-R⁸, -NR⁸-(C₁-C₆)-Alkylen-S(=O)₂-R⁹, -(C₀-C₆)-Alkylen-NR⁸R⁹, -O-(C₂-C₆)-Alkylen-NR⁸R⁹, - NR⁸-(C₂-C₆)-Alkylen-NR⁹R¹⁰, -(C₀-C₆)-Alkylen-S(=O)₂NR⁸R⁹, -O-(C₁-C₆)-Alkylen-S(=O)₂NR⁸R⁹, -NR⁸-(C₁-C₆)-Alkylen-S(=O)₂ NR⁹R¹⁰, -(C₀-C₆)-Alkylen-NR⁸-S(=O)₂R⁹, -O-(C₂-C₆)-Alkylen-NR⁸-S(=O)₂R⁹, -NR⁸-(C₂-C₆)-Alkylen-NR⁹-S(=O)₂R¹⁰, -(C₀-C₆)-Alkylen-C(=O) -NR⁸R⁹, -O-(C₁-C₆)-Alkylen-C(=O)-NR⁸R⁹, -NR⁸-(C₁-C₆)-Alkylen-C(=O)-NR⁹R¹⁰, - (C₀-C₆)-Alkylen-NR⁸C(=O)-R⁹, -O-(C₂-C₆)-Alkylen-NR⁸C(=O)-R⁹, -NR⁸-(C₂-C₆)-Alkylen-NR⁹C(=O)-R¹⁰, -(C₀-C₆)-Alkylen-OC(=O)-R⁸, -O-(C₂-C₆)-Alkylen-OC(=O)-R⁸, -NR⁸-(C₂-C₆)-Alkylen-OC(=O)-R⁹, -(C₀-C₆)-Alkylen-C(=O)-OR⁸, -O-(C₁-C₆)-Alkylen-C(=O)-OR⁸, -NR⁸-(C₁-C₆)-Alkylen-C(=O)-OR⁹, -(C₀-C₆)-Alkylen-C(=O)-R⁸, -O-(C₁-C₆)-Alkylene-C(=O)-R⁸, -NR⁸-(C₁-C₆)-Alkylen-C(=O)-R⁹,-(C₀-C₆)-Alkylen-NR⁸-C(=O)-OR⁹, -(C₀-C₆)-Alkylen-O-C(=O)-NR⁸R⁹, -(C₀-C₆)-Alkylen-NR⁸-C(=O)-NR⁹R¹⁰, -O-(C₂-C₆)-Alkylen-NR⁸-C(=O)-NR⁹R¹⁰, -NR⁸-(C₂-C₆)-Alkylen-NR⁹-C(=O)-NR¹⁰R¹¹, -(C₀-C₆)-Alkylen-NR⁸-C(=S)-NR⁹R¹⁰ und -(C₀-C₆)-Alkylen-NR⁸-C(=NR⁹)-NR¹⁰R¹¹;
R⁸, R⁹, R¹⁰ und R¹¹ jeweils unabhängig voneinander Wasserstoff oder ein wahlweise substituierter Rest sind, ausgewählt aus der Gruppe von -(C₁-C₆)-Haloalkyl, -(C₁-C₆)-Alkyl, -(C₁-C₆)-Cyanoalkyl, -(C₃-C₇)-Cycloalkyl, -(C₁-C₆)-Alkylen-(C₃-C₇)-Cycloalkyl, Heteroaryl, - (C₁-C₆)-Alkylen-Heteroaryl, Aryl, -(C₁-C₆)-Alkylen-Heterocyclus, Heterocyclus, -(C₁-C₆)-Alkylen-Aryl- -(C₀-C₆)-Alkylen-O-(C₀-C₆)Alkyl und -(C₀-C₆)-Alkylen-N-((C₀-C₆)-Alkyl)₂;
wobei wahlweise zwei beliebige Reste A mit den dazwischenliegenden Atomen einen 3- bis 10-gliedrigen bizyklischen Heterocyclus, einen Aryl- oder Heteroarylring bilden, wobei jeder Ring wahlweise weiter mit 1 bis 5 Resten substituiert ist, die unabhängig voneinander ausgewählt sind aus der Gruppe von Halogen, -CN, Nitro, -(C₁-C₆)-Alkyl, -(C₃-C₇)-Alkyl, - (C₀-C₆)-Alkylen-O-(C₀-C₆)-Alkyl und -(C₀-C₆)-Alkylen-N-((C₀-C₆)-Alkyl)₂;
wobei wahlweise zwei der Substituenten R⁴, R⁵, R⁶ oder R⁷ mit den dazwischenliegenden Atomen einen 3- bis 10-gliedrigen Heterocyclus, Aryl- oder Heteroarylring bilden, wobei jeder Ring wahlweise weiter mit 1 bis 5 Resten substituiert ist, die unabhängig voneinander ausgewählt sind aus der Gruppe von Halogen, Cyano, Nitro, -(C₁-C₆)-Alkyl, -(C₃-C₇)-Alkyl, -(C₀-C₆)-Alkylen-O-(C₀-C₆)-Alkyl und -(C₀-C₆)-Alkylen-N-((C₀-C₆)-Alkyl)₂;
wobei wahlweise zwei Substituenten aus R⁸, R⁹, R¹⁰ oder R¹¹ mit den dazwischenliegenden Atomen einen 3- bis 10-gliedrigen Heterocyclus, Aryl- oder Heteroarylring bilden, wobei jeder Ring wahlweise weiter mit 1 bis 5 Resten substituiert ist, die unabhängig voneinander ausgewählt sind aus der Gruppe von Halogen, Cyano, Nitro, -(C₁-C₆)-Alkyl, -(C₃-C₇)-Alkyl, -(C₀-C₆)-Alkylen-O-(C₀-C₆)-Alkyl und -(C₀-C₆)-Alkylen-N-((C₀-C₆)-Alkyl)₂; und
wobei wahlweise zwei beliebige Reste B mit den dazwischenliegenden Atomen einen 3- bis 10-gliedrigen bizyklischen Heterocyclus, einen Aryl- oder Heteroarylring bilden, wobei jeder Ring wahlweise weiter mit 1 bis 5 Resten substituiert ist, die unabhängig voneinander ausgewählt sind aus der Gruppe von Halogen, -CN, Nitro, -(C₁-C₆)-Alkyl, -(C₃-C₇)-Alkyl, - (C₀-C₆)-Alkylen-O-(C₀-C₆)-Alkyl und -(C₀-C₆)-Alkylen-N-((C₀-C₆)-Alkyl)₂.

2. Verbindung nach Anspruch 1 mit der Formel (I), wobei P ein -(C₁-C₆)-Alkyl oder ein Cycloalkyl, Heteroaryl, -(C₁-C₆)-Alkylen-Heteroaryl oder einen Heterocyclus der folgenden Formel darstellt: 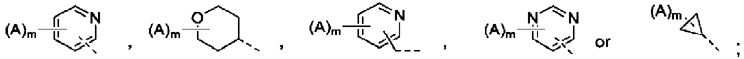 und/oder
wobei Q ein Aryl, Heteroaryl oder -(C5-C7)-Cycloalkenyl der folgenden Formel darstellt:

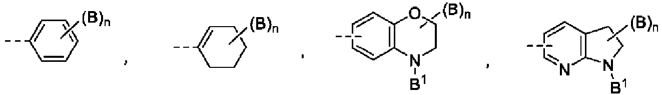

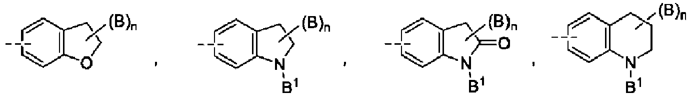

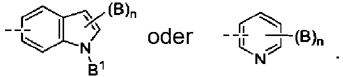

3. Verbindung nach einem vorstehenden Anspruch mit der Formel (I), wobei P ein Heteroaryl der folgenden Formel darstellt: 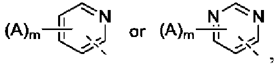
wobei jeder Rest wahlweise mit m Resten A substituiert ist, wobei m eine ganze Zahl gleich Null, 1, 2, 3 oder 4 ist; und/oder wobei Q eine Aryl- oder Heteroarylgruppe der folgenden Formel darstellt: 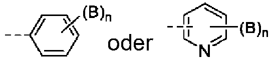
wobei jeder Rest wahlweise mit n Resten B substituiert ist, wobei n eine ganze Zahl gleich Null, 1, 2, 3, 4 oder 5 ist; und/oder wobei:
die Cycloalkyl-, Heterocyclus-, Aryl- und Heteroaryl-Ringsysteme von (A)ₘ ausgewählt sind aus der Gruppe von Azetidinyl, 2-Azabicyclo[2.2.1]heptan-2-yl, 7-Azabicyclo[2.2.1]heptan-7-yl, Benzimidazolyl, Benzisothiazolyl, Benzisoxazolyl, Benzofuryl, Benzopyrazolyl, Benzothiazolyl, Benzothiophenyl, Benzotriazolyl, Benzoxazolyl, Dihydrofuranyl, Dihydrothienyl, Dioxolanyl, 1,1-Dioxo-thiomorpholinyl, Furazanyl, Furyl, Imidazolidinyl, Imidazolinyl, Imidazolonyl, Imidazolyl, Imidazopyridazinyl, Imidazopyridyl, Indolyl, Isoindolyl, Isochinolinyl, Isothiazolinyl, Isothiazolyl, Isoxazolidinyl, Isoxazolinyl, Isoxazolyl, Morpholinyl, Naphthyl, Naphthyridinyl, Oxadiazolyl, Oxazolidinyl, Oxazolinyl, Oxazolonyl, Oxazolopyridazinyl, Oxazolopyridyl, Oxazolyl, Oxetanyl, Phenyl, Piperazinonyl, Piperazinyl, Piperidinonyl, Piperidinyl, Phthalazinyl, Pteridinyl, Purinyl, Pyranyl, Pyrazinyl, Pyrazolopyridinyl, Pyrazolyl, Pyridazinyl, Pyridonyl, Pyridyl, Pyrimidyl, Pyrrolidinonyl, Pyrrolidinyl, Pyrrolinyl, Pyrrolyl, Chinazolyl, Chinolyl, Chinoxalinyl, Tetrahydrofuranyl, Tetrahydropyranyl, Tetrahydrothiopyranyl, Tetrahydrotriazolopyridyl, Tetrahydrotriazolopyrimidinyl, Tetrazolyl, Thiadiazolyl, Thiazolidinyl, Thiazolinyl, Thiazolonyl, Thiazolopyridazinyl, Thiazolopyridyl, Thiazolyl, Thienyl, Thiomorpholinyl, Thionaphthyl, Thiopyranyl, Triazolinyl, Triazinyl, Triazolyl, Cyclopropyl, Cyclobutyl, Cyclopentyl, Cyclopentenyl, Cyclohexyl, Cyclohexenyl, Cycloheptyl, Cycloheptenyl, Cyclooctyl und Cyclooctenyl, und jeder Ring des Ringsystems wahlweise unabhängig mit 1 bis 4 Substituenten R⁴, R⁵, R⁶ oder R⁷ substituiert ist; und/oder wobei:
die Cycloalkyl-, Heterocyclus-, Aryl- und Heteroaryl-Ringsysteme von (B)ₙ ausgewählt sind aus der Gruppe von Azetidinyl, Benzimidazolyl, Benzisothiazolyl, Benzisoxazolyl, Benzofuryl, Benzopyrazolyl, Benzothiazolyl, Benzothiophenyl, Benzotriazolyl, Benzoxazolyl, Dihydrofuranyl, Dihydrothienyl, Dioxolanyl, 1,1-Dioxo-thiomorpholinyl, Furazanyl, Furyl, Imidazolidinyl, Imidazolinyl, Imidazolonyl, Imidazolyl, Imidazopyridazinyl, Imidazopyridyl, Indolyl, Isoindolyl, Isochinolinyl, Isothiazolinyl, Isothiazolyl, Isoxazolidinyl, Isoxazolinyl, Isoxazolyl, Morpholinyl, Naphthyl, Naphthyridinyl, Oxadiazolyl, Oxazolidinyl, Oxazolinyl, Oxazolonyl, Oxazolopyridazinyl, Oxazolopyridyl, Oxazolyl, Oxetanyl, Phenyl, Piperazinonyl, Piperazinyl, Piperidinonyl, Piperidinyl, Phthalazinyl, Pteridinyl, Purinyl, Pyranyl, Pyrazinyl, Pyrazolopyridinyl, Pyrazolyl, Pyridazinyl, Pyridonyl, Pyridyl, Pyrimidyl, Pyrrolidinonyl, Pyrrolidinyl, Pyrrolinyl, Pyrrolyl, Chinazolyl, Chinolyl, Chinoxalinyl, Tetrahydrofuranyl, Tetrahydropyranyl, Tetrahydrothiopyranyl, Tetrahydrotriazolopyridyl, Tetrahydrotriazolopyrimidinyl, Tetrazolyl, Thiadiazolyl, Thiazolidinyl, Thiazolinyl, Thiazolonyl, Thiazolopyridazinyl, Thiazolopyridyl, Thiazolyl, Thienyl, Thiomorpholinyl, Thionaphthyl, Thiopyranyl, Triazolinyl, Triazinyl, Triazolyl, Cyclopropyl, Cyclobutyl, Cyclopentyl, Cyclopentenyl, Cyclohexyl, Cyclohexenyl, Cycloheptyl, Cycloheptenyl, Cyclooctyl und Cyclooctenyl, und jeder Ring des Ringsystems wahlweise unabhängig mit 1 bis 4 Substituenten R⁸, R⁹, R¹⁰ oder R¹¹ substituiert ist; und/oder wobei:
die Cycloalkyl-, Heterocyclus-, Aryl- und Heteroaryl-Ringsysteme von R⁴, R⁵, R⁶, R⁷, R⁸, R⁹, R¹⁰ oder R¹¹ ausgewählt sind aus der Gruppe von Azetidinyl, Benzimidazolyl, Benzisothiazolyl, Benzisoxazolyl, Benzofuryl, Benzopyrazolyl, Benzothiazolyl, Benzothiophenyl, Benzotriazolyl, Benzoxazolyl, Dihydrofuranyl, Dihydrothienyl, Dioxolanyl, 1,1-Dioxo-thiomorpholinyl, Furazanyl, Furyl, Imidazolidinyl, Imidazolinyl, Imidazolonyl, Imidazolyl, Imidazopyridazinyl, Imidazopyridyl, Indolyl, Isoindolyl, Isochinolinyl, Isothiazolinyl, Isothiazolyl, Isoxazolidinyl, Isoxazolinyl, Isoxazolyl, Morpholinyl, Naphthyl, Naphthyridinyl, Oxadiazolyl, Oxazolidinyl, Oxazolinyl, Oxazolonyl, Oxazolopyridazinyl, Oxazolopyridyl, Oxazolyl, Oxetanyl, Phenyl, Piperazinonyl, Piperazinyl, Piperidinonyl, Piperidinyl, Phthalazinyl, Pteridinyl, Purinyl, Pyranyl, Pyrazinyl, Pyrazolopyridinyl, Pyrazolyl, Pyridazinyl, Pyridonyl, Pyridyl, Pyrimidyl, Pyrrolidinonyl, Pyrrolidinyl, Pyrrolinyl, Pyrrolyl, Chinazolyl, Chinolyl, Chinoxalinyl, Tetrahydrofuranyl, Tetrahydropyranyl, Tetrahydrothiopyranyl, Tetrahydrotriazolopyridyl, Tetrahydrotriazolopyrimidinyl, Tetrazolyl, Thiadiazolyl, Thiazolidinyl, Thiazolinyl, Thiazolonyl, Thiazolopyridazinyl, Thiazolopyridyl, Thiazolyl, Thienyl, Thiomorpholinyl, Thionaphthyl, Thiopyranyl, Triazolinyl, Triazinyl, Triazolyl, Cyclopropyl, Cyclobutyl, Cyclopentyl, Cyclopentenyl, Cyclohexyl, Cyclohexenyl, Cycloheptyl, Cycloheptenyl, Cyclooctyl und Cyclooctenyl, und jeder Ring des Ringsystems wahlweise mit 1-5 Resten substituiert ist, die unabhängig voneinander ausgewählt sind aus Wasserstoff, Halogen, -CN, Nitro, -(C₁-C₆)-Alkyl, -(C₀-C₆)-Alkylen-O-(C₀-C₆)-Alkyl und -(C₀-C₆)-Alkylen-N-((C₀-C₆)-Alkyl)₂.

4. Verbindung nach einem vorstehenden Anspruch mit der Formel (I), wobei R¹ Wasserstoff ist; und/oder wobei
R² und R³ jeweils unabhängig voneinander ausgewählt sind aus der Gruppe von Wasserstoff und Methyl; und/oder
wobei das oder jedes (A)ₘ unabhängig voneinander ausgewählt ist aus der Gruppe von Wasserstoff, Halogen, -CN, -OH, -CF₃ und einem wahlweise substituierten Rest, ausgewählt aus der Gruppe von -(C₁-C₆)-Alkyl, -(C₁-C₆)-Haloalkyl, -(C₃-C₇)-Cycloalkyl, -(C₁-C₆)-Cyanoalkyl, Aryl, Heterocyclus, -(C₀-C₆)-Alkylen-OR⁴, -O-(C₂-C₆)-Alkylen-OR⁴, - NR⁴(C₂-C₆)-Alkylen-OR⁵, -(C₀-C₆)-Alkylen-S(=O)₂-R⁴, -(C₀-C₆)-Alkylen-NR⁴R⁵, -O-(C₂-C₆)-Alkylen-NR⁴R⁵, -NR⁴-(C₂-C₆)-Alkylen-NR⁵R⁶, -(C₀-C₆)-Alkylen-S(=O)₂NR⁴R⁵, -(C₀-C₆)-Alkylen-C(=O)-NR⁴R⁵, -(C₀-C₆)-Alkylen-NR⁴C(=0)-R⁵, -(C₀-C₆)-Alkylen-C(=O)-OR⁴, -(C₀-C₆)-Alkylen-C(=O)-R⁴, -C(=O)-(C₁-C₆)-Alkylen-NR⁴-C(=O)-OR⁵ und -NR⁴-(C₀-C₆)-Alkylen-C(=O)-OR⁵; und/oder
wobei R⁴, R⁵ und R⁶ jeweils unabhängig voneinander Wasserstoff, -(C₁-C₆)-Alkyl, -(C₁-C₆)-Haloalkyl oder -C(=O)-O-(C₁-C₆)-Alkyl sind; und/oder
wobei das oder jedes (B)ₙ unabhängig ausgewählt ist aus der Gruppe von Wasserstoff, Halogen, -CN, -CF₃, und einem wahlweise substituierten Rest, ausgewählt aus der Gruppe -(C₁-C₆)-Alkyl, -(C₃-C₇)-Cycloalkyl, Aryl, Heteroaryl, Heterocyclus, -(C₀-C₆)-Alkylen-OR⁸, - NR⁸(C₂-C₆)-Alkylen-OR⁹, -(C₀-C₆)-Alkylen-NR⁸R⁹, -(C₀-C₆)-Alkylen-C(=O)-OR⁸ und -(C₀-C₆)-Alkylen-C(=O)-R⁸; und/oder
wobei R⁸ und R⁹ jeweils unabhängig voneinander ausgewählt sind aus der Gruppe von Wasserstoff, -(C₁-C₆)-Haloalkyl, -(C₁-C₆)-Alkyl, -(C₃-C₇)-Cycloalkyl und Aryl.

5. Verbindung nach einem vorstehenden Anspruch mit der Formel (II): 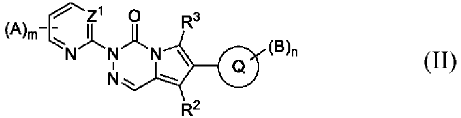
ein pharmazeutisch verträgliches Säure- oder Basenadditionssalz davon, eine stereochemisch isomere Form davon oder eine A-Oxid-Form davon, wobei Z¹ ausgewählt ist aus C oder N; oder mit der Formel (III): 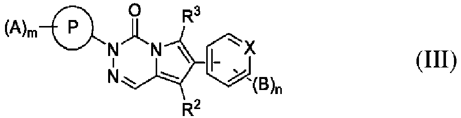
ein pharmazeutisch verträgliches Säure- oder Basenadditionssalz davon, eine stereochemisch isomere Form davon oder eine *N*-Oxid-Form davon, wobei X für C oder N steht.

6. Verbindung nach einem vorstehenden Anspruch mit der Formel (IV): 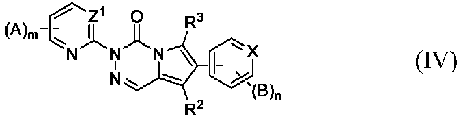
ein pharmazeutisch verträgliches Säure- oder Basenadditionssalz davon, eine stereochemisch isomere Form davon oder eine A-Oxid-Form davon, wobei Z¹ ausgewählt ist aus C oder N, und X ausgewählt ist aus C oder N, beispielsweise mit der Formel (V): 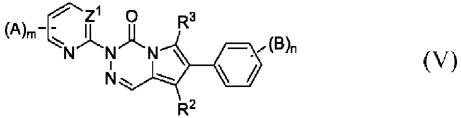
ein pharmazeutisch verträgliches Säure- oder Basenadditionssalz davon, eine stereochemisch isomere Form davon oder eine A-Oxid-Form davon; beispielsweise mit der Formel (VI): 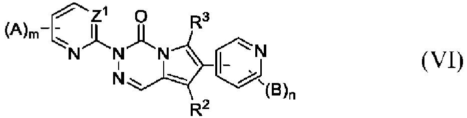
ein pharmazeutisch verträgliches Säure- oder Basenadditionssalz davon, eine stereochemisch isomere Form davon oder eine A-Oxid-Form davon.

7. Verbindung nach einem vorstehenden Anspruch, wobei:
R² Wasserstoff oder Methyl ist;
R³ Methyl oder Wasserstoff ist;
wobei das oder jedes (A)ₘ unabhängig ausgewählt ist aus der Gruppe von Wasserstoff, Halogen, -CF₃ und einem wahlweise substituierten Rest, ausgewählt aus der Gruppe von - (C₁-C₆)-Alkyl, -(C₁-C₆)-Haloalkyl, -(C₃-C₇)-Cycloalkyl, Heterocyclus, -(C₀-C₆)-Alkylen-OR⁴, - O-(C₂-C₆)-Alkylen-OR⁴ -NR⁴(C₂-C₆)-Alkylen-OR⁵, -(C₀-C₆)-Alkylen-NR⁴R⁵, -O-(C₂-C₆)-Alkylen-NR⁴R⁵, -NR⁴-(C₂-C₆)-Alkylen-NR⁵R⁶, -(C₀-C₆)-Alkylen-C(=O)-NR⁴R⁵, - (C₀-C₆)-Alkylen-NR⁴C(=O)-R⁵, -(C₀-C₆)-Alkylen-C(=O)-OR⁴, -(C₀-C₆)-Alkylen-C(=O)-R⁴, -C(=O)-(C₁-C₆)-Alkylen-NR⁴-C(=O)-OR⁵ und -NR⁴-(C₀-C₆)-Alkylen-C(=O)-OR⁵;
R⁴, R⁵ und R⁶ jeweils unabhängig voneinander Wasserstoff, -(C₁-C₆)-Alkyl, -(C₁-C₆)-Haloalkyl oder - C(=O)-O-(C₁-C₆)-Alkyl sind;
wobei das oder jedes (B)ₙ unabhängig ausgewählt ist aus der Gruppe von Wasserstoff, Halogen und einem wahlweise substituierten Rest, ausgewählt aus der Gruppe von -(C₁-C₆)-Alkyl, Heterocyclus, -(C₀-C₆)-Alkylen-OR⁸, -NR⁸(C₂-C₆)-Alkylen-OR⁹, -(C₀-C₆)-Alkylen-NR⁸R⁹, -(C₀-C₆)-Alkylen-C(=O)-OR⁸ und -(C₀-C₆)-Alkylen-C(=O)-R⁸; und
R⁸ und R⁹ jeweils unabhängig voneinander Wasserstoff, -(C₁-C₆)-Alkyl, -(C₃-C₇)-Cycloalkyl oder -(C₁-C₆)-Halogenalkyl sind.

8. Verbindung nach einem vorstehenden Anspruch mit der Formel (VII): 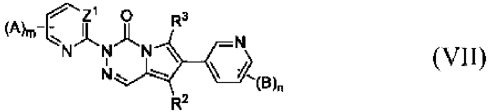 ein pharmazeutisch verträgliches Säure- oder Basenadditionssalz davon, eine stereochemisch isomere Form davon oder eine V-Oxid-Form davon, wobei:
Z¹ C oder N ist;
R2 Wasserstoff oder Methyl ist;
R3 Methyl oder Wasserstoff ist;
wobei das oder jedes (A)ₘ unabhängig ausgewählt ist aus der Gruppe von Wasserstoff, Halogen und einem wahlweise substituierten Rest, ausgewählt aus der Gruppe -(C₁-C₆)-Alkyl, Heterocyclus, -(C₀-C₆)-Alkylen-OR⁴, -NR⁴(C₂-C₆)-Alkylen-OR⁵, -(C₀-C₆)-Alkylen-NR⁴R⁵, -O-(C₂-C₆)-Alkylen-NR⁴R⁵, -NR⁴-(C₂-C₆)-Alkylen-NR⁵R⁶' -(C₀-C₆)-Alkylen-C(=O)-OR⁴ und -NR⁴-(C₀-C₆)-Alkylen-C(=O)-OR⁵;
R⁴, R⁵ und R⁶ jeweils unabhängig voneinander Wasserstoff, -(C₁-C₆)-Alkyl, -(C₁-C₆)-Haloalkyl oder -C(=O)-O-(C₁-C₆)-Alkyl sind;
wobei das oder jedes (B)ₙ unabhängig ausgewählt ist aus der Gruppe von Wasserstoff, Halogen und einem wahlweise substituierten Rest, ausgewählt aus der Gruppe von -(C₁-C₆)-Alkyl, Heterocyclus und - (C₀-C₆)-Alkylen-OR⁸; und
R8 Wasserstoff, -(C₁-C₆)-Alkyl, -(C₃-C₇)-Cycloalkyl oder -(C₁-C₆)-Halogenalkyl ist; oder mit der Formel (VIII): 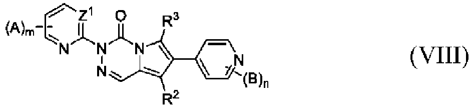
ein pharmazeutisch verträgliches Säure- oder Basenadditionssalz davon, eine stereochemisch isomere Form davon oder eine *N*-Oxid-Form davon, wobei:
Z¹ C oder N ist;
R2 Wasserstoff oder Methyl ist;
R3 Methyl oder Wasserstoff ist;
wobei das oder jedes (A)ₘ unabhängig ausgewählt ist aus der Gruppe von Wasserstoff, Halogen und einem wahlweise substituierten Rest, ausgewählt aus der Gruppe -(C₁-C₆)-Alkyl, Heterocyclus, -(C₀-C₆)-Alkylen-OR⁴, -NR⁴(C₂-C₆)-Alkylen-OR⁵, -(C₀-C₆)-Alkylen-NR⁴R⁵, -O-(C₂-C₆)-Alkylen-NR⁴R⁵, -NR⁴-(C₂-C₆)-Alkylen-NR⁵R⁶, -(C₀-C₆)-Alkylen-C(=O)-OR⁴ und -NR⁴-(C₀-C₆)-Alkylen-C(=O)-OR⁵;
R⁴, R⁵ und R⁶ jeweils unabhängig voneinander Wasserstoff, -(C₁-C₆)-Alkyl, -(C₁-C₆)-Haloalkyl oder - C(=O)-O-(C₁-C₆)-Alkyl sind;
wobei das oder jedes (B)ₙ unabhängig ausgewählt ist aus der Gruppe von Wasserstoff, Halogen und einem wahlweise substituierten Rest, ausgewählt aus der Gruppe von -(C₁-C₆)-Alkyl, Heterocyclus und -(C₀-C₆)-Alkylen-OR⁸; und
R⁸ Wasserstoff, -(C₁-C₆)-Alkyl, -(C₃-C₇)-Cycloalkyl oder-(C₁-C₆)-Halogenalkyl ist.

9. Verbindung nach einem vorstehenden Anspruch, wobei das oder jedes (A)ₘ unabhängig ausgewählt ist aus der Gruppe von Wasserstoff, Halogen und einem wahlweise substituierten Rest, ausgewählt aus der Gruppe von Heterocyclus, -(C₀-C₆)-Alkylen-OR⁴, - (C₀-C₆)-Alkylen-C(=O)-OR⁴, -NR⁴-(C₀-C₆)-Alkylen-C(=O)-OR⁵, -NR⁴(C₂-C₆)-Alkylen-OR⁵, - O-(C₂-C₆)-Alkylen-NR⁴R⁵, -NR⁴-(C₂-C₆)-Alkylen-NR⁵R⁶ und -(C₀-C₆)-Alkylen-NR⁴R⁵; und R⁴, R⁵ und R⁶ jeweils unabhängig voneinander Wasserstoff, -(C₁-C₆)-Alkyl, -(C₁-C₆)-Haloalkyl, oder -C(=O)-O-(C₁-C₆)-Alkyl sind; und/oder wobei das oder jedes (B)ₙ unabhängig ausgewählt ist aus der Gruppe von Wasserstoff, Halogen und einem wahlweise substituierten Rest, ausgewählt aus der Gruppe von -(C₁-C₆)-Alkyl, Heterocyclus und -(C₀-C₆)-Alkylen-OR⁸; und R⁸ Wasserstoff, -(C₁-C₆)-Alkyl, -(C₃-C₇)-Cycloalkyl oder -(C₁-C₆)-Haloalkyl ist; und/oder wobei das oder jedes (A)ₘ unabhängig ausgewählt ist aus der Gruppe von Wasserstoff, Halogen, -CH₂CH₂OH, -COOCH₂CH₃, -COH(CH₃)₂, -O-Methyl, - N(COOBu^{t})₂, NHCOOBu^{t}, Morpholinyl, -NH₂, -NHCH₂CH₂OCH₃, -NHCH₂CH₂N(CH₃)₂, - OCHF₂, -OCH₂CH₂N(CH₃)₂, CHOH(CH₃)₂, Methyl- und Hydroxy-substituiertes Pyrrolidinyl; und/oder wobei das oder jedes (B)ₙ unabhängig ausgewählt ist aus der Gruppe von Wasserstoff, Halogen, Azetidinyl, -OCHF₂, -OCF₃, Cyclopropyl, -O-Cyclopropyl, -O-Methyl, Methyl, Propyl, -O-Cyclobutyl und Azabicyclo[2.2.1]heptan-7-yl.

10. Verbindung nach Ansprüchen 1 bis 9, wobei die Verbindung als optische Isomere vorliegen kann, und wobei die Verbindung entweder ein racemisches Gemisch oder eines oder beide der einzelnen optischen Isomere ist.

11. Verbindung nach Ansprüchen 1 bis 10, wobei die Verbindung eines oder mehrere ist, ausgewählt aus: 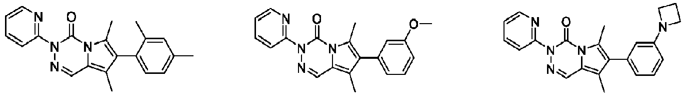 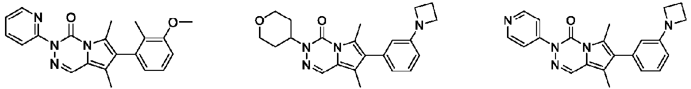 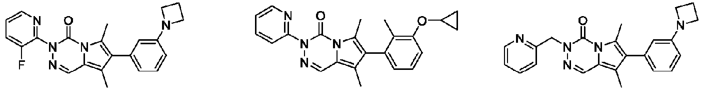 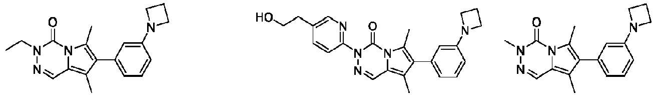 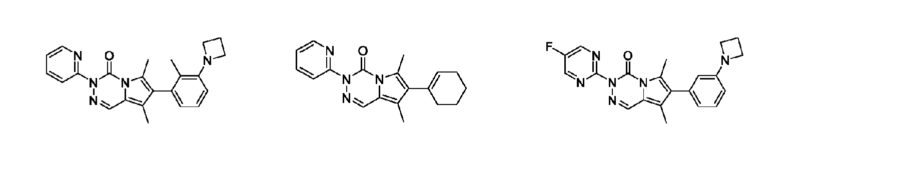 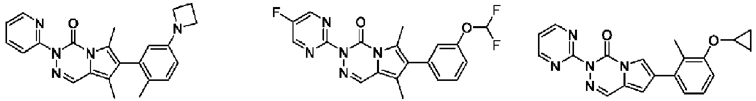 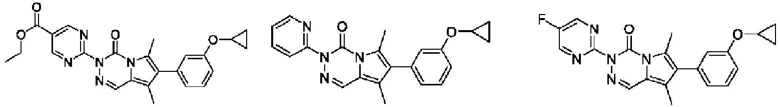 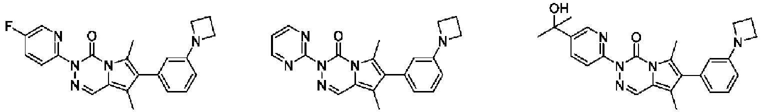 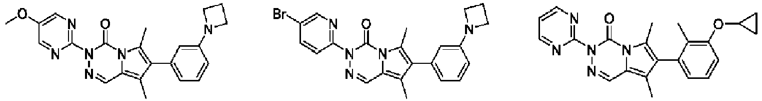 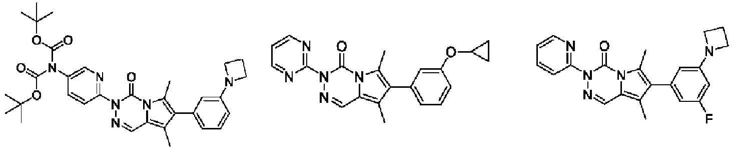 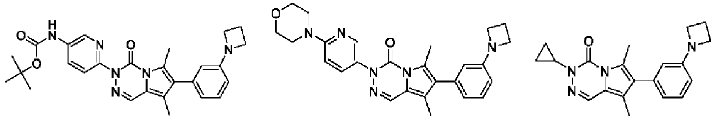 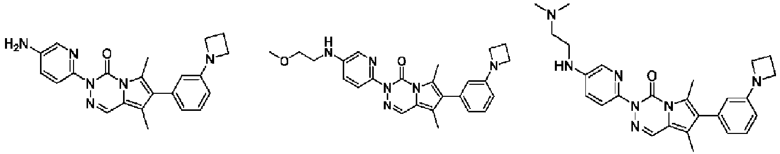 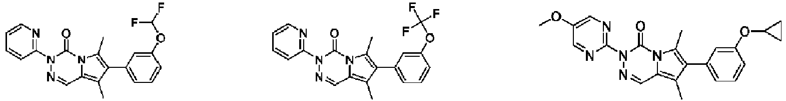 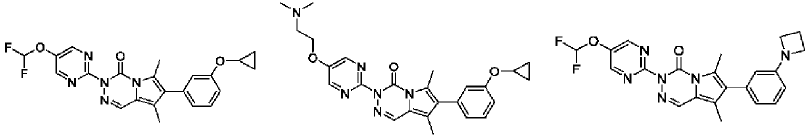 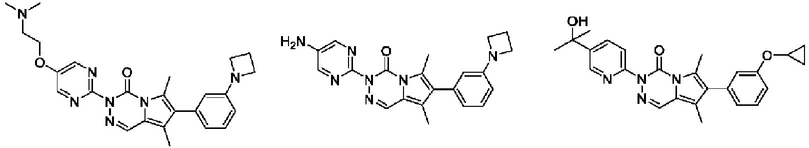 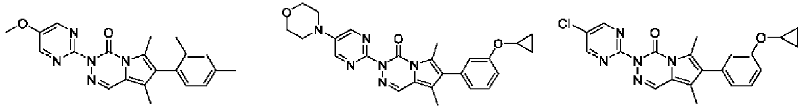 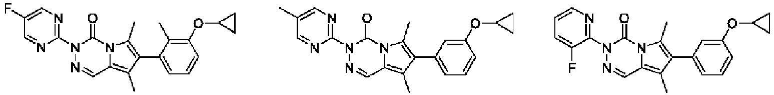 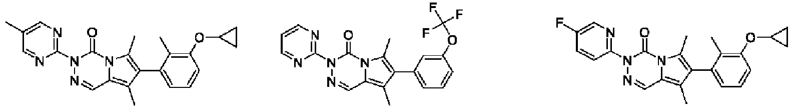 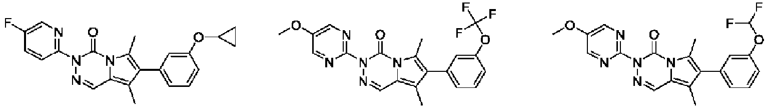 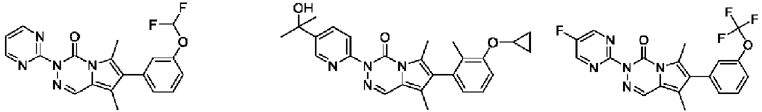 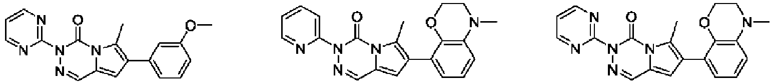 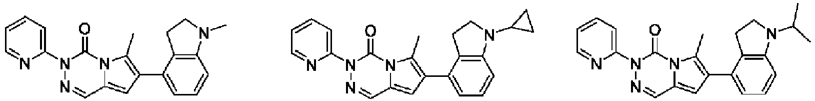 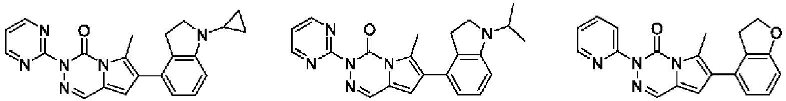 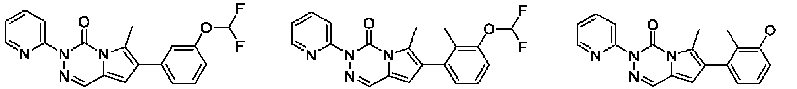 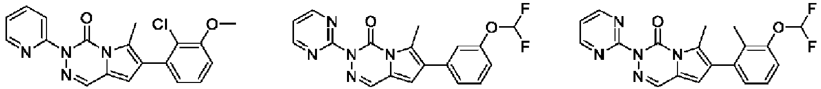 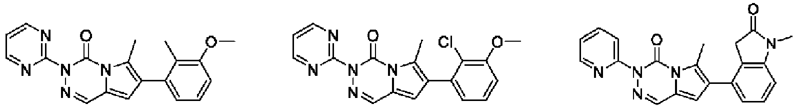 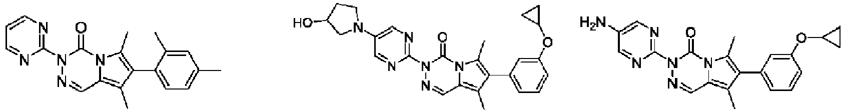 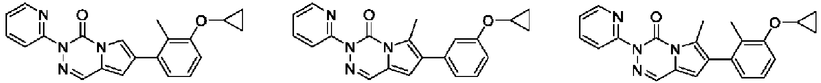 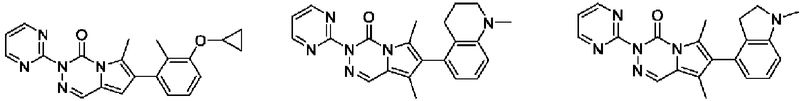 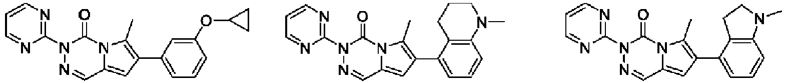 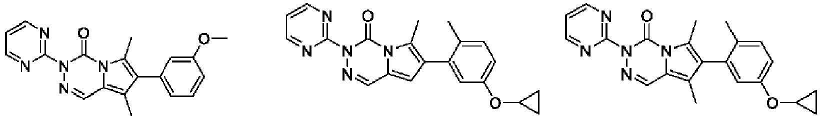 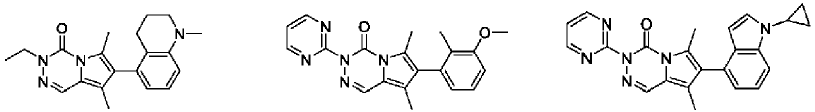 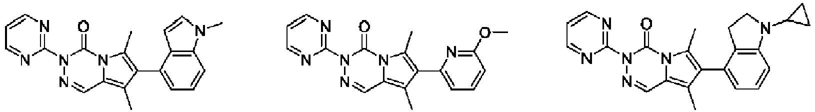 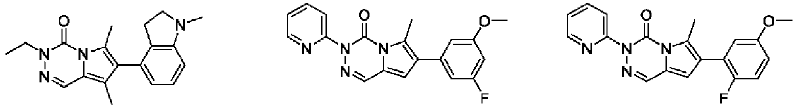 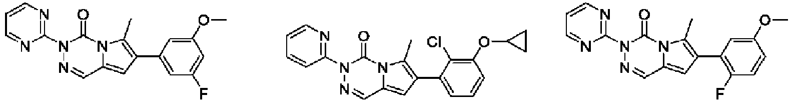 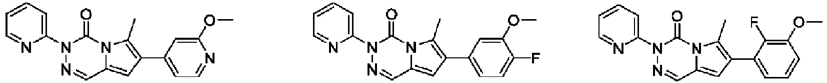 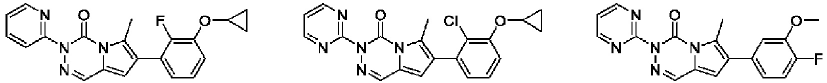 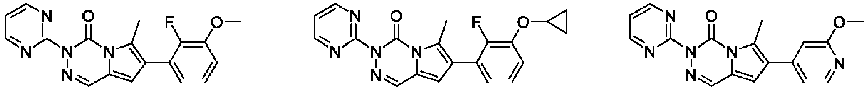 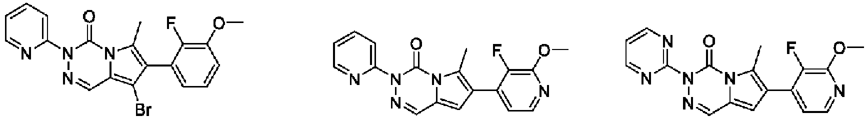 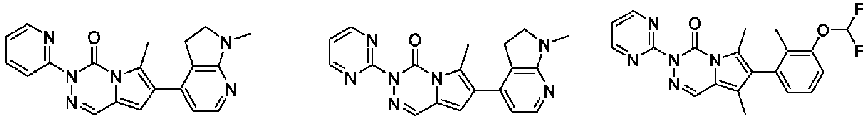 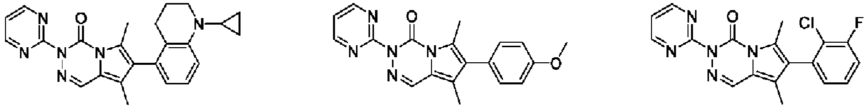 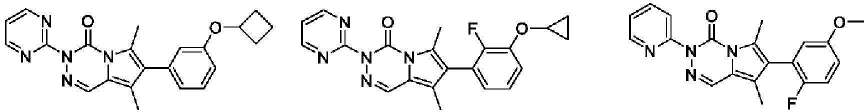 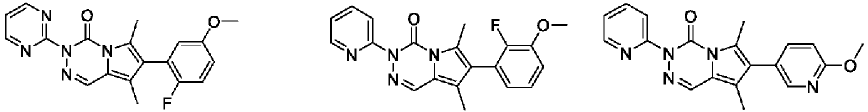 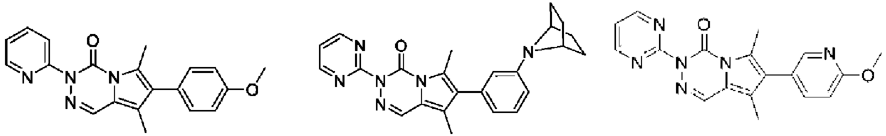 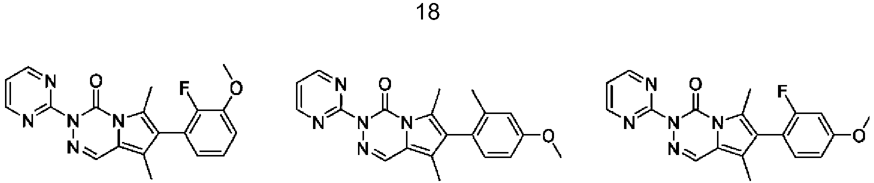 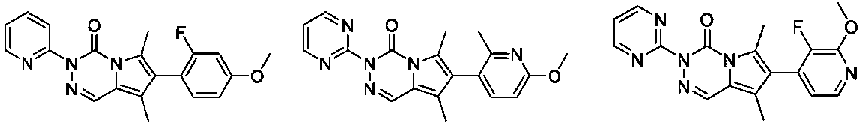 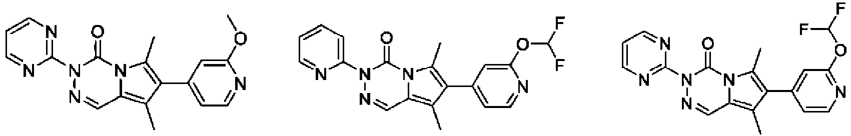 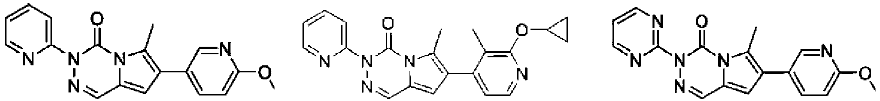 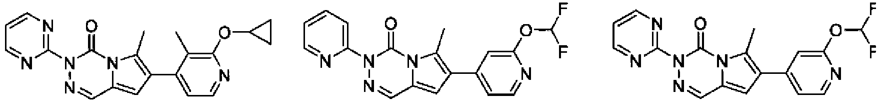 und ein pharmazeutisch verträgliches Säure- oder Basenadditionssalz davon, eine stereochemisch isomere Form davon oder eine A-Oxid-Form davon.

12. Pharmazeutische Zusammensetzung, umfassend eine therapeutisch wirksame Menge einer Verbindung nach einem der Ansprüche 1 bis 11 sowie einen pharmazeutisch verträglichen Träger und/oder Hilfsstoff.

13. Verbindung oder Zusammensetzung nach einem der Ansprüche 1 bis 12 zur Verwendung bei der Behandlung oder Vorbeugung einer Erkrankung bei einem Säugetier, die durch die modulierende Wirkung eines allosterischen mGlu7-Modulators, wie beispielsweise eines negativen allosterischen mGlu7-Modulators, beeinflusst oder begünstigt wird; oder zur Verwendung bei der Behandlung, Vorbeugung, Linderung, Kontrolle oder Verringerung des Risikos verschiedener neurologischer und psychiatrischer Störungen, die mit einer Glutamat-Dysfunktion bei einem Säugetier assoziiert sind, umfassend ein Verabreichen einer wirksamen Menge der Verbindung/Zusammensetzung an ein Säugetier, das einer solchen Behandlung oder Vorbeugung bedarf; beispielsweise wobei die Behandlung oder Vorbeugung durch die modulierende Wirkung eines negativen allosterischen mGlu7-Modulators beeinflusst oder begünstigt wird.

14. Verbindung oder Zusammensetzung zur Verwendung nach Anspruch 13, wobei es sich bei der Erkrankung um eine oder mehrere von einer Störung des zentralen Nervensystems, einer otischen Erkrankung oder Störung oder einer Schmerzerkrankung handelt; beispielsweise
wobei es sich bei der Störung des zentralen Nervensystems um eine Angststörung handelt, wie beispielsweise Agoraphobie, generalisierte Angststörung (GAD), Zwangsstörung (OCD), Panikstörung oder posttraumatische Belastungsstörung (PTSD); beispielsweise
wobei es sich bei der Störung des zentralen Nervensystems um eine psychotische Störung handelt, ausgewählt aus der Gruppe von Schizophrenie, Wahnstörung, schizoaffektiver Störung, schizophreniformer Störung und substanzinduzierter psychotischer Störung; beispielsweise
wobei es sich bei der ortischen Erkrankung und Störung um eine oder mehrere von einer Innenohrerkrankung, altersbedingter Hörbeeinträchtigung (Presbyakusis), Morbus Menière, plötzlichem Hörverlust, lärmbedingtem Hörverlust, Otitis media, autoimmun bedingter Innenohrerkrankung, akutem Tinnitus, chronischem Tinnitus, medikamentenbedingtem Hörverlust, verstecktem Hörverlust, Cisplatin-induziertem Hörverlust, Aminoglykosid-induziertem Hörverlust, Ototoxizität, zentraler auditiver Verarbeitungsstörung oder vestibulärer Störung handelt; beispielsweise
wobei es sich bei der Schmerzerkrankung um einen oder mehrere von neuropathischem Schmerz, entzündlichem Schmerz, viszeralem Schmerz, akutem Schmerz, chronischem Schmerz, starkem Schmerz, therapieresistentem Schmerz, posttraumatischem Schmerz, postoperativem Schmerz, Kopfschmerz oder Krebsschmerz handelt.

15. Verbindung oder Zusammensetzung nach einem der Ansprüche 1 bis 12, zur Verwendung als ein Arzneimittel.

## Revendications

1. Composé présentant la formule (I) : 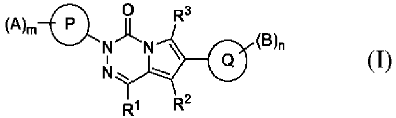 un sel d'addition d'acide ou de base pharmaceutiquement acceptable de celui-ci, une forme stéréochimiquement isomère de celui-ci ou une forme *N*-oxyde de celui-ci, dans lequel :
R¹ est sélectionné dans le groupe consistant en hydrogène, -CH₃ et -CF₃ ;
R² et R³ sont chacun indépendamment sélectionnés dans le groupe consistant en hydrogène, halogène, -(C₁-C₆)alkyle, -(C₁-C₆)haloalkyle et -CF₃ ;
P représente un -(C₁-C₆)alkyle, ou un cycloalkyle, un aryle, un hétéroaryle, un -(C₁-C₆)alkylène-hétéroaryle ou un hétérocycle de formule : 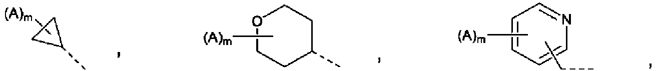 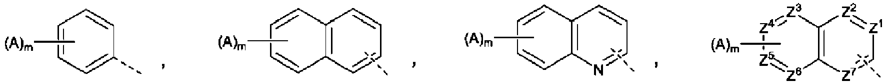 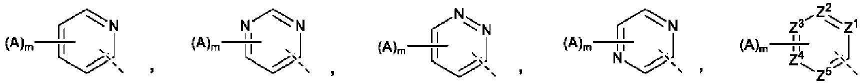 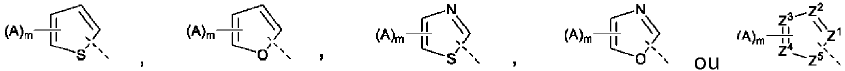
dans lequel chaque cycle cycloalkyle, aryle, hétéroaryle, -(C₁-C₆)alkylène-hétéroaryle ou hétérocycle est facultativement substitué par m radicaux A, dans lequel m est un nombre entier égal à zéro, 1, 2, 3 ou 4 ;
dans lequel Z¹, Z², Z³, Z⁴, Z⁵, Z⁶ et Z⁷ sont chacun indépendamment sélectionnés parmi C, N, O ou S ; à condition qu'au moins un parmi Z¹, Z², Z³, Z⁴, Z⁵, Z⁶ et Z⁷ soit N ;
le ou chaque (A)ₘ est indépendamment sélectionné dans le groupe consistant en hydrogène, halogène, -CN, -OH, -NO₂, -CF₃, -SH, -NH₂ et un radical facultativement substitué sélectionné dans le groupe consistant en -(C₁-C₆)alkyle, -(C₁-C₆)haloalkyle, -(C₂-C₆)alcynyle, -(C₂-C₆)alcényle, -(C₃-C₇)Cycloalkyle, -(C₁-C₆)alkylène-(C₃-C₇)cycloalkyle, - (C₃-C₈)cycloalcényle, -(C₁-C₆)cyanoalkyle, -(C₁-C₆)alkylène-hétéroaryle, -(C₁-C₆)alkylène-aryle, aryle, hétéroaryle, -(C₁-C₆)alkylène-hétérocycle, hétérocycle, -(C₀-C₆)alkylène-OR⁴, - O-(C₂-C₆)alkylène-OR⁴ -NR⁴(C₂-C₆)alkylène-OR⁵, -(C₃-C₆)alcynylène-OR⁴, -(C₃-C₆)alcynylène-NR⁴R⁵, -(C₃-C₆)alcénylène-OR⁴, -(C₃-C₆)alcénylène-NR⁴R⁵, -(C₀-C₆)alkylène-S-R⁴, -O-(C₂-C₆)alkylène-S-R⁴, -NR⁴-(C₂-C₆)alkylène-S-R⁵, -(C₀-C₆)alkylène-S(=O)-R⁴, -O-(C₁-C₆)alkylène-S(=O)-R⁴, -NR⁴-(C₁-C₆)alkylène-S(=O)-R⁵, -(C₀-C₆)alkylène-S(=O)₂-R⁴, - O-(C₁-C₆)alkylène-S(=O)₂-R⁴, -NR⁴-(C₁-C₆)alkylène-S(=O)₂-R⁵, -(C₀-C₆)alkylène- NR⁴R⁵, -O-(C₂-C₆)alkylène-NR⁴R⁵, -NR⁴-(C₂-C₆)alkylène-NR⁵R⁶, -(C₀-C₆)alkylène- S(=O)₂NR⁴R⁵, -O-(C₁-C₆)alkylène-S(=O)₂NR⁴R⁵, -NR⁴-(C₁-C₆)alkylène-S(=O)₂NR⁵R⁶, -(C₀-C₆)alkylène-NR⁴-S(=O)₂R⁵, -O-(C₂-C₆)alkylène-NR⁴-S(=O)₂R⁵, -NR⁴-(C₂-C₆)alkylène-NR⁵-S(=O)₂R⁶, -(C₀-C₆)alkylène-C(=O)-NR⁴R⁵, -O-(C₁-C₆)alkylène- C(=O)-NR⁴R⁵, -NR⁴-(C₁-C₆)alkylène-C(=O)-NR⁵R⁶, -(C₀-C₆)alkylène-NR⁴C(=O)-R⁵, -O-(C₂-C₆)alkylène-NR⁴C(=O)-R⁵, -NR⁴-(C₂-C₆)alkylène-NR⁵C(=O)-R⁶, -(C₀-C₆)alkylène-OC(=O)-R⁴, -O-(C₂-C₆)alkylène-OC(=O)-R⁴, -NR⁴-(C₂-C₆)alkylène- OC(=O)-R⁵, -(C₀-C₆)alkylène-C(=O)-OR⁴, -O-(C₁-C₆)alkylène-C(=O)-OR⁴, -NR⁴-(C₀-C₆)alkylène-C(=O)-OR⁵, -(Cₒ-C₆)alkylène-C(=O)-R⁴, -O-(C₁-C₆)alkylène-C(=O)-R⁴, - NR⁴-(C₁-C₆)alkylène-C(=O)-R⁵, -(C₀-C₆)alkylène-NR⁴-C(=O)-OR⁵, -C(=O)-(C₁-C₆)alkylène-NR⁴-C(=O)-OR⁵, -(C₀-C₆)alkylène-O-C(=O)-NR⁴R⁵, -(C₀-C₆)alkylène- NR⁴-C(=O)-NR⁵R⁶, -O-(C₂-C₆)alkylène-NR⁴-C(=O)-NR⁵R⁶, -NR⁴-(C₂-C₆)alkylène- NR⁵-C(=O)-NR⁶R⁷, -(C₀-C₆)alkylène-NR⁴-C(=S)-NR⁵R⁶ et -(C₀-C₆)alkylène-NR⁴- C(=NR⁵)-NR⁶R⁷ ;
R⁴, R⁵, R⁶ et R⁷ sont chacun indépendamment de l'hydrogène ou un radical facultativement substitué sélectionné dans le groupe consistant en -(C₁-C₆)haloalkyle, -(C₁-C₆)alkyle, -(C₁-C₆)cyanoalkyle, -(C₃-C₇)cycloalkyle, -(C₁-C₆)alkylène-(C₃-C₇)cycloalkyle, hétéroaryle, -(C₁-C₆)alkylène-hétéroaryle, aryle, -(C₁-C₆)alkylène-hétérocycle, hétérocycle, -(C₁-C₆)alkylène-aryle, -(C₀-C₆)alkylène-O-(C₀-C₆) alkyle, -(C₀-C₆)alkylène-N-((C₀-C₆)alkyle)₂ et -C(=O)-O-(C₁-C₆)alkyle ;
Q représente un aryle, un hétéroaryle ou un -(C₅-C₇)cycloalcényle de formule : 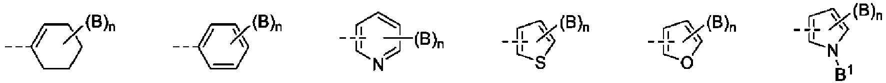 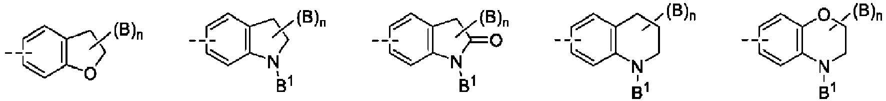 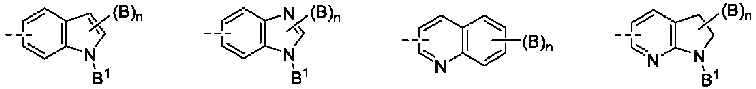
dans lequel chaque cycle aryle, hétéroaryle ou -(C₅-C₇)cycloalcényle est facultativement substitué par n radicaux B, dans lequel n est un nombre entier égal à zéro, 1, 2, 3, 4 ou 5 ; dans lequel B1 est un radical B ;
le ou chaque (B)ₙ est indépendamment sélectionné dans le groupe consistant en hydrogène, halogène, -CN, - OH, -NO₂, -CF₃, -SH, -NH₂ et un radical facultativement substitué sélectionné dans le groupe consistant en -(C₁-C₆) alkyle, -(C₁-C₆) haloalkyle, -(C₂-C₆) alcynyle, -(C₂-C₆) alcényle, -(C₃-C₇) cycloalkyle, -(C₁-C₆) alkylène-(C₃-C₇) cycloalkyle, - (C₃-C₈) cycloalcényle, -(C₁-C₆) cyanoalkyle, -(C₁-C₆) alkylène-hétéroaryle, -(C₁-C₆)alkylène-aryle, aryle, hétéroaryle, -(C₁-C₆) alkylène-hétérocycle, hétérocycle, -(C₀-C₆)alkylène-OR⁸, -O-(C₂-C₆) alkylène-OR⁸, -NR⁸(C₂- C₆) alkylène-OR⁹, -(C₃-C₆) alcynylène-OR⁸, -(C₃-C₆) alcynylène-NR⁸R⁹, -(C₃-C₆) alcénylène-OR⁸, -(C₃-C₆) alcénylène-NR⁸R⁹, -(C₀-C₆) alkylène-S-R⁸, -O-(C₂-C₆) alkylène-S-R⁸, -NR⁸-(C₂-C₆) alkylène-S-R⁹, -(C₀-C₆) alkylène-S(=O) -R⁸, - O-(C₁-C₆) alkylène-S(=O) -R⁸, -NR⁸-(C₁-C₆) alkylène-S(=O) -R⁹, -(C₀-C₆) alkylène-S(=O) ₂-R⁸, -O-(C₁-C₆) alkylène-S(=O)₂-R⁸, -NR⁸-(C₁-C₆) alkylène-S(=O)₂-R⁹, -(C₀-C₆) alkylène-NR⁸R⁹, -O-(C₂-C₆) alkylène-NR⁸R⁹, -NR⁸-(C₂-C₆) alkylène-NR⁹R¹⁰, -(C₀-C₆) alkylène-S(=O)₂NR⁸R⁹, -O-(C₁-C₆) alkylène-S(=O)₂NR⁸R⁹, -NR⁸-(C₁-C₆) alkylène-S(=O)₂ NR⁹R¹⁰, - (C₀-C₆) alkylène-NR⁸-S(=O)₂R⁹, -O-(C₂-C₆) alkylène-NR⁸-S(=O)₂R⁹, -NR⁸-(C₂-C₆) alkylène-NR⁹-S(=O)₂R¹⁰, -(C₀-C₆) alkylène-C(=O) -NR⁸R⁹, -O-(C₁-C₆)alkylène-C(=O)-NR⁸R⁹, -NR⁸-(C₁-C₆)alkylène-C(=O)-NR⁹R¹⁰, -(C₀-C₆)alkylène-NR⁸C(=O)-R⁹, -O-(C₂-C₆)alkylène-NR⁸C(=O)-R⁹, -NR⁸-(C₂-C₆)alkylène-NR⁹C(=O)-R¹⁰, -(C₀-C₆)alkylène-OC(=O)-R⁸, -O-(C₂-C₆)alkylène-OC(=O)-R⁸, -NR⁸-(C₂-C₆)alkylène-OC(=O)-R⁹, -(C₀-C₆)alkylène-C(=O)-OR⁸, - O-(C₁-C₆)alkylène-C(=O)-OR⁸, -NR⁸-(C₁-C₆)alkylène-C(=O)-OR⁹, -(C₀-C₆)alkylène-C(=O)-R⁸, -O-(C₁-C₆)alkylène-C(=O)-R⁸, -NR⁸-(C₁-C₆) alkylène-C(=O)-R⁹,-(C₀-C₆) alkylène-NR⁸-C(=O)-OR⁹, -(C₀-C₆) alkylène-O-C(=O)-NR⁸R⁹, -(C₀-C₆)alkylène-NR⁸-C(=O)-NR⁹R¹⁰, -O-(C₂-C₆)alkylène-NR⁸-C(=O)-NR⁹R¹⁰, -NR⁸-(C₂-C₆)alkylène-NR⁹-C(=O)-NR¹⁰R¹¹, -(C₀-C₆)alkylène-NR⁸-C(=S)-NR⁹R¹⁰ et -(C₀-C₆)alkylène-NR⁸-C(=NR⁹)-NR¹⁰R¹¹ ;
R⁸, R⁹, R¹⁰ et R¹¹ sont chacun indépendamment de l'hydrogène ou un radical facultativement substitué sélectionné dans le groupe consistant en -(C₁-C₆) haloalkyle, - (C₁-C₆) alkyle, -(C₁-C₆) cyanoalkyle, -(C₃-C₇) cycloalkyle, -(C₁-C₆) alkylène-(C₃-C₇) cycloalkyle, hétéroaryle, -(C₁-C₆) alkylène-hétéroaryle, aryle, -(C₁-C₆) alkylène-hétérocycle, hétérocycle, -(C₁-C₆) alkylène-aryle- -(C₀-C₆) alkylène-O-(C₀-C₆) alkyle et -(C₀-C₆) alkylène-N-((C₀-C₆) alkyle)₂ ;
dans lequel facultativement deux quelconques radicaux A sont combinés avec les atomes intermédiaires pour former un cycle bicyclique hétérocycle, aryle ou hétéroaryle de 3 à 10 chaînons, dans lequel chaque cycle est en outre facultativement substitué par 1 à 5 radicaux indépendamment sélectionnés dans le groupe consistant en halogène, -CN, nitro, -(C₁-C₆)alkyle, -(C₃-C₇)alkyle,-(C₀-C₆)alkylène-O-(C₀-C₆)alkyle et -(C₀-C₆) alkylène-N-((C₀-C₆) alkyle)₂ ;
dans lequel facultativement deux des substituants R⁴, R⁵, R⁶ ou R⁷ sont combinés avec les atomes intermédiaires pour former un cycle hétérocycle, aryle ou hétéroaryle de 3 à 10 chaînons, dans lequel chaque cycle est en outre facultativement substitué par 1 à 5 radicaux indépendamment sélectionnés dans le groupe consistant en halogène, cyano, nitro, -(C₁-C₆) alkyle, -(C₃-C₇) alkyle, -(C₀-C₆)alkylène-O-(C₀-C₆)alkyle et -(C₀-C₆)alkylène-N-((C₀-C₆)alkyle)₂ ;
dans lequel facultativement deux substituants parmi R⁸, R⁹, R¹⁰ ou R¹¹ sont combinés avec les atomes intermédiaires pour former un cycle hétérocycle, aryle ou hétéroaryle de 3 à 10 chaînons, dans lequel chaque cycle est en outre facultativement substitué par 1 à 5 radicaux indépendamment sélectionnés dans le groupe consistant en halogène, cyano, nitro, -(C₁-C₆) alkyle, -(C₃-C₇) alkyle, -(C₀-C₆)alkylène-O-(C₀-C₆)alkyle et -(C₀-C₆) alkylène-N-((C₀-C₆) alkyle)₂ ; et
dans lequel facultativement deux quelconques radicaux B sont combinés avec les atomes intermédiaires pour former un cycle bicyclique hétérocycle, aryle ou hétéroaryle de 3 à 10 chaînons, dans lequel chaque cycle est en outre facultativement substitué par 1 à 5 radicaux indépendamment sélectionnés dans le groupe consistant en halogène, -CN, nitro, -(C₁-C₆)alkyle, -(C₃-C₇)alkyle, -(C₀-C₆)alkylène-O-(C₀-C₆)alkyle et -(C₀-C₆) alkylène-N-((C₀-C₆) alkyle)₂.

2. Composé selon la revendication 1 présentant la formule (I), dans lequel P représente un -(C₁-C₆)alkyle, ou un cycloalkyle, hétéroaryle, -(C₁-C₆)alkylène-hétéroaryle ou hétérocycle de formule : 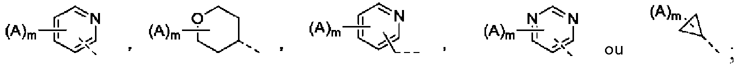 et/ou
dans lequel Q représente un aryle, hétéroaryle ou -(C5-C7)cycloalcényle de formule :

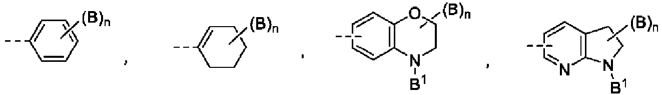

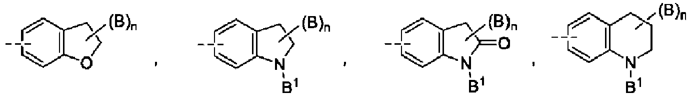

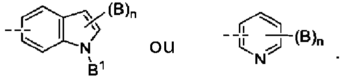

3. Composé selon une quelconque revendication précédente présentant la formule (I), dans lequel P représente un hétéroaryle de formule : 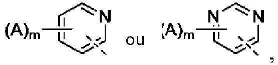
dans lequel chaque radical est facultativement substitué par m radicaux A, dans lequel m est un nombre entier égal à zéro, 1, 2, 3 ou 4 ; et/ou dans lequel Q représente un groupe aryle ou hétéroaryle de formule : 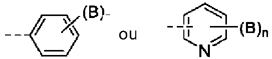
dans lequel chaque radical est facultativement substitué par n radicaux B, dans lequel n est un nombre entier égal à zéro, 1, 2, 3, 4 ou 5 ; et/ou dans lequel :
les systèmes cycliques cycloalkyle, hétérocycle, aryle et hétéroaryle de (A)ₘ sont sélectionnés dans le groupe consistué de azétidinyle, 2-azabicyclo[2.2.1]heptan-2-yle, 7-azabicyclo[2.2.1]heptan-7-yle, benzimidazolyle, benzisothiazolyle benzisoxazolyle, benzofuryle, benzopyrazolyle, benzothiazolyle, benzothiophényle, benzotriazolyle, benzoxazolyle, dihydrofurannyle, dihydrothiényle, dioxolannyle, 1,1-dioxo-thiomorpholinyle, furazannyle, furyle, imidazolidinyle, imidazolinyle, imidazolonyle, imidazolyle, imidazopyridazinyle, imidazopyridyle, indolyle, isoindolyle, isoquinolinyle, isothiazolinyle, isothiazolyle, isoxazolidinyle, isoxazolinyle, isoxazolyle, morpholinyle, naphtyle, naphtyridinyle, oxadiazolyle, oxazolidinyle, oxazolinyle, oxazolonyle, oxazolopyridazinyle, oxazolopyridyle, oxazolyle, oxétannyle, phényle, pipérazinonyle, pipérazinyle, pipéridinonyle, pipéridinyle, phtalazinyle, ptéridinyle, purinyle, pyrannyle, pyrazinyle, pyrazolopyridinyle, pyrazolyle, pyridazinyle, pyridonyle, pyridyle, pyrimidyle, pyrrolidinonyle, pyrrolidinyle, pyrrolinyle, pyrrolyle, quinazolyle, quinolyle, quinoxalinyle, tétrahydrofurannyle, tétrahydropyrannyle, tétrahydrothiopyrannyle, tétrahydrotriazolopyridyle, tétrahydrotriazolopyrimidinyle, tétrazolyle, thiadiazolyle, thiazolidinyle, thiazolinyle, thiazolonyle, thiazolopyridazinyle, thiazolopyridyle, thiazolyle, thiényle, thiomorpholinyle, thionaphtyle, thiopyrannyle, triazolinyle, triazinyle, triazolyle, cyclopropyle, cyclobutyle, cyclopentyle, cyclopentényle, cyclohexyle, cyclohexényle, cycloheptyle, cycloheptényle, cyclooctyle et cyclooctényle et chaque cycle dudit système cyclique est facultativement substitué indépendamment par 1 à 4 substituants R⁴, R⁵, R⁶ ou R7 ; et/ou dans lequel :
les systèmes cycliques cycloalkyle, hétérocycle, aryle et hétéroaryle de (B)ₙ sont sélectionnés dans le groupe consistant en azétidinyle, benzimidazolyle, benzisothiazolyle benzisoxazolyle, benzofuryle, benzopyrazolyle, benzothiazolyle, benzothiophényle, benzotriazolyle, benzoxazolyle, dihydrofurannyle, dihydrothiényle, dioxolannyle, 1,1-dioxo-thiomorpholinyle, furazannyle, furyle, imidazolidinyle, imidazolinyle, imidazolonyle, imidazolyle, imidazopyridazinyle, imidazopyridyle, indolyle, isoindolyle, isoquinolinyle, isothiazolinyle, isothiazolyle, isoxazolidinyle, isoxazolinyle, isoxazolyle, morpholinyle, naphtyle, naphtyridinyle, oxadiazolyle, oxazolidinyle, oxazolinyle, oxazolonyle, oxazolopyridazinyle, oxazolopyridyle, oxazolyle, oxétannyle, phényle, pipérazinonyle, pipérazinyle, pipéridinonyle, pipéridinyle, phtalazinyle, ptéridinyle, purinyle, pyrannyle, pyrazinyle, pyrazolopyridinyle, pyrazolyle, pyridazinyle, pyridonyle, pyridyle, pyrimidyle, pyrrolidinonyle, pyrrolidinyle, pyrrolinyle, pyrrolyle, quinazolyle, quinolyle, quinoxalinyle, tétrahydrofurannyle, tétrahydropyrannyle, tétrahydrothiopyrannyle, tétrahydrotriazolopyridyle, tétrahydrotriazolopyrimidinyle, tétrazolyle, thiadiazolyle, thiazolidinyle, thiazolinyle, thiazolonyle, thiazolopyridazinyle, thiazolopyridyle, thiazolyle, thiényle, thiomorpholinyle, thionaphtyle, thiopyrannyle, triazolinyle, triazinyle, triazolyle, cyclopropyle, cyclobutyle, cyclopentyle, cyclopentényle, cyclohexyle, cyclohexényle, cycloheptyle, cycloheptényle, cyclooctyle et cyclooctényle et chaque cycle dudit système cyclique est facultativement substitué indépendamment par 1 à 4 substituants R⁸, R⁹, R¹⁰ ou R¹¹ ; et/ou dans lequel :
les systèmes cycliques cycloalkyle, hétérocycle, aryle et hétéroaryle de R⁴, R⁵, R⁶, R⁷, R⁸, R⁹, R¹⁰ ou R¹¹ sont sélectionnés dans le groupe consistant en azétidinyle, benzimidazolyle, benzisothiazolyle benzisoxazolyle, benzofuryle, benzopyrazolyle, benzothiazolyle, benzothiophényle, benzotriazolyle, benzoxazolyle, dihydrofurannyle, dihydrothiényle, dioxolannyle, 1,1-dioxo-thiomorpholinyle, furazannyle, furyle, imidazolidinyle, imidazolinyle, imidazolonyle, imidazolyle, imidazopyridazinyle, imidazopyridyle, indolyle, isoindolyle, isoquinolinyle, isothiazolinyle, isothiazolyle, isoxazolidinyle, isoxazolinyle, isoxazolyle, morpholinyle, naphtyle, naphtyridinyle, oxadiazolyle, oxazolidinyle, oxazolinyle, oxazolonyle, oxazolopyridazinyle, oxazolopyridyle, oxazolyle, oxétannyle, phényle, pipérazinonyle, pipérazinyle, pipéridinonyle, pipéridinyle, phtalazinyle, ptéridinyle, purinyle, pyrannyle, pyrazinyle, pyrazolopyridinyle, pyrazolyle, pyridazinyle, pyridonyle, pyridyle, pyrimidyle, pyrrolidinonyle, pyrrolidinyle, pyrrolinyle, pyrrolyle, quinazolyle, quinolyle, quinoxalinyle, tétrahydrofurannyle, tétrahydropyrannyle, tétrahydrothiopyrannyle, tétrahydrotriazolopyridyle, tétrahydrotriazolopyrimidinyle, tétrazolyle, thiadiazolyle, thiazolidinyle, thiazolinyle, thiazolonyle, thiazolopyridazinyle, thiazolopyridyle, thiazolyle, thiényle, thiomorpholinyle, thionaphtyle, thiopyrannyle, triazolinyle, triazinyle, triazolyle, cyclopropyle, cyclobutyle, cyclopentyle, cyclopentényle, cyclohexyle, cyclohexényle, cycloheptyle, cycloheptényle, cyclooctyle et cyclooctényle et chaque cycle dudit système cyclique est facultativement substitué par 1-5 radicaux indépendamment sélectionnés parmi l'hydrogène, un halogène, -CN, nitro, -(C₁-C₆)alkyle, -(C₀-C₆)alkylène-O-(C₀-C₆)alkyle et -(C₀-C₆)alkylène-N-((C₀-C₆)alkyle)₂.

4. Composé selon une quelconque revendication précédente présentant la formule (I), dans lequel R¹ est l'hydrogène ; et/ou dans lequel
R² et R³ sont chacun indépendamment sélectionnés dans le groupe consistant en hydrogène et méthyle ; et/ou dans lequel le ou chaque (A)ₘ est indépendamment sélectionné dans le groupe consistant en hydrogène, halogène, -CN, -OH, -CF₃ et un radical facultativement substitué sélectionné dans le groupe consistant en -(C₁-C₆)alkyle, -(C₁-C₆)haloalkyle, -(C₃-C₇)cycloalkyle, -(C₁-C₆)cyanoalkyle, aryle, hétérocycle, -(C₀-C₆)alkylène-OR⁴, -O-(C₂-C₆)alkylène-OR⁴, -NR⁴(C₂-C₆)alkylène-OR⁵, -(C₀-C₆)alkylène-S(=O)₂-R⁴, -(C₀-C₆)alkylène-NR⁴R⁵, -O-(C₂-C₆)alkylène-NR⁴R⁵, -NR⁴-(C₂-C₆)alkylène-NR⁵R⁶, -(C₀-C₆)alkylène-S(=O)₂NR⁴R⁵, -(C₀-C₆)alkylène-C(=O)-NR⁴R⁵, -(C₀-C₆)alkylène-NR⁴C(=O)-R⁵, -(C₀-C₆)alkylène-C(=O)-OR⁴, -(C₀-C₆)alkylène-C(=O)-R⁴, -C(=O)-(C₁-C₆)alkylène-NR⁴-C(=O)-OR⁵ et -NR⁴-(C₀-C₆)alkylène-C(=O)-OR⁵ ; et/ou dans lequel R⁴, R⁵ et R⁶ sont chacun indépendamment de l'hydrogène, -(C₁-C₆)alkyle, -(C₁-C₆)haloalkyle ou -C(=O)-O-(C₁-C₆)alkyle ; et/ou
dans lequel le ou chaque (B)ₙ est indépendamment sélectionné dans le groupe consistant en hydrogène, halogène, -CN, -CF₃, et un radical facultativement substitué sélectionné dans le groupe consistant en -(C₁-C₆)alkyle, -(C₃-C₇)cycloalkyle, aryle, hétéroaryle, hétérocycle, -(C₀-C₆)alkylène-OR⁸, -NR⁸(C₂-C₆)alkylène-OR⁹, -(C₀-C₆)alkylène-NR⁸R⁹, - (C₀-C₆)alkylène-C(=O)-OR⁸ et -(C₀-C₆)alkylène-C(=O)-R⁸ ; et/ou
dans lequel R⁸ et R⁹ sont chacun indépendamment sélectionnés dans le groupe consistant en hydrogène, -(C₁-C₆) haloalkyle, -(C₁-C₆)alkyle, -(C₃-C₇)cycloalkyle et aryle.

5. Composé selon une quelconque revendication précédente présentant la formule (II) : 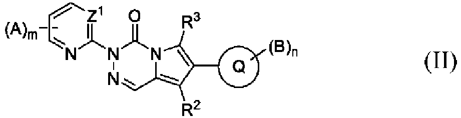
un sel d'addition d'acide ou de base pharmaceutiquement acceptable de celui-ci, une forme stéréochimiquement isomère de celui-ci ou une forme A-oxyde de celui-ci, dans lequel Z¹ est sélectionné parmi C ou N ; ou présentant la formule (III) : 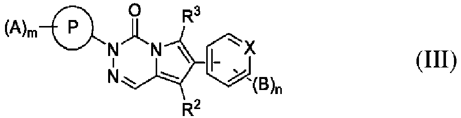
un sel d'addition d'acide ou de base pharmaceutiquement acceptable de celui-ci, une forme stéréochimiquement isomère de celui-ci ou une forme N-oxyde de celui-ci, dans lequel X est C ou N.

6. Composé selon une quelconque revendication précédente présentant la formule (IV) : 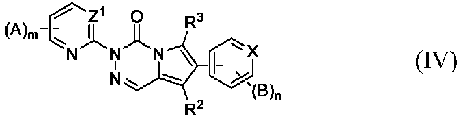
un sel d'addition d'acide ou de base pharmaceutiquement acceptable de celui-ci, une forme stéréochimiquement isomère de celui-ci ou une forme A-oxyde de celui-ci, dans lequel Z¹ est sélectionné parmi C ou N, et X est sélectionné parmi C ou N, par exemple présentant la formule (V) : 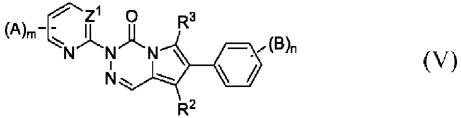
un sel d'addition d'acide ou de base pharmaceutiquement acceptable de celui-ci, une forme stéréochimiquement isomère de celui-ci ou une forme A-oxyde de celui-ci ; par exemple présentant la formule (VI) : 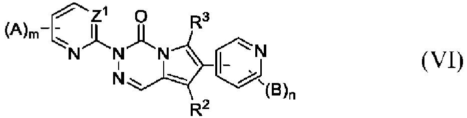
un sel d'addition d'acide ou de base pharmaceutiquement acceptable de celui-ci, une forme stéréochimiquement isomère de celui-ci ou une forme A-oxyde de celui-ci.

7. Composé selon une quelconque revendication précédente, dans lequel :
R² est de l'hydrogène ou du méthyle ;
R³ est du méthyle ou de l'hydrogène ;
le ou chaque (A)ₘ est indépendamment sélectionné dans le groupe consistant en hydrogène, halogène, -CF₃ et un radical facultativement substitué sélectionné dans le groupe consistant en -(C₁-C₆)alkyle, -(C₁-C₆)haloalkyle, -(C₃-C₇)cycloalkyle, hétérocycle, - (C₀-C₆)alkylène-OR⁴, -O-(C₂-C₆)alkylène-OR⁴ -NR⁴(C₂-C₆)alkylène-OR⁵, -(C₀-C₆)alkylène-NR⁴R⁵, -O-(C₂-C₆)alkylène-NR⁴R⁵, -NR⁴-(C₂-C₆)alkylène-NR⁵R⁶, -(C₀-C₆) alkylène-C(=O)-NR⁴R⁵, - (C₀-C₆)alkylène-NR⁴C(=O)-R⁵, -(C₀-C₆)alkylène-C(=O)-OR⁴, -(C₀-C₆) alkylène-C(=O)-R⁴, -C(=O)-(C₁-C₆)alkylène-NR⁴-C(=O)-OR⁵ et -NR⁴-(C₀-C₆)alkylène-C(=O)-OR⁵ ;
R⁴, R⁵ et R⁶ sont chacun indépendamment de l'hydrogène, -(C₁-C₆)alkyle, -(C₁-C₆)haloalkyle ou -C(=O)-O-(C₁-C₆)alkyle ;
le ou chaque (B)ₙ est indépendamment sélectionné dans le groupe consistant en hydrogène, halogène et un radical facultativement substitué sélectionné dans le groupe consistant en -(C₁-C₆)alkyle, hétérocycle, -(C₀-C₆)alkylène-OR⁸, -NR⁸(C₂-C₆)alkylène-OR⁹, -(C₀-C₆)alkylène-NR⁸R⁹, -(Co-C₆)alkylène-C(=O)-OR⁸ et -(C₀-C₆)alkylène-C(=O)-R⁸ ; et R⁸ et R⁹ sont chacun indépendamment de l'hydrogène, -(C₁-C₆)alkyle, -(C₃-C₇)cycloalkyle ou -(C₁-C₆) haloalkyle.

8. Composé selon une quelconque revendication précédente présentant la formule (VII) : 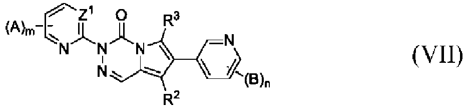 un sel d'addition d'acide ou de base pharmaceutiquement acceptable de celui-ci, une forme stéréochimiquement isomère de celui-ci ou une forme V-oxyde de celui-ci, dans lequel :
Z1 est sélectionné parmi C ou N ;
R2 est de l'hydrogène ou du méthyle ;
R3 est du méthyle ou de l'hydrogène ;
le ou chaque (A)ₘ est indépendamment sélectionné dans le groupe consistant en hydrogène, halogène, et un radical facultativement substitué sélectionné dans le groupe consistant en -(C₁-C₆)alkyle, hétérocycle, -(C₀-C₆)alkylène-OR⁴, -NR⁴(C₂-C₆)alkylène-OR⁵, -(C₀-C₆)alkylène-NR⁴R⁵, -O-(C₂-C₆)alkylène-NR⁴R⁵, -NR⁴-(C₂-C₆)alkylène-NR⁵R⁶' -(C₀-C₆)alkylène-C(=O)-OR⁴ et -NR⁴-(C₀-C₆)alkylène-C(=O)-OR⁵ ;
R⁴, R⁵ et R⁶ sont chacun indépendamment de l'hydrogène, -(C₁-C₆)alkyle, -(C₁-C₆)haloalkyle ou -C(=O)-O-(C₁-C₆)alkyle ;
le ou chaque (B)ₙ est indépendamment sélectionné dans le groupe consistant en hydrogène, halogène et un radical facultativement substitué sélectionné dans le groupe consistant en -(C₁-C₆)alkyle, hétérocycle et - (C₀-C₆)alkylène-OR⁸ ; et
R⁸ est de l'hydrogène, -(C₁-C₆)alkyle, -(C₃-C₇)cycloalkyle ou -(C₁-C₆)haloalkyle ; ou présentant la formule (VIII) : 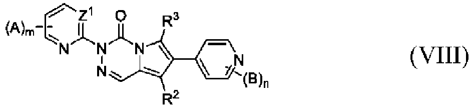
un sel d'addition d'acide ou de base pharmaceutiquement acceptable de celui-ci, une forme stéréochimiquement isomère de celui-ci ou une forme *N*-oxyde de celui-ci, dans lequel :
Z1 est sélectionné parmi C ou N ;
R2 est de l'hydrogène ou du méthyle ;
R3 est du méthyle ou de l'hydrogène ;
le ou chaque (A)ₘ est indépendamment sélectionné dans le groupe consistant en hydrogène, halogène, et un radical facultativement substitué sélectionné dans le groupe consistant en -(C₁-C₆)alkyle, hétérocycle, -(C₀-C₆)alkylène-OR⁴, -NR⁴(C₂-C₆)alkylène-OR⁵, -(C₀-C₆)alkylène-NR⁴R⁵, -O-(C₂-C₆)alkylène-NR⁴R⁵, -NR⁴-(C₂-C₆)alkylène-NR⁵R⁶, -(C₀-C₆)alkylène-C(=O)-OR⁴ et -NR⁴-(C₀-C₆)alkylène-C(=O)-OR⁵ ;
R⁴, R⁵ et R⁶ sont chacun indépendamment de l'hydrogène, -(C₁-C₆)alkyle, -(C₁-C₆)haloalkyle ou -C(=O)-O-(C₁-C₆)alkyle ;
le ou chaque (B)ₙ est indépendamment sélectionné dans le groupe consistant en hydrogène, halogène et un radical facultativement substitué sélectionné dans le groupe consistant en -(C₁-C₆)alkyle, hétérocycle et - (C₀-C₆)alkylène-OR⁸ ; et
R8 est de l'hydrogène, -(C₁-C₆)alkyle, -(C₃-C₇)cycloalkyle ou-(C₁-C₆)haloalkyle.

9. Composé selon une quelconque revendication précédente dans lequel le ou chaque (A)ₘ est indépendamment sélectionné dans le groupe consistant en hydrogène, halogène, et un radical facultativement substitué sélectionné dans le groupe consistant en hétérocycle, -(C₀-C₆)alkylène-OR⁴, -(C₀-C₆)alkylène-C(=O)-OR⁴, -NR⁴-(C₀-C₆)alkylène-C(=O)-OR⁵, -NR⁴(C₂-C₆)alkylène-OR⁵, -O-(C₂-C₆)alkylène-NR⁴R⁵, -NR⁴-(C₂-C₆)alkylène-NR⁵R⁶ et -(C₀-C₆)alkylène-NR⁴R⁵ ; et R⁴, R⁵ et R⁶ sont chacun indépendamment de l'hydrogène, -(C₁-C₆)alkyle, -(C₁-C₆)haloalkyle, ou -C(=O)-O-(C₁-C₆)alkyle ; et/ou dans lequel le ou chaque (B)ₙ est indépendamment sélectionné dans le groupe consistant en hydrogène, halogène et un radical facultativement substitué sélectionné dans le groupe consistant en -(C₁-C₆)alkyle, hétérocycle et -(C₀-C₆)alkylène-OR⁸ ; et R⁸ est de l'hydrogène, -(C₁-C₆)alkyle, -(C₃-C₇)cycloalkyle ou -(C₁-C₆)haloalkyle ; et/ou dans lequel le ou chaque (A)ₘ est indépendamment sélectionné dans le groupe consistant en hydrogène, halogène, -CH₂CH₂OH, -COOCH₂CH₃, -COH(CH₃)₂, -O-méthyle, -N(COOBu^{t})₂, NHCOOBu^{t}, morpholinyle, -NH₂, -NHCH₂CH₂OCH₃, -NHCH₂CH₂N(CH₃)₂, -OCHF₂, -OCH₂CH₂N(CH₃)₂, CHOH(CH₃)₂, pyrrolidinyle substituée par méthyle et hydroxy ; et/ou le ou chaque (B)ₙ est indépendamment sélectionné dans le groupe consistant en hydrogène, halogène, azétidinyle, -OCHF₂, -OCF₃, cyclopropyle, -O-cyclopropyle, -O-méthyle, méthyle, propyle, - O-cyclobutyle et azabicyclo[2.2.1]heptan-7-yle.

10. Composé selon les revendications 1 à 9, dans lequel le composé peut exister sous forme d'isomères optiques, et dans lequel le composé est soit un mélange racémique soit un ou les deux des isomères optiques individuels.

11. Composé selon les revendications 1 à 10, dans lequel ledit composé est un ou plusieurs éléments sélectionnés parmi : 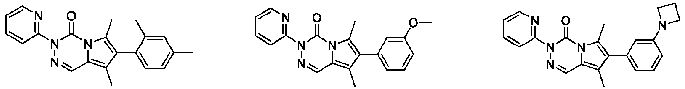 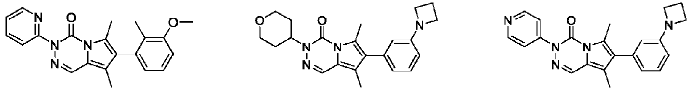 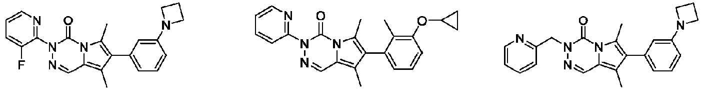 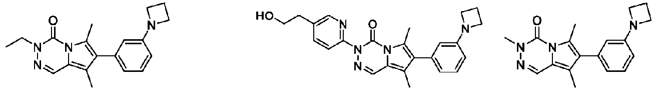 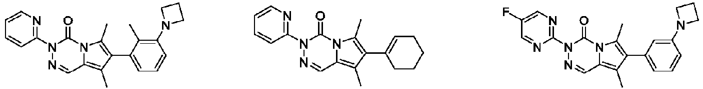 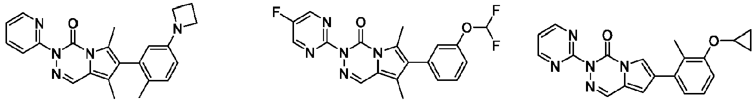 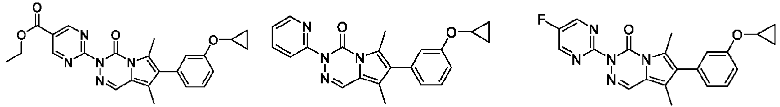 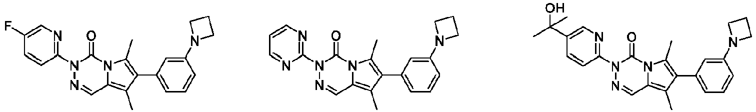 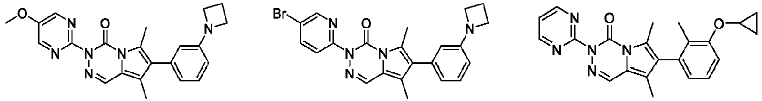 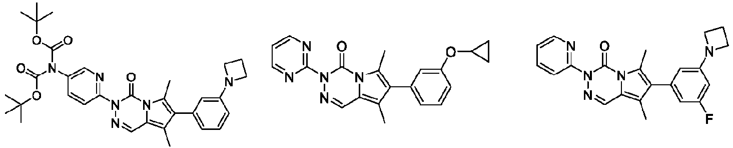 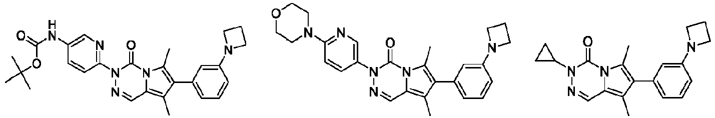 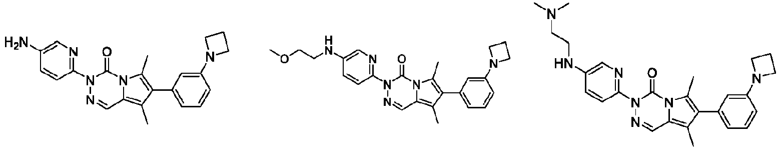 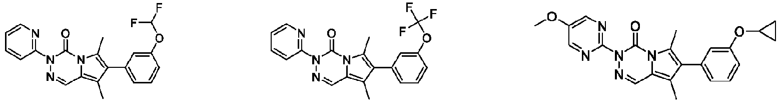 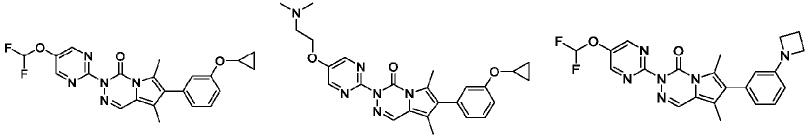 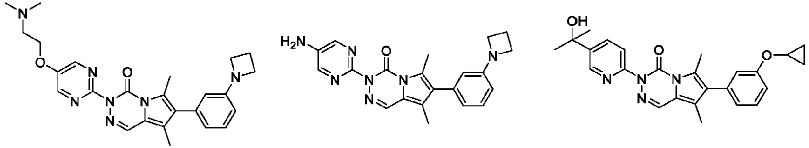 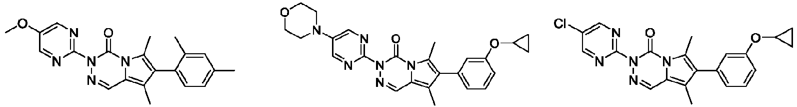 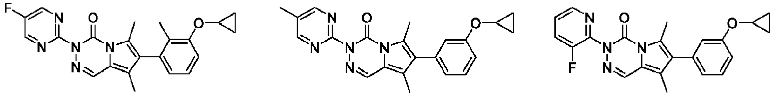 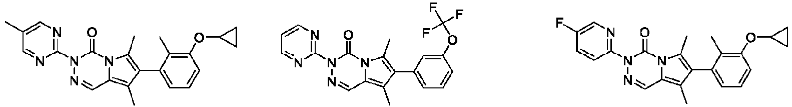 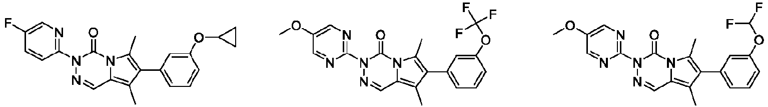 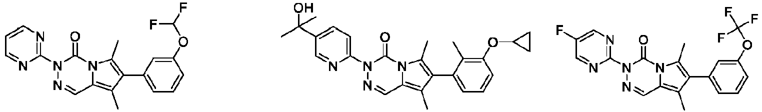 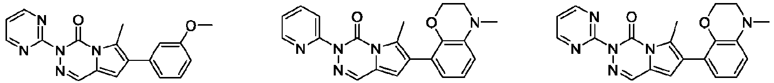 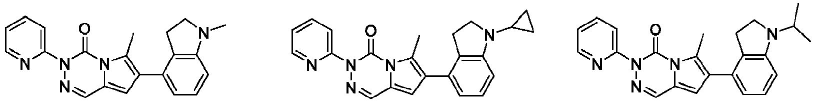 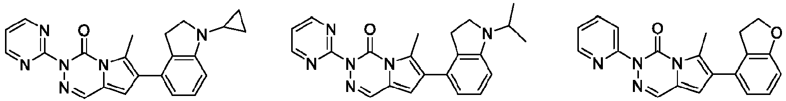 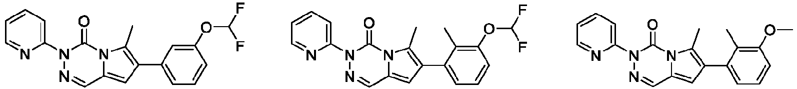 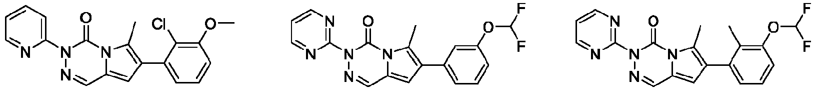 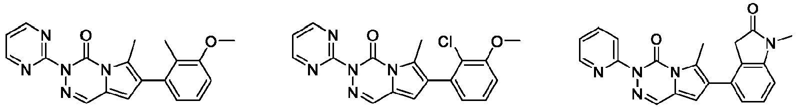 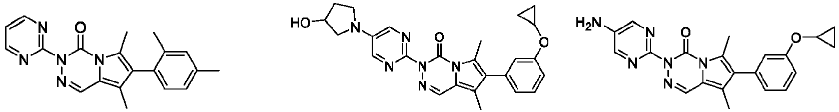 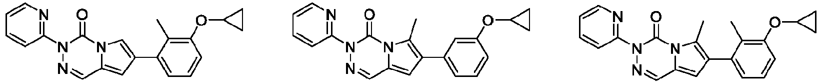 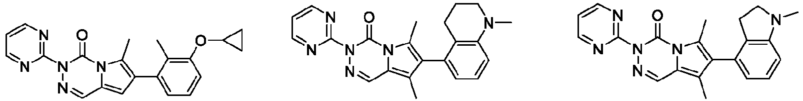 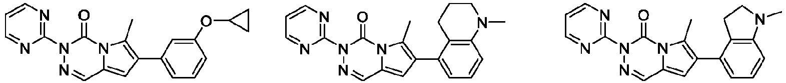 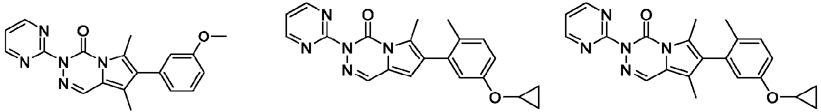 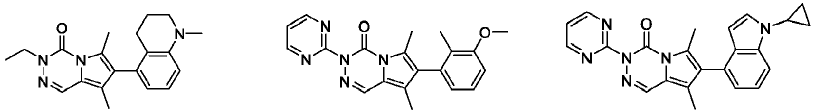 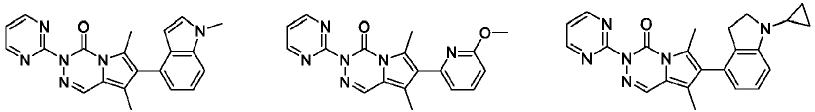 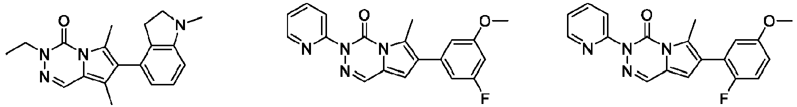 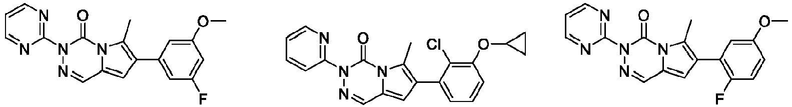 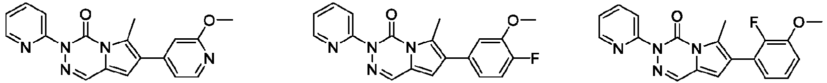 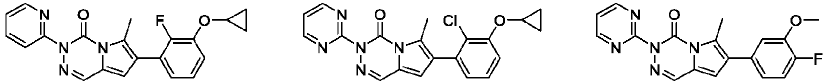 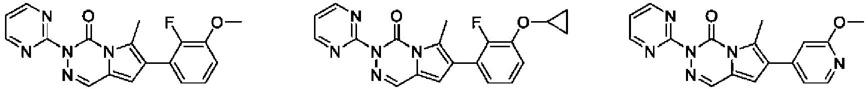 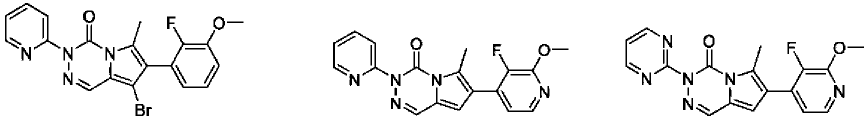 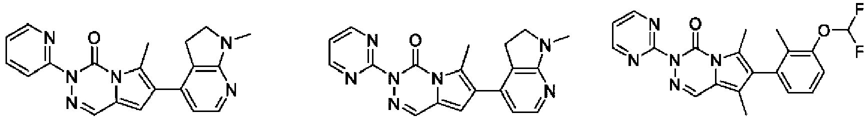 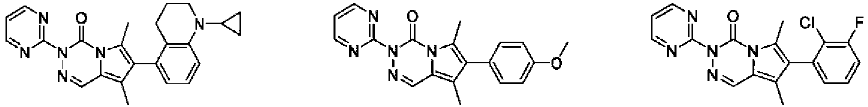 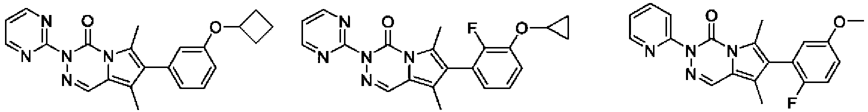 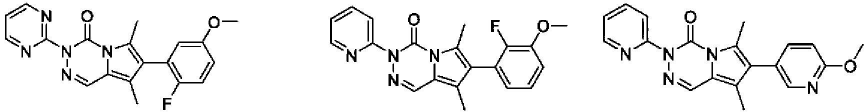 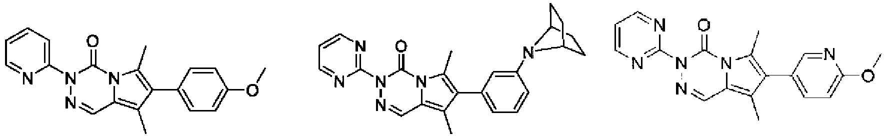 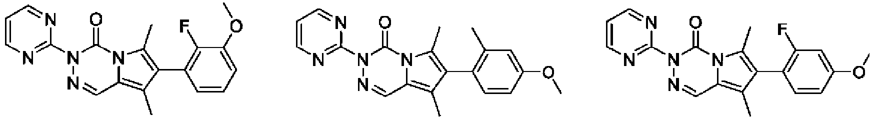 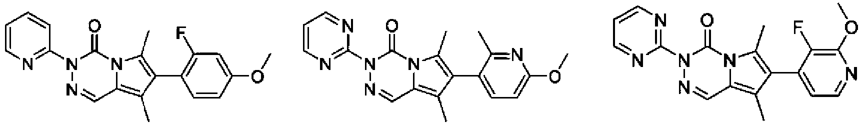 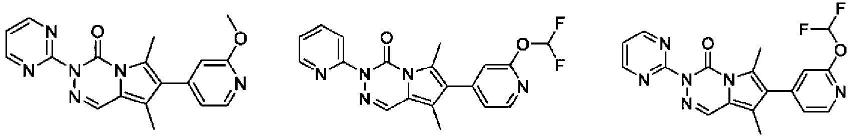 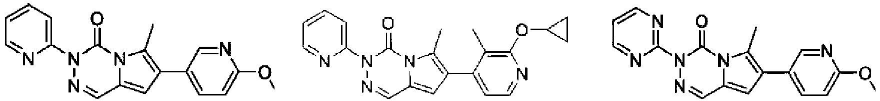 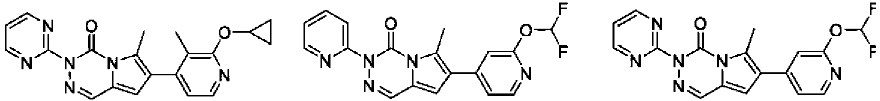 et un sel d'addition d'acide ou de base pharmaceutiquement acceptable de celui-ci, une forme stéréochimiquement isomère de celui-ci ou une forme A-oxyde de celui-ci.

12. Composition pharmaceutique comprenant une quantité thérapeutiquement efficace d'un composé selon l'une quelconque des revendications 1 à 11 et un véhicule et/ou excipient pharmaceutiquement acceptable.

13. Composé ou composition selon l'une quelconque des revendications 1 à 12 pour utilisation dans le traitement ou la prévention d'une maladie chez un mammifère affecté ou facilité par l'effet modulateur d'un modulateur allostérique de mGlu7, tel qu'un modulateur allostérique négatif de mGlu7 ; ou pour utilisation dans le traitement, la prévention, l'amélioration, la régulation ou la réduction du risque de divers troubles neurologiques et psychiatriques associés à un dysfonctionnement du glutamate chez un mammifère, comprenant l'administration à un mammifère ayant besoin d'un tel traitement ou prévention, d'une quantité efficace du composé/de la composition ; par exemple dans lequel le traitement ou la prévention est affecté ou facilité par l'effet modulateur d'un modulateur allostérique négatif de mGlu7.

14. Composé ou composition pour utilisation selon la revendication 13, dans lequel la maladie est un ou plusieurs parmi un trouble du système nerveux central, une maladie ou un trouble auriculaire ou un trouble de la douleur ; par exemple
dans lequel le trouble nerveux central est un trouble de l'anxiété tel que l'agoraphobie, un trouble anxieux généralisé (TAG), un trouble obsessionnel-compulsif (TOC), un trouble panique ou un trouble de stress post-traumatique (TSPT) ; par exemple
dans lequel le trouble du système nerveux central est un trouble psychotique sélectionné dans le groupe consistant en schizophrénie, trouble délirant, trouble schizoaffectif, trouble schizophréniforme et trouble psychotique induit par une substance ; par exemple
dans lequel la maladie et trouble auriculaire est un ou plusieurs parmi une déficience de l'oreille interne, une déficience auditive liée à l'âge (presbyacousie), la maladie de Ménière, une perte auditive soudaine, une perte auditive induite par le bruit, une otite moyenne, une maladie auto-immune de l'oreille interne, des acouphènes aigus, des acouphènes chroniques, une perte auditive induite par un médicament, une perte auditive cachée, une perte auditive induite par le cisplatine, une perte auditive induite par des aminoglycosides, une ototoxicité, un trouble central du traitement auditif ou un trouble vestibulaire ; par exemple
dans lequel le trouble de la douleur est un ou plusieurs parmi une douleur neuropathique, une douleur inflammatoire, une douleur viscérale, une douleur aiguë, une douleur chronique, une douleur sévère, une douleur réfractaire, une douleur post-traumatique, une douleur postopératoire, une céphalée douloureuse ou une douleur cancéreuse.

15. Composé ou composition selon l'une quelconque des revendications 1 à 12 pour utilisation en tant que médicament.
